# EUROPEAN PATENT SPECIFICATION

(11) **EP 1 670 946 B1**
(45) Date of publication and mention of the grant of the patent: **07.11.2012**
(21) Application number: 04788855.7
(22) Date of filing: 17.09.2004
(51) Int. Cl.: C12Q 1/68

(54) **PREDICTING BREAST CANCER TREATMENT OUTCOME**
VORHERSAGE DES AUSGANGS EINER BRUSTKREBSBEHANDLUNG
PREVISION DU RESULTAT D'UN TRAITEMENT CONTRE LE CANCER DU SEIN

(30) Priority: 19.09.2003 US 504087 P; 02.12.2003 US 727100; 06.02.2004 US 773761; 23.02.2004 US 547199 P
(43) Date of publication of application: 21.06.2006
(60) Divisional application: 10011854.6
(73) Proprietor: bioTheranostics, Inc., Mountain View, CA 94043 (US)
(72) Inventor: MA, Xiao-Jun, San Diego, CA 92130 (US); ERLANDER, Mark, G., Redwood City, CA 94063 (US); SGROI, Dennis, C., Winchester, MA 01890 (US); ENRIGHT, Edward, San Diego, CA 92130 (US)
(74) Representative: Roques, Sarah Elizabeth
(86) International application number: PCT/US2004/030789
(87) International publication number: WO 2005/028681

(56) References cited:
- WO-A-02/103320
- DAIDONE M G ET AL: "Biomarkers and outcome after tamoxifen treatment in node-positive breast cancers from elderly women" BRITISH JOURNAL OF CANCER, vol. 82, no. 2, January 2000 (2000-01), pages 270-277, XP002316786 ISSN: 0007-0920
- VAN DER FLIER SILVIA ET AL: "Bcar1/p130Cas protein and primary breast cancer: Prognosis and response to tamoxifen treatment" JOURNAL OF THE NATIONAL CANCER INSTITUTE (BETHESDA), vol. 92, no. 2, 19 January 2000 (2000-01-19), pages 120-127, XP002316787 ISSN: 0027-8874
- LUO L -Y ET AL: "Higher expression of human kallikrein 10 in breast cancer tissue predicts tamoxifen resistance" BRITISH JOURNAL OF CANCER, vol. 86, no. 11, 5 June 2002 (2002-06-05), pages 1790-1796, XP001205182 ISSN: 0007-0920
- ELLIS MATTHEW J ET AL: "Neoadjuvant comparisons of aromatase inhibitors and tamoxifen: Pretreatment determinants of response and on-treatment effect." JOURNAL OF STEROID BIOCHEMISTRY AND MOLECULAR BIOLOGY, vol. 86, no. 3-5, September 2003 (2003-09), pages 301-307, XP002316789 ISSN: 0960-0760
- HILSENBECK S G ET AL: "STATISTICAL ANALYSIS OF ARRAY EXPRESSION DATA AS APPLIED TO THE PROBLEM OF TAMOXIFEN RESISTANCE" JOURNAL OF THE NATIONAL CANCER INSTITUTE, US DEPT. OF HEALTH, EDICATIONAND WELFARE, PUBLIC HEALTH, US, vol. 91, no. 5, 26 January 1999 (1999-01-26), pages 453-459, XP009002755 ISSN: 0027-8874
- VEER VAN 'T L J ET AL: "Gene expression profiling predicts clinical outcome of breast cancer" NATURE, MACMILLAN JOURNALS LTD. LONDON, GB, vol. 415, no. 6871, 31 January 2002 (2002-01-31), pages 530-536, XP002259781 ISSN: 0028-0836
- MA XIAO-JUN ET AL: "A two-gene expression ratio predicts clinical outcome in breast cancer patients treated with tamoxifen" CANCER CELL, vol. 5, no. 6, June 2004 (2004-06), pages 607-616, XP002317299 ISSN: 1535-6108
- BECKER MICHAEL ET AL: "Distinct gene expression patterns in a tamoxifen-sensitive human mammary carcinoma xenograft and its tamoxifen-resistant subline MaCa 3366/TAM." MOLECULAR CANCER THERAPEUTICS. JAN 2005, vol. 4, no. 1, January 2005 (2005-01), pages 151-168, XP002317300 ISSN: 1535-7163

## Description

### Field of the Invention

The invention relates to the identification and use of gene expression profiles, or patterns, with clinical relevance to the treatment of breast cancer using tamoxifen (nolvadex) and other "antiestrogen" agents against breast cancer, including other "selective estrogen receptor modulators" ("SERM"s), "selective estrogen receptor downregulators" ("SERD"s), and aromatase inhibitors ("AI"s). In particular, the invention provides the identities of gene sequences the expression of which are correlated with patient survival and breast cancer recurrence in women treated with tamoxifen or other "antiestrogen" agents against breast cancer. The gene expression profiles, whether embodied in nucleic acid expression, protein expression, or other expression formats, may be used to select subjects afflicted with breast cancer who will likely respond positively to treatment with tamoxifen or another "antiestrogen" agent against breast cancer as well as those who will likely be non-responsive and thus candidates for other treatments. The invention also provides the identities of sets of sequences from multiple genes with expression patterns that are strongly predictive of responsiveness to tamoxifen and other "antiestrogen" agents against breast cancer.

### Background of the Invention

Breast cancer is by far the most common cancer among women. Each year, more than 180,000 and 1 million women in the U.S. and worldwide, respectively, are diagnosed with breast cancer. Breast cancer is the leading cause of death for women between ages 50-55, and is the most common non-preventable malignancy in women in the Western Hemisphere. An estimated 2,167,000 women in the United States are currently living with the disease (National Cancer Institute, Surveillance Epidemiology and End Results (NCI SEER) program, Cancer Statistics Review (CSR), www-seer.ims.nci.nih.gov/Publications/CSR1973 (1998)). Based on cancer rates from 1995 through 1997, a report from the National Cancer Institute (NCI) estimates that about 1 in 8 women in the United States (approximately 12.8 percent) will develop breast cancer during her lifetime (NCTs Surveillance, Epidemiology, and End Results Program (SEER) publication *SEER Cancer Statistics Review 1973-1997).* Breast cancer is the second most common form of cancer, after skin cancer, among women in the United States. An estimated 250,100 new cases of breast cancer are expected to be diagnosed in the United States in 2001. Of these, 192,200 new cases of more advanced (invasive) breast cancer are expected to occur among women (an increase of 5% over last year), 46,400 new cases of early stage (*in situ*) breast cancer are expected to occur among women (up 9% from last year), and about 1,500 new cases of breast cancer are expected to be diagnosed in men (Cancer Facts & Figures 2001 American Cancer Society). An estimated 40,600 deaths (40,300 women, 400 men) from breast cancer are expected in 2001. Breast cancer ranks second only to lung cancer among causes of cancer deaths in women. Nearly 86% of women who are diagnosed with breast cancer are likely to still be alive five years later, though 24% of them will die of breast cancer after 10 years, and nearly half (47%) will die of breast cancer after 20 years.

Every woman is at risk for breast cancer. Over 70 percent of breast cancers occur in women who have no identifiable risk factors other than age (U.S. General Accounting Office. Breast Cancer, 1971-1991: Prevention, Treatment and Research. GAO/PEMD-92-12; 1991). Only 5 to 10% of breast cancers are linked to a family history of breast cancer (Henderson IC, Breast Cancer. In: Murphy GP, Lawrence WL, Lenhard RE (eds). Clinical Oncology. Atlanta, GA: American Cancer Society; 1995:198-219).

Each breast has 15 to 20 sections called lobes. Within each lobe are many smaller lobules. Lobules end in dozens of tiny bulbs that can produce milk. The lobes, lobules, and bulbs are all linked by thin tubes called ducts. These ducts lead to the nipple in the center of a dark area of skin called the areola. Fat surrounds the lobules and ducts. There are no muscles in the breast, but muscles lie under each breast and cover the ribs. Each breast also contains blood vessels and lymph vessels. The lymph vessels carry colorless fluid called lymph, and lead to the lymph nodes. Clusters of lymph nodes are found near the breast in the axilla (under the arm), above the collarbone, and in the chest.

Breast tumors can be either benign or malignant. Benign tumors are not cancerous, they do not spread to other parts of the body, and are not a threat to life. They can usually be removed, and in most cases, do not come back. Malignant tumors are cancerous, and can invade and damage nearby tissues and organs. Malignant tumor cells may metastasize, entering the bloodstream or lymphatic system. When breast cancer cells metastasize outside the breast, they are often found in the lymph nodes under the arm (axillary lymph nodes). If the cancer has reached these nodes, it means that cancer cells may have spread to other lymph nodes or other organs, such as bones, liver, or lungs.

Major and intensive research has been focused on early detection, treatment and prevention. This has included an emphasis on determining the presence of precancerous or cancerous ductal epithelial cells. These cells are analyzed, for example, for cell morphology, for protein markers, for nucleic acid markers, for chromosomal abnormalities, for biochemical markers, and for other characteristic changes that would signal the presence of cancerous or precancerous cells. This has led to various molecular alterations that have been reported in breast cancer, few of which have been well characterised in human clinical breast specimens. Molecular alterations include presence/absence of estrogen and progesterone steroid receptors, HER-2 expression/amplification (Mark HF, et al. HER-2/neu gene amplification in stages I-IV breast cancer detected by fluorescent in situ hybridization. Genet Med; 1(3):98-103 1999), Ki-67 (an antigen that is present in all stages of the cell cycle except G0 and used as a marker for tumour cell proliferation, and prognostic markers (including oncogenes, tumour suppressor genes, and angiogenesis markers) like p53, p27, Cathepsin D, pS2, multi-drug resistance (MDR) gene, and CD31. WO 02/103320 relates to genetic markers, whose expression is correlated with breast cancer, and in particular which can be used to differentiate clinical conditions associated with breast cancer such as the presence or absence of the estrogen receptor ESR1, and BRCA1 and sporadic tumours.

Tamoxifen is the antiestrogen agent most frequently prescribed in women with both early stage and metastatic hormone receptor-positive breast cancer (for reviews, see Clarke, R et al. "Antiestrogen resistance in breast cancer and the role of estrogen receptor signalling". Oncogene 22, 7316-39 (2003) and Jordan, C. "Historical perspective on hormonal therapy of advanced breast Cancer". Clin. Ther. 24 Suppl A, A3-16 (2002)). In the adjuvant setting, tamoxifen therapy results in a 40-50% reduction in the annual risk of recurrence, leading to a 5.6% improvement in 10 year survival in lymph node negative patients, and a corresponding 10.9% improvement in node-positive patients (Group, E.B.C.T.C. Tamoxifen for early breast cancer. Cochrane Database Syst Rev, CD000486 (2001). Tamoxifen is thought to act primarily as a competitive inhibitor of estrogen binding to estrogen receptor (ER). The absolute levels of ER expression, as well as that of the progesterone receptor (PR, an indicator of a functional ER pathway), are currently the best predictors of tamoxifen response in the clinical setting (Group, (2001) and Bardou,. V.J. et al. "Progesterone receptor status significantly improves outcome prediction over estrogen receptor status alone for adjuvant endocrine therapy in two large breast cancer databases". J Clin Oncol 21, 1973-9 (2003)).

However, 25% of ER+/PR+ tumours, 66% of ER+/PR- cases and 55% of ER-/PR+ cases fail to respond, or develop early resistance to tamoxifen, through mechanisms that remain largely unclear (see Clarke *et al*.; Nicholson, R.I. et al. "The biology of antihormone failure in breast cancer". Breast Cancer Res Treat 80 Suppl 1, S29-34; discussion S35 (2003) and Osborne, C.K. et al. "Growth factor receptor cross-talk with estrogen receptor as a mechanism for tamoxifen resistance in breast cancer". Breast 12, 362-7 (2003)). Daidone et al., British Journal of Cancer 2000 Vol. 82 No. 2 pp270-277 seek to identify biomarkers and outcome after tamoxifen treatment in node-positive breast cancer for elderly women. Ellis et al., J. Ster. Biochem. & Mol. Bio. 86 (2003) 301-307 assess expression profiles in patients treated with aromatase inhibitors and tamoxifen. Van der Flier et al., J. Nat. Canc. Inst. Vol. 92 No. 2 (2000) 120-127 review Bcar1/p130Cas expression in resistance to tamoxifen therapy. Luo et al., Brit. J. Cancer 86 (2002) 1790-1796 monitor protein levels of human kallikrein 6 and human kallikrein 10 in breast cancer patients, and responsiveness to tamoxifen. Hilsenback et al., J. Nat. Canc. Inst. Vol. 91 No. 5 (1999) p453-459 analyses array expression data in acquired tamoxifen resistance.

Currently, no reliable means exist to allow the identification of these non-responders. In these patients, the use of alternative hormonal therapies, such as the aromatase inhibitors letrozole and anastrozole (Ellis, M.J. et al. "Letrozole is more effective neoadjuvant endocrine therapy than tamoxifen for ErbB-1- and/or ErbB-2-positive, estrogen receptorpositive primary breast cancer: evidence from a phase III randomized trial." J Clin Oncol 19, 3808-16 (2001); Buzdar, A.U. "Anastrozole: a new addition to the armamentarium against advanced breast cancer." Am J Clin Oncol 21, 161-6 (1998); and Goss, P.E. et al. "A randomized trial of letrozole in postmenopausal women after five years of tamoxifen therapy for early-stage breast cancer." N Engl J Med 349, 1793-802 (2003)); chemotherapeutic agents, or inhibitors of other signaling pathways, such as trastuzmab and gefitinib might offer the possibility of improving clinical outcome. Therefore, the ability to accurately predict tamoxifen treatment outcome should significantly advance the management of early stage breast cancer by identifying patients who are unlikely to benefit from TAM so that additional or alternative therapies may be sought

### Summary of the Invention

The present invention relates to the identification and use of gene expression patterns (or profiles or "signatures") and the expression levels of individual gene sequences which are clinically relevant to breast cancer. In particular, the identities of genes that are correlated with patient survival and breast cancer recurrence (e.g. metastasis of the breast cancer) are provided. The gene expression profiles, whether embodied in nucleic acid expression, protein expression, or other expression formats, may be used to predict survival of subjects afflicted with breast cancer and the likelihood of breast cancer recurrence, including cancer metastasis.

The invention thus provides for the identification and use of gene expression patterns (or profiles or "signatures") and the expression levels of individual gene sequences which correlate with (and thus are able to discriminate between) patients with good or poor survival outcomes. In one embodiment, the invention provides patterns that are able to distinguish patients with estrogen receptor (a isoform) positive (ER+) breast tumors into those with that are responsive, or likely to be responsive, to treatment with tamoxifen (TAM) or another "antiestrogen" agent against breast cancer (such as a "selective estrogen receptor modulator" ("SERM"), "selective estrogen receptor downregulator" ("SERD"), or aromatase inhibitor ("AI*)) and those that are non-responsive, or likely to be non-responsive, to such treatment. Responsiveness may be viewed in terms of better survival outcomes over time. These patterns are thus able to distinguish patients with ER+ breast tumours into at least two subtypes.

In a first aspect, the present invention provides a non-subjective means for the identification of patients with breast cancer (ER+ or ER-) as likely to have a good or poor survival outcome following treatment with TAM or another "antiestrogen" agent against breast cancer by assaying for the expression patterns disclosed herein. Thus where subjective interpretation may have been previously used to determine the prognosis and/or treatment of breast cancer patients, the present invention provides objective gene expression patterns, which may used alone or in combination with subjective criteria to provide a more accurate assessment of ER+ or ER- breast cancer patient outcomes or expected outcomes, including survival and the recurrence of cancer, following treatment with TAM or another "antiestrogen" agent against breast cancer. The expression patterns of the invention thus provides a means to determine ER+ or ER- breast cancer prognosis. Furthermore, the expression patterns can also be used as a means to assay small, node negative tumours that are not readily assayed by other means.

The gene expression patterns comprise genes capable of discriminating between breast cancer outcomes with significant accuracy. The gene sequences are identified as correlated with ER+ breast cancer outcomes such that the levels of their expression are relevant to a determination of the preferred treatment protocols for a patient, whether ER+ or ER-.

Thus in one embodiment, the invention provides a method to determine the risk of cancer recurrence in a subject afflicted with ER+ breast cancer if treated with tamoxifen, said method comprising assaying a sample of ER+ breast cancer cells from said subject for the expression of human HOXB13 and IL 17BR mRNAs to determine a ratio of HOXB13 and IL17BR expression levels, wherein said ratio is indicative of lack of cancer recurrence in said subject if treated with tamoxifen.

The ability to correlate gene expression with breast cancer outcome and responsiveness to TAM is particularly advantageous in light of the possibility that up to 40% of ER+ subjects that undergo TAM treatment are non-responders. Therefore, the ability to identify, with confidence, these non-responders at an early time point permits the consideration and/or application of alternative therapies (such as a different "antiestrogen" agent against breast cancer or other anti-breast cancer treatments) to the non-responders. Stated differently, the ability to identify TAM non-responder subjects permits medical personnel to consider and/or utilize alternative therapies for the treatment of the subjects before time is spent on ineffective TAM therapy. Time spent on an ineffective therapy often permits further cancer growth, and the likelihood of success with alternative therapies diminishes over time given such growth. Therefore, the invention also provides methods to improve the survival outcome of non-responders by use of the methods disclosed herein to identify non-responders for treatment with alternative therapies.

Gene expression patterns of the invention are identified as described below. Generally, a large sampling of the gene expression profile of a sample is obtained through quantifying the expression levels of mRNA corresponding to many genes. This profile is then analyzed to identify genes, the expression of which are positively, or negatively, correlated, with ER+ breast cancer outcome upon treatment with TAM or another "antiestrogen" agent against breast cancer. An expression profile of a subset of human genes may then be identified by the methods of the present disclosure correlated with a particular outcome. The use of multiple samples increases the confidence which a gene may be believed to be correlated with a particular survival outcome. Without sufficient confidence, it remains unpredictable whether expression of a particular gene is actually correlated with an outcome and also unpredictable whether expression of a particular gene may be successfully used to identify the outcome for a breast cancer patient The invention is practiced based on the identities of the gene sequences disclosed herein or the actual sequences used independent of identification

A profile of genes that are highly correlated with one outcome relative to another may be used to assay an sample from a subject afflicted with breast cancer to predict the likely responsiveness (or lack thereof) to TAM or another "antiestrogen" agent against breast cancer in the subject from whom the sample was obtained. Such an assay may be used as part of a method to determine the therapeutic treatment for said subject based upon the breast cancer outcome identified.

As discussed below, the correlated genes may be used in combination to increase the ability to accurately correlating a molecular expression phenotype with a breast cancer outcome. This correlation is a way to molecularly provide for the determination of survival outcomes as disclosed herein. Additional uses of the correlated genes are in the classification of cells and tissues; determination of diagnosis and/or prognosis; and determination and/or alteration of therapy.

The ability to discriminate is conferred by the identification of expression of the individual genes as relevant and not by the form of the assay used to determine the actual level of expression. An assay may utilize any identifying feature of an identified individual gene as disclosed herein as long as the assay reflects, quantitatively or qualitatively, expression of the gene in the "transcriptome" (the transcribed fraction of genes in a genome) or the "proteome" (the translated fraction of expressed genes in a genome). Additional assays include those based on the detection of polypeptide fragments of the relevant member or members of the proteome. Identifying features include, but are not limited to, unique nucleic acid sequences used to encode (DNA), or express (RNA), said gene or epitopes specific to, or activities of, a protein encoded by said gene. All that is required are the gene sequence(s) necessary to discriminate between breast cancer outcomes and an appropriate cell containing sample for use in an expression assay.

We also describe the identification of the gene expression patterns by analyzing global, or near global, gene expression from single cells or homogenous cell populations which have been dissected away from, or otherwise isolated or purified from, contaminating cells beyond that possible by a simple biopsy. Because the expression of numerous genes fluctuate between cells from different patients as well as between cells from the same patient sample, multiple data from expression of individual genes and gene expression patterns are used as reference data to generate models which in turn permit the identification of individual gene(s), the expression of which are most highly correlated with particular breast cancer outcomes.

We also describe physical and methodological means for detecting the expression of gene(s) identified by the models generated by individual expression patterns. These means may be directed to assaying one or more aspects of the DNA template(s) underlying the expression of the gene(s), of the RNA used as an intermediate to express the gene(s), or of the proteinaceous product expressed by the gene(s).

We also describe that, the gene(s) identified by a model as capable of discriminating between breast cancer outcomes may be used to identify the cellular state of an unknown sample of cell(s) from the breast. Preferably, the sample is isolated via non-invasive means. The expression of said gene(s) in said unknown sample may be determined and compared to the expression of said gene(s) in reference data of gene expression patterns correlated with breast cancer outcomes. Optionally, the comparison to reference samples may be by comparison to the model(s) constructed based on the reference samples.

One advantage provided by the present invention is that contaminating, non-breast cells (such as infiltrating lymphocytes or other immune system cells) are not present to possibly affect the genes identified or the subsequent analysis of gene expression to identify the survival outcomes of patients with breast cancer. Such contamination is present where a biopsy is used to generate gene expression profiles. However, and as noted herein, the invention includes the identity of genes that may be used with significant accuracy even in the presence of contaminating cells.

We also describe a non-subjective means based on the expression of multiple genes, or combinations thereof, for the identification of patients with breast cancer as likely to have a good or poor survival outcome following treatment with TAM or another "antiestrogen" agent against breast cancer. These genes are members of the expression patterns disclosed herein which have been found to be strongly predictive of clinical outcome following TAM treatment of ER+ breast cancer.

The present invention thus provides gene sequences identified as differentially expressed in ER+ breast cancer in correlation to TAM responsiveness. The sequences of two genes display increased expression in ER+ breast cells that respond to TAM treatment (and thus lack of increased expression in nonresponsive cases). The sequences of two other genes display decreased expression in ER+ breast cells that respond to TAM treatment (and thus lack of decreased expression in nonresponsive cases).

The first set of sequences found to be more highly expressed in TAM responsive, ER+ breast cells are these of interleukin 17 receptor B (IL17RB), which has been mapped to human chromosome 3 at 3p21.1. IL17RB is also referred to as interleukin 17B receptor (IL17BR) and sequences corresponding to it, and thus may be used in the practice of the instant invention, are identified by UniGene Cluster Hs.5470.

The second set of sequences found to be more highly expressed in TAM responsive, ER+ breast cells are those of a newly identified transcribed region of choline dehydrogenase (CHDH), which has been mapped to human chromosome 3 at 3p21.1. This is near the location mapped for the calcium channel, voltage-dependent, L type, alpha 1D subunit (CACNA1D) at 3p14.3. The invention is based in part on the unexpected discovery of an error in public databases that identified the sequence of AI24093 as corresponding to a transcribed part of CACNA1D (in Hs.399966). As detailed below, the transcribed regions of CHDH and CACNA1D are convergently oriented such that transcription proceeds from the regulatory regions of each toward the regulatory region of the other. Stated differently, they are convergently transcribed from complementary strands in the same region of chromosome 3.

Therefore, we also describe the identification of AI240933 being in the wrong orientation with respect to CACNA1D transcription but in the correct orientation as CHDH transcription and as located at the 3' end of CHDH transcription. Without being bound by theory, and offered to improve understanding of the disclosure, it is believed that the sequence of AI240933 is a part of the 3' end of the CHDH transcript. It is possibly part of the 3' untranslated region (UTR) of CHDH. The disclosure may be practiced with sequences corresponding to CHDH as well as those identified by Hs.126688.

The first set of sequences found to be expressed at lower levels in TAM responsive, ER+ breast cells are those of homeobox B 13 (HOXB 13), which has been mapped to human chromosome 17 at 17q21.2. Sequences corresponding to HOXB13, and thus may be used in the practice of the instant invention, are identified by UniGene Cluster Hs.66731.

The second set of sequences found to be expressed at lower levels in TAM responsive, ER+ breast cells are those of quinolinate phosphoribosyltransferase (QPRT, also known as nicotinate-nucleotide pyrophosphorylase, carboxylating), which has been mapped to human chromosome 16 at 16p12.1. Sequences corresponding to QPRT may be used in the practice of the instant invention, are identified by UniGene Cluster Hs.335116.

The invention may be practiced based on the identities of these gene sequences or the actual sequences used independent of the assigned identity.

The identified sequences may thus be used in methods of determining the responsiveness, or non-responsiveness, of a subject's ER+ or ER- breast cancer to TAM treatment, or treatment with another "antiestrogen" agent against breast cancer, via analysis of breast cells in a tissue or cell containing sample from a subject. As non-limiting examples, the lack of increased expression of IL17BR and/or CHDH sequences and/or the lack of decreased expression of HOXB 13 and/or QPRT sequences may be used as an indicator of nonresponsive cases. The present invention provides a non-empirical means for determining responsiveness to TAM or another SERM in ER+ patients. This provides advantages over the use of a "wait and see" approach following treatment with TAM or other "antiestrogen" agent against breast cancer. The expression levels of these sequences may also be used as a means to assay small, node negative tumors that are not readily assessed by conventional means.

The expression levels of the identified sequences may be used alone or in combination with other sequences capable of determining responsiveness to treatment with TAM or another "antiestrogen" agent against breast cancer. Preferably, the sequences of the invention are used alone or in combination with each other, such as in the format of a ratio of expression levels that can have improved predictive power over analysis based on expression of sequences corresponding to individual genes. The invention provides for ratios of the expression level of a sequence that is underexpressed to the expression level of a sequence that is overexpressed as a indicator of responsiveness or non-responsiveness.

The present invention provides means for correlating a molecular expression phenotype with a physiological response in a subject with ER+ breast cancer. This correlation provides a way to molecularly diagnose and/or determine treatment for a breast cancer afflicted subject Additional uses of the sequences are in the classification of cells and tissues; and determination of diagnosis and/or prognosis. Use of the sequences to identify cells of a sample as responsive, or not, to treatment with TAM or other "antiestrogen" agent against breast cancer may be used to determine the choice, or alteration, of therapy used to treat such cells in the subject, as well as the subject itself, from which the sample originated.

Such methods of the invention may be used to assist the determination of providing tamoxifen or another "antiestrogen" agent against breast cancer as a chemopreventive or chemoprotective agent to a subject at high risk for development of breast cancer. These methods of the invention are an advance over the studies of Fabian et al. (J Natl Cancer Inst. 92(15):1217-27, 2000), which proposed a combination of cytomorphology and the Gail risk model to identify high risk patients. The methods may be used in combination with assessments of relative risk of breast cancer such as that discussed by Tan-Chiu et al. (J Natl Cancer Inst. 95(4):302-307, 2003). Non-limiting examples include assaying of minimally invasive sampling, such as random (periareolar) fine needle aspirates or ductal lavage samples (such as that described by Fabian et al. and optionally in combination with or as an addition to a mammogram positive for benign or malignant breast cancer), of breast cells for the expression levels of gene sequences as disclosed herein to assist in the determination of administering therapy with an "antiestrogen" agent against breast cancer, such as that which may occur in cases of high risk subjects (like those described by Tan-Chiu et al.). The assays would thus lead to the identification of subjects for who the application of an "antiestrogen" agent against breast cancer would likely be beneficial as a chemopreventive or chemoprotective agent. It is contemplated that such application as enabled by the instant invention could lead to beneficial effects such as those seen with the administration of tamoxifen (see for example, Wickerham D.L., Breast Cancer Res. and Treatment 75 Suppl 1:S7-12, Discussion S33-5, 2000). Other applications of the invention include assaying of advanced breast cancer, including metastatic cancer, to determine the responsiveness, or non-responsiveness, thereof to treatment with an "antiestrogen" agent against breast cancer.

An assay of the invention may utilize a means related to the expression level of the sequences disclosed herein as long as the assay reflects, quantitatively or qualitatively, expression of the sequence. Preferably, however, a quantitative assay means is preferred. The ability to determine responsiveness to TAM or other "antiestrogen" agent against breast cancer and thus outcome of treatment therewith is provided by the recognition of the relevancy of the level of expression of the identified sequences and not by the form of the assay used to determine the actual level of expression. Identifying features of the sequences include, but are not limited to, unique nucleic acid sequences used to encode (DNA), or express (RNA), the disclosed sequences or epitopes specific to, or activities of, proteins encoded by the sequences. Alternative means include detection of nucleic acid amplification as indicative of increased expression levels and nucleic acid inactivation, deletion, or methylation, as indicative of decreased expression levels. Stated differently, the invention may be practiced by assaying one or more aspect of the DNA template(s) underlying the expression of the disclosed sequence(s), of the RNA used as an intermediate to express the sequence(s), or of the proteinaceous product expressed by the sequence(s), as well as proteolytic fragments of such products. As such, the detection of the presence of, amount of, stability of, or degradation (including rate) of, such DNA, RNA and proteinaceous molecules may be used in the practice of the disclosure

The practice of the present invention is unaffected by the presence of minor mismatches between the disclosed sequences and those expressed by cells of a subject's sample. A non-limiting example of the existence of such mismatches are seen in cases of sequence polymorphisms between individuals of a species, such as individual human patients within *Homo sapiens.* Knowledge that expression of the disclosed sequences (and sequences that vary due to minor mismatches) is correlated with the presence of non-normal or abnormal breast cells and breast cancer is sufficient for the practice of the invention with an appropriate cell containing sample via an assay for expression.

In one embodiment, the invention provides for the identification of the expression levels of the disclosed sequences by analysis of their expression in a sample containing ER+ or ER- breast cells. In one preferred embodiment, the sample contains single cells or homogenous cell populations which have been dissected away from, or otherwise isolated or purified from, contaminating cells beyond that possible by a simple biopsy. Alternatively, undissected cells within a "section" of tissue may be used. Multiple means for such analysis are available, including detection of expression within an assay for global, or near global, gene expression in a sample (e.g. as part of a gene expression profiling analysis such as on a microarray) or by specific detection, such as quantitative PCR (Q-PCR), or real time, quantitative PCR.

Preferably, the sample is isolated via non-invasive or minimally invasive means. The expression of the disclosed sequence(s) in the sample may be determined and compared to the expression of said sequence(s) in reference data of non-normal or cancerous breast cells. Alternatively, the expression level may be compared to expression levels in normal or non-cancerous cells, preferably from the same sample or subject. In embodiments of the invention utilizing Q-PCR, the expression level may be compared to expression levels of reference genes in the same sample or a ratio of expression levels may be used.

When individual breast cells are isolated in the practice of the invention, one benefit is that contaminating, non-breast cells (such as infiltrating lymphocytes or other immune system cells) are not present to possibly affect detection of expression of the disclosed sequence(s). Such contamination is present where a biopsy is used to generate gene expression profiles. However, analysis of differential gene expression and correlation to ER+ breast cancer outcomes with both isolated and non-isolated samples, as described herein, increases the confidence level of the disclosed sequences as capable of having significant predictive power with either type of sample.

While the present invention is described mainly in the context of human breast cancer, it may be practiced in the context of breast cancer of any animal known to be potentially afflicted by breast cancer. Preferred animals for the application of the present invention are mammals, particularly those important to agricultural applications (such as, but not limited to, cattle, sheep, horses, and other "farm animals"), animal models of breast cancer, and animals for human companionship (such as, but not limited to, dogs and cats).

The above aspects and embodiments of the invention may be applied equally with respect to use of more than one "antiestrogen" agent against breast cancer. In the case of a combination of agents, any combination of more than one SERM, SERD, or AI may be used in place of TAM or another "antiestrogen" agent against breast cancer. Aromatase is an enzyme that provides a major source of estrogen in body tissues including the breast, liver, muscle and fat. Without being bound by theory, and solely provided to assist in a better understanding of the invention, AIs are understood to function in a manner comparable to TAM and other "antiestrogen" agents against breast cancer, which are thought to act as antagonists of estrogen receptor in breast tissues and thus as against breast cancer. AIs may be either nonsteroidal or steroidal agents. Examples of the former, which inhibit aromatase via the heme prosthetic group) include, but are not limited to, anastrozole (arimidex), letrozole (femara), and vorozole (rivisor), which have been used or contemplated as treatments for metastatic breast cancer. Examples of steroidal AIs, which inactivate aromatase, include, but are not limited to, exemestane (aromasin), androstenedione, and formestane (lentaron).

Other forms of therapy to reduce estrogen levels include surgical or chemical ovarian ablation. The former is physical removal of the ovaries while the latter is the use of agents to block ovarian production of estrogen. One non-limiting example of the latter are agonists of gonadotropin releasing hormone (GnRH), such as goserelin (zoladex).

The invention disclosed herein is based in part on the performance of a genome-wide microarray analysis of hormone receptor-positive invasive breast tumors from 60 patients treated with adjuvant tamoxifen alone, leading to the identification of a two-gene expression ratio that is highly predictive of clinical outcome. This expression ratio, which is readily adapted to PCR-based analysis of standard paraffin-embedded clinical specimens, was validated in an independent set of 20 patients as described below.

### Brief Description of the Drawings

Figure 1 shows receiver operating characteristic (ROC) analyses of IL17BR, HOXB13, and CACNA1D expression levels as predictors of breast cancer outcomes in whole tissue sections (top 3 graphs) and laser microdissected cells (bottom 3 graphs). AUC refers to area under the curve.

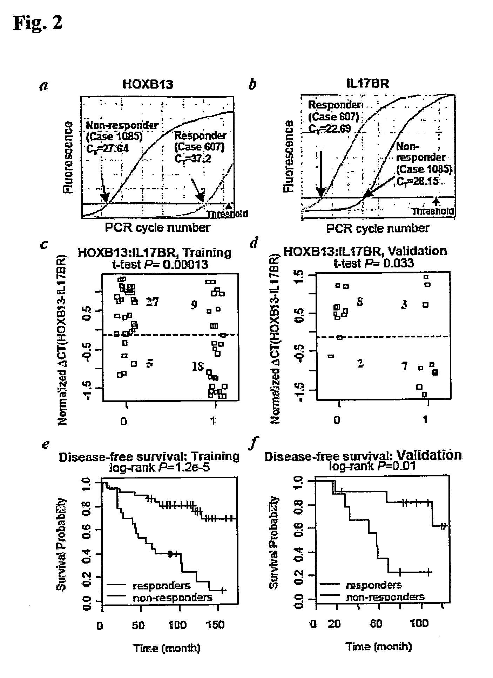

Figure 2 contains six parts relating to the validation of a ratio of HOXB 13 expression to IL17BR expression as an indicator of responsiveness, or lack thereof, to TAM. Parts a and b show the results of gene expression analysis of HOXB13 and IL17BR sequences by Q-PCR in both Responder and Non-responder samples. Plots of the Responder and Non-responder training and validation data sets are shown in Parts c and d, where "0" indicates Responder datapoints in both and "1" indicates Non-responder datapoints in both. Parts e and f show plots of the Responder and Non-responder training and validation data sets as a function of survival, where the upper line in each Part represents the Responders and the lower line represents the Non-responders.
Figure 3 shows a schematic representation of the known 3' region of the CHDH gene sequence in combination with additional CHDH 3' untranslated sequences identified by the instant disclosure.
Figure 4 shows the results of a PCR amplification reaction wherein an amplicon consistent with that expected from the schematic of Figure 1 is produced. The PCR primers used were as follows: forward CHDH primer: 5'-AAAGTCTTGGGAAATGAGACAAGT-3'; reverse primers 83R: 5'-AGCTGTCATTTGCCAGTGAGA-3' and 81R: 5'-CTGTCATTTGCCAGTGAGAGC-3'.
Figure 5 shows the alignment of 28 sequences to identify a contig comprising the CHDH 3' end region. The alignment includes the sequence of AI240933, which includes the 3' end of the assembled consensus sequence.
Figure 6 shows the sequence of an assembled contig containing the new 3' end of CHDH.
Figure 7 shows a representation of a region of human chromosome 3 wherein the location of CACNA1D is identified via "Hs.399966" and the location of CHDH is identified via "Hs.126688".

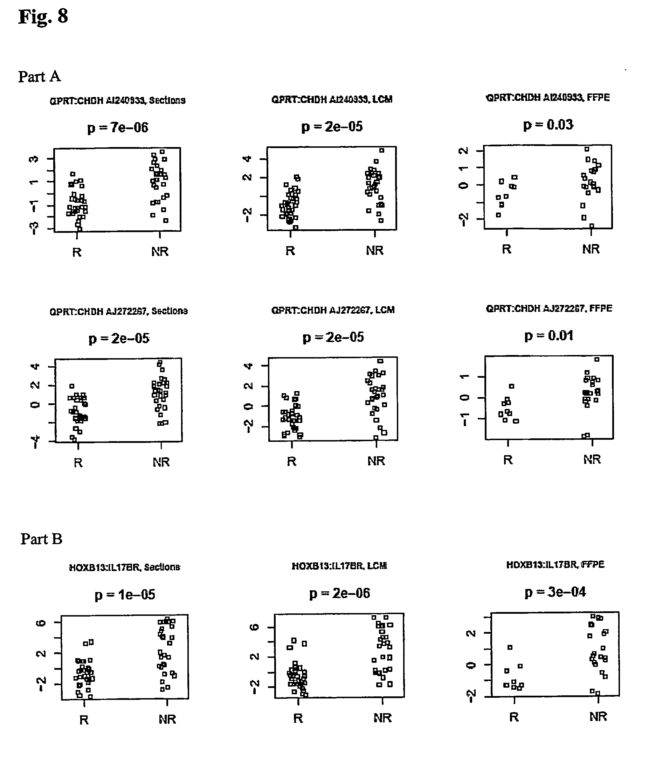

Figure 8, Part A contains six parts relating to the validation of a ratio of QPRT expression to CHDH expression as an indicator of responsiveness, or lack thereof, to TAM. The three portions identified by "QPRT:CHDH AI240933" reflect the ratio using a probe for expression of the GenBank AI240933 sequence. The three portions identified by "QPRT:CHDH AJ272267" reflect the ratio using a probe for expression of the GenBank AJ272267 sequence, identified as that of a partial mRNA for CHDH. Part B contains analogous use of a ratio of HOXB13 expression to IL17BR expression as an indicator of TAM responsiveness. Plots of the Responder ("R") and Non-responder ("NR") data sets are shown. P values are two-sample t-test.

### Modes of Practicing the Invention

Definitions of terms as used herein:
A gene expression "pattern" or "profile" or "signature" refers to the relative expression of genes correlated with responsiveness to treatment of ER+ breast cancer with TAM or another "antiestrogen" agent against breast cancer. Responsiveness or lack thereof may be expressed as survival outcomes which are correlated with an expression "pattern" or "profile" or "signature" that is able to distinguish between, and predict, said outcomes.

A "selective estrogen receptor modulator" or SERM is an "antiestrogen" agent that in some tissues act like estrogens (agonist) but block estrogen action in other tissues (antagonist). A "selective estrogen receptor downregulators" (or "SERD"s) or "pure" antiestrogens includes agents which block estrogen activity in all tissues. See Howell et al. (Best Bractice & Res. Clin. Endocrinol. Metab. 18(1):47-66, 2004). Preferred SERMs of the invention are those that are antagonists of estrogen in breast tissues and cells, including those of breast cancer. Non-limiting examples of such include TAM, raloxifene, GW5638, and ICI 182,780. The possible mechanisms of action by various SERMs have been reviewed (see for example Jordan et al., 2003, Breast Cancer Res. 5:281-283; Hall et al., 2001, J. Biol. Chem. 276(40):36869-36872; Dutertre et al. 2000, J. Pharmacol. Exp. Therap. 295(2):431-437; and Wijayaratne et al., 1999, Endocrinology 140(12):5828-5840). Other non-limiting examples of SERMs in the context of the invention include triphenylethylenes, such as tamoxifen, GW5638, TAT-59, clomiphene, toremifene, droloxifene, and idoxifene; benzothiophenes, such as arzoxiphene (LY353381 or LY353381-HCl); benzopyrans, such as EM-800; naphthalenes, such as CP-336,156; and ERA-923.

Non-limiting examples of SERD or "pure" antiestrogens include agents such as ICI 182,780 (fulvestrant or faslodex) or the oral analogue SR16243 and ZK 191703 as well as aromatase inhibitors and chemical ovarian ablation agents as described herein.

Other agents encompassed by SERM as used herein include progesterone receptor inhibitors and related drugs, such as progestomimetics like medroxyprogesterone acetate, megace, and RU-486; and peptide based inhibitors of ER action, such as LH-RH analogs (leuprolide, zoladex, [D-Trp6]LH-RH), somatostatin analogs, and LXXLL motif mimics ofER as well as tibolone and resveratrol. As noted above, preferred SERMs of the invention are those that are antagonist of estrogen in breast tissues and cells, including those of breast cancer. Non-limiting examples of preferred SERMs include the actual or contemplated metabolites (in vivo) of any SERM, such as, but not limited to, 4-hydroxytamoxifen (metabolite of tamoxifen), EM652 (or SCH 57068 where EM-800 is a prodrug of EM-652), and GW7604 (metabolite of GW5638). See Willson et al. (1997, Endocrinology 138(9):3901-3911) and Dauvois et al. (1992, Proc. Nat'l. Acad. Sci., USA 89:4037-4041) for discussions of some specific SERMs.

Other preferred SERMs are those that produce the same relevant gene expression profile as tamoxifen or 4-hydroxytamoxifen. One example of means to identify such SERMs is provided by Levenson et al. (2002, Cancer Res. 62:4419-4426).

A "gene" is a polynucleotide that encodes a discrete product, whether RNA or proteinaceous in nature. It is appreciated that more than one polynucleotide may be capable of encoding a discrete product The term includes alleles and polymorphisms of a gene that encodes the same product, or a functionally associated (including gain, loss, or modulation of function) analog thereof, based upon chromosomal location and ability to recombine during normal mitosis.

A "sequence" or "gene sequence" as used herein is a nucleic acid molecule or polynucleotide composed of a discrete order of nucleotide bases. The term includes the ordering of bases that encodes a discrete product (i.e. "coding region"), whether RNA or proteinaceous in nature, as well as the ordered bases that precede or follow a "coding region". Non-limiting examples of the latter include 5' and 3' untranslated regions of a gene. It is appreciated that more than one polynucleotide may be capable of encoding a discrete product It is also appreciated that alleles and polymorphisms of the disclosed sequences may exist and may be used in the practice of the invention to identify the expression level(s) of the disclosed sequences or the allele or polymorphism. Identification of an allele or polymorphism depends in part upon chromosomal location and ability to recombine during mitosis.

The terms "correlate" or "correlation" or equivalents thereof refer to an association between expression of one or more genes and a physiological response of a breast cancer cell and/or a breast cancer patient in comparison to the lack of the response. A gene may be expressed at higher or lower levels and still be correlated with responsiveness, non-responsiveness or breast cancer survival or outcome. We describe, for example, for the correlation between increases in expression of IL17BR and/or CHDH sequences and responsiveness of ER+ breast cells to TAM or another "antiestrogen" agent against, breast cancer. Thus increases are indicative of responsiveness. Conversely, the lack of increases, including unchanged expression levels, are indicators of non-responsiveness. Similarly, we describe, for example, for the correlation between decreases in expression of HOXB13 and/or QPRT sequences and responsiveness of ER+ breast cells to TAM or another SERM. Thus decreases are indicative of responsiveness while the lack of decreases, including unchanged expression levels, are indicators of non-responsiveness. Increases and decreases may be readily expressed in the form of a ratio between expression in a non-normal cell and a normal cell such that a ratio of one (1) indicates no difference while ratios of two (2) and one-half indicate twice as much, and half as much, expression in the non-normal cell versus the normal cell, respectively. Expression levels can be readily determined by quantitative methods as described below.

For example, increases in gene expression can be indicated by ratios of or about 1.1, of or about 1.2, of or about 1.3, of or about 1.4, of or about 1.5, of or about 1.6, of or about 1.7, of or about 1.8, of or about 1.9, of or about 2, of or about 2.5, of or about 3, of or about 3.5, of or about 4, of or about 4.5, of or about 5, of or about 5.5, of or about 6, of or about 6.5, of or about 7, of or about 7.5, of or about 8, of or about 8.5, of or about 9, of or about 9.5, of or about 10, of or about 15, of or about 20, of or about 30, of or about 40, of or about 50, of or about 60, of or about 70, of or about 80, of or about 90, of or about 100, of or about 150, of or about 200, of or about 300, of or about 400, of or about 500, of or about 600, of or about 700, of or about 800, of or about 900, or of or about 1000. A ratio of 2 is a 100% (or a two-fold) increase in expression. Decreases in gene expression can be indicated by ratios of or about 0.9, of or about 0.8, of or about 0.7, of or about 0.6, of or about 0.5, of or about 0.4, of or about 0.3, of or about 0.2, of or about 0.1, of or about 0.05, of or about 0.01, of or about 0.005, of or about 0.001, of or about 0.0005, of or about 0.0001, of or about 0.00005, of or about 0.00001, of or about 0.000005, or of or about 0.000001.

For a given phenotype, a ratio of the expression of a gene sequence expressed at increased levels in correlation with the phenotype to the expression of a gene sequence expressed at decreased levels in correlation with the phenotype may also be used as an indicator of the phenotype. As a non-limiting example, the phenotype of non-responsiveness to tamoxifen treatment of breast cancer is correlated with increased expression of HOXB13 and/or QPRT as well as decreased expression of IL17BR and/or CHDH. Therefore, a ratio of the expression levels of HOXB13 or QPRT to IL17BR or CHDH may be used as an indicator of non-responsiveness.

A "polynucleotide" is a polymeric form of nucleotides of any length, either ribonucleotides or deoxyribonucleotides. This term refers only to the primary structure of the molecule. Thus, this term includes double- and single-stranded DNA and RNA. It also includes known types of modifications including labels known in the art, methylation, "caps", substitution of one or more of the naturally occurring nucleotides with an analog, and intemucleotide modifications such as uncharged linkages (e.g., phosphorothioates, phosphorodithioates, etc.), as well as unmodified forms of the polynucleotide.

The term "amplify" is used in the broad sense to mean creating an amplification product can be made enzymatically with DNA or RNA polymerases. "Amplification," as used herein, generally refers to the process of producing multiple copies of a desired sequence, particularly those of a sample. "Multiple copies" mean at least 2 copies. A "copy" does not necessarily mean perfect sequence complementarity or identity to the template sequence. Methods for amplifying mRNA are generally know in the art, and include reverse transcription PCR (RT-PCR) and those described in U.S. Patent Application 10/062,857 (filed on October 25, 2001), as well as U.S. Provisional Patent Applications 60/298,847 (filed June 15, 2001) and 60/257,801 (filed December 22, 2000). Another method which may be used is quantitative PCR (or Q-PCR). Alternatively, RNA may be directly labeled as the corresponding cDNA by methods known in the art.

By "corresponding", it is meant that a nucleic acid molecule shares a substantial amount of sequence identity with another nucleic acid molecule. Substantial amount means at least 95%, usually at least 98% and more usually at least 99%, and sequence identity is determined using the BLAST algorithm, as described in Altschul et al. (1990), J. Mol. Biol. 215:403-410 (using the published default setting, i.e. parameters w=4, t=17).

A "microarray" is a linear or two-dimensional or three dimensional (and solid phase) array of preferably discrete regions, each having a defined area, formed on the surface of a solid support such as, but not limited to, glass, plastic, or synthetic membrane. The density of the discrete regions on a microarray is determined by the total numbers of immobilized polynucleotides to be detected on the surface of a single solid phase support, preferably at least about 50/cm², more preferably at least about 100/cm², even more preferably at least about 500/cm², but preferably below about 1,000/cm². Preferably, the arrays contain less than about 500, about 1000, about 1500, about 2000, about 2500, or about 3000 immobilized polynucleotides in total. As used herein, a DNA microarray is an array of oligonucleotides or polynucleotides placed on a chip or other surfaces used to hybridize to amplified or cloned polynucleotides from a sample. Since the position of each particular group of primers in the array is known, the identities of a sample polynucleotides can be determined based on their binding to a particular position in the microarray. As an alternative to the use of a microarray, an array of any size may be used in the practice of the invention, including an arrangement of one or more position of a two-dimensional or three dimensional arrangement in a solid phase to detect expression of a single gene sequence.

Because the invention relies upon the identification of genes that are over- or underexpressed, one embodiment of the invention involves determining expression by hybridization of mRNA, or an amplified or cloned version thereof, of a sample cell to a polynucleotide that is unique to a particular gene sequence. Preferred polynucleotides of this type contain at least about 16, at least about 18, at least about 20, at least about 22, at least about 24, at least about 26, at least about 28, at least about 30, or at least about 32 consecutive basepairs of a gene sequence that is not found in other gene sequences. The term "about" as used in the previous sentence refers to an increase or decrease of 1 from the stated numerical value. Even more preferred are polynucleotides of at least or about 50, at least or about 100, at least about or 150, at least or about 200, at least or about 250, at least or about 300, at least or about 350, at least or about 400, , at least or about 450, or at least or about 500 consecutive bases of a sequence that is not found in other gene sequences. The term "about" as used in the preceding sentence refers to an increase or decrease of 10% from the stated numerical value. Longer polynucleotides may of course contain minor mismatches (e.g. via the presence of mutations) which do not affect hybridization to the nucleic acids of a sample. Such polynucleotides may also be referred to as polynucleotide probes that are capable of hybridizing to sequences of the genes, or unique portions thereof, described herein. Such polynucleotides may be labeled to assist in their detection. Preferably, the sequences are those of mRNA encoded by the genes, the corresponding cDNA to such mRNAs, and/or amplified versions of such sequences. In preferred embodiments of the invention, the polynucleotide probes are immobilized on an array, other solid support devices, or in individual spots that localize the probes.

In another embodiment of the invention, all or part of a disclosed sequence may be amplified and detected by methods such as the polymerase chain reaction (PCR) and variations thereof, such as, but not limited to, quantitative PCR (Q-PCR), reverse transcription PCR (RT-PCR), and real-time PCR (including as a means of measuring the initial amounts ofmRNA copies for each sequence in a sample), optionally real-time RT-PCR or real-time Q-PCR. Such methods would utilize one or two primers that are complementary to portions of a disclosed sequence, where the primers are used to prime nucleic acid synthesis. The newly synthesized nucleic acids are optionally labeled and may be detected directly or by hybridization to a polynucleotide of the invention. The newly synthesized nucleic acids may be contacted with polynucleotides (containing sequences) of the invention under conditions which allow for their hybridization. Additional methods to detect the expression of expressed nucleic acids include RNAse protection assays, including liquid phase hybridizations, and *in situ* hybridization of cells.

Alternatively, and in yet another embodiment of the disclosure, gene expression may be determined by analysis of expressed protein in a cell sample of interest by use of one or more antibodies specific for one or more epitopes of individual gene products (proteins), or proteolytic fragments thereof, in said cell sample or in a bodily fluid of a subject. The cell sample may be one of breast cancer epithelial cells enriched from the blood of a subject, such as by use of labeled antibodies against cell surface markers followed by fluorescence activated cell sorting (FACS). Such antibodies are preferably labeled to permit their easy detection after binding to the gene product. Detection methodologies suitable for use in the practice of the diclosure include, but are not limited to, immunohistochemistry of cell containing samples or tissue, enzyme linked immunosorbent assays (ELISAs) including antibody sandwich assays of cell containing tissues or blood samples, mass spectroscopy, and immuno-PCR.

The term "label" refers to a composition capable of producing a detectable signal indicative of the presence of the labeled molecule. Suitable labels include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical or chemical means.

The term "support" refers to conventional supports such as beads, particles, dipsticks, fibers, filters, membranes and silane or silicate supports such as glass slides.

As used herein, a "breast tissue sample" or "breast cell sample" refers to a sample of breast tissue or fluid isolated from an individual suspected of being afflicted with, or at risk of developing, breast cancer. Such samples are primary isolates (in contrast to cultured cells) and may be collected by any non-invasive or minimally invasive means, including, but not limited to, ductal lavage, fine needle aspiration, needle biopsy, the devices and methods described in U.S. Patent 6,328,709, or any other suitable means recognized in the art. Alternatively, the "sample" may be collected by an invasive method, including, but not limited to, surgical biopsy.

"Expression" and "gene expression" include transcription and/or translation of nucleic acid material.

As used herein, the term "comprising" and its cognates are used in their inclusive sense; that is, equivalent to the term "including" and its corresponding cognates.

Conditions that "allow" an event to occur or conditions that are "suitable" for an event to occur, such as hybridization, strand extension, and the like, or "suitable" conditions are conditions that do not prevent such events from occurring. Thus, these conditions permit, enhance, facilitate, and/or are conducive to the event. Such conditions, known in the art and described herein, depend upon, for example, the nature of the nucleotide sequence, temperature, and buffer conditions. These conditions also depend on what event is desired, such as hybridization, cleavage, strand extension or transcription.

Sequence "mutation," as used herein, refers to any sequence alteration in the sequence of a gene disclosed herein interest in comparison to a reference sequence. A sequence mutation includes single nucleotide changes, or alterations of more than one nucleotide in a sequence, due to mechanisms such as substitution, deletion or insertion. Single nucleotide polymorphism (SNP) is also a sequence mutation as used herein. Because the present invention is based on the relative level of gene expression, mutations in non-coding regions of genes as disclosed herein may also be assayed in the practice of the invention.

"Detection" includes any means of detecting, including direct and indirect detection of gene expression and changes therein. For example, "detectably less" products may be observed directly or indirectly, and the term indicates any reduction (including the absence of detectable signal). Similarly, "detectably more" product means any increase, whether observed directly or indirectly.

Increases and decreases in expression of the disclosed sequences are defined in the following terms based upon percent or fold changes over expression in normal cells. Increases may be of 10, 20, 30, 40, 50, 60, 70, 80, 90, 100, 120, 140, 160, 180, or 200% relative to expression levels in normal cells. Alternatively, fold increases may be of 1, 1.5, 2, 2.5, 3, 3.5, 4, 4.5, 5, 5.5, 6, 6.5, 7, 7.5, 8, 8.5, 9, 9.5, or 10 fold over expression levels in normal cells. Decreases may be of 10, 20, 30, 40, 50, 55, 60, 65, 70, 75, 80, 82, 84, 86, 88, 90, 92, 94, 96, 98, 99 or 100% relative to expression levels in normal cells.

Unless defined otherwise all technical and scientific terms used herein have the same meaning as commonly understood to one of ordinary skill in the art to which this invention belongs.

### Embodiments of the Invention

In a first aspect, the disclosed invention relates to the identification and use of gene expression patterns (or profiles or "signatures") which discriminate between (or are correlated with) breast cancer survival in a subject treated with tamoxifen (TAM) or another "antiestrogen" agent against breast cancer. Such patterns may be determined by the methods of the invention by use of a number of reference cell or tissue samples, such as those reviewed by a pathologist of ordinary skill in the pathology of breast cancer, which reflect breast cancer cells as opposed to normal or other non-cancerous cells. The outcomes experienced by the subjects from whom the samples may be correlated with expression data to identify patterns that correlate with the outcomes following treatment with TAM or another "antiestrogen" agent against breast cancer. Because the overall gene expression profile differs from person to person, cancer to cancer, and cancer cell to cancer cell, correlations between certain cells and genes expressed or underexpressed may be made as disclosed herein to identify genes that are capable of discriminating between breast cancer outcomes.

The present disclosure may be practiced with any number of the genes believed, or likely to be, differentially expressed with respect to breast cancer outcomes, particularly in cases of ER+ breast cancer. The identification may be made by using expression profiles of various homogenous breast cancer cell populations, which were isolated by microdissection, such as, but not limited to, laser capture microdissection (LCM) of 100-1000 cells. The expression level of each gene of the expression profile may be correlated with a particular outcome. Alternatively, the expression levels of multiple genes may be clustered to identify correlations with particular outcomes.

Genes with significant correlations to breast cancer survival when the subject is treated with tamoxifen may be used to generate models of gene expressions that would maximally discriminate between outcomes where a subj ect responds to treatment with tamoxifen or another "antiestrogen" agent against breast cancer and outcomes where the treatment is not successful. Alternatively, genes with significant correlations may be used in combination with genes with lower correlations without significant loss of ability to discriminate between outcomes. Such models may be generated by any appropriate means recognized in the art, including, but not limited to, cluster analysis, supported vector machines, neural networks or other algorithm known in the art. The models are capable of predicting the classification of a unknown sample based upon the expression of the genes used for discrimination in the models. "Leave one out" cross-validation may be used to test the performance of various models and to help identify weights (genes) that are uninformative or detrimental to the predictive ability of the models. Cross-validation may also be used to identify genes that enhance the predictive ability of the models.

The gene(s) identified as correlated with particular breast cancer outcomes relating to tamoxifen treatment by the above models provide the ability to focus gene expression analysis to only those genes that contribute to the ability to identify a subject as likely to have a particular outcome relative to another. The expression of other genes in a breast cancer cell would be relatively unable to provide information concerning, and thus assist in the discrimination of, a breast cancer outcome.

As will be appreciated by those skilled in the art, the models are highly useful with even a small set of reference gene expression data and can become increasingly accurate with the inclusion of more reference data although the incremental increase in accuracy will likely diminish with each additional datum. The preparation of additional reference gene expression data using genes identified and disclosed herein for discriminating between different outcomes in breast cancer following treatment with tamoxifen or another "antiestrogen" agent against breast cancer is routine and may be readily performed by the skilled artisan to permit the generation of models as described above to predict the status of an unknown sample based upon the expression levels of those genes.

To determine the (increased or decreased) expression levels of genes in the practice of the present invention, any method known in the art may be utilized. In one preferred embodiment of the invention, expression based on detection of RNA which hybridizes to the genes identified and disclosed herein is used. This is readily performed by any RNA detection or amplification+detection method known or recognized as equivalent in the art such as, but not limited to, reverse transcription-PCR, the methods disclosed in U.S. Patent 6,794,141, and methods to detect the presence, or absence, of RNA stabilizing or destabilizing sequences.

Alternatively, expression based on detection of DNA status may be used. Detection of the DNA of an identified gene as methylated or deleted may be used for genes that have decreased expression in correlation with a particular breast cancer outcome. This may be readily performed by PCR based methods known in the art, including, but not limited to, Q-PCR. Conversely, detection of the DNA of an identified gene as amplified may be used for genes that have increased expression in correlation with a particular breast cancer outcome. This may be readily performed by PCR based, fluorescent *in situ* hybridization (FISH) and chromosome *in situ* hybridization (CISH) methods known in the art.

Expression based on detection of a presence, increase, or decrease in protein levels or activity may also be used. Detection may be performed by any immunohistochemistry (IHC) based, blood based (especially for secreted proteins), antibody (including autoantibodies against the protein) based, exfoliate cell (from the cancer) based, mass spectroscopy based, and image (including used of labeled ligand) based method known in the art and recognized as appropriate for the detection of the protein. Antibody and image based methods are additionally useful for the localization of tumors after determination of cancer by use of cells obtained by a non-invasive procedure (such as ductal lavage or fine needle aspiration), where the source of the cancerous cells is not known. A labeled antibody or ligand may be used to localize the carcinoma(s) within a patient or to assist in the enrichment of exfoliated cancer cells from a bodily fluid.

A preferred embodiment using a nucleic acid based assay to determine expression is by immobilization of one or more sequences of the genes identified herein on a solid support, including, but not limited to, a solid substrate as an array or to beads or bead based technology as known in the art. Alternatively, solution based expression assays known in the art may also be used. The immobilized gene(s) may be in the form of polynucleotides that are unique or otherwise specific to the gene(s) such that the polynucleotide would be capable of hybridizing to a DNA or RNA corresponding to the gene(s). These polynucleotides may be the full length of the gene(s) or be short sequences of the genes (up to one nucleotide shorter than the full length sequence known in the art by deletion from the 5' or 3' end of the sequence) that are optionally minimally interrupted (such as by mismatches or inserted non-complementary basepairs) such that hybridization with a DNA or RNA corresponding to the gene(s) is not affected. Preferably, the polynucleotides used are from the 3' end of the gene, such as within about 350, about 300, about 250, about 200, about 150, about 100, or about 50 nucleotides from the polyadenylation signal or polyadenylation site of a gene or expressed sequence. Polynucleotides containing mutations relative to the sequences of the disclosed genes may also be used so long as the presence of the mutations still allows hybridization to produce a detectable signal.

The immobilized gene(s), or sequences complementary thereto, may be used to determine the state of nucleic acid samples prepared from sample breast cell(s) for which the outcome of the sample's subject (e.g. patient from whom the sample is obtained) is not known or for confirmation of an outcome that is already assigned to the sample's subject. Without limiting the disclosure, such a cell may be from a patient with ER+ or ER- breast cancer. The immobilized polynucleotide(s) need only be sufficient to specifically hybridize to the corresponding nucleic acid molecules derived from the sample under suitable conditions. While even a single correlated gene sequence may to able to provide adequate accuracy in discriminating between two breast cancer outcomes, two or more, three or more, four or more, five or more, six or more, seven or more, eight or more, nine or more, ten or more, eleven or more, or any integer number of the genes identified herein may be used as a subset capable of discriminating may be used in combination to increase the accuracy of the method. The disclosure specifically contemplates the selection of more than one, two or more, three or more, four or more, five or more, six or more, seven or more, eight or more, nine or more, ten or more, eleven or more, or any integer number of the genes disclosed in the tables and figures herein for use as a subset in the identification of breast cancer survival outcome.

Of course 1 or more, 2 or more, 3 or more, 4 or more, 5 or more, 6 or more, 7 or more, 8 or more, 9 or more, any integer number of, or all the genes provided in Tables 2, 3 and/or XXX below may be used. "Accession" as used in the context of the Tables herein refers to the GenBank accession number of a sequence of each gene. P value refers to values assigned as described in the Examples below. The indications of "E-xx" where "xx" is a two digit number refers to alternative notation for exponential figures where "E-xx" is "10'^{-xx}". Thus in combination with the numbers to the left of "E-xx", the value being represented is the numbers to the left times 10^{-xx}. "Description" as used in the Tables provides a brief identifier of what the sequence/gene encodes.

Genes with a correlation identified by a p value below or about 0.02, below or about 0.01, below or about 0.005, or below or about 0.001 are preferred for use in the practice of the disclosure. The present disclosure includes the use of gene(s) the expression of which identify different breast cancer outcomes after treatment with TAM or another "antiestrogen" agent against breast cancer to permit simultaneous identification of breast cancer survival outcome of a patient based upon assaying a breast cancer sample from said patient.

In a second aspect, which also serves as embodiments of the use of a subset of the genes disclosed herein, the present disclosure relates to the identification and use of multiple sets of sequences for the determination of responsiveness of ER+ breast cancer to treatment with TAM or another "antiestrogen" agent against breast cancer. The differential expression of these sequences in breast cancer relative to normal breast cells is used to predict responsiveness to TAM or another "antiestrogen" agent against breast cancer in a subject.

To identify gene expression patterns in ER positive, early stage invasive breast cancers that might predict response to hormonal therapy, microarray gene expression analysis was performed on tumors from 60 women uniformly treated with adjuvant tamoxifen alone. These patients were identified from a total of 103 ER+ early stage cases presenting to Massachusetts General Hospital between 1987 and 1997, from whom tumor specimens were snap frozen and for whom minimal 5 year follow-up was available (see Table 1 for details). Within this cohort, 28 (46%) women developed distant metastasis with a median time to recurrence of 4 years ("tamoxifen non-responders") and 32 (54%) women remained disease-free with median follow-up of 10 years ("tamoxifen responders"). Responders were matched with non-responder cases with respect to TNM staging (see Singletary, S.E. et al. "Revision of the American Joint Committee on Cancer staging system for breast cancer." J Clin Oncol 20, 3628-36 (2002)) and tumor grade (see Dalton, L.W. et al. "Histologic grading of breast cancer: linkage of patient outcome with level of pathologist agreement." Mod Pathol 13, 730-5. (2000)).

Previous studies linking gene expression profiles to clinical outcome in breast cancer have demonstrated that the potential for distant metastasis and overall survival probability may be predictable through biological characteristics of the primary tumor at the time of diagnosis (see Huang, E. et al. "Gene expression predictors of breast cancer outcomes." Lancet 361, 1590-6 (2003); Sorlie, T. et al. "Gene expression patterns of breast carcinomas distinguish tumor subclasses with clinical implications." Proc Natl Acad Sci U S A 98:10869-74 (2001); Sorlie, T. et al. "Repeated observation of breast tumor subtypes in independent gene expression data sets." Proc Natl Acad Sci U S A 100, 8418-23 (2003); Sotiriou, C. et al. "Breast cancer classification and prognosis based on gene expression profiles from a population-based study." Proc Natl Acad Sci U S A 100, 10393-8 (2003); van't Veer, L.J. et al. "Gene expression profiling predicts clinical outcome of breast cancer." Nature 415, 530-6 (2002); and van de Vijver, M.J. et al. "A gene-expression signature as a predictor of survival in breast cancer." N Engl J Med 347, 1999-2009 (2002)). In particular, a 70-gene expression signature has proven to be a strong prognostic factor, out-performing all known clinicopathological parameters. However, in those studies patients either received no adjuvant therapy (van 't Veer, L.J. et al. Nature 2002) or were treated non-uniformly with hormonal and chemotherapeutic regimens (Huang, E. et al.; Sorlie, T. et al.; Sorlie, T. et al.; Sotiriou, C. et al.; and van de Vijver, M.J. et al. N Engl J Med 2002). Patients with ER+ early-stage breast cancer treated with tamoxifen alone, such as the cohort studied here, represent only a subset of the population tested with the 70-gene signature. Of note, 61 of the genes in the 70-gene signature were present on the microarray used as described below, but no significant association with clinical outcome was observed in the defined subset of patients.

In comparison with existing biomarkers, including ER1, PGR, ERBB2 and EGFR, the sets of gene sequences disclosed herein are significantly more predictive of responsiveness to TAM treatment. Multivariate analysis indicated that these three genes were significant predictors of clinical outcome independent of tumor size, nodal status and tumor grade. ER and progesterone receptor (PR) expression have been the major clinicopathological predictors for response to TAM. However, up to 40% of ER+ tumors fail to respond or develop resistance to TAM. The invention thus provides for the use of the identified biomarkers to allow better patient management by identifying patients who are more likely to benefit from TAM or other endocrine therapy and those who are likely to develop resistance and tumor recurrence.

As noted herein, the sequences(s) identified by the present invention are expressed in correlation with ER+ breast cancer cells. For example, IL17BR, identified by LM.A.G.E. Consortium Clusters NM_018725 and NM_172234 ("The I.M.A.G.E. Consortium: An Integrated Molecular Analysis of Genomes and their Expression," Lennon et al., 1996, Genomics 33:151-152; see also image.llnl.gov) has been found to be useful in predicting responsiveness to TAM treatment.

In preferred embodiments of the invention, any sequence, or unique portion thereof, of the IL17BR sequences of the cluster, as well as the UniGene *Homo sapiens* cluster Hs.5470, may be used. Similarly, any sequence encoding all or a part of the protein encoded by any IL17BR sequence disclosed herein may be used. Consensus sequences of LM.A.G.E. Consortium clusters are as follows, with the assigned coding region (ending with a termination codon) underlined and preceded by the 5' untranslated and/or non-coding region and followed by the 3' untranslated and/or non-coding region:

agcgcagcgt gcgggtggcc tggatcccgc gcagtggccc ggcgatgtcg ctcgtgctgc
taagcctggc cgcgctgtgc aggagcgccg taccccgaga gccgaccgtt caatgtggct
ctgaaactgg gccatctcca gagtggatgc tacaacatga tctaatcccc ggagacttga
gggacctccg agtagaacct gttacaacta gtgttgcaac aggggactat tcaattttga
tgaatgtaag ctgggtactc cgggcagatg ccagcatccg cttgttgaag gccaccaaga
tttgtgtgac gggcaaaagc aacttccagt cctacagctg tgtgaggtgc aattacacag
aggccttcca gactcagacc agaccctctg gtggtaaatg gacattttcc tacatcggct
tccctgtaga gctgaacaca gtctatttca ttggggccca taatattcct aatgcaaata
tgaatgaaga tggcccttcc atgtctgtga atttcacctc accaggctgc ctagaccaca
taatgaaata taaaaaaaag tgtgtcaagg ccggaagcct gtgggatccg aacatcactg
cttgtaagaa gaatgaggag acagtagaag tgaacttcac aaccactccc ctgggaaaca
gatacatggc tcttatccaa cacagcacta tcatcgggtt ttctcaggtg tttgagccac
accagaagaa acaaacgcga gcttcagtgg tgattccagt gactggggat agtgaaggtg
ctacggtgca gctgactcca tattttccta cttgtggcag cgactgcatc cgacataaag
gaacagttgt gctctgccca caaacaggcg tccctttccc tctggataac aacaaaagca
agccgggagg ctggctgcct ctcctcctgc tgtctctgct ggtggccaca tgggtgctgg
tggcagggat ctatctaatg tggaggcacg aaaggatcaa gaagacttcc ttttctacca
ccacactact gccccccatt aaggttcttg tggtttaccc atctgaaata tgtttccatc
acacaatttg ttacttcact gaatttcttc aaaaccattg cagaagtgag gtcatccttg
aaaagtggca gaaaaagaaa atagcagaga tgggtccagt gcagtggctt gccactcaaa
agaaggcagc agacaaagtc gtcttccttc tttccaatga cgtcaacagt gtgtgcgatg
gtacctgtgg caagagcgag ggcagtccca gtgagaactc tcaagacctc ttcccccttg
cctttaacct tttctgcagt gatctaagaa gccagattca tctgcacaaa tacgtggtgg
tctactttag agagattgat acaaaagacg attacaatgc tctcagtgtc tgccccaagt
accacctcat gaaggatgcc actgctttct gtgcagaact tctccatgtc aagcagcagg

tgtcagcagg aaaaagatca caagcctgcc acgatggctg ctgctccttg tagcccaccc
atgagaagca agagacctta aaggcttcct atcccaccaa ttacagggaa aaaacgtgtg
atgatcctga agcttactat gcagcctaca aacagcctta gtaattaaaa catttatac
caataaaatt ttcaaatatt gctaactaat gtagcattaa ctaacgattg gaaactacat
ttacaacttc aaagctgttt tatacataga aatcaattac agttttaatt gaaaactata
accattttga taatgcaaca ataaagcatc ttcagccaaa catctagtct tccatagacc
atgcattgca gtgtacccag aactgtttag ctaatattct atgtttaatt aatgaatact
aactctaaga acccctcact gattcactca atagcatctt aagtgaaaaa ccttctatta
catgcaaaaa atcattgttt ttaagataac aaaagtaggg aataaacaag ctgaacccac
ttttaaaaaa aaaaaaaaaa aaaaaaaaaa aaaaaaa

SEQ ID NO:2 (consensus sequence for IL17BR, transcript variant 2, identified as NM_172234 or NM_172234.1):

agcgcagcgt gcgggtggcc tggatcccgc gcagtggccc ggcgatgtcg ctcgtgctgc
taagcctggc cgcgctgtgc aggagcgccg taccccgaga gccgaccgtt caatgtggct
ctgaaactgg gccatctcca gagtggatgc tacaacatga tctaatcccc ggagacttga
gggacctccg agtagaacct gttacaacta gtgttgcaac aggggactat tcaattttga
tgaatgtaag ctgggtactc cgggcagatg ccagcatccg cttgttgaag gccaccaaga
tttgtgtgac gggcaaaagc aacttccagt cctacagctg tgtgaggtgc aattacacag
aggccttcca gactcagacc agaccctctg gtggtaaatg gacattttcc tacatcggct
tccctgtaga gctgaacaca gtctatttca ttggggccca taatattcct aatgcaaata
tgaatgaaga tggcccttcc atgtctgtga atttcacctc accaggctgc ctagaccaca
taatgaaata taaaaaaaag tgtgtcaagg ccggaagcct gtgggatccg aacatcactg
cttgtaagaa gaatgaggag acagtagaag tgaacttcac aaccactccc ctgggaaaca
gatacatggc tcttatccaa cacagcacta tcatcgggtt ttctcaggtg tttgagccac
accagaagaa acaaacgcga gcttcagtgg tgattccagt gactggggat agtgaaggtg
ctacggtgca ggtaaagttc agtgagctgc tctggggagg gaagggacat agaagactgt
tccatcattc attgctttta aggatgagtt ctctcttgtc aaatgcactt ctgccagcag
acaccagtta agtggcgttc atgggggctc tttcgctgca gcctccaccg tgctgaggtc
aggaggccga cgtggcagtt gtggtccctt ttgcttgtat taatggctgc tgaccttcca
aagcactttt tattttcatt ttctgtcaca gacactcagg gatagcagta ccattttact
tccgcaagcc tttaactgca agatgaagct gcaaagggtt tgaaatggga aggtttgagt
tccaggcagc gtatgaactc tggagagggg ctgccagtcc tctctgggcc gcagcggacc
cagctggaac acaggaagtt ggagcagtag gtgctccttc acctctcagt atgtctcttt
caactctagt ttttgaggtg gggacacagg aggtccagtg ggacacagcc actccccaaa
gagtaaggag cttccatgct tcattccctg gcataaaaag tgctcaaaca caccagaggg
ggcaggcacc agccagggta tgatggctac tacccttttc tggagaacca tagacttccc
ttactacagg gacttgcatg tcctaaagca ctggctgaag gaagccaaga ggatcactgc
tgctcctttt ttctagagga aatgtttgtc tacgtggtaa gatatgacct agccctttta
ggtaagcgaa ctggtatgtt agtaacgtgt acaaagttta ggttcagacc ccgggagtct
tgggcacgtg ggtctcgggt cactggtttt gactttaggg ctttgttaca gatgtgtgac
caaggggaaa atgtgcatga caacactaga ggtatgggcg aagccagaaa gaagggaagt
tttggctgaa gtaggagtct tggtgagatt ttgctctgat gcatggtgtg aactttctga
gcctcttgtt tttcctcagc tgactccata ttttcctact tgtggcagcg actgcatccg
acataaagga acagttgtgc tctgcccaca aacaggcgtc cctttccctc tggataacaa
caaaagcaag ccgggaggct ggctgcctct cctcctgctg tctctgctgg tggccacatg
ggtgctggtg gcagggatct atctaatgtg gaggcacgaa aggatcaaga agacttcctt
ttctaccacc acactactgc cccccattaa ggttcttgtg gtttacccat ctgaaatatg
tttccatcac acaatttgtt acttcactga atttcttcaa aaccattgca gaagtgaggt
catccttgaa aagtggcaga aaaagaaaat agcagagatg ggtccagtgc agtggcttgc
cactcaaaag aaggcagcag acaaagtcgt cttccttctt tccaatgacg tcaacagtgt

gtgcgatggt acctgtggca agagcgaggg cagtcccagt gagaactctc aagacctctt
cccccttgcc tttaaccttt tctgcagtga tctaagaagc cagattcatc tgcacaaata
cgtggtggtc tactttagag agattgatac aaaagacgat tacaatgctc tcagtgtctg
ccccaagtac cacctcatga aggatgccac tgctttctgt gcagaacttc tccatgtcaa
gcagcaggtg tcagcaggaa aaagatcaca agcctgccac gatggctgct gctccttgta
gcccacccat gagaagcaag agaccttaaa ggcttcctat cccaccaatt acagggaaaa
aacgtgtgat gatcctgaag cttactatgc agcctacaaa cagccttagt aattaaaaca
ttttatacca ataaaatttt caaatattgc taactaatgt agcattaact aacgattgga
aactacattt acaacttcaa agctgtttta tacatagaaa tcaattacag ttttaattga
aaactataac cattttgata atgcaacaat aaagcatctt cagccaaaca tctagtcttc
catagaccat gcattgcagt gtacccagaa ctgtttagct aatattctat gtttaattaa
tgaatactaa ctctaagaac ccctcactga ttcactcaat agcatcttaa gtgaaaaacc
ttctattaca tgcaaaaaat cattgttttt aagataacaa aagtagggaa taaacaagct
gaacccactt ttaaaaaaaa aaaaaaaaaa aaaaaaaaaa aaaaa

I.M.A.G.E. Consortium Clone ID numbers and the corresponding GenBank accession numbers of sequences identified as belonging to the I.M.A.G.E. Consortium and UniGene clusters, are listed below. Also included are sequences that are not identified as having a Clone ID number but still identified as being those of IL17BR. The sequences include those of the "sense" and complementary strands sequences corresponding to IL17BR. The sequence of each GenBank accession number is presented in the attached Appendix.

| Clone ID numbers | GenBank accession numbers |
|---|---|
| 2985728 | AW675096, AW673932, BC000980 |
| 5286745 | BI602183 |
| 5278067 | B1458542 |
| 5182255 | BI823321 |
| 924000 | AA514396 |
| 3566736 | IBF110326 |
| 3195409 | BE466508 |
| 3576775 | BF740045 |
| 2772915 | AW299271 |
| 1368826 | AA836217 |
| 1744837 | AI203628 |
| 2285564 | AI627783 |
| 2217709 | AI744263 |
| 2103651 | AI401622 |
| 2419487 | AI826949 |
| 3125592 | BE047752 |
| 2284721 | AI911549 |
| 3643302 | BF194822 |
| 1646910 | AI034244 |
| 1647001 | AI033911 |
| 3323709 | BF064177 |
| 1419779 | AA847767 |
| 2205190 | AI538624 |
| 2295838 | AI913613 |
| 2461335 | AI942234 |
| 12130362 | JAI580483 |
| 2385555 | AI831909 |
| 2283817 | AI672344 |
| 2525596 | AW025192 |
| 454687 | AA677205 |
| 1285273 | AA721647 |
| 3134106 | BF115018 |
| 342259 | W61238, W61239 |
| 1651991 | AI032064 |
| 2687714 | AW236941 |
| 3302808 | BG057174 |
| 2544461 | AW058532 |
| 122014 | T98360, T98361 |
| 2139250 | AI470845 |
| 2133899 | AI497731 |
| 121300 T96629, T96740 | T96629, T96740 |
| 162274 | H25975, He941 |
| 3446667 | BE539514, BX282554 |
| 156864 | R74038, R74129 |
| 4611491 | BG433769 |
| 4697316 | BG530489 |
| 429376 | AA007528, AA007529 |
| 5112415 | BI260259 |
| 701357 | AA287951, AA287911 |
| 121909 | T97852, T97745 |
| 268037 | N40294 |
| 1307489 | AA809841 |
| 1357543 | AA832389 |
| 48442 | H14692 |
| 1302619 | AA732635 |
| 1562857 | AA928257 |
| 1731938 | AI184427 |
| 1896025 | AI298577 |
| 2336350 | AI692717 |
| 1520997 | AA910922 |
| 240506 | H90761 |
| 2258560 | AI620122 |
| 1569921 | AI793318, AA962325, AI733290 |
| 6064627 | BQ226353 |
| 299018 | W04990 |
| 5500181 | BM455231 |
| 2484011 | BI492426 |
| 4746376 | BG674622 |
| 233783 | BX111256 |
| 1569921 | BX1117618 |
| 450450 | AA682806 |
| 1943085 | AI202376 |
| 2250390 | AI658949 |
| 4526156 | BG403405 |
| 3249181 | BE673417 |
| 2484395 | AW021469 |
| 30515867 | CF455736 |
| 285815 | AW339874 |
| 4556884 | BG399724 |
| 3254505 | BF475787 |
| 3650593 | BF437145 |
| 233783 | H64601 |
| None (mRNA sequences) | AF212365, AKp95091 AF208110, AF208111, AF250309, AK095091 |
| None | BM983744, CB305764, BM715988, BM670929, BI792416, BI715216, N56060, CB241389, AV660618, BX088671, CB154426, CA434589, CA412162, CA314073, BF921554, BF920093, AV685699, AV650175, BX483104, CD675121, BE081436, AW970151, AW837146, AW368264, D25960, AV709899, BX431018, AL535617, AL525465, BX453536, BX453537, AV728945, AV728939, AV727345 |

In one preferred embodiment, any sequence, or unique portion thereof, of the following IL17BR sequence, identified by AF208111 or AF208111.1, may be used in the practice of the invention.
SEQ ID NO:3 (sequence for IL17BR):

CGGCGATGTCGCTCGTGCTGATAAGCCTGGCCGCGCTGTGCAGGAGCGCCGTACCCCGAG
AGCCGACCGTTCAATGTGGCTCTGAAACTGGGCCATCTCCAGAGTGGATGCTACAACATG
ATCTAATCCCCGGAGACTTGAGGGACCTCCGAGTAGAACCTGTTACAACTAGTGTTGCAA
CAGGGGACTATTCAATTTTGATGAATGTAAGCTGGGTACTCCGGGCAGATGCCAGCATCC
GCTTGTTGAAGGCCACCAAGATTTGTGTGACGGCAAAAGCAACTTCCAGTCCTACAGCT
GTGTGAGGTGCAATTACACAGAGGCCTTCCAGACTCAGACCAGACCCTCTGGTGGTAAAT
GGACATTTTCCTATATCGGCTTCCCTGTAGAGCTGAACACAGTCTATTTCATTGGGGCCC
ATAATATTCCTAATGCAAATATGAATGAAGATGGCCCTTCCATGTCTGTGAATTTCACCT
CACCAGGCTGCCTAGACCACATAATGAAATATAAAAAAAGTGTGTCAAGGCCGGAAGCC
TGTGGGATCCGAACATCACTGCTTGTAAGAAGAATGAGGAGACAGTAGAAGTGAACTTCA
CAACCACTCCCCTGGGAAACAGATACATGGCTCTTATCCAACACAGCACTATCATCGGGT
TTTCTCAGGTGTTTGAGCCACACCAGAAGAAACAAACGCGAGCTTCAGTGGTGATTCCAG
TGACTGGGGATAGTGAAGGTGCTACGGTGCAGGTAAAGTTCAGTGAGCTGCTCTGGGGAG
GGAAGGGACATAGAAGACTGTTCCATCATTCATTGCTTTTAAGGATGAGTTCTCTCTTGT
CAAATGCACTTCTGCCAGCAGACACCAGTTAAGTGGCGTTCATGGGGGTTCTTTCGCTGC
AGCCTCCACCGTGCTGAGGTCAGGAGGCCGACGTGGCAGTTGTGGTCCCTTTTGCTTGTA
TTAATGGCTGCTGACCTTCCAAAGCACTTTTTATTTTCATTTTCTGTCACAGACACTCAG
GGATAGCAGTACCATTTTACTTCCGCAAGCCTTTAACTGCAAGATGAAGCTGCAAAGGGT
TTGAAATGGGAAGGTTTGAGTTCCAGGCAGCGTATGAACTCTGGAGAGGGGCTGCCAGTC
CTCTCTGGGCCGCAGCGGACCCAGCTGGAACACAGGAAGTTGGAGCAGTAGGTGCTCCTT
CACCTCTCAGTATGTCTCTTTCAACTCTAGTTTTTGAAGTGGGGACACAGGAAGTCCAGT
GGGGACACAGCCACTCCCCAAAGAATAAGGAACTTCCATGCTTCATTCCCTGGCATAAAA
AGTGNTCAAACACACCAGAGGGGGCAGGCACCAGCCAGGGTATGATGGGTACTACCCTTT
TCTGGAGAACCATAGACTTCCCTTACTACAGGGACTTGCATGTCCTAAAGCACTGGCTGA
AGGAAGCCAAGAGGATCACTGCTGCTCCTTTTTTGTAGAGGAAATGTTTGTGTACGTGGT
AAGATATGACCTAGCCCTTTTAGGTAAGCGAACTGGTATGTTAGTAACGTGTACAAAGTT
TAGGTTCAGACCCCGGGAGTCTTGGGCATGTGGGTCTCGGGTCACTGGTTTTGACTTTAG
GGCTTTGTTACAGATGTGTGACCAAGGGGAAAATGTGCATGACAACACTAGAGGTAGGGG
CGAAGCCAGAAAGAAGGGAAGTTTTGGCTGAAGTAGGAGTCTTGGTGAGATTTTGCTGTG
ATGCATGGTGTGAACTTTCTGAGCCTCTTGTTTTTCCTCAGCTGACTCCATATTTTCCTA
CTTGTGGCAGCGACTGCATCCGACATAAAGGAACAGTTGTGCTCTGCCCACAAACAGGCG
TCCCTTTCCCTCTGGATAACAACAAAAGCAAGCCGGGAGGCTGGCTGCCTCTCCTCCTGC
TGTCTCTGCTGGTGGCCACATGGGTGCTGGTGGCAGGGATCTATCTAATGTGGAGGCACG
AAAGGATCAAGAAGACTTCCTTTTCTACCACCACTACTGCCCCCCATTAAGGTTCTTG
TGGTTTACCCATCTGAAATATGTTTCCATCACACAATTTGTTACTTCACTGAATTTCTTC
AAAACCATTGCAGAAGTGAGGTCATCCTTGAAAAGTGGCAGAAAAAGAAAATAGCAGAGA
TGGGTCCAGTGCAGTGGCTTGCCACTCAAAAGAAGGCAGCAGACAAAGTCGTCTTCCTTC
TTTCCAATGACGTCAACAGTGTGTGCGATGGTACCTGTGGCAAGAGCGAGGGCAGTCCCA
GTGAGAACTCTCAAGACCTCTTCCCCCTTGCCTTTAACCTTTTCTGCAGTGATCTAAGAA
GCCAGATTCATCTGCACAAATACGTGGTGGTCTACTTTAGAGAGATTGATACAAAAGACG
ATTACAATGCTCTCAGTGTCTGCCCCAAGTACCACTTCATGAAGGATGCCACTGCTTTCT
GTGCAGAACTTCTCCATGTCAAGCAGCAGGTGTCAGCAGGAAAAAGATCACAAGCCTGCC
ACGATGGCTGCTGCTCCTTGTAGCCCACCCATGAGAAGCAAGAGACCTTAAAGGCTTCCT
ATCCCACCAATTACAGGGAAAAAACGTGTGATGATCCTGAAGCTTACTATGCAGCCTACA

AACAGCCTTAGTAATTAAAACATTTTATACCAATAAAATTTTCAAATATTACTAACTAAT
GTAGCATTAACTAACGATTGGAAACTACATTTACAACTTCAAAGCTGTTTTATACATAGA
AATCAATTACAGCTTTAATTGAAAACTGTAACCATTTTGATAATGCAACAATAAAGCATC
TTCCAAAAAAAAAAAAAAAAAAAAAAAAAAAAAAA

In preferred embodiments of the disclosure, any sequence, or unique portion thereof, of the CHDH sequences of the cluster, as well as the UniGene Homo sapiens cluster Hs. 126688, may be used. Similarly, any sequence encoding all or a part of the protein encoded by any CHDH sequence disclosed herein, including sequences of the new assembled contig, may be used. Consensus sequences of I.M.A.G.E. Consortium clusters are as follows, with the assigned coding region (ending with a termination codon) underlined and preceded by the 5' untranslated and/or non-coding region and followed by the previously identified 3' untranslated and/or non-coding region:
SEQ ID NO:4 (consensus sequence for CHDH, identified as NM_018397 or NM_018397.1):

agcgggccgc ggccacccgc tcctcccgct ccggtcccga ctgtcgggct ctcggccgag
tcgccccgga caatcacaaa gagtgtgtag gccagccccg gtcacagagt gcaccgtatc
ctgtcacttc tggatgtgag ggagaagtga gtcatctcat tcccctccgt ggatcagagg
acttggacta gatagaagca tgtggtgtct cctacgaggc ctgggccggc ctggagccct
ggcacgggga gccctggggc agcagcaatc cctgggtgcc cgggccctgg ccagcgcagg
ctctgagagc cgggacgagt acagctatgt ggtggtgggc gcgggctcgg cgggctgcgt
gctggctggg aggctcacgg aggaccccgc cgagcgcgtg ctgctgctgg aggccgggcc
caaggacgtg cgcgcgggga gcaagcggct ctcgtggaag atccacatgc ccgcggccct
ggtggccaac ctgtgcgacg acaggtacaa ctggtgctac cacacagagg tgcagcgggg
cctggacggc cgcgtgctgt actggccacg cggccgcgtc tggggtggct cctcatccct
caatgccatg gtctacgtcc gtgggcacgc cgaggactac gagcgctggc agcgccaggg
cgcccgcggc tgggactacg cgcactgcct gccctacttc cgcaaggcgc agggccacga
gctgggcgcc agccggtacc ggggcgccga tggcccgctg cgggtgtccc ggggcaagac
caaccacccg ctgcactgcg cattcctgga ggccacgcag caggccggct acccgctcac
cgaggacatg aatggcttcc agcaggaggg cttcggctgg atggacatga ccatccatga
aggcaaacgg tggagcgcag cctgtgccta cctgcaccca gcactgagcc gcaccaacct
caaggccgag gccgagacgc ttgtgagcag ggtgctattt gagggcaccc gtgcagtggg
cgtggagtat gtcaagaatg gccagagcca cagggcttat gccagcaagg aggtgattct
gagtggaggt gccatcaact ctccacagct gctcatgctc tctggcatcg ggaatgctga
tgacctcaag aaactgggca tccctgtggt gtgccaccta cctggggttg gccagaacct
gcaagaccac ctggagatct acattcagca ggcatgcacc cgccctatca ccctccattc
agcacagaag cccctgcgga aggtctgcat tggtctggag tggctctgga aattcacagg

ggagggagcc actgcccatc tggaaacagg tgggttcatc cgcagccagc ctggggtccc
ccacccggac atccagttcc atttcctgcc atcccaagtg attgaccacg ggcgggtccc
cacccagcag gaggcttacc aggtacatgt ggggcccatg cggggcacga gtgtgggctg
gctcaaactg agaagtgcca atccccaaga ccaccctgtg atccagccca actacttgtc
aacagaaact gatattgagg atttccgtct gtgtgtgaag ctcaccagag aaatttttgc
acaggaagcc ctggctccgt tccgagggaa agagctccag ccaggaagcc acattcagtc
agataaagag atagatgcct ttgtgcgggc aaaagccgac agcgcctacc acccctcgtg
cacctgtaag atgggccagc cctccgatcc cactgccgtg gtggatccgc agacaagggt
cctcggggtg gaaaacctca gggtcgtcga tgcctccatc atgcctagca tggtcagcgg
caacctgaac gcccccacaa tcatgatcgc agagaaggca gctgacatta tcaaggggca
gcctgcactc tgggacaaag atgtccctgt ctacaagccc aggacgctgg ccacccagcg
ctaagacagt tgctgctgga ggatgaccag ggaagccccc tgataagcca agaggccag
cacagccctt gctcccaggc tcctgcctga aactatctag cacactagga cccaggtggt
accctactca gtggctgaga attggataaa gtcttgggaa atgagacaaa aaaaaaaaaa
aa

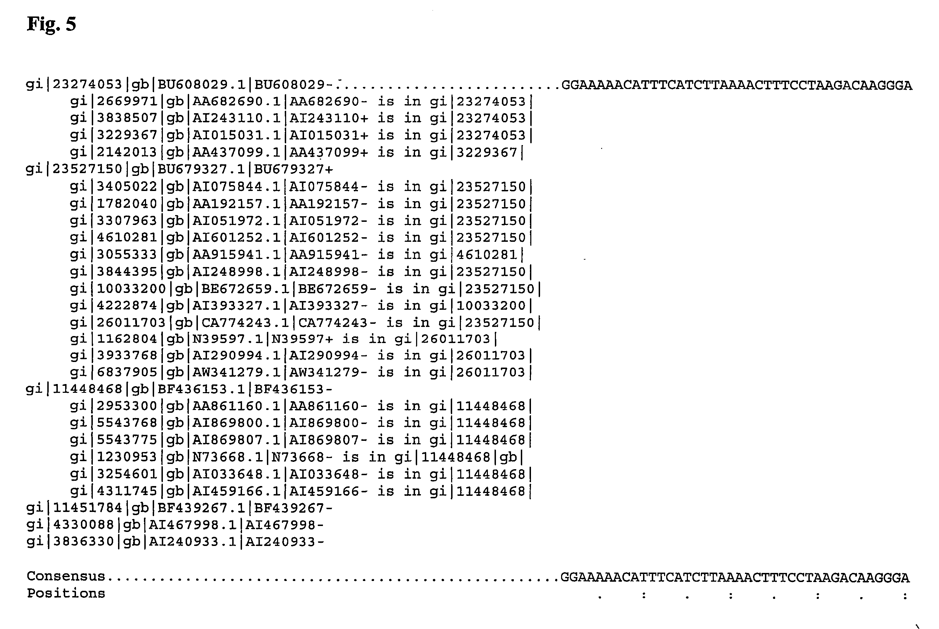

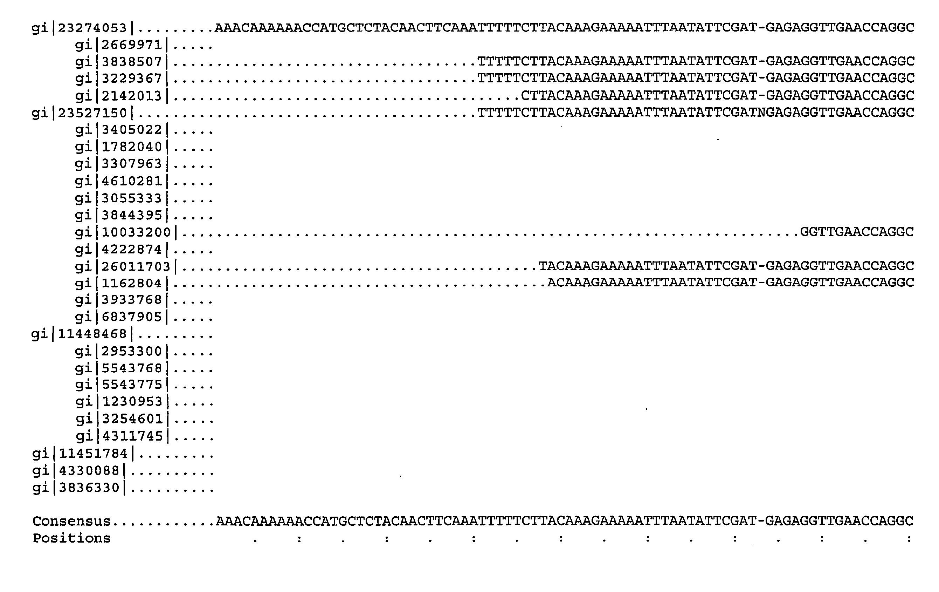

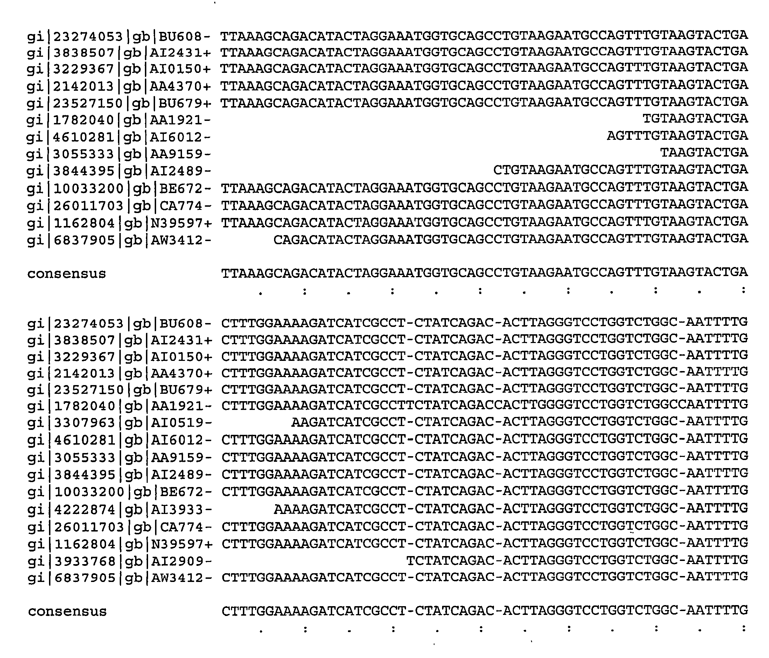

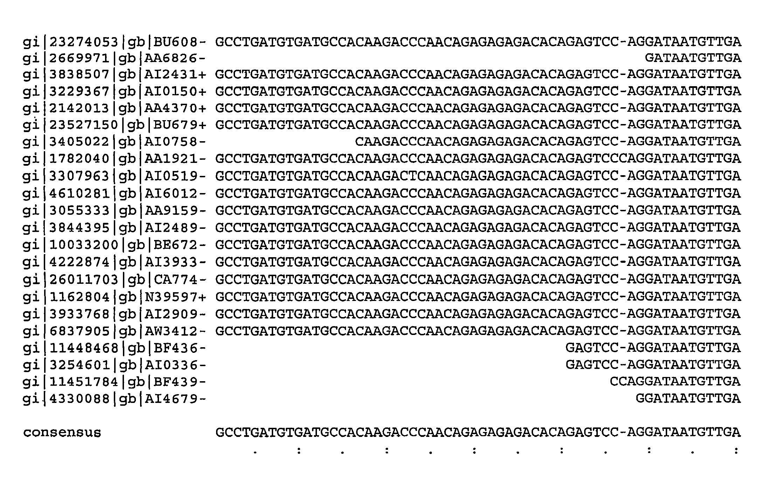

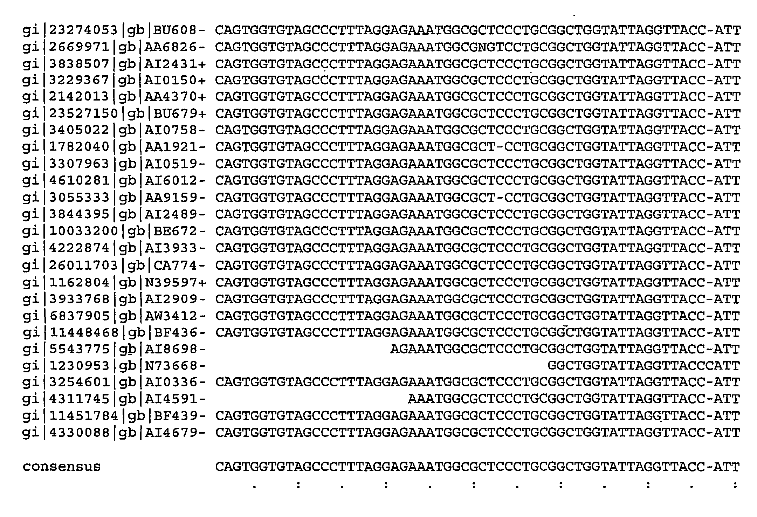

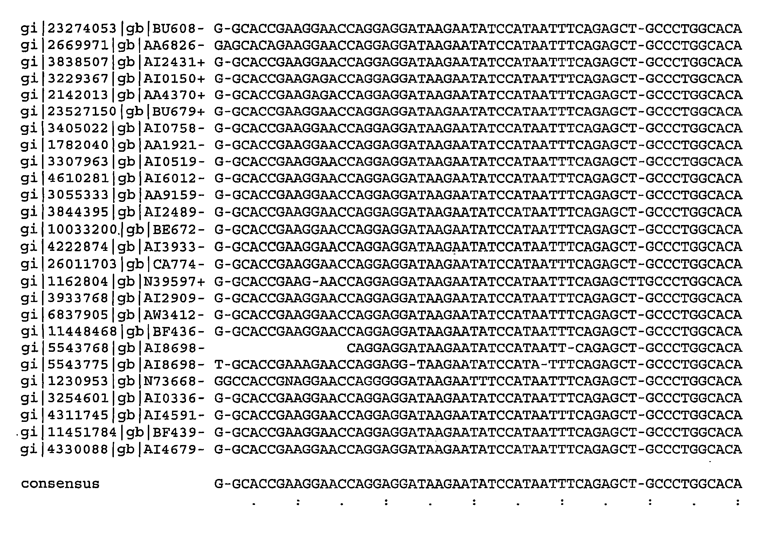

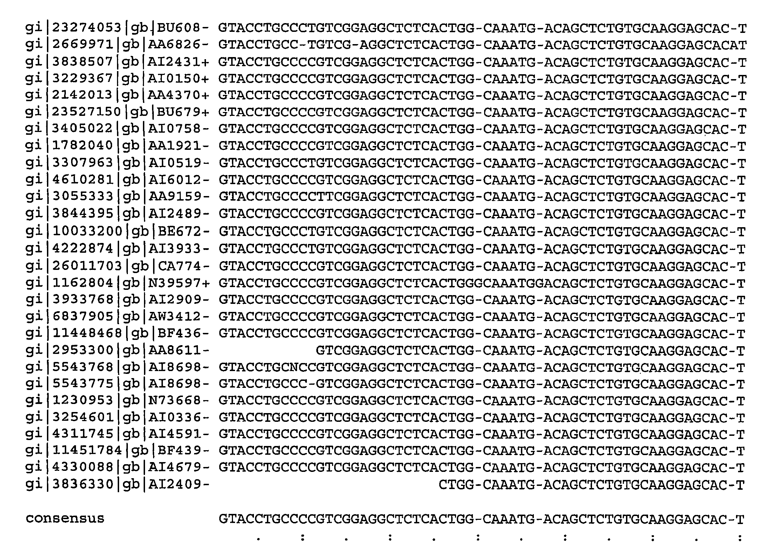

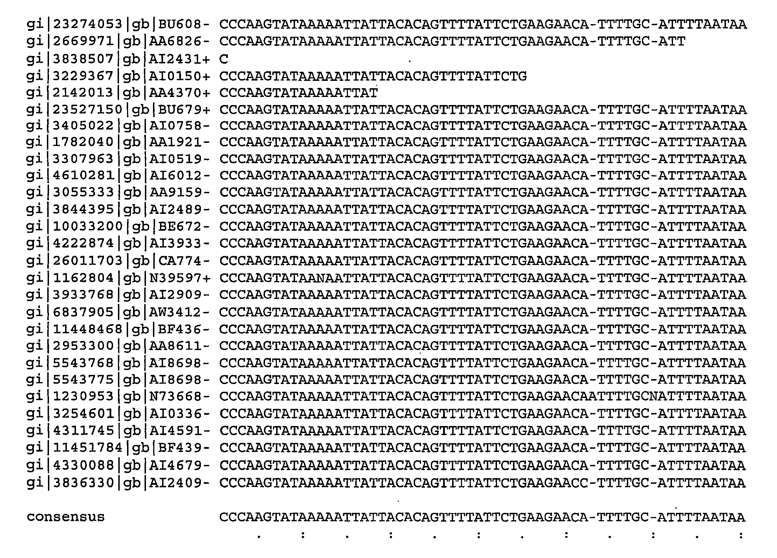

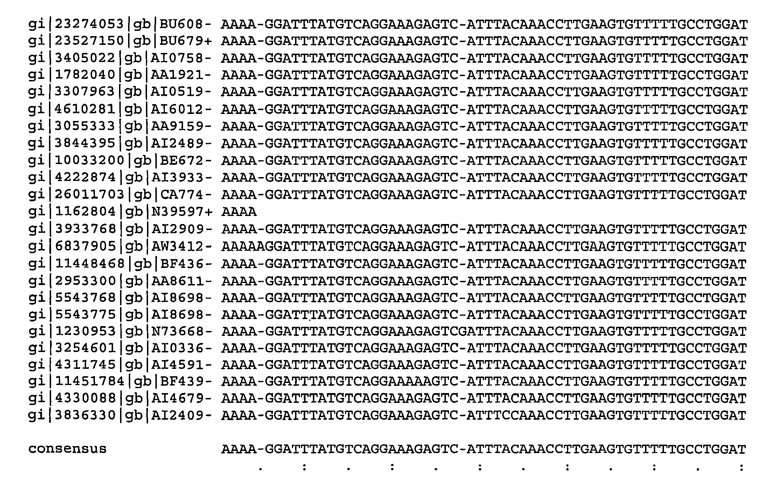

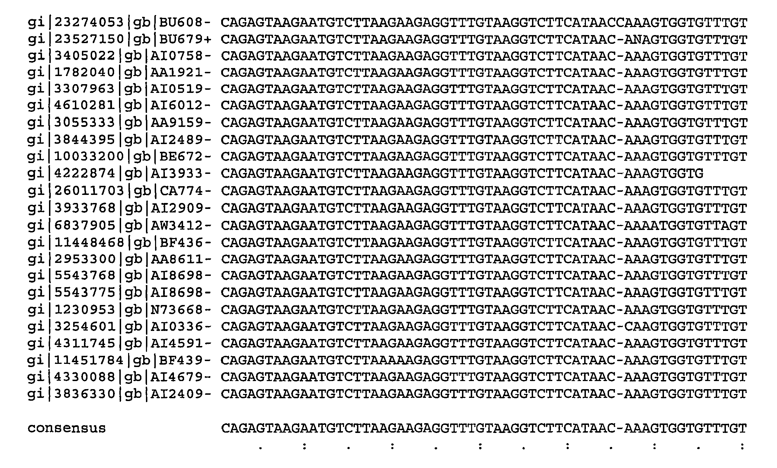

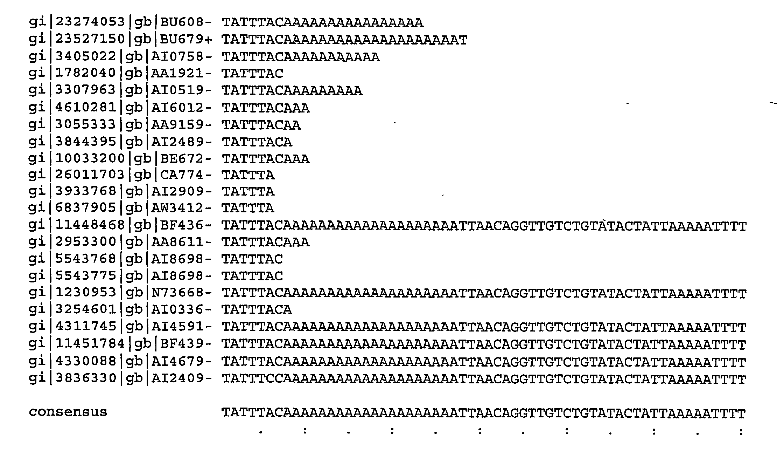

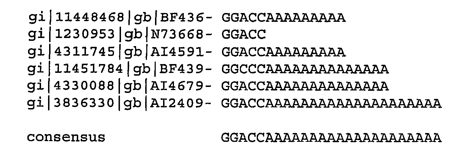

I.M.A.G.E. Consortium Clone ID numbers and the corresponding GenBank accession numbers of sequences identified as belonging to the I.M.A.G.E. Consortium and UniGene clusters, are listed below. Also included are sequences that are not identified as having a Clone ID number but still identified as being those of CHDH. The sequences include those of the "sense" and complementary strands sequences corresponding to CHDH. Additional sequences for use in the practice of the disclosure are those aligned in Figure 5, which are also provided in the attached Appendix.

| Clone ID number/ GenBank accession number(s) |
|---|
| 4824572/BC034502 and BG720228 |
| 5191415/BI765156 |
| 5311690/B1667529 |
| 5267676/BI460380 |
| 11031605/AA609488 |
| 3842653/BE732217 |
| 4543273/BG336766 |
| 3504516/BE279319 |
| 3140587BE279968 |
| 6297066/BQ648069 |
| 2714263/AW449121 |
| 2735859/AW450678 |
| 2720363/AW139168 |
| 3642981/BF195860 |
| 5931105/BQ066460 |
| 3574335/BF430927 |
| 3268842/BF435866 |
| 3267752/BF435185 |
| 1868020/AI264647 and BX116752 and AI733810 and AI792632 |
| 2365837/AI741739 |
| 3085519BF510364 |
| 1647746/AI034449 |
| 2695349/AW194S22 |
| 2285283/AI628996 |
| 2694067/AW235087 |
| 2285315/AI629023 |
| 2463061/AI928186 |
| 2462306/AI927042 |
| 2381448/AI768443 |
| 2298488/AI650346 |
| 3134601/BF197300 |
| 2300327/AI631941 |
| 2697626/AW167538 |
| 3034918/AW779820 |
| 2525301/AW024823 |
| 2300291/AI631914 |
| 2137091/AI473735 |
| 4147169/BG060119 |
| 2772286/AW299654 |
| 2172535/AI564145 |
| 2690214/AW241612 |
| 1868068/AI241086 |
| 1608918/AA991365 |
| 3134810/BF197431 |
| 1869723/AI245204 |
| 2691133/AW242403 |
| 6109050/BU500214 |
| 2384051/AI796286 |
| 12055388/AI308167 |
| 3032446/AW771262 |
| 2907815/AW340332 |
| 1636795//AI792354 and AI017355 |
| 2299592/AI640195 |
| 2054920/AI334627 |
| 2690173/AW237735 |
| 1869819/AI245373 |
| 3195030BE464406 |
| 1646613/AI025866 |
| 2773291/AW299629 |
| 2461358/AI942245 |
| 5678397/BM142449 and BM142311 |
| 5672209/BM052814 and BM053126 |
| 2137904/AI800207 |
| 511224/A088689 and AA088826 |
| 2734357/AW449405 |
| 381379/AA052926 and AA052927 |
| 2337545/AI914219 |
| 2528186/AW337722 |
| 2028284/AI262965 |
| 3436048BF940636 |
| 2344677/AI695649 |
| 123940/R00867 and R01524/ |
| 240988/H90906 and H91018 |
| 240077/H82409 and H82667 |
| None (mRNA sequences)/NM_018397.1 and AJ272267.1 and AK055402.1 |
| None/AA772473.1 and BM682615.1 and BM713059.1 and BM716959.1^{.}and BU738538.1 and AA324019.1 and AA302740.1 and C20981.1 and BF930030.1 and BQ303877.1 and BM769931.1 and AW900269.1 and F26419.1 and CB147231.1 and BE765491.1 AV656671.1 |

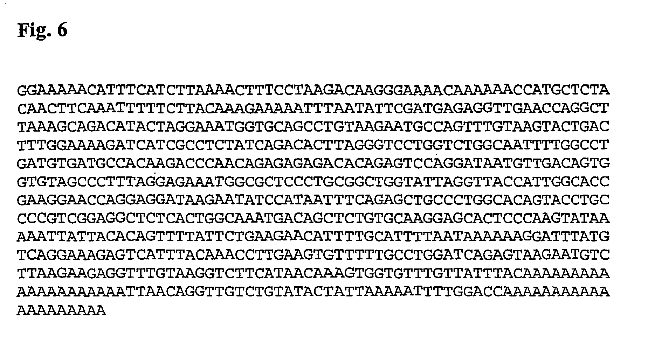

In one preferred embodiment, any sequence, or unique portion thereof, of the CHDH sequences in Figures 5 or 6 may be used in the practice of the disclosure

In another set of preferred embodiments of the disclosure any sequence, or unique portion thereof, of the QPRT sequences of the I.M.A.G.E. Consortium cluster NM_014298, as well as the UniGene Homo sapiens cluster Hs.126688, may be used. Similarly, any sequence encoding all or a part of the protein encoded by any QPRT sequence disclosed herein may be used. The consensus sequence of the I.M.A.G.E. Consortium cluster is as follows, with the assigned coding region (ending with a termination codon) underlined and preceded by the 5' untranslated and/or non-coding region and followed by the 3' untranslated and/or non-coding region:
SEQ ID NO:5 (consensus sequence for QPRT, identified as NM_014298 or NM_014298.2):

gtcctgagca gccaacacac cagcccagac agctgcaagt caccatggac gctgaaggcc
tggcgctgct gctgccgccc gtcaccctgg cagccctggt ggacagctgg ctccgagagg
actgcccagg gctcaactac gcagccttgg tcagcggggc aggcccctcg caggcggcgc
tgtgggccaa atcccctggg gtactggcag ggcagccttt cttcgatgcc atatttaccc
aactcaactg ccaagtctcc tggttcctcc ccgagggatc gaagctggtg ccggtggcca
gagtggccga ggtccggggc cctgcccact gcctgctgct gggggaacgg gtggccctca
acacgctggc ccgctgcagt ggcattgcca gtgctgccgc cgctgcagtg gaggccgcca
ggggggccgg ctggactggg cacgtggcag gcacgaggaa gaccacgcca ggcttccggc
tggtggagaa gtatgggctc ctggtgggcg gggccgcctc gcaccgctac gacctgggag
ggctggtgat gttgaaggat aaccatgtgg tgccccccgg tggcgtggag aaggcggtgc
gggcggccag acaggcggct gacttcgctc tgaaggtgga agtggaatgc agcagcctgc
aggaggtcgt ccaggcagct gaggctggcg ccgaccttgt cctgctggac aacttcaagc
cagaggagct gcaccccacg gccaccgcgc tgaaggccca gttcccgagt gtggctgtgg
aagccagtgg gggcatcacc ctggacaacc tcccccagtt ctgcgggccg cacatagacg
tcatctccat ggggatgctg acccaggcgg tcccagccct tgatttctcc ctcaagctgt
ttgccaaaga ggtggctcca gtgcccaaaa tccactagtc ctaaaccgga agaggatgac
accggccatg ggttaacgtg gctcctcagg accctctggg tcacacatct ttagggtcag
tgaacaatgg ggcacatttg gcactagctt gagcccaact ctggctctgc cacctgctgc
tcctgtgacc tgtcagggct gacttcacct ctgctcatct cagtttccta atctgtaaaa
tgggtctaat aaaggatcaa ccaaaaaaaa aaaaaaaaaa aa

I.M.A.G.E. Consortium Clone ID numbers and the corresponding GenBank accession numbers of sequences identified as belonging to the I.M.A.G.E. Consortium and UniGene clusters, are listed below. Also included are sequences that are not identified as having a Clone ID number but still identified as being those of QPRT. The sequences include those of the "sense" and complementary strands sequences corresponding to QPRT. Representative sequences of GenBank accession numbers below are presented in the attached Appendix.

| Clone ID number/ GenBank accession number(s) |
|---|
| 2960170/BC005060 and BE299670 and BE299712 □ |
| 3506460/BE273102 and BC010033 □ |
| 3959973/BC018910 and BE902622 □ |
| 267692/N23182 and N32648 □ |
| 3843834/BE735342 □ |
| 4872092/BX283118 and BG769505 □ |
| 4845859/13G750434 □ |
| 4868806/BG766440 □ |
| 4594651/BG441877 □ |
| 4553618/BG337811 □ |
| 4554044/BG338063 □ |
| 44731611/BG251163 □ |
| 14581127/BG396079 □ |
| 4136221/BF316915 □ |
| 4127089/BF313098 □ |
| 4508387/BG257831 □ |
| 4125826/BF312975 □ |
| 4411920/BG115486 |
| 4842556/BG748194 |
| 4395232/BF980859 |
| 4122808/BF304964 |
| 6305325/BQ643384 |
| 4107138/BF204965 |
| 4875437/BG753310/ |
| 6337913/BU501237 |
| 4136491/BF317004 |
| 4131857/13F307788 |
| 4302204/BF684687 |
| 5092370/BI195027 |
| 3353576/BE257622 |
| 4473768/BG252578 |
| 3912491/BE887856 |
| 160124003/BU186666 |
| 4873695/BG751315 |
| .4873694/BG751234 |
| 4080072/BF237708 |
| 6300166/BQ876922 |
| 896716/BI198351 |
| 4877853/BG770209 |
| 4896715/BI198375 |
| 5087154/BI252426 and BI252874 |
| 6085289/BU174626 |
| 5741237/BM558378 |
| 4995462/BI088884 |
| 6720160/CA488404 |
| 5764841/BM926410 |
| 6208509/BQ879962 |
| 4581968/BG396587 |
| 5554997/BM477735 |
| 3451668/BE538581 |
| 5803440/BQ069150 |
| 6250974/BQ688755 |
| 6251079/BQ685759 |
| 5535758/BM468306 |
| 6146330/BU165540 |
| 1740729/AI191477 |
| 2729947/AW293885 |
| 6082577BU174653 |
| 2753118/AW275889 |
| 2437568/AI884372 |
| 2507497/AI961218 |
| 5207705/BI771713 |
| 2067750/AI383718 |
| 263894/N28522 and H99843 |
| 1148416/AA627205 |
| 138014/R63144 |
| 70610/T49073 and T49074 |
| 5531252/BM800219 |
| 3629874/BE409186 |
| 5001398/BI093643 |
| 4361336/BF971224 |
| 4451023/BG121013 |
| 3844815/BE730924 |
| 4361451/BF970190 |
| 4154033/BF346117 |
| 4915206/BG818225 |
| 4444686/BG118070 |
| 6086243/BU149745 |
| 4899066/BG829478 |
| 6086128/BU180123 |
| 4366207/BG108477 |
| 3140355/BE280221 |
| 5459527/BM012505 |
| 3627907/BE382922 |
| 5418599/BM016313 |
| 4862852/BG765156 |
| 14877780/BG769917 |
| 3162024/BE262076 |
| 5182393/BI518189 and BI517759 |
| 5417445/BM015407 |
| 16015713/BU175170 |
| 417111/W87557 and W87461 |
| 4580196/BG395022 |
| 16271908/BQ648651 |
| 6298174BQ652789 |
| 6271910/BQ653475 |
| 6271630/BQ647246 |
| 798664/BM928534 |
| 6652195/BU860925 |
| 6299767/BQ651366 |
| 5225259/BI838658 |
| 4895399/BI198873 |
| 740128/A477534 and AA479051 |
| 617256/BU178924 |
| 4562784/BG326197 |
| 3957711/BE902093 |
| 6293406/BQ650920 |
| None (mRNA sequences)/BT007231.1 and NM_014298.2 and AK090801.1 and D78177.1 |
| None/CB156177.1 and BM711970.1 and BM675916.1 and BM675420.1 and BM714918.1 and AA337770.1 and AA305670.1 and AA305611.1 and and BU622082.1 and AV705250.1 and AL528086.2 and AL531128.2 and AL543783.2 and AL548817.2 and AL554386.2 and AL563056.2 and AL563955.2 and AL570131.2 and AL573234.2 and BF956608.1 and AV648116.1 and AV645766.1 and BF742969.1 and AL577191.2 and CD050133.1 and CD049103.1 and BX417895.1 and CB529044.1 and CD250136.1 and AA054830.1 and BX508036.1 and BX454610.1 |

In another set of preferred embodiments of the invention, any sequence, or unique portion thereof, of the HOXB13 sequences of the I.M.A.G.E. Consortium cluster NM_006361, as well as the UniGene Homo sapiens cluster Hs.66731, may be used. Similarly, any sequence encoding all or a part of the protein encoded by any HOXB13 sequence disclosed herein may be used. The consensus sequence of the I.M.A.G.E. Consortium cluster is as follows, with the assigned coding region (ending with a termination codon) underlined and preceded by the 5' untranslated and/or non-coding region and followed by the 3' untranslated and/or non-coding region:
SEQ ID NO:6 (consensus sequence for HOXB13, identified as NM_006361 or NM_006361.2):

cgaatgcagg cgacttgcga gctgggagcg atttaaaacg ctttggattc ccccggcctg
ggtggggaga gcgagctggg tgccccctag attccccgcc cccgcacctc atgagccgac
cctcggctcc atggagcccg gcaattatgc caccttggat ggagccaagg atatcgaagg
cttgctggga gcgggagggg ggcggaatct ggtcgcccac tcccctctga ccagccaccc
agcggcgcct acgctgatgc ctgctgtcaa ctatgccccc ttggatctgc caggctcggc
ggagccgcca aagcaatgcc acccatgccc tggggtgccc caggggacgt ccccagctcc
cgtgccttat ggttactttg gaggcgggta ctactcctgc cgagtgtccc ggagctcgct
gaaaccctgt gcccaggcag ccaccctggc cgcgtacccc gcggagactc ccacggccgg
ggaagagtac cccagtcgcc ccactgagtt tgccttctat ccgggatatc cgggaaccta
ccacgctatg gccagttacc tggacgtgtc tgtggtgcag actctgggtg ctcctggaga
accgcgacat gactccctgt tgcctgtgga cagttaccag tcttgggctc tcgctggtgg
ctggaacagc cagatgtgtt gccagggaga acagaaccca ccaggtccct tttggaaggc
agcatttgca gactccagcg ggcagcaccc tcctgacgcc tgcgcctttc gtcgcggccg
caagaaacgc attccgtaca gcaaggggca gttgcgggag ctggagcggg agtatgcggc
taacaagttc atcaccaagg acaagaggcg caagatctcg gcagccacca gcctctcgga
gcgccagatt accatctggt ttcagaaccg ccgggtcaaa gagaagaagg ttctcgccaa
ggtgaagaac agcgctaccc cttaagagat ctccttgcct gggtgggagg agcgaaagtg
ggggtgtcct ggggagacca gaaacctgcc aagcccaggc tggggccaag gactctgctg
agaggcccct agagacaaca cccttcccag gccactggct gctggactgt tcctcaggag
cggcctgggt acccagtatg tgcagggaga cggaacccca tgtgacaggc ccactccacc
agggttccca aagaacctgg cccagtcata atcattcatc ctcacagtgg caataatcac
gataaccagt

I.M.A.G.E. Consortium Clone ID numbers and the corresponding GenBank accession numbers of sequences identified as belonging to the I.M.A.G.E. Consortium and UniGene clusters, are listed below. Also included are sequences that are not identified as having a Clone ID number but still identified as being those of HOXB13. The sequences include those of the "sense" and complementary strands sequences corresponding to HOXB 13. The sequence of each GenBank accession number is presented in the attached Appendix.

| Clone ID numbers | GenBank accession numbers |
|---|---|
| 4250486 | BF676461, BC007092 |
| 5518335 | BM462617 |
| 4874541 | BG752489 |
| 4806039 | BG778198 |
| 3272315 | CB050884, CB050885 |
| 4356740 | BF96519 |
| 6668163 | BU930208 |
| 1218366 | AA807966 |
| 2437746 | AI884491 |
| 11187697 | AA652388 |
| 3647557 | BF446158 |
| 1207949 | AA657924 |
| 1047774 | AA644637 |
| 3649397 | BF222357 |
| 971664 | AA527613 |
| 996191 | AA533227 |
| 813481 | AA456069, AA455572, BX117624 |
| 6256333 | BQ673782 |
| 2408470 | AI814453 |
| 2114743 | AI417272 |
| 998548 | AA535663 |
| 2116027 | AI400493 |
| 3040843 | AW779219 |
| 1101311 | AA594847 |
| 1752062 | AI150430 |
| 898712 | AA494387 |
| 1218874_{.} | AA662643 |
| 2460189 | AI935940 |
| 986283 | AA532530 |
| 1435135 | AA857572 |
| 1871750 | AI261980 |
| 3915135 | BE888751 |
| 2069668 | AI378797 |
| 667188 | AA234220, AA236353 |
| 1101561 | AA588193 |
| 1170268 | AI821103, AI821851, AA635855 |
| 2095067 | AI420753 |
| 4432770 | BG180547 |
| 783296 | AA468306, AA468232 |
| 3271646 | CB050115, CB050116 |
| 1219276 | AA661819 |
| 30570598 | CF146837 |
| 30570517 | CF146763 |
| 30568921 | CF144902 |
| 3099071 | CF141511 |
| 3096992 | CF139563 |
| 3096870 | CF139372 |
| 3096623 | CF139319 |
| 3096798 | CF139275 |
| 30572408 | CF122893 |
| 2490082 | AI972423 |
| 2251055 | AI918975 |
| 2419308 | AI826991 |
| 2249105 | AI686312 |
| 2243362 | AI655923 |
| 30570697 | CF146922 |
| 3255712 | BF476369 |
| 3478356 | BF057410 |
| 3287977 | BE645544 |
| 3287746 | BE645408 |
| 3621499 | BE388501 |
| 30571128 | CF147366 |
| 30570954 | CF147143 |
| None (mRNA sequences) | BT007410, BC007092, U57052, U81599 |
| None | CB120119, CB125764, AU098628, CB126130, BI023924, BM767063, BM794275, BQ363211, BM932052, AA357646, AW609525, CB126919, AW609336, AW609244, BF855145, AU126914, CB126449, AW582404, BX641644 |

In one preferred embodiment, any sequence, or unique portion thereof, of the following HOXB13 sequence, identified by BC007092 or BC007092.1, may be used in the practice of the invention.
SEQ ID NO:7 (sequence for HOXB 13):

GGATTCCCCGGCCTGGGTGGGAGAGCGAGCTGGGTGCCCCCTAGATTCCCCGCCCCCG
CACCTCATGAGCCGACCCTCGGCTCCATGGAGCCGGCAATTATGCCACCTTGGATGGAG
CCAAGGATATCGAAGGCTTGCTGGAGCGGAGGGGGCGGAATCTGGTCGCCCACTCCC
CTCTGACCAGCCACCCAGCGGCGCCTACGCTGATGCCTGCTGTCAACTATGCCCCCTTGG
ATCTGCCAGGCTCGGCGGAGCCGCCAAAGCAATGCCACCCATGCCCTGGGGTGCCCCAGG
GGACGTCCCCAGCTCCCGTGCCTTATGGTTACTTTGGAGGCGGGTACTACTCCTGCCGAG
TGTCCCGGAGCTCGCTGAAACCCTGTGCCCAGGCAGCCACCCTGGCCGCGTACCCCGCGG

AGACTCCCACGGCCGGGAAGAGTACCCAGCCGCCCACTGAGTTTGCCTTCTATCCGG
GATATCCGGAACCTACCAGCCTATGGCCAGTTACCTGGACGTGTCTGTGGTGCAGACTC
TGGGTGCTCCTGGAGAACCGCGACATGACTCCCTGTTGCCTGTGGACAGTTACCAGTCTT
GGGCTCTCGCTGGTGGCTGGAACAGCCAGATGTGTTGCCAGGGAGAACAGAACCCACCAG
GTCCCTTTTGGAAGGCAGCATTTGCAGACTCCAGCGGGCAGCACCCTCCTGACGCCTGCG
CCTTTCGTCGCGGCCGCAAGAAACGCATTCCGTACAGCAAGGGGCAGTTGCGGGAGCTGG
AGCGGGAGTATGCGGCTAACAAGTTCATCACCAAGGACAAGAGGCGCAAGATCTCGGCAG
CCACCAGCCTCTCGGAGCGCCAGATTACCATCTGGTTTCAGAACCGCCGGGTCAAAGAGA
AGAAGGTTCTCGCCAAGGTGAAGAACAGCGCTACCCCTTAAGAGATCTCCTTGCCTGGGT
GGGAGGAGCGAAAGTGGGGGTGTCCTGGGGAGACCAGGAACCTGCCAAGCCCAGGCTGGG
GCCAAGGACTCTGCTGAGAGGCCCCTAGAGACAACACCCTTCCCAGGCCACTGGCTGCTG
GACTGTTCCTCAGGAGCGGCCTGGGTACCCAGTATGTGCAGGGAGACGGAACCCCATGTG
ACAGCCCACTCCACCAGGGTTCCCAAAGAACCTGGCCCAGTCATAATCATTCATCCTGAC
AGTGGCAATAATCACGATAACCAGTACTAGCTGCCATGATCGTTAGCCTCATATTTTCTA
TCTAGAGCTCTGTAGAGCACTTTAGAAACCGCTTTCATGAATTGAGCTAATTATGAATAA
ATTTGGAAAAAAAAAAAAAAAAAAAAAAAAAAAAA

Sequences identified by SEQ ID NO. are provided using conventional representations of a DNA strand starting from the 5' phosphate linked end to the 3' hydroxyl linked end. The assignment of coding regions is generally by comparison to available consensus sequence(s) and therefore may contain inconsistencies relative to other sequences assigned to the same cluster. These have no effect on the practice of the invention because the invention can be practiced by use of shorter segments (or combinations thereof) of sequences unique to each of the three sets described above and not affected by inconsistencies. As non-limiting examples, a segment of IL 17BR, CHDH, QPRT, or HOXB13 nucleic acid sequence composed of a 3' untranslated region sequence and/or a sequence from the 3' end of the coding region may be used as a probe for the detection of IL17BR, CHDH, QPRT, or HOXB13 expression, respectively, without being affected by the presence of any inconsistency in the coding regions due to differences between sequences. Similarly, the use of an antibody which specifically recognizes a protein, or fragment thereof, encoded by the IL17BR, CHDH, QPRT, or HOXB13 sequences described herein, to detect its expression would not be affected by the presence of any inconsistency in the representation of the coding regions provided above.

As will be appreciated by those skilled in the art, some of the above sequences include 3' poly A (or poly T on the complementary strand) stretches that do not contribute to the uniqueness of the disclosed sequences. The invention may thus be practiced with sequences lacking the 3' poly A (or poly T) stretches. The uniqueness of the disclosed sequences refers to the portions or entireties of the sequences which are found only in IL17BR, CHDH, QPRT, or HOXB13 nucleic acids, including unique sequences found at the 3' untranslated portion of the genes. Preferred unique sequences for the practice of the disclosure are those which contribute to the consensus sequences for each of the three sets such that the unique sequences will be useful in detecting expression in a variety of individuals rather than being specific for a polymorphism present in some individuals. Alternatively, sequences unique to an individual or a subpopulation may be used. The preferred unique sequences are preferably of the lengths of polynucleotides of the invention as discussed herein.

To determine the (increased or decreased) expression levels of the above described sequences in the practice of the present invention, any method known in the art may be utilized. In one preferred embodiment of the invention, expression based on detection of RNA which hybridizes to polynucleotides containing the above described sequences is used. This is readily performed by any RNA detection or amplification+detection method known or recognized as equivalent in the art such as, but not limited to, reverse transcription-PCR (optionally real-time PCR), the methods disclosed in U.S. Patent 6,794,141, the methods disclosed in U.S. Patent 6,291,170, and quantitative PCR. Methods to identify increased RNA stability (resulting in an observation of increased expression) or decreased RNA stability (resulting in an observation of decreased expression) may also be used. These methods include the detection of sequences that increase or decrease the stability of mRNAs containing the IL17BR, CHDH, QPRT, or HOXB13 sequences disclosed herein. These methods also include the detection of increased mRNA degradation.

In particularly preferred embodiments of the disclosure, polynucleotides having sequences present in the 3' untranslated and/or non-coding regions of the above disclosed sequences are used to detect expression or non-expression of IL17BR, CHDH, QPRT, or HOXB13 sequences in breast cells in the practice of the disclosure. Such polynucleotides may optionally contain sequences found in the 3' portions of the coding regions of the above disclosed sequences. Polynucleotides containing a combination of sequences from the coding and 3' non-coding regions preferably have the sequences arranged contiguously, with no intervening heterologous sequence(s).

Alternatively, the disclosure may be practiced with polynucleotides having sequences present in the 5' untranslated and/or non-coding regions of IL17BR, CHDH, QPRT, or HOXB13 sequences in breast cells to detect their levels of expression. Such polynucleotides may optionally contain sequences found in the 5' portions of the coding regions. Polynucleotides containing a combination of sequences from the coding and 5' non-coding regions preferably have the sequences arranged contiguously, with no intervening heterologous sequence(s). The disclosure may also be practiced with sequences present in the coding regions of IL17BR, CHDH, QPRT, or HOXB13.

Preferred polynucleotides contain sequences from 3' or 5' untranslated and/or non-coding regions of at least about 16, at least about 18, at least about 20, at least about 22, at least about 24, at least about 26, at least about 28, at least about 30, at least about 32, at least about 34, at least about 36, at least about 38, at least about 40, at least about 42, at least about 44, or at least about 46 consecutive nucleotides. The term "about" as used in the previous sentence refers to an increase or decrease of 1 from the stated numerical value. Even more preferred are polynucleotides containing sequences of at least or about 50, at least or about 100, at least about or 150, at least or about 200, at least or about 250, at least or about 300, at least or about 350, or at least or about 400 consecutive nucleotides. The term "about" as used in the preceding sentence refers to an increase or decrease of 10% from the stated numerical value.

Sequences from the 3' or 5' end of the above described coding regions as found in polynucleotides of the invention are of the same lengths as those described above, except that they would naturally be limited by the length of the coding region. The 3' end of a coding region may include sequences up to the 3' half of the coding region. Conversely, the 5' end of a coding region may include sequences up the 5' half of the coding region. Of course the above described sequences, or the coding regions and polynucleotides containing portions thereof, may be used in their entireties.

Polynucleotides combining the sequences from a 3' untranslated and/or non-coding region and the associated 3' end of the coding region are preferably at least or about 100, at least about or 150, at least or about 200, at least or about 250, at least or about 300, at least or about 350, or at least or about 400 consecutive nucleotides. Preferably, the polynucleotides used are from the 3' end of the gene, such as within about 350, about 300, about 250, about 200, about 150, about 100, or about 50 nucleotides from the polyadenylation signal or polyadenylation site of a gene or expressed sequence. Polynucleotides containing mutations relative to the sequences of the disclosed genes may also be used so long as the presence of the mutations still allows hybridization to produce a detectable signal.

In another embodiment of the invention, polynucleotides containing deletions of nucleotides from the 5' and/or 3' end of the above disclosed sequences may be used. The deletions are preferably of 1-5, 5-10, 10-15, 15-20, 20-25, 25-30, 30-35, 35-40, 40-45, 45-50, 50-60, 60-70, 70-80, 80-90, 90-100, 100-125, 125-150, 150-175, or 175-200 nucleotides from the 5' and/or 3' end, although the extent of the deletions would naturally be limited by the length of the disclosed sequences and the need to be able to use the polynucleotides for the detection of expression levels.

Other polynucleotides of the invention from the 3' end of the above disclosed sequences include those of primers and optional probes for quantitative PCR. Preferably, the primers and probes are those which amplify a region less than about 350, less than about 300, less than about 250, less than about 200, less than about 150, less than about 100, or less than about 50 nucleotides from the from the polyadenylation signal or polyadenylation site of a gene or expressed sequence.

In yet another embodiment of the invention, polynucleotides containing portions of the above disclosed sequences including the 3' end may be used in the practice of the invention. Such polynucleotides would contain at least or about 50, at least or about 100, at least about or 150, at least or about 200, at least or about 250, at least or about 300, at least or about 350, or at least or about 400 consecutive nucleotides from the 3' end of the disclosed sequences.

The disclosure thus also includes polynucleotides used to detect IL17BR, CHDH, QPRT, or HOXB13 expression in breast cells. The polynucleotides may comprise a shorter polynucleotide consisting of sequences found in the above provided SEQ ID NOS in combination with heterologous sequences not naturally found in combination with IL17BR, CHDH, QPRT, or HOXB13 sequences.

As non-limiting examples, a polynucleotide comprising one of the following sequences may be used in the practice of the invention.
SEQ ID NO:8:
   GCTCTCACTGGCAAATGACAGCTCTGTGCAAGGAGCACTCCCAAGTATAAAAATTATTAC
SEQ ID NO:9:
   TGCCTAATTTCACTCTCAGAGTGAGGCAGGTAACTGGGGCTCCACTGGGTCACTCTGAGA
SEQ ID NO:10:
   GATCGTTAGCCTCATATTTTCTATCTAGAGCTCTGTAGAGCACTTTAGAAACCGCTTTCA

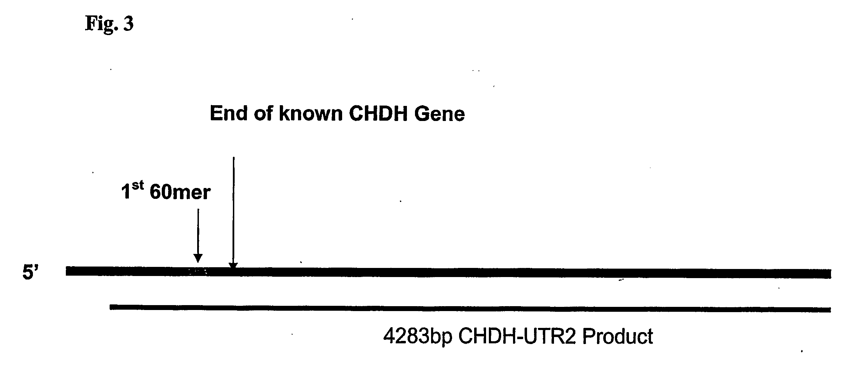

SEQ ID NO:8 is a portion of the AI240933 sequence while SEQ ID NO:9 is a portion of the AJ272267 (CHDH mRNA) sequence. They correspond to the two "60mer" positions indicated in Figure 3. SEQ ID NO:10 is a polynucleotide capable of hybridizing to some HOXB13 sequences as described herein.

Thus, the disclosure may be practiced with a polynucleotide consisting of the sequence of SEQ ID NOS:8, 9 or 10 in combination with one or more heterologous sequences that are not normally found with SEQ ID NOS:8, 9 or 10. Alternatively, the disclosure may also be practiced with a polynucleotide consisting of the sequence of SEQ ID NOS:8, 9 or 10 in combination with one or more naturally occurring sequences that are normally found with SEQ ID NOS:8, 9 or 10.

Polynucleotides with sequences comprising SEQ ID NOS:8 or 9, either naturally occurring or synthetic, may be used to detect nucleic acids which are over expressed in breast cancer cells that are responsive, and those which are not over expressed in breast cancer cells that are non-responsive, to treatment with TAM or another "antiestrogen" agent against breast cancer. Polynucleotides with sequences comprising SEQ ID NO:10, either naturally occurring or synthetic, may be used to detect nucleic acids which are under expressed in breast cancer cells that are responsive, and those which are not under expressed in breast cancer cells that are non-responsive, to treatment with TAM or another "antiestrogen" agent against breast cancer.

Additional sequences that may be used in polynucleotides as described above for SEQ ID NOS:8 8 and 9 is the following, which is complementary to a portion of IL17BR sequences disclosed herein:
SEQ ID NO:11: TCCAATCGTTAGTTAATGCTACATTAGTT

Additional sequences that may be used in polynucleotides as described above for SEQ ID NO:10 are the following, which is complementary to a portion of IL17BR sequences disclosed herein:
SEQ ID NO:12: CAATTCATGAAAGCGGTTTCTAAAG

Additionally, primers of defined sequences may be used to PCR amplify portions of CHDH sequences to determine their level of expression. For example, primers comprising the following sequences may be used to amplify a portion of the AI240933 sequence.
Forward primer (SEQ ID NO:13): TGAAGTGTTTTTGCCTGGATCA
Reverse primer (SEQ ID NO:14): CACCACTTTGTTATGAAGACCTTACAA

In some embodiments of the disclosure, the primers may be used in quantitative RT-PCR methods known in the art, optionally in the presence of a labeled or detectable probe that binds double stranded nucleic acids (such as Sybr Green™) or a specific probe such as a "TaqMan" probe. In one embodiment, such a probe may comprise the sequence AGTAAGAATGTCTTAAGAAGAGG (SEQ ID NO:15) for the detection of AI240933 expression.

Additionally, polynucleotides containing other sequences, particularly unique sequences, present in naturally occurring nucleic acid molecules comprising SEQ ID NOS:8-15 may be used in the practice of the disclosure.

Other polynucleotides for use in the practice of the disclosure include those that have sufficient homology to those described above to detect expression by use of hybridization techniques. Such polynucleotides preferably have about or 95%, about or 96%, about or 97%, about or 98%, or about or 99% identity with IL17BR, CHDH, QPRT, or HOXB13 sequences as described herein. Identity is determined using the BLAST algorithm, as described above. The other polynucleotides for use in the practice of the disclosure may also be described on the basis of the ability to hybridize to polynucleotides of the invention under stringent conditions of about 30% v/v to about 50% formamide and from about 0.01M to about 0.15M salt for hybridization and from about 0.01M to about 0.15M salt for wash conditions at about 55 to about 65°C or higher, or conditions equivalent thereto.

In a further embodiment of the disclosure, a population of single stranded nucleic acid In a further embodiment of the disclosure, a population of single stranded nucleic acid molecules comprising one or both strands of a human IL17BR, CHDH, QPRT, or HOXB13 sequence is provided as a probe such that at least a portion of said population may be hybridized to one or both strands of a nucleic acid molecule quantitatively amplified from RNA of a breast cancer cell. The population may be only the antisense strand of a human IL17BR, CHDH, QPRT, or HOXB13 sequence such that a sense strand of a molecule from, or amplified from, a breast cancer cell may be hybridized to a portion of said population. In the case of IL17BR or CHDH, the population preferably comprises a sufficiently excess amount of said one or both strands of a human IL17BR or CHDH sequence in comparison to the amount of expressed (or amplified) nucleic acid molecules containing a complementary IL17BR or CHDH sequence from a normal breast cell. This condition of excess permits the increased amount of nucleic acid expression in a breast cancer cell to be readily detectable as an increase.

Alternatively, the population of single stranded molecules is equal to or in excess of all of one or both strands of the nucleic acid molecules amplified from a breast cancer cell such that the population is sufficient to hybridize to all of one or both strands. Preferred cells are those of a breast cancer patient that is ER+ or for whom treatment with tamoxifen or one or more other "antiestrogen" agent against breast cancer is contemplated. The single stranded molecules may of course be the denatured form of any IL I 7BR, CHDH, QPRT, or HOXB13 sequence containing double stranded nucleic acid molecule or polynucleotide as described herein.

The population may also be described as being hybridized to an IL17BR or CHDH sequence containing nucleic acid molecules at a level of at least twice as much as that by nucleic acid molecules of a normal breast cell. As in the embodiments described above, the nucleic acid molecules may be those quantitatively amplified from a breast cancer cell such that they reflect the amount of expression in said cell.

The population is preferably immobilized on a solid support, optionally in the form of a location on a microarray. A portion of the population is preferably hybridized to nucleic acid molecules quantitatively amplified from a non-normal or abnormal breast cell by RNA amplification. The amplified RNA may be that derived from a breast cancer cell, as long as the amplification used was quantitative with respect to IL17BR, CHDH, QPRT, or HOXB13 containing sequences.

In another embodiment of the disclosure, expression based on detection of DNA status may be used. Detection of the QPRT or HOXB13 DNA as methylated, deleted or otherwise inactivated, may be used as an indication of decreased expression as found in non-normal breast cells. This may be readily performed by PCR based methods known in the art. The status of the promoter regions of QPRT or HOXB13 may also be assayed as an indication of decreased expression of QPRT or HOXB13 sequences. A non-limiting example is the methylation status of sequences found in the promoter region.

Conversely, detection of the DNA of a sequence as amplified may be used for as an indication of increased expression as found in non-normal breast cells. This may be readily performed by PCR based, fluorescent *in situ* hybridization (FISH) and chromosome *in situ* hybridization (CISH) methods known in the art.

A preferred embodiment using a nucleic acid based assay to determine expression is by immobilization of one or more of the sequences identified herein on a solid support, including, but not limited to, a solid substrate as an array or to beads or bead based technology as known in the art. Alternatively, solution based expression assays known in the art may also be used. The immobilized sequence(s) may be in the form of polynucleotides as described herein such that the polynucleotide would be capable of hybridizing to a DNA or RNA corresponding to the sequence(s).

The immobilized polynucleotide(s) may be used to determine the state of nucleic acid samples prepared from sample breast cancer cell(s), optionally as part of a method to detect ER status in said cell(s). Without limiting the disclosure, such a cell may be from a patient suspected of being afflicted with, or at risk of developing, breast cancer. The immobilized polynucleotide(s) need only be sufficient to specifically hybridize to the corresponding nucleic acid molecules derived from the sample (and to the exclusion of detectable or significant hybridization to other nucleic acid molecules).

In yet another embodiment of the disclosure, a ratio of the expression levels of two of the disclosed genes may be used to predict response to treatment with TAM or another SERM. Preferably, the ratio is that of two genes with opposing patterns of expression, such as an underexpressed gene to an overexpressed gene, in correlation to the same phenotype. Non-limiting examples include the ratio of HOXB13 over IL17BR or the ratio of QPRT over CHDH. This aspect of the invention is based in part on the observation that such a ratio has a stronger correlation with TAM treatment outcome than the expression level of either gene alone. For example, the ratio of HOXB13 over IL17BR has an observed classification accuracy of 77%.

As a non-limiting example, the Ct values from Q-PCR based detection of gene expression levels may be used to derive a ratio to predict the response to treatment with one or more "antiestrogen" agent against breast cancer.

### Additional Embodiments of the Invention

In embodiments where only one or a few genes are to be analyzed, the nucleic acid derived from the sample breast cancer cell(s) may be preferentially amplified by use of appropriate primers such that only the genes to be analyzed are amplified to reduce contaminating background signals from other genes expressed in the breast cell. Alternatively, and where multiple genes are to be analyzed or where very few cells (or one cell) is used, the nucleic acid from the sample may be globally amplified before hybridization to the immobilized polynucleotides. Of course RNA, or the cDNA counterpart thereof may be directly labeled and used, without amplification, by methods known in the art.

Sequence expression based on detection of a presence, increase, or decrease in protein levels or activity may also be used. Detection may be performed by any immunohistochemistry (IHC) based, bodily fluid based (where a IL17BR, CHDH, QPRT, or HOXB13 polypeptide, or fragment thereof, is found in a bodily fluid, such as but not limited to blood), antibody (including autoantibodies against the protein where present) based, ex foliate cell (from the cancer) based, mass spectroscopy based, and image (including used of labeled ligand where available) based method known in the art and recognized as appropriate for the detection of the protein. Antibody and image based methods are additionally useful for the localization of tumors after determination of cancer by use of cells obtained by a non-invasive procedure (such as ductal lavage or fine needle aspiration), where the source of the cancerous cells is not known. A labeled antibody or ligand may be used to localize the carcinoma(s) within a patient.

Antibodies for use in such methods of detection include polyclonal antibodies, optionally isolated from naturally occurring sources where available, and monoclonal antibodies, including those prepared by use of IL17BR, CHDH, QPRT, or HOXB13 polypeptides (or fragments thereof) as antigens. Such antibodies, as well as fragments thereof (including but not limited to F_{ab} fragments) function to detect or diagnose non-normal or cancerous breast cells by virtue of their ability to specifically bind IL17BR, CHDH, QPRT, or HOXB13 polypeptides to the exclusion of other polypeptides to produce a detectable signal. Recombinant, synthetic, and hybrid antibodies with the same ability may also be used in the practice of the disclosure. Antibodies may be readily generated by immunization with a IL17BR, CHDH, QPRT, or HOXB13 polypeptide (or fragment thereof), and polyclonal sera may also be used in the practice of the disclosure.

Antibody based detection methods are well known in the art and include sandwich and ELISA assays as well as Western blot and flow cytometry based assays as non-limiting examples. Samples for analysis in such methods include any that contain IL17BR, CHDH, QPRT, or HOXB13 polypeptides or fragments thereof. Non-limiting examples include those containing breast cells and cell contents as well as bodily fluids (including blood, serum, saliva, lymphatic fluid, as well as mucosal and other cellular secretions as non-limiting examples) that contain the polypeptides.

The above assay embodiments may be used in a number of different ways to identify or detect the response to treatment with TAM or another "antiestrogen" agent against breast cancer based on gene expression in a breast cancer cell sample from a patient. In some cases, this would reflect a secondary screen for the patient, who may have already undergone mammography or physical exam as a primary screen. If positive from the primary screen, the subsequent needle biopsy, ductal lavage, fine needle aspiration, or other analogous minimally invasive method may provide the sample for use in the assay embodiments before, simultaneous with, or after assaying for ER status. The present invention is particularly useful in combination with non-invasive protocols, such as ductal lavage or fine needle aspiration, to prepare a breast cell sample.

The present invention provides a more objective set of criteria, in the form of gene expression profiles of a discrete set of genes, to discriminate (or delineate) between breast cancer outcomes. In particularly preferred embodiments of the invention, the assays are used to discriminate between good and poor outcomes after treatment with tamoxifen or another "antiestrogen" agent against breast cancer. Comparisons that discriminate between outcomes after about 10, about 20, about 30, about 40, about 50, about 60, about 70, about 80, about 90, about 100, or about 150 months may be performed.

While good and poor survival outcomes may be defined relatively in comparison to each other, a "good" outcome may be viewed as a better than 50% survival rate after about 60 months post surgical intervention to remove breast cancer tumor(s). A "good" outcome may also be a better than about 60%, about 70%, about 80% or about 90% survival rate after about 60 months post surgical intervention. A "poor" outcome may be viewed as a 50% or less survival rate after about 60 months post surgical intervention to remove breast cancer tumor(s). A "poor" outcome may also be about a 70% or less survival rate after about 40 months, or about a 80% or less survival rate after about 20 months, post surgical intervention.

In another embodiment of the disclosure based on the expression of a few genes, the isolation and analysis of a breast cancer cell sample may be performed as follows:
(1) Ductal lavage or other non-invasive procedure is performed on a patient to obtain a sample.
(2) Sample is prepared and coated onto a microscope slide. Note that ductal lavage results in clusters of cells that are cytologically examined as stated above.
(3) Pathologist or image analysis software scans the sample for the presence of atypical cells.
(4) If atypical cells are observed, those cells are harvested (e.g. by microdissection such as LCM).
(5) RNA is extracted from the harvested cells.
(6) RNA is assayed, directly or after conversion to cDNA or amplification therefrom, for the expression of IL17BR, CHDH, QPRT, and/or HOXB13 sequences.

With use of the present invention, skilled physicians may prescribe or withhold treatment with TAM or another "antiestrogen" agent against breast cancer based on prognosis determined via practice of the instant invention.

The above discussion is also applicable where a palpable lesion is detected followed by fine needle aspiration or needle biopsy of cells from the breast. The cells are plated and reviewed by a pathologist or automated imaging system which selects cells for analysis as described above.

The present invention may also be used, however, with solid tissue biopsies, including those stored as an FFPE specimen. For example, a solid biopsy may be collected and prepared for visualization followed by determination of expression of one or more genes identified herein to determine the breast cancer outcome. As another non-limiting example, a solid biopsy may be collected and prepared for visualization followed by determination of IL17BR, CHDH, QPRT, and/or HOXB13 expression. One preferred means is by use of *in situ* hybridization with polynucleotide or protein identifying probe(s) for assaying expression of said gene(s).

In an alternative method, the solid tissue biopsy may be used to extract molecules followed by analysis for expression of one or more gene(s). This provides the possibility of leaving out the need for visualization and collection of only cancer cells or cells suspected of being cancerous. This method may of course be modified such that only cells that have been positively selected are collected and used to extract molecules for analysis. This would require visualization and selection as a prerequisite to gene expression analysis. In the case of an FFPE sample, cells may be obtained followed by RNA extraction, amplification and detection as described herein.

In an alternative to the above, the sequence(s) identified herein may be used as part of a simple PCR or array based assay simply to determine the response to treatment with TAM or another "antiestrogen" agent against breast cancer by use of a sample from a non-invasive or minimally invasive sampling procedure. The detection of sequence expression from samples may be by use of a single microarray able to assay expression of the disclosed sequences as well as other sequences, including sequences known not to vary in expression levels between normal and non-normal breast cells, for convenience and improved accuracy.

Other uses of the present invention include providing the ability to identify breast cancer cell samples as having different responses to treatment with TAM or another "antiestrogen" agent against breast cancer for further research or study. This provides an advance based on objective genetic/molecular criteria.

In yet another embodiment of the disclosure based on the expression of multiple genes in an expression pattern or profile, the isolation and analysis of a breast cancer cell sample may be performed as follows:
(1) Ductal lavage or other non-invasive procedure is performed on a patient to obtain a sample.
(2) Sample is prepared and coated onto a microscope slide. Note that ductal lavage results in clusters of cells that are cytologically examined as stated above.
(3) Pathologist or image analysis software scans the sample for the presence of non-normal and/or atypical breast cancer cells.
(4) If such cells are observed, those cells are harvested (e.g. by microdissection such as LCM).
(5) RNA is extracted from the harvested cells.
(6) RNA is purified, amplified, and labeled.
(7) Labeled nucleic acid is contacted with a microarray containing polynucleotides complementary to all or part of one or more of the genes identified herein as correlated to discriminations between breast cancer outcomes under suitable hybridization conditions, then processed and scanned to obtain a pattern of intensities of each spot (relative to a control for general gene expression in cells) which determine the level of expression of the gene(s) in the cells.
(8) The pattern of intensities is analyzed by comparison to the expression patterns of the genes in known samples of breast cancer cells correlated with outcomes (relative to the same control).

A specific example of the above method would be performing ductal lavage following a primary screen, observing and collecting non-normal and/or atypical cells for analysis. The comparison to known expression patterns, such as that made possible by a model generated by an algorithm (such as, but not limited to nearest neighbor type analysis, SVM, or neural networks) with reference gene expression data for the different breast cancer survival outcomes, identifies the cells as being correlated with subjects with good or poor outcomes. Another example would be taking a breast tumor removed from a subject after surgical intervention, optionally converting all or part of it to an FFPE sample prior to subsequent isolation and preparation of breast cancer cells from the tumor for determination/identification of atypical, non-normal, or cancer cells, and isolation of said cells followed by steps 5 through 8 above.

Alternatively, the sample may permit the collection of both normal as well as cancer cells for analysis. The gene expression patterns for each of these two samples will be compared to each other as well as the model and the normal versus individual comparisons therein based upon the reference data set. This approach can be significantly more powerful that the cancer cells only approach because it utilizes significantly more information from the normal cells and the differences between normal and cancer cells (in both the sample and reference data sets) to determine the breast cancer outcome of the patient based on gene expression in the cancer cells from the sample.

The genes identified herein also may be used to generate a model capable of predicting the breast cancer survival and recurrence outcomes of an ER+ breast cell sample based on the expression of the identified genes in the sample. Such a model may be generated by any of the algorithms described herein or otherwise known in the art as well as those recognized as equivalent in the art using gene(s) (and subsets thereof) disclosed herein for the identification of breast cancer outcomes. The model provides a means for comparing expression profiles of gene(s) of the subset from the sample against the profiles of reference data used to build the model. The model can compare the sample profile against each of the reference profiles or against a model defining delineations made based upon the reference profiles. Additionally, relative values from the sample profile may be used in comparison with the model or reference profiles.

In a preferred embodiment of the invention, breast cell samples identified as normal and cancerous from the same subject may be analyzed, optionally by use of a single microarray, for their expression profiles of the genes used to generate the model. This provides an advantageous means of identifying survival and recurrence outcomes based on relative differences from the expression profile of the normal sample. These differences can then be used in comparison to differences between normal and individual cancerous reference data which was also used to generate the model.

### Articles of Manufacture

The materials and methods of the present invention are ideally suited for preparation of kits produced in accordance with well known procedures. We describe kits comprising agents (like the polynucleotides and/or antibodies described herein as non-limiting examples) for the detection of expression of the disclosed sequences. Such kits, optionally comprising the agent with an identifying description or label or instructions relating to their use in the methods of the present invention, are provided. Such a kit may comprise containers, each with one or more of the various reagents (typically in concentrated form) utilized in the methods, including, for example, prefabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more primer complexes of the present invention (e.g., appropriate length poly(T) or random primers linked to a promoter reactive with the RNA polymerase). A set of instructions will also typically be included.

The methods provided by the present invention may also be automated in whole or in part. All aspects of the present invention may also be practiced such that they consist essentially of a subset of the disclosed genes to the exclusion of material irrelevant to the identification of breast cancer survival outcomes via a cell containing sample.

Having now generally described the invention, the same will be more readily understood through reference to the following examples which are provided by way of illustration, and are not intended to be limiting of the present invention, unless specified.

### Examples

### Example 1

### General methods

### Patient and tumor selection criteria and study design

Patient inclusion criteria for this study were: Women diagnosed at the Massachusetts General Hospital (MGH) between 1987 and 2000 with ER positive breast cancer, treatment with standard breast surgery (modified radical mastectomy or lumpectomy) and radiation followed by five years of systemic adjuvant tamoxifen; no patient received chemotherapy prior to recurrence. Clinical and follow-up data were derived from the MGH tumor registry. There were no missing registry data and all available medical records were reviewed as a second tier of data confirmation.

All tumor specimens collected at the time of initial diagnosis were obtained from frozen and formalin fixed paraffin-embedded (FFPE) tissue repositories at the Massachusetts General Hospital. Tumor samples with greater than 20% tumor cells were selected with a median of greater than 75% for all samples. Each sample was evaluated for the following features: tumor type (ductal vs. lobular), tumor size, and Nottingham combined histological grade. Estrogen and progesterone receptor expression were determined by biochemical hormone binding analysis and/or by immunohistochemical staining as described (Long, A.A. et al. "High-specificity in-situ hybridization. Methods and application." Diagn Mol Pathol 1, 45-57 (1992)); receptor positivity was defined as greater than 3 fmol/mg tumor tissue (Long et al.) and greater than 1% nuclear staining for the biochemical and immunohistochemical assays, respectively.

Study design is as follows: A training set of 60 frozen breast cancer specimens was selected to identify gene expression signatures predictive of outcome or response, in the setting of adjuvant tamoxifen therapy. Tumors from responders were matched to the non-responders with respect to TNM staging and tumor grade. Differential gene expression identified in the training set was validated in an independent group of 20 invasive breast tumors with formalin fixed paraffin-embedded (FFPE) tissue samples.

### LCM, RNA isolation and amplification

With each frozen tumor sample within the 60-case cohort, RNA was isolated from both a whole tissue section of 8µm in thickness and a highly enriched population of 4,000-5,000 malignant epithelial cells acquired by laser capture microdissection using a PixCell IIe LCM system (Arcturus, Mountain View, CA). From each tumor sample within the 20-case test set, RNA was isolated from four 8µm-thick FFPE tissue sections. Isolated RNA was subjected to one round of T7 polymerase *in vitro* transcription using the RiboAmp^{™} kit (frozen samples) or another system for FFPE samples according to manufacturer's instructions (Arcturus Bioscience, Inc., Mountain View, CA for RiboAmp^{™}). Labeled cRNA was generated by a second round of T7-based RNA in vitro transcription in the presence of 5-[3-Aminoallyl]uridine 5'-triphosphate (Sigma-Aldrich, St. Louis, MO). Universal Human Reference RNA (Stratagene, San Diego, CA) was amplified in the same manner. The purified aRNA was later conjugated to Cy5 (experimental samples) or Cy3 (reference sample) dye (Amersham Biosciences).

### Microarray analysis

A custom designed 22,000-gene oligonucleotide (60mer) microarray was fabricated using ink-jet in-situ synthesis technology (Agilent Technologies, Palo Alto, CA). Cy5-labeled sample RNA and Cy3-labeled reference RNA were co-hybridized at 65°C, 1X hybridization buffer (Agilent Technologies). Slides were washed at 37°C with 0.1X SSC/0.005% Triton X-102. Image analysis was performed using Agilent's image analysis software. Raw Cy5/Cy3 ratios were normalized using intensity-dependent non-linear regression.

A data matrix consisting of normalized Cy5/Cy3 ratios from all samples were median centered for each gene. The variance of each gene over all samples was calculated and the top 25% high variance genes (5,475) selected for further analysis. Identification and permutation testing for significance of differential gene expression were performed using BRB ArrayTools, developed by Dr. Richard Simon and Amy Peng (see http site at linus.nci.nih.gov/BRB-ArrayTools.html). Hierarchical cluster analysis was performed with GeneMaths software (Applied-Maths, Belgium) using cosine correlation and complete linkage. All other statistical procedures (two-sample t-test, receiver operating characteristic analysis, multivariate logistic regression and survival analysis) were performed in the open source R statistical environment (see http site at www.r-project.org). Statistical test of significance of ROC curves was by the method of DeLong ("Comparing the areas under two or more correlated receiver operating characteristic curves: a nonparametric approach." Biometrics 44, 837-45 (1988)). Disease free survival was calculated from the date of diagnosis. Events were scored as the first distant metastasis, and patients remaining disease-free at the last follow-up were censored. Survival curves were calculated by the Kaplan-Meier estimates and compared by log-rank tests.

### Real-Time Quantitative PCR analysis

Real-time PCR was performed on 59 of the 60-case training samples (one case was excluded due to insufficient materials) and the 20-case validation samples. Briefly, 2 µg of amplified RNA was converted into double stranded cDNA. For each case 12ng of cDNA in triplicates was used for real-time PCR with an ABI 7900HT (Applied Biosystems) as described (Gelmini, S. et al. "Quantitative polymerase chain reaction-based homogeneous assay with fluorogenic probes to measure c-erbB-2 oncogene amplification." Clin Chem 43, 752-8 (1997)). The sequences of the PCR primer pairs and fluorogenic MGB probe (5' to 3'), respectively, that were used for each gene are as follows:
HoxB 13
   TTCATCCTGACAGTGGCAATAATC,
   CTAGATAGAAAATATGAGGCTAACGATCAT,
   VIC- CGATAACCAGTACTAGCTG;
IL17BR
   GCATTAACTAACGATTGGAAACTACATT,
   GGAAGATGCTTTATTGTTGCATTATC,
   VIC-ACAACTTCAAAGCTGTTTTA.

Relative expression levels of HOXB13 in normal, DCIS and IDC samples were calculated as follows. First, all CT values are adjusted by subtracting the highest CT (40) among all samples, then relative expression = 1 / 2^CT.

### In Situ Hybridization

Dig-labeled RNA probes were prepared using DIG RNA labeling kit (SP6/T7) from Roche Applied Science, following the protocol provided with the kit. *In situ* hybridization was performed on frozen tissue sections as described (Long et al.).

**Table 1. Patients and tumor characteristics of training set.**

| **Sample ID** | **Tumor type** | **Size** | **Grade** | **Nodes** | **ER** | **PR** | **Age** | ***DFS*** | **Status** |
|---|---|---|---|---|---|---|---|---|---|
| 1389 | D | 1.7 | 2 | 0/1 | Pos | Pos | 80 | 94 | 0 |
| 648 | D | 1.1 | 2 | 0/15 | Pos | ND | 62 | 160 | 0 |
| 289 | D | 3 | 2 | 0/15 | Pos | ND | 75 | 63 | 1 |
| 749 | D | 1.8 | 2 | 2/9 | Pos | Pos | 61 | 137 | 0 |
| 420 | D/L | 2 | 3 | ND | Pos | Pos | 72 | 58 | 1 |
| 633 | D | 2.7 | 3 | 0/11 | Pos | ND | 61 | 20 | 1 |
| 662 | D | 1 | 3 | 6/11 | Pos | Pos | 79 | 27 | 1 |
| 849 | D | 2 | 1 | 0/26 | Pos | Neg | 75 | 23 | 1 |
| 356 | D | 1 | 2 | 2/20 | Pos | ND | 58 | 24 | 1 |
| 1304 | D | 2 | 3 | 0/14 | Pos | Pos | 57 | 20 | 1 |
| 1419 | D | 2.5 | 2 | 1/8 | Pos | Pos | 59 | 86 | 0 |
| 1093 | D | 1 | 3 | 1/14 | Pos | Pos | 66 | 85 | 0 |
| 1047 | D/L | 2.6 | 2 | 0/18 | Pos | Neg | 70 | 128 | 0 |
| 1037 | D/L | 1.5 | 2 | 0/4 | Pos | Pos | 85 | 83 | 0 |
| 319 | D | 4 | 2 | 1/13 | Pos | ND | 67 | 44 | 1 |
| 25 | D | 3.5 | 2 | 0/9 | Neg | Pos | 62 | 75 | 1 |
| 180 | D | 1.6 | 2 | 2/19 | Pos | Pos | 69 | 169 | 0 |
| 687 | D | 3.5 | 3 | 3/16 | Pos | ND | 73 | 142 | 0 |
| 856 | D | 1.6 | 2 | 0/16 | Pos | Pos | 73 | 88 | 0 |
| 1045 | D | 2.5 | 3 | 1/12 | Pos | Neg | 73 | 121 | 0 |
| 1205 | D | 2.7 | 2 | 1/19 | Pos | Pos | 71 | 88 | 0 |
| 1437 | D | 1.7 | 2 | 2/22 | Pos | Pos | 67 | 89 | 0 |
| 1507 | D | 3.7 | 3 | 0/40 | Pos | Pos | 70 | 70 | 0 |
| 469 | D | 1 | 1 | 0/19 | Pos | ND | 66 | 161 | 0 |
| 829 | D | 1.2 | 2 | 0/9 | Pos | ND | 69 | 136 | 0 |
| 868 | D | 3 | 3 | 0/13 | Pos | Pos | 65 | 130 | 0 |
| 1206 | D | 4.1 | 3 | 0/15 | Pos | Neg | 84 | 56 | 1 |
| 843 | D | 3.4 | 2 | 11/20 | Pos | Neg | 76 | 122 | 1 |
| 342 | D | 3 | 2 | 9/21 | Pos | ND | 62 | 102 | 1 |
| 1218 | D | 4.5 | 1 | 3/16 | Pos | Pos | 62 | 10 | 1 |
| 547 | D/L | 1.5 | 2 | ND | Pos | ND | 74 | 129 | 1 |
| 1125 | D | 2.6 | 2 | 0/18 | Pos | Pos | 54 | 123 | 0 |
| 1368 | D | 2.6 | 2 | ND | Pos | Pos | 82 | 63 | 0 |
| 605 | D | 2.2 | 2 | 6/18 | Pos | ND | 70 | 110 | 0 |
| 59 | L | 3 | 2 | 33/38 | Pos | ND | 70 | 21 | 1 |
| 68 | D | 3 | 2 | 0/17 | Pos | ND | 53 | 38 | 1 |
| 317 | D | 1.2 | 3 | 1/10 | Pos | Pos | 71 | 5 | 1 |
| 374 | D | 1 | 3 | 0/15 | Pos | Neg | 57 | 47 | 1 |
| 823 | D | 2 | 2 | 0/6 | Pos | Pos | 51 | 69 | 1 |
| 280 | D | 2.2 | 3 | 0/12 | Pos | ND | 66 | 44 | 1 |
| 651 | D | 4.7 | 3 | 10/13 | Pos | ND | 48 | 137 | 1 |
| 763 | D | 1.8 | 2 | 0/14 | Pos | Pos | 63 | 118 | 0 |
| 1085 | D | 4.7 | 2 | 0/8 | Pos | Pos | 48 | 101 | 1 |
| 1363 | D | 2.1 | 2 | 0/15 | Pos | Pos | 56 | 114 | 0 |
| 295 | D | 3.5 | 2 | 3/21 | Pos | Pos | 52 | 118 | 1 |
| 871 | D | 4 | 3 | 0/16 | Pos | Neg | 61 | 6 | 1 |
| 1343 | D | 2.5 | 3 | ND | Pos | Pos | 79 | 21 | 1 |
| 140 | L | >2.0 | 2 | 18/28 | Pos | ND | 63 | 43 | 1 |
| 260 | D/L | 0.9 | 2 | 1/13 | Pos | ND | 73 | 42 | 1 |
| 297 | D | 0.8 | 2 | 1/16 | Pos | Pos | 66 | 169 | 0 |
| 1260 | D | 3.5 | 2 | 0/14 | Pos | Pos | 58 | 79 | 0 |
| 1405 | D | 1 | 3 | ND | Pos | Pos | 81 | 95 | 0 |
| 518 | L | 5.5 | 2 | 3/20 | Pos | ND | 68 | 156 | 0 |
| 607 | D | 1.2 | 2 | 5/14 | Pos | Pos | 76 | 114 | 0 |
| 638 | D | 2 | 2 | 1/24 | Pos | Pos | 67 | 148 | 0 |
| 655 | D | 2 | 3 | ND | Pos | Pos | 73 | 143 | 0 |
| 772 | D | 2.5 | 2 | 0/18 | Pos | Pos | 68 | 69 | 1 |
| 878 | D/L | 1.6 | 2 | 0/9 | Pos | Neg | 76 | 138 | 0 |
| 1279 | D | 2 | 2 | 0/12 | Pos | Pos | 68 | 102 | 0 |
| 1370 | D | 2 | 2 | ND | Pos | Pos | 73 | 61 | 0 |

| | | | | | | | | | |
|---|---|---|---|---|---|---|---|---|---|
| Abbreviations: D, ductal; L, lobular; D/L. ductal and lobular features; pos, positive; neg, negative; ND, not determined; ER, estrogen receptor; PR, progesterone receptor; DFS, disease-free survival (number of months); status=1, recurred; status=0, disease-free. | | | | | | | | | |

### Example 2

### Identification of differentially expressed genes

Gene expression profiling was performed using a 22,000-gene oligonucleotide microarray as described above. In the initial analysis, isolated RNA from frozen tumor-tissue sections taken from the archived primary biopsies were used. The resulting expression dataset was first filtered based on overall variance of each gene with the top 5,475 high-variance genes (75th percentile) selected for further analysis. Using this reduced dataset, t-test was performed on each gene comparing the tamoxifen responders and non-responders, leading to identification of 19 differentially expressed genes at the P value cutoff of 0.001 (Table 2). The probability of selecting this many or more differentially expressed genes by chance was estimated to be 0.04 by randomly permuting the patient class with respect to treatment outcome and repeating the t-test procedure 2,000 times. This analysis thus demonstrated the existence of statistically significant differences in gene expression between the primary breast cancers of tamoxifen responders and non-responders.

**Table 2. 19-gene signature identified by t-test in the Sections dataset**

| | **Parametric p-value** | **Mean in responde rs** | **Mean in non-respond ers** | **Fold differen ce of means** | **GB acc** | **Description** |
|---|---|---|---|---|---|---|
| **1** | 1.96E-05 | 0.759 | 1.317 | 0.576 | AW006861 | SCYA4 \| small inducible cytokine A4 |
| **2** | 2.43E-05 | 1.31 | 0.704 | 1.861 | AI240933 | ESTs |
| **3** | 8.08E-05 | 0.768 | 1.424 | 0.539 | X59770 | IL1R2 \| interleukin 1 receptor, type II |
| **4** | 9.57E-05 | 0.883 | 1.425 | 0.62 | AB000520 | APS adaptor protein with pleckstrin homology and src homology 2 domains |
| **5** | 9.91 E-05 | 1.704 | 0.659 | 2.586 | AF208111 | IL17BR \| interleukin 17B receptor |
| **6** | 0.0001833 | 0.831 | 1.33 | 0.625 | AI820604 | ESTs |
| **7** | 0.0001935 | 0.853 | 1.459 | 0.585 | AI087057 | DOK2 \| docking protein 2, 56kD |
| **8** | 0.0001959 | 1.29 | 0.641 | 2.012 | AJ272267 | CHDH \| choline dehydrogenase |
| **9** | 0.0002218 | 1.801 | 0.943 | 1.91 | N30081 | ESTs, Weakly similar to 138022 hypothetical protein [H.sapiens] |
| **10** | 0.0004234 | 1.055 | 2.443 | 0.432 | AI700363 | ESTs |
| **11** | 0.0004357 | 0.451 | 1.57 | 0.287 | AL117406 | ABCC11 \| ATP-binding cassette, sub-family C (CFTR/MRP), member 11 |
| **12** | 0.0004372 | 1.12 | 3.702 | 0.303 | BC007092 | HOXB13 homeo box B13 |
| **13** | 0.0005436 | 0.754 | 1.613 | 0.467 | M92432 | GUCY2D guanylate cyclase 2D, membrane (retina-specific) |
| **14** | 0.0005859 | 1.315 | 0.578 | 2.275 | AL050227 | Homo sapiens mRNA; cDNA DKFZp586M0723 (from clone DKFZp586M0723) |
| **15** | 0.000635 | 1.382 | 0.576 | 2.399 | AW613732 | Homo sapiens cDNA FLJ31137 fis, clone IMR322001049 |
| **16** | 0.0008714 | 0.794 | 1.252 | 0.634 | BC007783 | SCYA3 small inducible cytokine A3 |
| **17** | 0.0008912 | 2.572 | 1.033 | 2.49 | X81896 | C11 orf25 \| chromosome 11 open reading frame 25 |
| **18** | 0.0009108 | 0.939 | 1.913 | 0.491 | BC004960 | MGC10955 \| hypothetical protein MGC10955 |
| **19** | 0.0009924 | 1.145 | 0.719 | 1.592 | AK027250 | Homo sapiens cDNA: FLJ23597 fis, clone LNG15281 |

To refine our analysis to the tumor cells and circumvent potential variability attributable to stromal cell contamination, the same cohort was reanalyzed following laser-capture microdissection (LCM) of tumor cells within each tissue section. Using variance based gene filtering and t-test screening identical to that utilized for the whole tissue section dataset, 9 differentially expressed gene sequences were identified with P < 0.001 (Table 3).

**Table 3. 9-gene signature identified by t-test in the LCM dataset**

| | **Parametric p-value** | **Mean in responders** | **Mean in non-responders** | **Fold difference of means** | **GB acc** | **Description** |
|---|---|---|---|---|---|---|
| **1** | 2.67E-05 | 1.101 | 4.891 | 0.225 | BC007092 | HOXB13 homeo box B13 |
| **2** | 0.0003393 | 1.045 | 2.607 | 0.401 | AI700363 | ESTs |
| **3** | 0.0003736 | 0.64 | 1.414 | 0.453 | NM_014298 | QPRT \| quinolinate phosphoribosyltransferase (nicotinate-nucleotide pyrophosphorylase (carboxylating)) |
| **4** | 0.0003777 | 1.642 | 0.694 | 2.366 | AF208111 | IL17BR \| interleukin 17B receptor |
| **5** | 0.0003895 | 0.631 | 1.651 | 0.382 | AF033199 | ZNF204 \| zinc finger protein 204 |
| **6** | 0.0004524 | 1.97 | 0.576 | 3.42 | AI688494 | FLJ13189 hypothetical protein FLJ13189 |
| **7** | 0.0005329 | 1.178 | 0.694 | 1.697 | AI240933 | ESTs |
| **8** | 0.0007403 | 0.99 | 1.671 | 0.592 | AL157459 | Homo sapiens mRNA; cDNA DKFZp434B0425 (from clone DKFZp434B0425) |
| **9** | 0.0007739 | 0.723 | 1.228 | 0.589 | BC002480 | FLJ13352 \| hypothetical protein FLJ13352 |

Only 3 genes were identified as differentially expressed in both the LCM and whole tissue section analyses: the homeobox gene HOXB13 (identified twice as AI700363 and BC007092), the interleukin 17B receptor IL17BR (AF208111), and the voltage-gated calcium channel CACNA1D (AI240933). HOXB 13 was differentially overexpressed in tamoxifen nonresponsive cases, whereas IL17BR and CACNA1D were overexpressed in tamoxifen responsive cases. Interestingly, the QPRT sequence had similarities to the HOXB13 sequence in relation to expression levels in responders and non-responders. Based on their identification as tumor-derived markers significantly associated with clinical outcome in two independent analyses, the utility of each of these genes was evaluated by itself and in combination with the others.

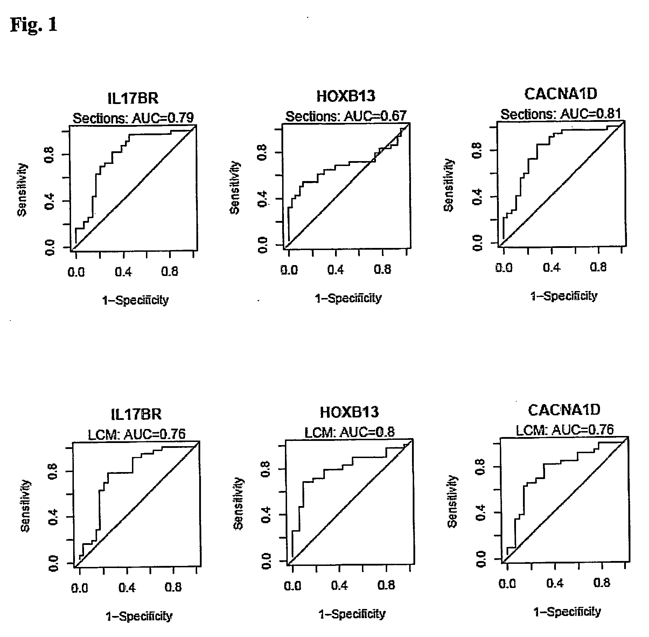

To define the sensitivity and specificity of HOXB13, IL17BR and CACNA1D expression as markers of clinical outcome, Receiver Operating Characteristic (ROC) analysis (Pepe, M.S. "An interpretation for the ROC curve and inference using GLM procedures." Biometrics 56, 352-9 (2000)) was used. For data derived from whole tissue sections, the Area Under the Curve (AUC) values for IL17BR, HOXB 13 and CACNAID were 0.79, 0.67 and 0.81 for IL17BR, HOXB 13 and CACNA1D, respectively (see Table 4 and Fig. 1, upper portion). ROC analysis of the data generated from the microdissected tumor cells yielded AUC values of 0.76, 0.8, and 0.76 for these genes (see Table 4 and Fig. 1, lower portion).

**Table 4. ROC analysis of using IL17BR, CACNA1D and HOXB13 expression to predict tamoxifen response**

| | **Tissue Sections** | | | **LCM** |
|---|---|---|---|---|
| | **AUC** | **P *value*** | **AUC** | *P* **value** |
| **IL17BR** | 0.79 | 1.58E-06 | 0.76 | 2.73E-05 |
| **CACNA1D** | 0.81 | 3.02E-08 | 0.76 | 1.59E-05 |
| **HOXB13** | 0.67 | 0.012 | 0.79 | 9.94E-07 |
| **ESR1** | 0.55 | 0.277 | 0.63 | 0.038 |
| **PGR** | 0.63 | 0.036 | 0.63 | 0.033 |
| **ERBB2** | 0.69 | 0.004 | 0.64 | 0.027 |
| **EGFR** | 0.56 | 0.200 | 0.61 | 0.068 |

| | | | | |
|---|---|---|---|---|
| AUC, area under the curve; *P* values are AUC > 0.5. | | | | |

A statistical test of significance indicated that these AUC values are all significantly greater than 0.5, the expected value from the null model that predicts clinical outcome randomly. Therefore, these three genes have potential utility for predicting clinical outcome of adjuvant tamoxifen therapy. As comparison, markers that are currently useful in evaluating the likelihood of response to tamoxifen were analyzed in comparison. The levels of ER (gene symbol ESR1) and progesterone receptor (PR, gene symbol PGR) are known to be positively correlated with tamoxifen response (see Fernandez, M.D., et al. "Quantitative oestrogen and progesterone receptor values in primary breast cancer and predictability of response to endocrine therapy." Clin Oncol 9, 245-50 (1983); Ferno, M. et al. "Results of two or five years of adjuvant tamoxifen correlated to steroid receptor and S-phase levels." South Sweden Breast Cancer Group, and South-East Sweden Breast Cancer Group. Breast Cancer Res Treat 59, 69-76 (2000); Nardelli, G.B., et al. "Estrogen and progesterone receptors status in the prediction of response of breast cancer to endocrine therapy (preliminary report)." Eur J Gynaecol Oncol 7, 151-8 (1986); and Osbome, C.K., et al. "The value of estrogen and progesterone receptors in the treatment of breast cancer." U 46, 2884-8 (1980)).

In addition, growth factor signaling pathways (EGFR, ERBB2) are thought to negatively regulate estrogen-dependent signaling, and hence contribute to loss of responsiveness to tamoxifen (see Dowsett, M. "Overexpression of HER-2 as a resistance mechanism to hormonal therapy for breast cancer." Endocr Relat Cancer 8, 191-5 (2001)). ROC analysis of these genes confirmed their correlation with clinical outcome, but with AUC values ranging only from 0.55 to 0.69, reaching statistical significance for PGR and ERBB2 (see Table 4).

The LCM dataset is particularly relevant, since EGFR, ERBB2, ESR1 and PGR are currently measured at the tumor cell level using either immunohistochemistry or fluorescence *in situ* hybridization. As individual markers of clinical outcome, HOXB13, IL17BR and CAC1D all outperformed ER1, PGR, EGFR and ERBB2 (see Table 4).

### Example 3

### Identification of the HOXB13:IL17BR Expression Ratio

HOXB13:IL17BR expression ratio was identified as a robust composite predictor of outcome as follows. Since HOXB13 and IL17BR have opposing patterns of expression, the expression ratio of HOXB13 over IL17BR was examined to determine whether it provides a better composite predictor of tamoxifen response. Indeed, both t-test and ROC analyses demonstrated that the two-gene ratio had a stronger correlation with treatment outcome than either gene alone, both in the whole tissue sections and LCM datasets (see Table 5). AUC values for HOXB13:IL17BR reached 0.81 for the tissue sections dataset and 0.84 for the LCM dataset. Pairing HOXB13 with CACNA1D or analysis of all three markers together did not provide additional predictive power.

**Table 5. HOXB13:IL17BR ratio is a stronger predictor of treatment outcome**

| | | ***t*-test** | | **ROC** |
|---|---|---|---|---|
| | | ***t*-statistic** | ***P* value** | **AUC *P* value** |
| | **IL17BR** | 4.15 | 1.15E-04 | 0.79 1.58E-06 |
| **Tissue** | **HOXB13** | -3.57 | 1.03E-03 | 0.67 0.01 |
| **Section** | **HOXB13:IL17B R** | **-4.91** | **1.48E-05** | **0.81 1.08E-07** |
| **LCM** | **IL17BR** | 3.70 | 5.44E-04 | 0.76 2.73E-05 |
| | | **HOXB13** | -4.39 | 8.00E-05 0.79 9.94E-07 |
| | | **HOXB13:IL17B R** | **-5.42** | **2.47E-06 0.84 4.40E-11** |

| | | | | |
|---|---|---|---|---|
| AUC, area under the curve; *P* values are AUC > 0.5. | | | | |

The HOXB13/IL7BR ratio was compared to well-established prognostic factors for breast cancer, such as patient age, tumor size, grade and lymph node status (see Fitzgibbons, P.L. et al. "Prognostic factors in breast cancer. College of American Pathologists Consensus Statement 1999." Arch Pathol Lab Med 124, 966-78 (2000)). Univariate logistic regression analysis indicated that only tumor size was marginally significant in this cohort (P=0.04); this was not surprising given that the responder group was closely matched to the non-responder group with respect to tumor size, tumor grade and lymph node status during patient selection. Among the known positive (ESR1 and PGR) and negative (ERBB2 and EGFR) predictors of tamoxifen response, ROC analysis of the tissue sections data indicated that only PGR and ERBB2 were significant (see Table 4). Therefore, a comparison of logistic regression models containing the HOXB13:IL17BR ratio either by itself or in combination with tumor size, and expression levels of PGR and ERBB2, were made (see Table 6). The HOXB13:IL17BR ratio alone was a highly significant predictor (P=0.0003) and had an odds ratio of 10.2 (95%CI 2.9-35.6). In the multivariate model, HOXB13:IL17BR ratio is the only significant variable (P=0.002) with an odds ratio of 7.3 (95%CI 2.1-26). Thus, the expression ratio of HOXB13:IL17BR is a strong independent predictor of treatment outcome in the setting of adjuvant tamoxifen therapy.

**Table 6. Logistic Regression Analysis**

| **Univariate Model** | | | |
|---|---|---|---|
| **Predictor** | Odds Ratio | 95% CI | P Value |
| *HOXB13:IL17BR* | 10.17 | 2.9-35.6 | 0.0003 |

| **Multivariate Model** | | | |
|---|---|---|---|
| **Predictors** | Odds Ratio | 95% CI | P Value |
| *Tumor size* | 1.5 | 0.7-3.5 | 0.3289 |
| *PGR* | 0.8 | 0.3-1.8 | 0.5600 |
| *ERBB2* | 1.7 | 0.8-3.8 | 0.1620 |
| *HOXB13:IL17BR* | 7.3 | 2.1-26.3 | 0.0022 |

| | | | |
|---|---|---|---|
| All predictors are continuous variables. Gene expression values were from microarray measurements. Odds ratio is the inter-quartile odds ratio, based on the difference of a predictor from its lower quartile (0.25) to its upper quartile (0.75); CI, confidence interval. | | | |

### Example 4

### Independent validation of HOXB13:IL17BR expression ratio

The reduction of a complex microarray signature to a two-gene expression ratio allows the use of simpler detection strategies, such as real-time quantitative PCR (RT-QPCR) analysis. The HOXB13:IL17BR expression ratio by RT-QPCR using frozen tissue sections that were available from 59 of the 60 training cases were analyzed (Fig 2, part a). RT-QPCR data were highly concordant with the microarray data of frozen tumor specimens (correlation coefficient r=0.83 for HOXB13, 0.93 for IL17BR). In addition, the PCR-derived HOXB13:IL17BR ratios, represented as ΔCTs, where CT is the PCR amplification cycles to reach a predetermined threshold amount (e.g., Fig. 2, parts a and b) and ΔCT is the CT difference between HOXB13 and IL17BR, were highly correlated with the microarray-derived data (r= 0.83) and with treatment outcome (t test P=0.0001, Fig. 2, part c). Thus, conventional RT-QPCR analysis for the expression ratio of HOXB13 to IL17BR appears to be equivalent to microarray-based analysis of frozen tumor specimens.

To validate the predictive utility of HOXB13:IL17BR expression ratio in an independent patient cohort, 20 additional ER-positive early-stage primary breast tumors from women treated with adjuvant tamoxifen only at MGH between 1991 and 2000, and for which medical records and paraffin-embedded tissues were available, were identified. Of the 20 archival cases, 10 had recurred with a median time to recurrence of 5 years, and 10 had remained disease-free with a median follow up of 9 years (see Table 7 for details).

**Table 7. Patient and tumor characteristics of the validation set.**

| **Sample** | **Tumor Type** | **Size** | **Grade** | **Nodes** | **ER** | **PR** | **Age** | **DFS** | **Status** |
|---|---|---|---|---|---|---|---|---|---|
| | | | | | | | | | |
| Test 1 | D | 1.9 | 3 | 0/10 | Pos | Pos | 69 | 15 | 1 |
| Test 2 | D | 1.7 | 3 | 0/19 | Pos | Pos | 61 | 117 | 1 |
| Test 3 | D | 1.7 | 2 | 0/26 | Pos | ND | 65 | 18 | 1 |
| Test 4 | D | 1.2 | 2 | 0/19 | Pos | Pos | 63 | 69 | 1 |
| Test 5 | D | 1.7 | 2 | 2/2 | Pos | Pos | 60 | 52 | 1 |
| Test 6 | D | 1.1 | 1 | 0/10 | Pos | Pos | 54 | 59 | 1 |
| Test 7 | D | >1.6 | 2 | 0/17 | Pos | Neg | 66 | 32 | 1 |
| Test 8 | L | 2.6 | 1-2 | 0/14 | Pos | Pos | 58 | 67 | 1 |
| Test 9 | D | 1.2 | 2 | ND | Pos | Pos | 93 | 58 | 1 |
| Test 10 | D | 4 | 3 | 0/20 | Pos | Pos | 66 | 27 | 1 |
| | | | | | | | | | |
| Test11 | D | 1.1 | 2 | 0/19 | Pos | Pos | 64 | 97 | 0 |
| Test 12 | D | 2.7 | 2 | 0/10 | Pos | Pos | 66 | 120 | 0 |
| Test 13 | D | 0.9 | 1 | 0/22 | Pos | Pos | 66 | 123 | 0 |
| Test 14 | D | 2.1 | 2 | 0/16 | Pos | Pos | 57 | 83 | 0 |
| Test 15 | D | 0.8 | 1-2 | 0/8 | Pos | Pos | 74 | 80 | 0 |
| Test 16 | D | 1 | 2 | 0/13 | Pos | Pos | 74 | 93 | 0 |
| Test 17 | D | 1.6 | 2 | 0/29 | Pos | Pos | 66 | 121 | 0 |
| Test 18 | L | 1.5 | 1-2 | 0/8 | Pos | Pos | 65 | 25 | 0 |
| Test 19 | D | 1.5 | 3 | 0/19 | Pos | Pos | 60 | 108 | 0 |
| [Test 20* | L | 4 | 1-2 | 0/19 | Pos | Pos | 60 | 108 | 0 |

| | | | | | | | | | |
|---|---|---|---|---|---|---|---|---|---|
| Abbreviations: Same as supplemental Table 1. * Patient received tamoxifen for 2 years. | | | | | | | | | |

RNA was extracted from formalin-fixed paraffin-embedded (FFPE) whole tissue sections, linearly amplified, and used as template for RT-QPCR analysis. Consistent with the results of the training cohort, the HOXB13:IL17BR expression ratio in this independent patient cohort was highly correlated with clinical outcome (t test P=0.035) with higher HOXB13 expression (lower ΔCTs) correlating with poor outcome (Fig. 2, part d). To test the predictive accuracy of the HOXB13:IL17BR ratio, the RT-QPCR data from the frozen tissue sections (n=59) was used to build a logistic regression model. In this training set, the model predicted treatment outcome with an overall accuracy of 76% (P=0.000065, 95% confidence interval 63%-86%). The positive and negative predictive values were 78% and 75%, respectively. Applying this model to the 20 independent patients in the validation cohort, treatment outcome for 15 of the 20 patients was correctly predicted (overall accuracy 75%, P=0.04, 95% confidence interval 51%-91%), with positive and negative predictive values of 78% and 73%, respectively.

Kaplan-Meier analysis of the patient groups as predicted by the model resulted in significantly different disease-free survival curves in both the training set and the independent test set (Fig. 2, parts e and f).

A further representative example of the application of the ratio to section samples and LCM samples of the 60 patient cohort is shown in Figure 8, Part B (indicated by "Sections" and "LCM" respectively). Also shown therein is an exemplary application of the ratio to 31 FFPE samples (indicated by "FFPE").

### Example 5

### Identification of additional sequences as the result of CHDH expression

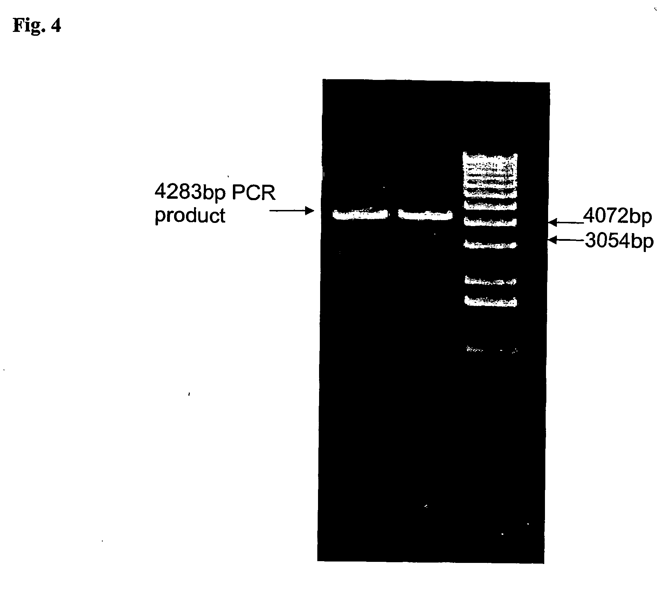

The fact that the sequence of AI240933 was complementary to the coding strand of CACNA1D led to the question of whether expression of a sequence other than that of CACNA1D was detected in Example 2 above. Therefore an assembly of sequences was made, which led to the identification of a larger 3' region of the known sequences expressed as part of CHDH (see Figures 5 and 3). This larger sequence was confirmed to be expressed by PCR analysis using probes that are capable of amplifying a calculated 4283 nucleotide region that spans both portions of previously identified CHDH sequence and the AI240933 sequence (see Figure 4).

The assembly of sequences in Figure 5 led to the identification of a larger contig of sequences shown in Figure 6. Additional support for the likelihood of the larger contig was provided by an alignment of the contig to mouse CHDH sequences.

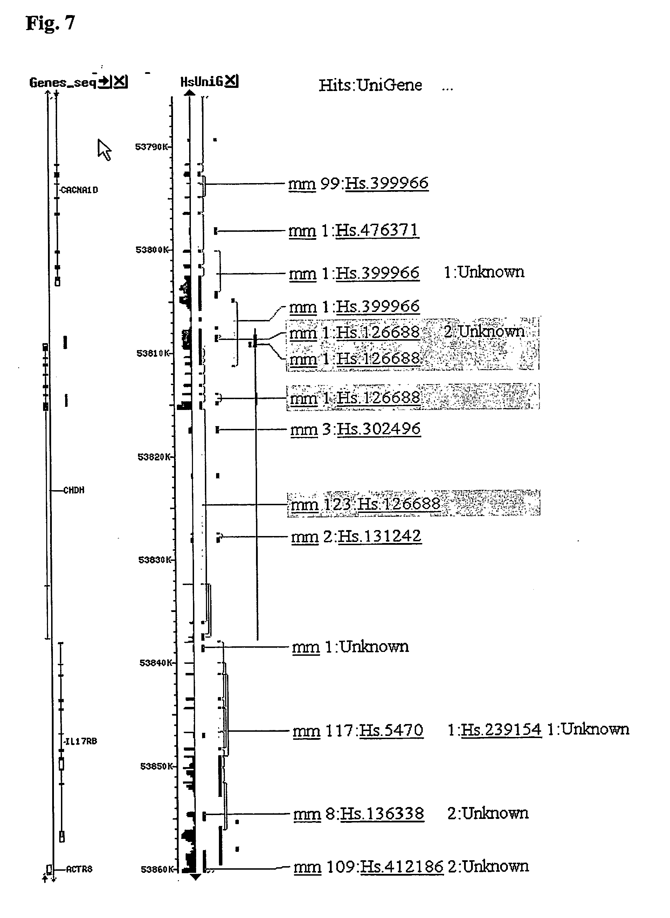

The likely relationship between CHDH and CACNA1D sequences is shown in Figure 7.

### Example 6

### Identification and use of the QPRT:CHDH Expression Ratio

A QPRT:CHDH expression ratio was identified as a robust composite predictor of outcome in a manner similar to that described in Example 3 above. Since QPRT and CHDH have opposing patterns of expression, the expression ratio of QPRT over CHDH was examined to determine its ability to function as a composite predictor of tamoxifen response. Results from the application of the ratio to section samples and LCM samples of the 60 patient cohort is shown in Figure 8, Part A (indicated by "Sections" and "LCM" respectively). Also shown therein is an exemplary application of the ratio to 31 FFPE samples (indicated by "FFPE").

### Additional References

Ma, X.J. et al. Gene expression profiles of human breast cancer progression. Proc Natl Acad Sci U S A 100, 5974-9 (2003).
Nicholson, R.I. et al. Epidermal growth factor receptor expression in breast cancer: association with response to endocrine therapy. Breast Cancer Res Treat 29, 117-25 (1994).

### Appendix

Sequences identified as those of IL17BR cluster
AW675096 
CCGGCGATGTCGCTCGTGCTGCTAAGCCTGGCCGCGCTGTGCAGGAGCGCCGTACCCCGA
GAGCCGACCGTTCAATGTGGCTCTGAAACTGGGCCATCTCCAGAGTGGATGCTACAACAT
GATCTAATCCCGGGAGACTTGAGGGACCTCCGAGTAGAACCTGTTACAACTAGTGTTGCA
ACAGGGGACTATTCAATTTTGATGAATGTAAGCTGGGTACTCCGGGCAGATGCCAGCATC
CGCTTGTTGAAGGCCACCAAGATTTGTGTGACGGGCAAAAGCAACTTCCAGTCCTACAGC
TGTGTGAGGTGCAATTACACAGAGGCCTTCCAGACTCAGACCAGACCCTCTGGTGGTAAA
TGGACATTTTCCTACATCGGCTTCCCTGTAGAGCTGAACACAGTCTATTTCATTGGGGCC
CATAATATTCCTAATGCAAATATGAATGAAGATGGCCCTTCCATGTCTGTGAATNTCACC
TCACCAGGCTGCCTAGACCACATAATGAAATATAAAAAAAAGTGTGTCAAGGCCGGAAGC
CTGTGGGATCCGAACATCACT
AW673932 
TTTTTTTTTTTTTTTTTTTTAAAAGTGGGTTCAGCTTGTTTATTCCCTACTTTTGTTATCT
TAAAAACAATGATTTTTGCATGTAATAGAAGGTTTTCACTTAAGATGCTATTGAGTGA
ATCAGTGAGGGGTTCTTAGAGTTAGTATTCATTAATTAAACATAGAATATTAGCTAAACA
GTTCTGGGTACACTGCAATGCATGGTCTATGGAAGACTAGATGTTTGGCTGAAGATGCTT
TATTGTTGCATTATCAAAATGGTTATAGTTTTCAATTAAAACTGTAATTGATTTCTATGT
ATAAAACAGCTTTGAAGTTGTAAATGTAGTTTCCAATCGTTAGTTAATGCTACATTAGTT
AGCAATATTTGAAAATTTTATTGGTATAAAATGTTTTAATTACTAAGGCTGTTTGTAGGC
TGCATAGTAAGCTTCAGGATCATCACACGTTTTTTCCCTGTAATTGG
BC000980 
ggcccggcga tgtcgctcgt gctgctaagc ctggccgcgc tgtgcaggag cgccgtaccc
cgagagccga ccgttcaatg tggctctgaa actgggccat ctccagagtg gatgctacaa
catgatctaa tcccgggaga cttgagggac ctccgagtag aacctgttac aactagtgtt
gcaacagggg actattcaat tttgatgaat gtaagctggg tactccgggc agatgccagc
atccgcttgt tgaaggccac caagatttgt gtgacgggca aaagcaactt ccagtcctac
agctgtgtga ggtgcaatta cacagaggcc ttccagactc agaccagacc ctctggtggt
aaatggacat tttcctacat cggcttccct gtagagctga acacagtcta tttcattggg
gcccataata ttcctaatgc aaatatgaat gaagatggcc cttccatgtc tgtgaatttc
acctcaccag gctgcctaga ccacataatg aaatataaaa aaaagtgtgt caaggccgga
agcctgtggg atccgaacat cactgcttgt aagaagaatg aggagacagt agaagtgaac
ttcacaacca ctcccctggg aaacagatac atggctctta tccaacacag cactatcatc
gggttttctc aggtgtttga gccacaccag aagaaacaaa cgcgagcttc agtggtgatt
ccagtgactg gggatagtga aggtgctacg gtgcagctga ctccatattt tcctacttgt
ggcagcgact gcatccgaca taaaggaaca gttgtgctct gcccacaaac aggcgtccct
ttccctctgg ataacaacaa aagcaagccg ggaggctggc tgcctctcct cctgctgtct
ctgctggtgg ccacatgggt gctggtggca gggatctatc taatgtggag gcacgaaagg
atcaagaaga cttccttttc taccaccaca ctactgcccc ccattaaggt tcttgtggtt
tacccatctg aaatatgttt ccatcacaca atttgttact tcactgaatt tcttcaaaac
cattgcagaa gtgaggtcat ccttgaaaag tggcagaaaa agaaaatagc agagatgggt
ccagtgcagt ggcttgccac tcaaaagaag gcagcagaca aagtcgtctt ccttctttcc
aatgacgtca acagtgtgtg cgatggtacc tgtggcaaga gcgagggcag tcccagtgag
 
aactctcaag acctcttccc ccttgccttt aaccttttct gcagtgatct aagaagccag
attcatctgc acaaatacgt ggtggtctac tttagagaga ttgatacaaa agacgattac
aatgctctca gtgtctgccc caagtaccac ctcatgaagg atgccactgc tttctgtgca
gaacttctcc atgtcaagca gcaggtgtca gcaggaaaaa gatcacaagc ctgccacgat
ggctgctgct ccttgtagcc cacccatgag aagcaagaga ccttaaaggc ttcctatccc
accaattaca gggaaaaaac gtgtgatgat cctgaagctt actatgcagc ctacaaacag
ccttagtaat taaaacattt tataccaata aaattttcaa atattgctaa ctaatgtagc
attaactaac gattggaaac tacatttaca acttcaaagc tgttttatac atagaaatca
attacagttt taattgaaaa ctataaccat tttgataatg caacaataaa gcatcttcag
ccaaacatct agtcttccat agaccatgca ttgcagtgta cccagaactg tttagctaat
attctatgtt taattaatga atactaactc taagaacccc tcactgattc actcaatagc
atcttaagtg aaaaaccttc tattacatgc aaaaaatcat tgtttttaag ataacaaaag
tagggaataa acaagctgaa cccactttta aaaaaaaaaa aaaaaaaaaa aaaaaaaaaa
aa
BI602183 
AGCGGAGCTGCGGGTGGCCTGGATCCCGCAGTGGCCCGGCGATGTCGCTCGTGCTGCT
AAGCCTGGCCACGCTGTGCAGGAGCGCCGTACCCCGAGAGCCGACCGTTCAATGTGGCTC
TGAAACTGTGGACATTTTCCTATATCGGCTTCCCTGTAGAGCTGAAAACAGTCTATTTCA
TTGGGGCCCATAATATTCCTAATGCAAATATGAATGAAGATGGCCCTTCCATGTCTGTGA
ATTTCACCTCACCAGGCTGCCTAGACCACATAATGAAATATAAAAAAGTGTGTCAAGGC
CGGAAGCCTGTGGGATCCGAACATCACTGCTTGTAAGAAGAATGAGGAGACAGTAGAAGT
GAACTTCACAACCACTCCCCTGGGAAACAGATACATGGCTCATCCAACACAGCACTATCA
TCGGGTTTTCTCAGGTGTTTGAGCCACACCAGAAGAAACAAACGCGAGCTTCAGTGGTGA
TTCCAGTGACTGGGGATAGTGAAGGTGCTACGGTGCAGCTGACTCCATATTTTCCTACTT
GTGGCAGCGACTGCATCCGACATAAAGGAACAGTTGTGCTCTGCCCACAAACAGGCGTCC
CTTTCCCCTCTGGATAACAACAAAAGCAAGCCGGGAGGCTGGCTGCCTCTCCTCCTGCTG
TCTCTGCTGGTTGGCCACATTGGGTGCTGGTGGCAGGGATCTATCTAATGTGGAGGCACG
AAAGGATCCAGAAGACTTCCTTTTCTACCACAAACTACTGCCCCCATTAAGGTCCTGTGG
TTACCCATCTTGAAATATGTTCCTCACACAATTTGTTACTTCACTGAATTCTTCAAAACC
TG
BI458542 
AGCGGAGCGTGCGGGTGGCCTGGATCCCGCAGTGGCCCGGCGATGTCGCTCGTGCTGC
TAAGCCTGGCCACGCTGTGCAGGAGCGCCGTACCCCGAGAGCCGACCGTTCAATGTGGCT
CTGAAACTGTGGACATTTTCCTATATCGGCTTCCCTGTAGAGCTGAAAACAGTCTATTTC
ATTGGGGCCCATAATATTCCTAATGCAAATATGAATGAAGATGGCCCTTCCATGTCTGTG
AATTTCACCTCACCAGGCTGCCTAGACCACATAATGAAATATAAAAAAAAGTGTGTCAAG
GCCGGAAGCCTGTGGGATCCGAACATCACTGCTTGTAAGAAGAATGAGGAGACAGTAGAA
GTGAACTTCACAACCACTCCCCTGGGAAACAGATACATGGCTCATCCAACACAGCACTAT
CATCGGGTTTTCTCAGGTGTTTGAGCCACACCAGAAGAAACAAACGCGAGCTTCAGTGGT
GATTCCAGTGACTGGGGATAGTGAAGGTGCTACGGTGCAGCTGACTCCATATTTTCCTAC
TTGTGGCAGCGACTGCATCCGACATAAAGGAACAGTTGTGCTCTGCCCACAAACAGGCGT
CCCTTTCCCTCTGGATAACAACAAAAGCAAGCCGGGAGGCTGGCTGCCTCTCCTCCTGCT
GTCTCTGCTGGTGGNCACATTGGGTGCTGGTGGCAGGGATCTATCTAATGTGGAGGCACG
AAAGGATCAGAAGACTTCCTTTTCTACCACCACATACTGCCCCCCATTAAGGTTCTTGTG
GTTTACCC
BI823321 GGCGATGTCGCTCGTGCTGCTAAGCCTGGCCGCGCTGTGCAGGAGCGCCGTACCCCGAGA 
GCCGACCGTTCAATGTGGCTCTGAAACTGGGCCATCTCCAGAGTGGATGCTACAACATGA
TCTAATCCCGGAGACTTGAGGGACCTCCGAGTAGAACCTGTTACAACTAGTGTTGCAAC
AGGGGACTATTCAATTTTGATGAATGTAAGCTGGGTACTCCGGGCAGATGCCAGCATCCG
CTTGTTGAAGGCCACCAAGATTTGTGTGACGGGCAAAAGCAACTTCCAGTCCTACAGCTG
TGTGAGGTGCAATTACACAGAGGCCTTCCAGACTCAGACCAGACCCTCTGGTGGTAAATG
GACATTTTCCTATATCGGCTTCCCTGTAGAGCTGAACACAGTCTATTTCATTGGGGCCCA
TAATATTCCTAATGCAAATATGAATGAAGATGGCCCTTCCATGTCTGTGAATTTCACCTC
ACCAGGAAGCCTGTGGGATCCGAACATCACTGCTTGTAAGAAAGAATGAGGAGACAGTAG
AAGTGAACTTCACAACCACTCCCCTGGGAAACAGATACATGGCTCTTATCCAACACAGCA
CTATCATCGGGTTTCTCAGGTGTTTGAGCCACACCAGAAGAAACAAACGCGAGCTTCAGT
GGTGATTCCAGTGACTGGGGATAGTGAAGGTGCTACGGTGCAGCTGACTCCATATTTTCC
TACTTGTGGCAGCGACTGCAATCCGACATAAAGGAACAGTTGTGCTCTGCCCACAAACAG
GCGTCCCTTTCCCTCTTGGATAGCAACAGAAGCAAGCCGGGAGGCTGGTGCCTCTTCTTC
TGGTGTCTCTGCTGGTGGCACATTGAGTGCTGGTGGCAGGATCCATCTAATGTGGAGGCC
CCAAAGGACCAGGAAAGACTTCCTTTATTAGCACCAAGTATTGCCC
AA514396 
TGGCTGAAGATGCTTTATTGTTGCATTATCAAAATGGTTATAGTTTTCAATTAAAACTGT
AATTGATTTCTATGTATAAAACAGCTTTGAAGTTGTAAATGTAGTTTCCAATCGTTAGTT
AATGCTACATTAGTTAGCAATATTTGAAAATTTTATTGGTATAAAATGTTTTAATTACTA
AGGCTGTTTGTAGGCTGCATAGTAAGCTTCAGGATCATCACACGTTTTTTCCCTGTAATT
GGTGGGATAGGAAGCCTTTAAGGTCTCTTGCTTCTCATGGGTGGGCTACAAGGAGCAGCA
GCCATCGTGGCAGGCTTGTGATCTTTTTCCTGCTGACACCTGCTACTTGACATGGAGAAG
TTCTGCACAGAAAGCAGTGGCATCCTTCATGAGGTGGTACTTGGGGCAGACACTGAGAGC
ATTGTAATCGTCTTTTGTATCAATCTCTCTAAAGTAGACCACCACGTATTTGTGCAGATG
AATCTGGC
BF110326 
TTTGTTTGGCTGAAGATGCTTTATTGTTGCATTATCAAAATGGTTATAGTTTTCAATTAA
AACTGTAATTGATTTCTATGTATAAAACAGCTTTGAAGTTGTAAATGTAGTTTCCAATCG
TTAGTTAATGCTACATTAGTTAGCAATATTTGAAAATTTTATTGGTATAAAATGTTTTAA
TTACTAAGGCTGTTTGTAGGCTGCATAGTAAGCTTCAGGATCATCACACGTTTTTCCCT
GTAATTGGTGGGATAGGAAGCCTTTAAGGTCTCTTGCTTTTCATGGGTGGGCTACAAGGA
GCAGCAGCCATCGTGGCAGGCTTGTGATCTTTTTCCTGCTGACACCTGCTGCTTGACATG
GAGAAGTTCTGCACAGAAAGCAGTGGCATCCTTCATGAGGTGGTACTTGGGGCAGACACT
GAGAGCATTGTAATCGTCTTTTGTATCAATCTCTCTAAAGTAGACCACCACGTATTTGTG
CAGATGAATCTGGCTTCTTAGATCACTGC
BE466508 
TGGCATGAGATGCTATATTGTTGCATTATCAAAATGGGTTTAGTCTTCAATTAACACTGT
AATTGATTTCTATGTATAAAACAGCTTTGAAGTTGTAAATGTGGTTTCCAATCGTCAGTT
AATGCTACATTAGTTAGCAATATTTGAAAATTTTATTGGTATAAAATGTTTTAATTACTA
AGGCTGTTTGTAGGCTGCATAGTAAGCTTCAGGATCATCACACGTTTTTTCCCTGTAATT
GGTGGGATAGGAAGCCTTTAAGGTCTCTTGCTTCTCATGGGTGGGCTACAAGGAGCAGCA
GCCATCGTGGCAGGCTTGTGATCTTTTTCCTGCTGACACCTGCTGCTTGACATGGAGAAG
TTCTGCACAGAAAGCAGTGGCATCCTTCATGAGGTGGTACTTGGGGCAGACACTGAGAGC
ATTGTAATCGTCTTTTGTATCAATCTCTCTAAAGTAGACCACCACGTATTTGTGCAGATG
AATCTGGCTTCTTAGATCACTG
BF740045 
GTTTGGCTGAAGATGCTTTATTGTTGCATTATCAAAATGGTTATAGTTTTCAATTAAAAC
TGTAATTGATTTCTATGTATAAAACACGCTTTGAAGTTGTAAATGTAGTTTCCAATCGTT
AGTTAATGCTACATTAGTTAGCAATATTTGAAAATTTTATTGGTATAAAATGTTTTAATT
ACTAAGGCTGTTTGTAGGCTGCATAGTAAGCTTCAGGATCATCACACGTTTTTCCCTGT
AATTGGTGGGATAGGAAGCCTTTAAGGTCTCTTGCTTCTCATGGGTGGGCTACAAGGAGC
AGCAGCCATCGTGGCAGGCTTGTGATCTTTTTCCTGCTGACACCTGCTGCTTGACATGGA
GAAGTTCTGCACAGAAAGCAGTGGCATCCTTCATGAGGTGGTACTTGGGGCAGACACTGA
GAGCATTGTAATCGTCTTTTGTATCAATCTCTCTAAAGTA
AW299271 
TGGCTGAAGATGCTTTATTGTTGCATTATCAAAATGGTTATAGTTTTCAATTAAAACTGT
AATTGATTTCTATGTATAAAACAGCGTTGAAGTTGTAAATGTAGTTTCCAATCGTTAGTT
AATGCTACATTAGTTAGCAATATTTGAAAATTTTATTGGTATAAAATGTTTTAATTACTA
AGGCTGTTTGTAGGCTGCATAGTAAGCTTCAGGATCATCACACGTTTTTTCCCTGTAATT
GGTGGGATAGGAAGCCTTTAAGGTCTCTTGCTTCTCATGGGTGGGCTACAAGGAGCAGCA
GCCATCGTGGCAGGCTTGTGATCTTTTTCCTGCTGACACCTGCTGCTTGACATGGAGAAG
TTCTGCACAGAAAGCAGTGGCATCCTTCATGAGGTGGTACTTGGGGCAGACACTGAGAGC
ATTGTAATCGTCTTTTGTATCAATCTCTCTAAAGTAGACCACCACGTATTTGTGCAGATG
AATCTGGCTTCTTAGATCACTGCAGAAAAG
AA836217 
TTTTTTTTTTACAACTTCAAAGCTGTTTATACATAGAAATCAATTACAGTTTTAATTGA
AAACTATAACCATTTTGATAATGCAACAATAAAGCATCTTCAGCCAAACATCTAGTCTTC
CATAGACCATGCATTGCAGTGTACCCAGAACTGTTTAGCTAATATTCTATGTTTAATTAA
TGAATACTAACTCTAAGAACCCCTCACTGATTCACTCAATAGCATCTTAAGTGAAAAACC
TTCTATTACATGCAAAAAATCATTGTTTTAAGATAACAAAAGTAGGGAATAAACAAGCT
GAACCCACTTTTACTGGACCAAATGATCTATTATATGTGTACCACTTGTATGATTTGGTA
TTTGCATAAGACCTTCCCTCTACAAACTAGATTCATATCTTGATTCTTGTACAGGTGCCT
TTTAACATGAACAACAAAATACCCACAAACTTGTCTACTTTTGCC
AI203628 
TAGTAATTAAAACATTTTATACCAATAAAATTTTCAAATATTGCTAACTAATGTAGCATT
AACTAACGATTGGAAACTACATTTACAACTTCAAAGCTGTTTATACATAGAAATCAATT
ACAGTTTTAATTGAAAACTATAACCATTTTGATAATGCAACAATAAAGCATCTTCAGCCA
AACATCTAGTCTTCCATAGACCATGCATTGCAGTGTACCCAGAACTGTTTAGCTAATATT
CTATGTTTAATTAATGAATACTAACTCTAAGAACCCCTCACTGATTCACTCAATAGCATC
TTAAGTGAAAAACCTTCTATTACATGCAAAAAATCATTGTTTTTAAGATAACAAAAGTAG
GGAATAAACAAGCTGAACCCACTTTTACTGGACCAAATGATCTATTATATGTGTAACCAC
TTGTATGATTTGGTATTTGCATAAGACCTTCCCTCTACAAACTAGATTCATATCTTGATT
CTTGTACAGGTGCCTTTTAACATGAA
AI627783 
TTTTTTTTTTTTTTTTAGCAATATTGAAAATTTATTGGTATAAAATGTTTTAATTACT
AAGGCTGTTTGTAGGCTGCATAGTAAGCTTCAGGATCATCACACGTTTTTCCCTGTAAT
TGGTGGGATAGGAAGCCTTTAAGGTCTCTTGCTTCTCATGGGTGGGCTACAAGGAGCAGC
AGCCATCGTGGCAGGCTTGTGATCTTTTTCCTGCTGACACCTGCTACTTGACATGGAGAA
GTTCTGCACAGAAAGCAGTGGCATCCTTCATGAGGTGGTACTTGGGGCAGACACTGAGAG
 
CATTGTAATCGTCTTTTGTATCAATCTCTCTAAAGTAGACCACCACGTATTTGTGCAGAT
GAATCTGGCTTCTTAGATCACTGCAGAAAAGGTTAAAGGCAAGGGGGAAGAGGTCTTGAG
AGTTCTC
AI744263 
TTAAAGTGGGTTCAGCTTGTTTATTCCCTACTTTTGTTATCTTAAAAACAATGATTTTTT
GCATGTAATAGAAGGTTTTTCACTTAAGATGCTATTGAGTGAATCAGTGAGGGGTTCTTA
GAGTTAGTATTCATTAATTAAACATAGAATATTAGCTAAACAGTTCTGGGTACACTGCAA
TGCATGGTCTATGGAAGACTAGATGTTTGGCTGAAGATGCTTTATTGTTGCATTATCAAA
ATGGTTACAGTTTTCAATTAAAGCTGTAATTGATTTCTATGTATAAAACAGCTTTGAAGT
TGTAAATGTAGTTTCCAATCGTTAGTTAATGCTACATTAGTTAGCAATATTTGAAAATTT
TATTGGTATAAAATGTTTTAATTACTAAGGCTGTTTGTAGGCTGCATAGTAAGCTTCAGG
ATCATCACACGTTNTTTCCCTGTAATTGGTGGATAGGAAGCCTTTA
AI401622 
AGTTAGCAATATTTGAAAATTTTATTGGTATAAAATGTTTTAATTACTAAGGCTGTTTGT
AGGCTGCATAGTAAGCTTCAGGATCATCACACGTTTTTCCCTGTAATTGGTGGATAGG
AAGCCTTTAAGGTCTCTTGCTTCTCATGGGTGGCTACAAGGAGCAGCAGCCATCGTGGC
AGGCTTGTGATCTTTTTCCTGCTGACACCTGCTACTTGACATGGAGAAGTTCTGCACAGA
AAGCAGTGGCATCCTTCATGAGGTGGTACTTGGGGCAGACACTGAGAGCATTGTAATCGT
CTTTTGTATCAATCTCTCTAAAGTAGACCACCACGTATTTGTGCAGATGAATCTGGCTTC
TTAGATCACTGCAGAAAAGGTTAAAGGCAAGGGGGAAGAGGTCTTGAGAGTTCTCACTGG
AI826949 
TTGGCTGAAGATGCTTTATTGTTGCATTATCAAAATGGTTATAGTTTTCAATTAAAACTG
TAATTGATTTCTATGTATAAAACAGCTTTGAAGTTGTAAATGTAGTTTCCAATCGTTAGT
TAATGCTACATTAGTTAGCAATATTTGAAAATTTTATTGGTATAAAATGTTTTAATTACT
AAGGCTGTTTGTAGGCTTGCATAGAAGCTTCAGGATCATCACACGTTTTTTCCCTGTAAT
TGGTGGGATAGGAAGCCTTTAAGGTCTCTTGCTTCTCATGGGTGGGCTACAAGGAGCAGC
AGCCATCGTGGCAGGCTTGTGATCTTTTTCCTGCTGACACCTGCTGCTTGACATGGAGAA
GTTCTGCACAGAAAGCAGTGGCATCCTTCATGAGGTGGTACTTGGGGCAGACACTGAGAG
CATTGTAATCGTCT
BE047352 
TTTTTTTTTTAGCAATATTTGAAAATTTTATTGGTATAAAATGTTTTAATTACTAAGGCT
GTTTGTAGGCTGCATAGTAAGCTTCAGGATCATCACACGTTTTTCCCTGTAATTGGTGG
GATAGGAAGCCTTTAAGGTCTCTTGCTTCTCATGGGTGGGCTACAAGGAGCAGCAGCCAT
CGTGGCAGGCTTGTGATCTTTTTCCTGCTGACACCTGCTACTTGACATGGAGAAGTTCTG
CACAGAAAGCAGTGGCATCCTTCATGAGGTGGTACTTGGGGCAGACACTGAGAGCATTGT
AATCGTCTTTTGTATCAATCTCTCTAAAGTAGACCACCACGTATTTGTGCAGATGAATCT
GGCTTCTTAGATCACTGCAGAAAAGGTTAAAGGCAAGGGGGAAGAGGTCTTGAGAG
AI911549 
TTTGGCTGAAGATGCTTTATTGTTGCATTATCAAAATGGTTACAGTTTTCAATTAAAGCT
GTAATTGATTTCTATGTATAAAACAGCTTTGAAGTTGTAAATGTAGTTTCCAATCGTTAG
TTAATGCTACATTAGTTAGCAATATTTGAAAATTTATTGGTATAAAATGTTTTAATTAC
TAAGGCTGTTTGTAGGCTGCATAGTAAGCTTCAGGATCATCACACGTTTTTTCCCTGTAA
 
TTGGTGGGATAGGAAGCCTTTAAGGTCTCTTGCTTCTCATGGGTGGGCTACAAGGAGCAG
CAGCCATCGTGGCAGGCTTGTGATCTTTTTCCTGCTGACACCTGCTGCTTGACATGGAGA
AGTTCTGCACAGAAAGCAGTGGCATCCTTCATGAGGTGGTACTTGGGGCAGACA
BF194822 
TTCTCTGGCTGAAGATGCTTTATTGTTGCATTATCAAAATGGTTACAGTTTTCAATTAAA
GCTGTAATTGATTTCTATGTATAAAACAGCTTTGAAGTTGTAAATGTAGTTTCCAATCGT
TAGTTAATGCTACATTAGTTAGCAATATTTGAAAATTTTATTGGTATAAAATGTTTTAAT
TACTAAGGCTGTTTGTAGGCTGCATAGTAAGCTTCAGGATCATCACACGTTTTTCCCTG
TAATTGGTGGGATAGGAAGCCTTTAAGGTCTCTTGCTTCTCATGGGTGGCTACAAGGAG
CAGCAGCCATCGTGGCAGGCTTGTGATCTTTTTCCTGCTGACACCTGCTGCTTGACATGG
AGAAGTTCTGCACAGAAAGCAGTGGCATCCTTCATGAGGTGGTACTTGG
AI034244 
TTTTTTTTTTTTTTTTACAACCTTGAAAGCTGTTTATACATAGAAATCAATTACAGTTT
TAATTGAAAACTATAACCATTTTGATAATGCAACAATAAAGCATCTTCAGCCAAACATCT
AGTCTTCCATAGACCATGCATTGCAGTGTACCCAGAACTGTTTAGCTAATATTCTATGTT
TAATTAATGAATACTAACTCTAAGAACCCCTCACTGATTCACTCAATAGCATCTTAAGTG
AAAAACCTTCTATTACATGCAAAAAATCATTGTTTTTAAGATAACAAAAGTAGGGAATAA
ACAAGCTGAACCCACTTTTACTGGACCAAATGATCTATTATATGTGTAACCACTTGTATG
ATTTGGATTTGCATAAGACCTTCCCTCTACAAACTAGATTCATATCTTGATTCT
AI033911 
TTTTTTTTTTTTTTTACAACTGCAAAGCTGTTTATACATAGAAATCAATTACAGTTTT
AATTGAAAACTATAACCATTTTGATAATGCAACAATAAAGCATCTTCAGCCAAACATCTA
GTCTTCCATAGACCATGCATTGCAGTGTACCCAGAACTGTTTAGCTAATATTCTATGTTT
AATTAATGAATACTAACTCTAAGAACCCCTCACTGATTCACTCAATAGCATCTTAAGTGA
AAAACCTTCTATTACATGCAAAAAATCATTGTTTTTAAGATAACAAAAGTAGGGAATAAA
CAAGCTGAACCCACTTTTACTGGACCAAATGATCTATTATATGTGTAACCACTTGTATGA
TTTGGTATTTGCATAAGACCTTCCCTCTACAAACTAGATTCATATCTTGATTCT
BF064177 
TTTTTTAGTTAGCAATATTTGAAAATTTTATTGGTATAAAATGTTTTAATTACTAAGGCT
GTTTGTAGGCTGCATAGTAAGCTTCAGGATCATCACACGTTTTTCCCTGTAATTGGTGG
GATAGGAAGCCTTTAAGGTCTCTTGCTTCTCATGGGTGGGCTACAAGGAGCAGCAGCCAT
CGTGGCAGGCTTGTGATCTTTTTCCTGCTGACACCTGCTACTTGACATGGAGAAGTTCTG
CACAGAAAGCAGTGGCATCCTTCATGAGGTGGTACTTGGGGCAGACACTGAGAGCATTGT
AATCGTCTTTTGTATCAATCTCTCTAAAGTAGACCACCACGTATTTGTGCAGATGAATCT
GGCTTCTTAGATCACTGCAGAAAAGGTTAAAGGCAAGGGGGAAGAGGTCTTGAGAGTTCT
CACTGGGACTGCCCTCGCTCTTGCCACAGGTACCATCGCACACACTGTTGACGTCATTGG
AAAG
AA847767 
GGCTGAAGATGCTTTATTGTTGCATTATCAAAATGGTTATAGTTTTCAATTAAAACTGTA
ATTGATTTCTATGTATAAAACAGCTTTGAAGTTGTAAATGTAGTTTCCAATCGTTAGTTA
ATGCTACATTAGTTAGCAATATTTGAAAATTTTATTGGTATAAAATGTTTTAATTACTAA
GGCTGTTTGTAGGCTGCATAGTAAGCTTCAGGATCATCACACGTTTTTTCCCTGTAATTG
 
GTGGGATAGGAAGCCTTTAAGGTCTCTTGCTTCTCATGGGTGGCTACAAGGAGCAGCAG
CCATCGTGGCAGGCTTGTGATCTTTTTCCTGCTGACACCTGCTGCTTGACATGGAGAAGT
TCTGCACAGAAAGCAGTGGCATCCTTCATGAGGTGGTA
AI538624 
TTGGCTGAAGATGCTTTATTGTTGCATTATCAAAATGGTTACAGTTTTCAATTAAAGCTG
TAATTGATTTCTATGTATAAAACAGCTTTGAAGTTGTAAATGTAGTTTCCAATCGTTAGT
TAATGCTACATTAGTTAGCAATATTTGAAAATTTTATTGGTATAAAATGTTTTAATTACT
AAGGCTGTTTGTAGGCTGCATAGTAAGCTTCAGGATCATCACACGTTTTTTCCCTGTAAT
TGGTGGGATAGGAAGCCTTTAAGGTCTCTTGCTTCTCATGGGTGGGCTACAAGGAGCAGC
AGCCATCGTGGCAGGCTTGTGATCTTTTTCCTGCTGACACCTGCTGCTTGACATGGAGAA
GTTCTGCACAGAAAGCAGTGGCATCCTTCATGAGGTGGTAC
AI913613 
TTGGCTGAAGATGCTTTATTGTTGCATTATCAAAATGGTTATAGTTTTCAATTAAAACTG
TAATTGATTTCTATGTATAAAACAGCTTTGAAGTTGTAAATGTAGTTTCCAATCGTTAGT
TAATGCTACATTAGTTAGCAATATTTGAAAATTTTATTGGTATAAAATGTTTTAATTACT
AAGGCTGTTTGTAGGCTGCATAGTAAGCTTCAGGATCATCACACGTTNTTTCCCTGTAAT
TGGTGGGATAGGAAGCCTTTAAGGTCTCTTGCTTCTCATGGGTGGGCTACAAGGAGCAGC
AGCCATCGTGGCAGGCTTGTGATCTTTTTCCTGCTGACACCTGCTGCTTGACATGGAGAA
GTTCTGCACAGAAAGCAGTGGCATCCTTCATG
AI942234 
GTTTGGCTGAAGATGCTTTATTGTTGCATTATCAAAATGGTTATAGTTTTCAATTAAAAC
TGTAATTGATTTCTATGTATAAAACAGCTTTGAAGTTGTAAATGTAGTTTCCAATCGTTA
GTTAATGCTACATTAGTTAGCAATATTTGAAAATTTTATTGGTATAAAATGTTTTAATTA
CTAAGGCTGTTTGTAGGCTGCATAGTAAGCTTCAGGATCATCACACGTTTTTTCCCTGTA
ATTGGTGGGATAGGAAGCCTTTAAGGTCTCTTGCTTCTCATGGGTGGGCTACAAGGAGCA
GCAGCCATCGTGGCAGCTTGGTGATCTTTTTCCTGCTGACACCTGCTGCTTGACATGAAG
AAGTTCTGCACAGAAAGCAGTGGCAT
AI580483 
GTTTGGCTGAAGATGCTTTATTGTTGCATTATCAAAATGGTTATAGTTTTCAATTAAAAC
TGTAATTGATTTCTATGTATAAAACAGCTTTGAAGTTGTAAATGTAGTTTCCAATCGTTA
GTTAATGCTACATTAGTTAGCAATATTTGAAAATTTTATTGGTATAAAATGTTTTAATTA
CTAAGGCTGTTTGTAGGCTGCATAGTAAGCTTCAGGATCATCACACGTTTTTTCCCTGTA
ATTGGTGGGATAGGAAGCCTTTAAGGTCTCTTGCTTCTCATGGGTGGGCTACAAGGAGCA
GCAGCCATCGTGGCAGGCTTGGATCTTTTTCCTGCTGACACCTGCTGCTTGACATTGGAA
AGTTCTGCACAGAAAGCAGTGGCATC
AI831909 
TTTTGGCTGATGATGCTTTATTGTTGCATTATCAAAATGGTTACAGTTTTCAATTAAAGC
TGTAATTGATTTCTATGTATAAAACAGCTTTGAAGTTGTAAATGTAGTTTCCAATCGTTA
GTTAATGCTACATTAGTTAGCAATATTTGAAAATTTATTGGTATAAAATGTTTTAATTA
CTAAGGCTGTTTGTAGGCTGCATAGTAAGCTTCAGGATCATCACACGTTTTTCCCTGTA
ATTGGTGGGATAGGAAGCCTTTAAGGTCTCTTGCTTCTCATGGGTGGGCTACAAGGAGCA
GCAGCCATCGTGGCAGGCTTGTGATCTTTTTCCTGCTGACACCTGCTGCTTGACATGGAG
 AAGTTCTGCACAGAAAGCAGTGGCAT
AI672344 
GGCTGAAGATGCTTTATTGTTGCATTATCAAAATGGTTACAGTTTTCAATTAAAGCTGTA
ATTGATTTCTATGTATAAAACAGCTTTGAAGTTGTAAATGTAGTTTCCAATCGTTAGTTA
ATGCTACATTAGTTAGCAATATTTGAAAATTTTATTGGTATAAAATGTTTTAATTACTAA
GGCTGTTTGTAGGCTGCATAGTAAGCTTCAGGATCATCACACGTTTTTTCCCTGTAATTG
GTGGGATAGGAAGCCTTTAAGGTCTCTTGCTTCTCATGGGTGGGCTACAAGGAGCAGCAG
CCATCGTGGCAGGCTTGTGATCTTTTTCCTGCTGACACCTGCTGCTTGACATGGAGAAGT
TCTGCACAGAAAG
AW025192 
GATTGGCTGTTTTATGCTTTATTGTTGCATTATCAAAATGGTTATAGTTTTCAATTAAAA
CTGTAATTGATTTCTATGTATAAAACAGCTTTGAAGTTGTAAATGTAGTTTCCAATCGTT
AGTTAATGCTACATTAGTTAGCAATATTTGAAAATTTTATTGGTATAAAATGTTTTAATT
ACTAAGGCTGTTTGTAGGCTGCATAGTAAGCTTCAGGATCATCACACGTTTTTTCCCTGT
TATTGGTGGGATAGGAAGCCTTTAAGGTCTCTTGCTTCTCATGGGTGGGCTACAAGGAGC
AGCAGCCATCGTGGCAGGCTTGTGATCTTTTTCCTGCTGACACCTGCTGCTTGACATGGA
GAAGTTCTGCACAAAAAGCAGTGGCATCCTTCATGAGGTGGTA
AA677205 
GCAATATTTTAAAATTTTATTGGTATAAAATGTTTTAATTACTAAGGCTGTTTGTAGGCT
GCATAGTAAGCTTCAGGATCATCACACGTTTTTCCCTGTAATTGGTGGCATAGGAAGCC
TTTAAGGTCTCTTGCTTCTCATGGTGTGGCTACAAGGAGCAGCAGCCATCGTGGCAGGC
TTGTGATCTTTTTCCTGCTGACACCTGCTGCTTGACATGGAGAAGTTCTGCACAGAAAGC
AGTGGCATCCTTCATGAGGTGGTACTTGGGGCAGACACTGAGAGCATTGTAATCGTCTTT
TGTATCAATCTCTCTAAAGTAGACCACCACGTATTTGTGCAGATGAATCTGGCTTCTTAG
ATCACTGCAGAAAAGGTTAAAGGCAAGGGGGAAGAGGTCTTGAGAGTTCTCACTGGGACT
GCCCTCGCTCTTGCCACAGGTACCATCGCACACACTG
AA721647 
TTTTTTTTTTACAACTTCAAAGCTGTTTATACATAGAAATCAATTACAGTTTTAATTGA
AAACTATAACCATTTTGATAATGCAACAATAAAGCATCTTCAGCCAAACATCTAGTCTTC
CATAGACCATGCATTGCAGTGTACCCAGAACTGTTTAGCTAATATTCTATGTTTAATTAA
TGAATACTAACTCTAAGAACCCCTCACTGATTCACTCAATAGCATCTTAAGTGAAAAACC
TTCTATTACATGCAAAAAATCATTGTTTTTAAGATAACAAAAGTAGGGAATAAACAAGCT
GAACCCACTTTTACTGGACCAAATGATCTATTATATGTGTAACCACTTGTATGATTTGGT
ATTTG
BF115018 
GTTTCGCTGAAGATGCTTTATTGTTGCATTATCAAAATGGTTATAGTTTTCAATTAAAAC
TGTAATTGATTTCTATGTATAAAACAGCTTTGAAGTTGTAAATGTAGTTTCCAATCGTTA
GTTAATGCTACATTAGTTAGCAATATTTGAAAATTTTATTGGTATAAAATGTTTTAATTA
CTAAGGCTGTTTGTAGGCTGCATAGTAAGCTTAAGGCCCATCACACGTTTTTTCCCTGTA
ATTGGTGGGATAGGAAGCCTTTAAGGTCTCTTGCTTNCATGGGTGGGCTACAAGGAGCA
GCAGCCATCGTGGCAGGCTTGNGATCTTTTTCCTGCTGGCCCCTGCTGCTTGACAT
W61238 
NAAAGCACTGGCTGAAGGAAGCCAAGAGGATCACTGCTGCTCCTTTTTTCTAGAGGAAAT
GTTTGTCTACGTGGTAAGATATGACCTAGCCCTTTTAGGTAAGCGAACTGGTATGTTAGT
AACGTGTACAAAGTTTAGGTTCAGACCCCGGGAGTCTTGGGCACGTGGGTCTCGGGTCAC
TGGTTTTGACTTTAGGGCTTTGTTACAGATGTGTGACCAAGGGGAAAATGTGCATGACAA
CACTAGAGGTATGGGCGACACGANAACGAACGGGAAGTTTTGGCTGAAGTAGGAGTCTTG
GTGAGATTTTGCTCTGATGCATGGTGTGAACTTTCTGAGCCTCTTGTTTTTCCTCAAGCT
GACTCCATATTTTCCTACTTGTGGCAGCGACTGCATCCGACATAAAGGAACAG
W61239 
TAGCAATATTTGAAAATTTTATTGGTATAAAATGTTTTAATTACTAAGGCTGTTTGTAGG
CTGCATAGTAAGCTTCAGGATCATCACACGTTTTTCCCTGTAATTGGTGGATAGGAAG
CCTTTAAGGTCTCTTGCTTCTCATGGGTGGGCTACAAGGAGCAGCAGCCATCGTGGCAGG
CTTGTGATCTTTTTCCTGCTGACACCTGCTGCTTGACATGGAGAAGTTCTGCACAGAAAG
CAGTGGCATCCTTCATGAGGTGGTACTTGGGGCAGACACTGAGAGCATTGTAATCGTCTT
TTGTATCAATCTCTCTAAAGTAGACCACCACGTATTTGTGCAGATGAATCTGGCTTCTTA
GATCACTGCAGAAAAGGTTAAAGGCAAGGGGGGA
AI032064 
AGCAATATTTGAAAATTTTATTGGTATAAAATGTTTTAATTACTAAGGCTGTTTGTAGGC
TGCATAGTAAGCTTCAGGATCATCACACGTTTTTCCCTGTAATTGGTGGCATAGGAAGC
CTTTAAGGTCTCTTGCTTCTCATGGGTGGGCTACAAGGAGCAGCAGCCATCGTGGCAGGC
TTGTGATCTTTTTCCTGCTGACACCTGCTGCTTGACATGGAGAAGTTCTGCACAGAAAGC
AGTGGCATCCTTCATGAGGTGGTACTTGGGGCAGACACTGAGAGCATTGTAATCGTCTTT
TGTATCAATCTCTCTAAAGTAGACCACCACGTATTTGTGCAGATGAATCTGGCTTCTTAG
ATCACTGCAGAAAAGGTTAAAGGCAAGGGGGAAGAGGTCTTGAGAGTTCTCACTGGGACT
GCCCTCGCTCTTGCCAC
AW236941 
TTTTTTTTTTTAGCAATATTTGAAAATTTTATTGGTATAAAATGTTTTAATTACTAAGGC
TGTTTGTAGGCTGCATAGTAAGCTTCAGGATCATCACACGTTTTTCCCTGTAATTGGTG
GGATAGGAAGCCTTTAAGGTCTCTTGCTTCTCATGGGTGGGCTACAAGGAGCAGCAGCCA
TCGTGGCAGGCTTGTGATCTTTTTCCTGCTGACACCTGCTGCTTGACATGGAGAAGTTCT
GCACAAAAAGCAGTGGCATCCTTCATGAGGTGGTACTTGGGGCAGACACTGAGAGCATTG
TAATCGTCTTTTGTATCAATC
AW236941 
TTTTTTTTTTTAGCAATATTTGAAAATTTATTGGTATAAAATGTTTTAATTACTAAGGC
TGTTTGTAGGCTGCATAGTAAGCTTCAGGATCATCACACGTTTTTCCCTGTAATTGGTG
GGATAGGAAGCCTTTAAGGTCTCTTGCTTCTCATGGGTGGCTACAAGGAGCAGCAGCCA
TCGTGGCAGGCTTGTGATCTTTTTCCTGCTGACACCTGCTGCTTGACATGGAGAAGTTCT
GCACAAAAAGCAGTGGCATCCTTCATGAGGTGGTACTTGGGGCAGACACTGAGAGCATTG
TAATCGTCTTTTGTATCAATC
BG057174 
TTTTATACATAGAAATCAATTACAGCTTTAATTGAAAACTATAACCATTTTGATAATGCA
ACAATAAAGCATCTTCAGCCAAACATCTAGTCTTCCATAGACCATGCATTGCAGTGTACC
 
CAGAACTGTTTAGCTAATATTCTATGTTTAATTAATGAATACTAACTCTAAGAACCCCTC
ACTGATTCACTCAATAGCATCTTAAGTGAAAAACCTTCTATTACATGCAAAAAATCATTG
TTTTTAAGATAACAAAAGTAGGGAATAAACAAGCTGAACCCACTTTTACTGGACCAAATG
ATCTATTATATGTG
AW058532 
GGCTGAAGATGCTTTATTGTTGCATTATCAAAATGGTTATAGTTTTCAATTAAAACTGTA
ATTGATTTCTATGTATAAAACAGCTTTGAAGTTGTAAATGTAGTTTCCAATCGTTAGTTA
ATGCTACATTAGTTAGCAATATTTGAAAATTTTATTGGTATAAAATGTTTTAATTACTAA
GGCTGTTTGTAGGCTGCATAGTAAGCTTCAGGATCATCACACGTTTTTTCCCCTGTATGG
GTGGGATAGGAAGCCTTTAAGGTCTCTTGCTTCTCATGGGTGGGCT
T98360 
TNAGGAANGAGAAGAAGCGAGATNNANNTNNAGAAATANGTGGTGGCNTANTTTAGAGAG
ATTGATNCAAAAGCNGATTNCAATNNNCTCAGTGNCTNCCCAAGTNCCNCCTCATGAAGG
ATNCACTNCTTTCTGTGCAGACTNNNCATGTCAAGCAGCAGGTGTCAGCAGGAAAAAGAN
CACAAGCTCCNCGATGGCTGCTGCTCCTTGTAGCCCNCCATGAGAAGCAAGAGNCTTAAA
GGCTTCCTATCCCACCAATTACAGGGAAAAACGTGTGATGACCTGAGCTTACTATGCAGC
CTACAANCAGCCTTAGTAATTAAACCNTTTATT
T98361 
NANNATGAAGATGCTTTATTGTTGCATTATCAAAATGGTTACAGTTTTCAATTAAAGCTG
TAATTGATTTCTATGTATAAAACAGCTTTGAAGTTGTAAATGTAGTTTCCAATCGTTAGT
TAATGCTACATTAGTTAGCAATATTTGAAAATTTTATTGGNATAAAATGTTTTAATTACT
AAGGCTGTTTGTAGGCTGCATAGTAAGCTTCAGGATCATCACACGTTTTTNCCCTGTAAT
TGGGTGGGGATAGGGAAGCCCTTTAAGGGTCTCTTGCTTCTCATGGGGTGGGGCCTACNA
AGGGAGCAGCCAGCCCATCGTGGCCAGGGCCTTGTGGANCCTTTTTCCCTGCCTGGACAC
CCTGCCTGCCTTGGACCATGGGGAGGAAGGTTCTGGCACCAGGAAAGCCAGGTGGCCCAT
CCCTTCCATGAGGGTGGGGTACTTNGGGGGGCCAGGACCACTGAGGNGCCATTGGTAATC
CGTCCTTTTNGTATCCAATCCCCTCCTAAGGTAGGNCCCCCC
AI470845 
TTTTGTGGGTTCAGCTTGTTTATTCCCTACTTTTGTTATCTTAAAAACAATGATTTTTTG
CATGTAATAGAAGGTTTTTCACTTAAGATGCTATTGAGTGAATCAGTGAGGGGTTCTTAG
AGTTAGTATTCATTAATTAAACATAGAATATTAGCTAAACAGTTCTGGGTACACTGCAAT
GCATGGTCTATGGAAGACTAGATGTTTGGCTGAAGATGCTTTTATTGTTGCATTATCAAN
ATGGTTTATAGTTTTCAATTAAAACTGTAATTGATTT
AI497731 
GGCTGAAGATGCTTTATTGTTGCATTATCAAAATGGTTATAGTTTTCAATTAAAACTGTA
ATTGATTTCTATGTATAAAACAGCTTTGAAGTTGTAAATGTAGTTTCCAATCGTTAGTTA
ATGCTACATTAGTTAGCAATATTTGAAAATTTTATTGGTATAAAATGTTTTAATTACTAA
GGCTGTTTGTAGGCTGCATAGTAAGCTTAANGATCATACNCACGTTTTTCCCTGAATTTG
GTGGGATAANGAAGCCTTTAAAGGT
T96629 
TTGAAAATTTATTGGNATAAAATGTTTTAATTACTAAGGCTGTTTGTAGGCTGCATAGT
AAGCTTCAGGANCATCACACGTTTTTCCCTGTAATTGGTGGCATAGGAAGCCTTTAAGG
TCTCTTGCTTCTCATGGGTGGCTACAAGGAGCAGCAGCCATCGTGGCAGGCTTGTGATC
TTTTTCCTGCTGACACCTGCTGCTTGACATGGAGAAGTTCTGCACAGAAAGCAGTGGCAT
CCTTCATGAGGTGGTACTTGGGGCAGACACTGAGAGCATTGTAATCGTCTTTTGTATCAA
TCTCTCTAAAGTAGACCACCACCGTNTTTGTGCAGATGGANTCTGGCTTC
T96740 
AGGCACTATCATCGGGTTTTCTCAGGTGTTTGAGCCACACCAGAAGAAACAAACGCGAGC
TTCAGTGGTGATTCCAGTGACTGGGATAGTGAAGGTGCTACGGTGCAGCTGACTCCATA
TTTTCCTACTTGTGGCAGCGACTGCATCCGACATAAAGGAACAGTTGTGCTCTGCCCACA
AACAGGCGTCCCTTTCCCTCTGGATAACAACAAAAGCAAGCCGGGANGGNCTGNCCTCTC
CTCCTGCTGTCTCTGCTGGTGGCCACATGGGTGCTGGTGGCAGGGATCTATCTAATGTGG
AGGCACGAAAGGATCAAGAAGACTTCCTTTTCTAACCACCACATTACTGCCCCCCATTTA
AGGTTCTTGTGGTTTTACCCATCTGGAAATATGTTTTCCCTTCACACATTTGTTTATTTC
ATTGATTTNTTTCAAAACCTTGGCAGGAGTTT
H25975 
GGGTCCAGTGCAGTGGCTTGCNTGCAGAAAGAAGGCAGCAGACAAAGTCGTCTTCCTTCT
TTCCAATGACGTCAACAGTGTGTGCGATGGTACCTGTGGCAAGAGCGAGGGCAGTCCCAG
TGAGAACTCTCAAGACCTCTTCCCCCTTGCCTTTAACCTTTTCTGCAGTGATCTAAGAAG
CCAGATTCATCTGCACAAATACGTGGTGGTCTACTTTAGAGAGATTGATACAAAAGACGA
TTACAATGCTCTCAGTGTCTGCCCCAAGTACCACCTCATGAAGGATGCCACTGCTTTCTG
TGCAGAACTTCTCCATGTCAAGCAGCAGGTGTCAGCAGGAAAAAGATTCACAAGCCTGCC
ACGATGGCTGCTTGCTTCCTTTGTAGCCCACCCATGAGGAAGNCAAGAGACCTTNAAAGG
GTTCCTTTTCCCATCANTTTACAGGGGANAAAACGTGTGATGATC
H25941 
TTTTGTTTGGCTNATNTNNTTCTTATTGTTGCATTATCAAAATGGTTATAGTTTTCAATT
AAAACTGTAATTGATTNCTATGTATAAAACAGCTTTGAAGTTGTAAATGTAGTTTCCAAT
CGTTAGTTAATGCTACATTAGTTAGCAATATTTGAAAATTTTATTGGTATAAAANGTTTT
AATTACTAAGGCTGTTTGTAGGCTGCATAGTAAGCTTCAGGATCATCACACGTTTTTCCC
CTGTAATTGGTGGGATAGGAAGCCTTTAAGGTCTCTNGCTTCTCATGGGTGGGCTACAAG
GAGCAGCAGCCATCGTGGCAGGCTTGTGANCTTTTNCCTGCTGACACCTGCTGCTTGACA
TGGGAGAAGTTCTGCACAGAAAGGCAGTGGGCATCCTTCATGAGGTGGGTACTTGGGGGN
CAGACACTGAGGAGCATTGT
BE539514 
ACTCAAAAGAAGGCAGCAGACAAAGTCGTCTTCCTTCTTTCCAATGACGTCAACAGTGTG
TGCGATGGTACCTGTGGCAAGAGCGAGGGCAGTCCCAGTGAGAACTCTCAAGACCTCTTC
CCCCTTGCCTTTAACCTTTTCTGCAGTGATCTAAGAAGCCAGATTCATCTGCACAAATAC
GTGGTGGTCTACTTTAGAGAGATTGATACAAAAGACGATTACAGTGCTCTCAGTGTCTGC
CCCAAGTACCACCTCATGAAGGATGCCACTGCTTTCTGTGCAGAACTTCTCCATGTCAAG
CAGCAGGTGTCAGCAGGAAAAAGATCACAAGCCTGCCACGATGGCCGCTGCTCCTTGTAG
CCCACCCATGAGAAGCAAGAGACCTTAAAGGCTTCCTATCCCACCAATTACAGGAAAAA
ACGTGTGATGATCCTGAAGCTTACTATGCAGCCTACAAACAGCCTTAGTAATTAAAACAT
TTTATACCAATAAAATTTTCAAATATGCTAACTAATGTAGCATTAACTAACGATTGGAAA
CTACATTTACAACTTCAAAGCTGTTTTATACATAGAAATCAATTACAGCTTTAATTGAAA
ACTGTAACCATTTTGATAATGCAACAATAAAGCATCTTCAG
BX282554 
GTCCAGTGCAGTGGCTTGCCACTCAAAAGAAGGCAGCAGACAAAGTCGTCTTCCTTCTTT
CCAATGACGTCAACAGTGTGTGCGATGGTACCTGTGGCAAGAGCGAGGGCAGTCCCAGTG
AGAACTCTCAAGACCTCTTCCCCCTTGCCTTTAACCTTTTCTGCAGTGATCTAAGAAGCC
AGATTCATCTGCACAAATACGTGGTGGTCTACTTTAGAGAGATTGATACAAAAGACGATT
ACAGTGCTCTCAGTGTCTGCCCCAAGTACCACCTCATGAAGGATGCCACTGCTTTCTGTG
CAGAACTTCTCCATGTCAAGCAGCAGGTGTCAGCAGGAAAAAGATCACAAGCCTGCCACG
ATGGCCGCTGCTCCTTGTAGCCCACCCATGAGAAGCAAGAGACCTTAAAGGCTTCCTATC
CCACCAATTACAGGGGAAAAAACGTGTGATGATCCTGAAGCTTACTAT
R74038 
TATTGTTGCATTATCAAAATGGTTATAGTTTTCAATTAAAACTGTAATTGATTTCTATGT
ATAAAACAGCTTTGAAGTTGTAAATGTAGTTTCCAATCGTTAGTTAATGCTACATTAGTT
AGCAATATTTGAAAATTTTATTGGTATAAAATGTTTTAATTACTAAGGCTGTTTGTAGGC
TGCATAGTAAGCTTCAGGATCATCACACGTTTTTNCCCTGTAATTGGGTGGGGATAGGGA
AGCCTTTAAGGTCTCTTGCTTCTCATGGGGTGGGGCTACAAGGGAGGCAGGCAGCCATCG
TGGGCAGGGCTTGTGATCTTTTTCCCTGCTGACACCTGCTGCTTGACATGGGGGGAAGGT
TCTGGCACAGAAAGCAGTGGGCATCCTTCATGAGGGTGGTACTTGGGGGGCAGACACTGA
GGAGGCNTTGTAAATCGNCTTTTTNGTATCCAANCTCTNCTAAAGTAGGGNCCACCNCGT
TTTTTNTTGCAGGTGGATNCGGGGCTN
R74129 
GGGTCCAGTGCAGTGGCTTGCNTNCAAAAGAAGGCAGCAGACAAAGTCGTCTTCCTTCTT
TCCAATGACGTCAACAGTGTGTGCGATGGTACCTGTGGCAAGAGCGAGGGCAGTCCCAGT
GAGAACTCTCAAGACCTCTTCCCCCTTGCCTTTAACCTTTTCTGCAGTGATCTAAGAAGC
CAGATTCATCTGCACAAATACGTGGTGGTCTACTTTAGAGAGATTGATACAAAAGACGAT
TACAATGCTCTCAGTGTCTGCCCCAAGTACCACCTCATGAAGGATGCCACTGCTTTCTGT
GCAGAACTTCTCCATGTCAAGCAGCAGGTGTCAGCAGGAAAAAGATCACAAGCCTGCCAC
GATNGCTGCTGCTCCTTGTAGNCCACCCATGAGAAGCAAGTGACCTTTAAAGGNTTTCCT
ATTNCCACCNATTTACAGGG
BG433769 
GACTAGATGTTTGGCTGAAGATGCTTTATTGTTGCATTATCAAAATGGTTATAGTTTTCA
ATTAAAACTGTAATTGATTTCTATGTATAAAACAGCTTTGAAGTTGTAAATGTAGTTTCC
AATCGTTAGTTAATGCTACATTAGTTAGCAATATTTGAAAATTTTATTGGTATAAAATGT
TTTAATTACTAAGGCTGTTTGTAGGCTGCATAGTAAGCTTCAGGATCATCACACGTTTTT
TCCCTGTAATTGGTGGGATAGGAAGCCTTTAAGGTCTCTTGCTTCTCATGGGTGGGCTAC
AAGGAGCAGCAGCCATCGTGGCAGGCTTGTGATCTTTTTCCTGCTGACACCTGCTGCTTG
ACATGGAGAAGTTCTGCACAGAAAGCAGTGGCATCCTTCATGAGGTGGTACTTGGGGCAG
ACACTGAGAGCATTGTAATCGTCTTTTGTATCAATCTCTCTAAAGTAGACCACCACGTAT
TTGTGCAGATGAATCTGGCTTCTTAGATCACTGCAGAAAAGGTTAAAGGCAAGGGGGAAG
AGGTCTTGAGAGTTCTCACTGGGACTGCCCTCGCTCTTGCCACAGGTACCATCGCACACA
CTGTTGACGTCATTGGAAAAGAAGGAAGAC
BG530489 GAGTTCTCACTGGGACTGCCCTCGCTCTTGCCACAGGTACCATCGCACACACTGTTGACG 
TCATTGGAAAGAAGGAAGACGACCTTGTCTGCTACCTTCTTTTGAGTGGCAAGCCACTGC
ACTGGACCCATCTCTGCTATTTCTTTTTCTGCCACTTTTCAAGGATGACCTCACTTCTG
CAATGGTTTTGAAGAAATTCAGTGAAGTAACAAATTGTGTGATGGAAACATATTTCAGAT
GGGTAAACCACAAGAACCTTAATGGGGGCAGTAGTGTGGTGGTAGAAAAGGAAGTCTTC
TTGATCCTTTCTGTGAGAGGAGAAAAGCATTTGTTATCTGTGAATAGCAAACAGCAGGCT
TTCACTCTGTAAACCATCCCTGACAAATGATCCCTTGCTAGAGAATGTCAGCTGAGCACC
AAGGGCCTTGTTAGTGACAGCAAGGAAAAACATCCTGATGTTCCTTTTGAACACATCACC
TGAAACACACTGATGCTTAAACCTTAACTTTTTTTTTTTGGGGACATAGTCTCACTCTG
TCGCCCAGGCTGGAGTGCGTGGAGAGGACCTCGGAAAGACTGGCAAGCATCCGCATACA
AGGGAGTAACAGCACAATACTCCGTGAACTTCGGAGCCCTCCAAAGGAATACTCAAGGGC
GGGTAAAGGATGGCAAGGGTCGACGGAGAGCCCACGAGGAGAGCGGAAGGTAGAGAGGAG
ACAAGCATAAGACGCGAGAGGAACTCCAAGGCGGGGCCAAAGAGAGAAACCACGGTCACC
AACAGAAG
AA007528 
AGAAGCCAGATTCATCTGCACAAATACGTGGTGNTCTACTTTAGAGAGATTGATACAAAA
GACGATTACAATGCTCTCAGTGTCTGCCCCAAGTACCACCTCATGAAGGATGCCACTGCT
TTCTGTGCAGAACTTCTCCATGTCAAGCAGCAGGTGTCAGCAGGAAAAAGATCACAAGCC
TGCCACGATGGCTGCTGCTCCTTGTAGCCCACCCATGAGAAGCAAGAGACCTTAAAGGCT
TCCTATCCCACCAATTACAGGGNAAAAACNGTAGTGATNATCCCTGACAGCTTACTATGC
CAGCCNT
AA007529 
TTGGCTGAAGATGCTTTATTGTTGCATTATCAAAATCGGTTACAGTTTTCAATTAAAGCT
GTAATTNGATTTCTATGTATAAAACAGCTTTGAAGTTGTAAATGTAGTTTCCAATCGTTA
GTTAATGCTACATTAGTTAGCAATATTTGAAAATTTTATTGGTATAAAATGTTTTAATTA
CTAAGGCTGTTTGTAGGCTGCATAGTAAGCTTCAGGATCATCACACGTTTTTTCCCTGTA
ATTGGGTGGGATAGGAAGCCTTTAAGGTCTCTTGCTTCTCATTGGGTGGGCTACAAGGAG
CAGCAGCCATCCGTNGGCAAGGCTTTGTGGATNCT
BI260259 
GGAAGAGAAAGATCGTCCAGAGGTTCCATCGCACACACTGTATGACGTCATTGGAAATGA
AGGAAGACGACTTTGTCTGCTGGCTTCTTGTGAGTGGCAAGCCACTGCAGTGGACCCATC
TCTGCTATTTTCTTTATTCTGCCACTTTTCAAGGATGACCTCACTTCTGCAATGGTTTTG
AAGAAAGTTCAGTGAAGTAACAAATTGTGTGATGGAAACATATTTCAGATGGGTAAACCA
CAAGAACCTTAATGGGGGGCAGTAGTGTGGTGGTAGAAAAGGAAGTCTTCTTGATCCTTT
CTGTGAGAGGAGAAAAGCATTAGTTATCTGTGAACAGCAAACAGCAGGCATTTCACATCT
GTAAACCATCCCTGACAAATGATCCCTTGCTAGAGAATGTCAGCTGAGCACCAAGGGGCC
TTGTTAGTGACAGCAAGGACAAAACATCCTGATGTTCCTTTTGAACACATCAGCTGAAAC
ACACTGATGCTCTAAACCGTTAACTATTTATTAATGGGGGAACATAGGTCTCAACTCATG
TACGACCAGGCTGGAGTGCAGTGGGGTTGAACATCGACAGACATAGCAAACCACCGATCA
CTAGGGAAACAACGCACAGAACTCCAGACTTAAAACACC
AA287951 
ATTCGGCACCTGGGGGCAGACACTGAGAGCATTGTAATCGTCTTTTGTATCAATCTCTC
TAAAGTAGACCACCACGTATTTGTGCAGATGAATCTGGCTTCTTAGATCACTGCAGAAAA
GGTTAAAGGCAAGGGGGAAGAGGTCTTGAGAGTTCTCACTGGGACTGCCCTCGCTCTTGC
CACAGGTACCATCGCACACACTGTTGACGTCATTGGAAAGAAGGAAGACGACTTTGTCTG
CTGCCTTCTTTTGAGTGGCAAGCCACTGCACTGGACCCATCTCTGCTATTTTCTTTTTCT
 
GCCACTTTTCAAGGATGACCTCACTTCTGCAATGGTTTTGAAGAAATTCAGTGAAGTAAC
AAATNTGTGTGATGGAAACATATTTCAGATGGGTAAACCACAAGAACCTTAATGGGGGGC
AGTAGTGTGGTGGTAGAAAAGGAAGTCTTCTTGATCCTTTCTGTGAGAGGAGAAAGC
AA287911 
TTTTGATGGTCCACTTCCATTTAATGAATTAGTAAATATCTTTTCTCATGATTTTAATTA
CATTTTTTTCTCTAGCTTACTTTATTATAATACAGCACATAATACACCTAACATGCAAAA
TATGTGTTAATTGGCTGTTTATGTTATTGGTAAGACTTCCAGTCAACAGTAGGCTATTAG
AAGTTAAGTTGTGGAAAATCAAAGGTTATAGGAGATTTTCAACTGCATGCAGGGCCGGT
GCCCTCCCCACTGTGTTGTTCAAGGGTCAGCTGTACTCTCTAAGGGCTTTGCTAACTTCA
AAACATGGAGTATTTGAATACAGAAACCAGAGCATTTACATACTCAGCTCAAGGCAGAGC
TATTAAAAAAACTCCTCTTCTCCATATGTAGGAAAGGAAATACAAATGCATCCTTTGAGT
CATTTGTGATGT
T97852 
AACAGTTGTGCTCTGCCCACAAACAGGCGTCCCTTTCCCTCTGGATAACAACAAAAGCAA
GCCGGGANGNCTGNCGCTCTCCTCCTGCTGTCTCTGCTGGTGGCCACATGGGTGCTGGTN
GCAGGGATCTATCTAATGTNGAGGCACGAAAGGGATCAAGAGGACTTCCTTTTCTACCAC
CACACTACTGCCCCCCATTAAGGTTCTTGTNGGTTTACCCATCTGGAAATATGTTTCCAT
CACACAATTTGTTACTTCACTGGAATTTCTTCAAAACCATTGGCAGGANGTGAGGGTCAT
CCTTGGAAAAGTGGGC
T97745 
CCTCACTTCTGCAATGGTTTTGAAGAAATTCAGTGAAGTAACAAATTGTGTGATGGAAAC
ATATTTCAGATGGGTAAACCACAAGAACCTTAATGGGGGCAGTAGTGTGGTGGTAGAAA
AGGAAGTCTTCTTGATCCTTTCGTGCCTCCACATTAGATAGATCCCTGCCACCAGCACCC
ATGTGGCCACCAGCAGAGACAGCAGGAGGAGAGGCAGCCAGCCTCCCGGCTTTGCTTTTG
TTGTTATCCAGAGGGGAAAGGGGACGCCTGTTTNTGGGGCAGAGCACAACTGTTTCCCTC
GTGCCCGAATTCTTTGGGCCTTCGAGGGGCCAAATTTCCCTATTAGGTGAGGTCGTATTT
TAAATTTCGGTAATTCATGGTCATAGGCTTGTTTTTCCCCG
N40294 
GTTTCAACACAATTTTGGATCAGCTGCCTGTTTGCAAAAACATAATATATTTCTGTTAAA
CAGTTCTTCACCTAACAGCATATTGCTCTTATAACTGGTAGAGCTGTTTCAAAGGAAGTT
GGTTTCTGGTCCAAGTTTTGACCTAAACCATGTCCATCTTCTATTACCAGCACTTACAAG
CACTGTGAAAACTGATCATGACAAATAAGTAAAATTTGCTACATTAAACATATTGCCTCA
GCCATTACTAAGCGTCCACTTGTAAAGCTGGACACAGTTTTTACTTTATGCTTCATTTTG
ATTTTTTATCCGTAAGACATAAATTAGAAGGCATGAGGTGGCCCTTTAAGGATAATCTGC
AAATATACACATTTTAAATAGTCATCCATCTGGAAATCGNTCCACCATTCCAGGGGAAGG
ATTCCAGGTATTGGTGCTGTGGTGGAAATAAAGCATTCCCCNGGGAAAAAAACCATTTTA
TGNCTAAATAATTACCACCATTAACCTCNTGGGGTT
AA809841 
GAATACTAACTCTAAGAACCCCTCACTGATTCACTCAATAGCATCTTAAGTGAAAAACCT
TCTATTACATGCAAAAAATCATTGTTTTTAAGATAACAAAAGTAGGAATAAACAAGCTG
AACCCACTTTTACTGGACCAAATGATCTATTATATGTGTAACCACTTGTATGATTTGGGA
TTTGCAT
AA832389 
TTTTTTACAACTTCAAAGCTGTTTATACATAGAAATCAATTACAGTTTTAATTGAAAAC
TATAACCATTTTGATAATGCAACAATAAAGCATCTTCAGCCAAACATCTAGTCTTCCATA
GACCATGCATTGCAGTGTACCCAGAACTGTTTAGCT
H14692 
CTGAGTGTGATGGTGTAAGCCTGTGGTCCCAGCTACTAGGGAGGCTGAGATGGATTACA
GGTGTGAGCCACGGCGCCTGGCCTAAAAGCATCTTTTTCTTTAACGCAGAGGTTATGTTG
TATTATTAGCATAAATGTTTTTTTCTGGGAATGCTTATTTCACACAGCACAATACTGAAT
CTTCTCTGGAATGTGGATCGATTTCAGATGGATGACTATTAAAATGTGTATATTTGCAGA
TTATCCTTAAAGGGCCACCTCATGCCTTCTAATTTATGTCTTACGGATAAAAAATCAAAA
TGAAGCATAAAGTAAAAACTGTGTCCAGCTTTACAAGTGGACGCTTAGTAATGGCTGAGG
CAATATGTTTAATGTAGCCAAATTTTACTTATTTGTCCATGATCCAGTTTTTCACAGTGC
TTGTTAAGTGCTGGTAATTAGGAAGGTGGGACATGGGTTAGGTCAAAACTTGGGACCNGA
AACCAACTTGN
AA732635 
TTTTTTTTTACAACTTCAAAGCTGTTTATACATAGAAATCAATTACAGTTTAATTGA
AAACTATAACCATTTTGATAATGCAACAATAAAGCATCTTCAGCCAAACATCTAGTCTTC
CATAGACCATGCATTGCATTGTACCCAGAACTGTTTAGCTAATATTCTATGTTTAATTAA
TGAATACTAACTCTAAGAACCCCTCACTGATTCACTCAATAGCATCTTAAGTGAAAAACC
TTCTATTACATGCAAAAAATCATTGGTTTT
AA928257 
TTTTCTGAGTAAGAACAGGCTTTATTTGTAAAACCACTCGTGACTCTTTACAAAGCAGGA
TACACAGAAGGAAAAAAATACACAGTGCAAAATGGATGTTCTGAGTGCCACAAGGATCT
GCTGAAAAAAGCCAAAGATGTAAGATGGCTGGGTATATATGAGAATGAATATTTCACTAT
ATTCTGATTCAATTACCAGTCTCAGTGGCCCAGGATGAGCTTTTGGTGTGGTCACATGGC
CAACATTTGGATAACAAATGAGGAATAATGGTACCGCCTCACTAGTGCCTGAGAACAGCA
TGTTCTGGAAAATGTCTCTGGAGTTAGAGATGTGTTAGCTTTTTCATTACAGATGGAGAA
ATACAATGTTTACACAACAGTCCAGGGGTGGGGTCAAAAGTTGGAAGGTGTCATTAGACG
CAGCCAAATAAAGTGAAGACAACCCAGGTGACTGGCAGCCCTGACTTGTGCGTGGGCG
AI184427 
TTTCTGAGTAAGAACAGGCTTTATTTGTAAAACCACTCGTGACTCTTTACAAAGCAGGAT
ACACAGAAGGAAAAAAATACACAGTGCAAAATGGATGTTCTGAGTGCCACAAGGATCTG
CTGAAAAAAGCCAAAGATGTAAGATGGCTGGGTATATATGAGAATGAATATTTCACTATA
TTCTGATTCAATTACCAGTCTCAGTGGCCCAGGATGAGCTTTGGTGGTGGTCACATGGCC
AACATTTGGATAACAAATGAGGA
AI298577 
GAGATGGAGGTCTCGCTTTGTGACGTAGCCTGGTCTTGAGCGATCCTTTTGCCTTGGCCT
TGCCAAAGTGCTGGGATTGGAGGCATGAGCCACTGCACCCACCCCTGTTTTTTTTTAAG
TAAACCATTATAATAACTCATTTATAAAAAGGTTACTTCAAGAGGGCTTTCAACTTAAGA
ATTATTTTCATTTTGAACATGAAAAGTTAAATAGTAACTAAGAAACTGAGAACTCTGACA
 
GTGACCTCTAATAGGTAACTTTAGGCAAAAGTAGACAAGTTTGTGGGTATTTTGTTGTTC
ATGTTAAAAGGCACCTGTACAAGAATCAAGATATGAATCTAGTTTGTAGAGGGAAGGTCT
TATGCAAATACCAAATCATACAAGTGGT
AI692717 
AGAGATGTTGGTCTCGCTTTGTGACGTAGCCTGGGCTTGAGCGATCCTTTTGCCTTGGCC
TTGCCAAAGTGCTGGGATTGGAGGCATGAGCCACTGCACCCACCCCTGTTTTTTTTTAA
GTAAACCATTATAATAACTCATTTATAAAAGGTTACTTCAAGAGGGCTTTCAACTTAAG
AATTATTTTCATTTTGAACATGAAAAGTTAAATAGTAACTAAGAAACTGAGAACTCTGAC
AGTGACCTCTAATAGGTAACTTTAGGCAAAAGTAGACAAGTTTGTGGGTATTTTGTTGTT
CATGTTAAAAGGCACCTGTACAAGAATCAAGATATGAATCTAGTTTGTAGAGGGAAGGTC
TTATGCAAATACCAAATCATACAAGTGGTTACACATATAATAGATCATTTGGTCCAGTAA
AAGTGGGTTCAGCTTGTTTATTCCCTACTT
AA910922 
GAGATGGAGGTCTCGCTTTGTGACGTAGCCTGGTCTTGAGCGATCCTTTTGCCTTGGCTT
GCAAAGTGCTGGGATTGGAGGCATGAGCACTGCACCCACCCCTGTTTTTTTTTTTAAGTA
AACCATTATAATAACTCATTTATAAAAAGGTTACTTCAAGAG
H90761 
TTCACTCAATAGCATCTTAAGTGAAAAACCTTCTATTACATGCAAAAAATCATTGTTTTT
AAGATAACAAAAGTAGGAATAAACAAGCTGAACCCACTTTTACTGGACCAAATGANCTA
TTATATGTATAACCACTTGTATGATTTGGTATTTGCATAAGACCTTCCCTCTACAAACTA
GATTCATATCTTGATTCTTGTACAGGTGCCTTTTTAATATTCTGTGATGAAATCGTTCAC
AGTCAGAGTACATGTCTGCTGCATATGGGAAATAGGGACTGTTGTTCTGAGGGACAAGGC
ACTCAATTCAGCCGTAAAGGCTGACCCGGGCTACTTTTTTTCCANGGGAATACAATTTTT
TTACCTTGGAATAAAATNGGGCCCGACNGGAC
AI620122 
TTTTTTTTTTTGAGTAAGAACAGGCTTTATTGTAAAACCACTCGTGACTCTTTACAAAG
CAGGATACACAGAAGGAAAAAAATACACAGTGCAAAATGGATGTTCTGAGTGCCACAAG
GATCTGCTGAAAAAAAGCCAAAGATGTAAGATGGCTGGGTATATATGAGAATGAATATTT
CACTATATTCTGATTCAATTACCAGTCTCAGTGGCCCAGGATGAGCTTTTGGTGTGGTCA
CATGGCCAACATTTGGATAACAAATGAGGAATAATGGTACCGCCTCACTAGTGCCTGAGA
ACAGCATGTTCTGGAAAATGTCTCTGGAGTTAGAGATGTGTTAGCTTTTTCATTACAGAT
GGAGAAATACAATGTTTACACAACAGTCCAGGGGTGGGGTCAAAAGTTGGAAGGTGTCAT
TAGACGCA
AI793318 
AAATTTTTAACTTTTAATAGTTAAAATAGTTAACTATTGGTATGGTAGGAAATGATAAAG
TAGACTAGTATCTGTATACATTTTCTGCATTTATGACATACCTTTTTCTTCATTTTTTTC
AATATTTTAATTGAAAAGTTCATCCGAGTTTCATCTAAGTTTTTTCAAAGTGATACAAAT
CTCCAAAAAATTTTCCAATATATGTATTGAAAAAATCCAGGTGTAAGTGGCTCTGCGCAG
TCCAAACCTGTGTTGTTCAAGGGTCAACTGTGTATGAATCCAAGCGAAAGCTTTTCTTAA
CACCTCATAAGAACTATTTTTTAAAAAACAGGAACTAGCATAGAGTAACCATCACAGGTA
AAGTGTAATTTGTTATCAGCCATCTTTTGCCCATTTCAGTACTGGTAGAAGGCTCAATGG
TAAAAATAAA
AA962325 
TTTTTTTTTTTTTTTTTTTTTTTCTGACTGTCCCGTTTTTATTTTTACCATTGAGCCTT
CTACCAGTACTGAAATGGGCAAAAGATGGCTGATAACAAATTACACTTTACCTGTGATGG
TTACTCTATGCTAGTTCCTGTTTTTTAAAAAATAGTTCTTATGAGGTGTTAAGAAAAGCT
TTCGCTTGGATTCATACACAGTTGACCCTTGAACAACACAGGTTTGGACTGCGCAGACCA
CTTACACCTGGATTTTTTCAATACATATATTGGAAAATTTTTTGGGGATTTGTATCACTT
TGAAAAAACTTAGATGAAACTCGGATGGACTTTTCCATTAAAATATTGGAAAAAATGAAG
AAAAAGGT
AI733290 
TTTTTTTTTTTTTTTTTTTTTTTTCTGACTGGCCCGTTTTATTTTTACCATTGAGCCTT
CTACCAGTACTGAAATGGGCAAAAGATGGCTGATAACAAATTACACTTTACCTGGGATGG
TTACTCTATGCTAGTTCCTGTTTTTAAAAAATAGTTCTTATGAGGGGTTAAAAAAAGCT
TTCGCTTGGATTCATACACAGTTGACCCTTGAACAACACAGGTTTGGACTGCGCAGAGCC
ACTTACACCTGGATTTTTTCAATACATATATTGGAAAATTTTTTGGAGATTTGTATCACT
TTGAAAAAACTTAGATGAAACTCGGATGAACTTTTCAATTAAAATATTGAAAAAAATGAA
GAAAAAGGTATGTCATAAATGCAGAAAATGTATACAGATACTAGTCTACTTTATCATTTC
CTACCATACCAATAG
BQ226353 
TAAAGGAACAGTTGTGCTCTGCCCACAAACAGGCGTCCCTTTCCCTCTGGATAACAGTAA
GTGCCCAGTAACTTCAACCAGATGATCAAAGTGGCTCACACACAGTCACTGCCCCCCACT
CAGTATGTGGAAGGGTTGTGTGTATGTGGGCAGTGCAAGGGGTCGCTGCCTGTGTACACT
GAACTGGGGTGCAGAGAAAGCCAACAGTGCTGTCCCAGAGAACCTAGAATCTGAGTAAGA
ACAGGCTTTATTTGTAAAACCACTCGTGACTCTTTACAAAGCAGGATACACAGAAGGGAA
AAAAATACACAGTGCAAAATGGATGTTCTGAGTGCCACAAGGATCTGCTGAAAAAAGCCA
AAGATGTAAGATGGCTGGGTATATATGAGAATGAATATTTCACTATATTCTGATTCAATT
ACCAGTCTCAGTGGCCCAGGATGAGCTTTTGGTGTGGTCACATGGCCAACATTTGGATAA
CAAATGAGGAATAATGGTACCGCCTCACTAGTGCCTGAGAACAGCATGTTCTGGAAAATG
TCTCTGGAGTTAGAGATGTGTTAGCTTTTTCATTACAGATGGAGAAATACAATGTTTACA
CAACAGTCCAGGGGTGGGGGTCAAAAGTTGGAAGGTGTCATTAGACGCAGCCAAATAAAG
TGAAGACCACCCAGGTGACTGGCAGCCCTGACTTGTGCGTGGGCGAAACCTTACAGATTC
CTGGGGCACTCTGTGCCTGAACTTACCTGGATGGTCTTTGTGAGGCGGGTGGGCACTTAT
CCTCCATNAATGGTCAGTCTAACAAGACCGGCCTGTAAAAATGGCATCTAATAGGGGCTA
TGGAATGGAAAACAGTTGGTACCCAGAAATAACTTTAATT
W04890 
GACAGTCTGGGAGCCCAGAGCTCTGGGAGGAGTNGGGAAAATGCTGCTTCCTGCTGCTTG
CTTCTAGGCACCTGCTTCCGCCATCTCACTTACCATGGCTAGAGATGGGGGTGAGACTGG
GGAAGGACAAAAGCAGGGAACAGATAAGGGATGGAAATCAGAAGGGAATATAGAAAGAAC
TCTGGATATGCNAGAAATGCCGGTACCTGAGCATTTTGTATCAATGGGAGTACCCTCTGT
AACTGCTCAGTAGGTTACAAATGAAGAGTCCACCAGTATTAGAAACAATTTAAACTTGCC
AGTACCAACTGGGATGTGTGCCTTCAATTTGAAAATTGTATGTTTTATTTTTTAAATTTG
GTTAACAGCATTAATTTATAGAGTATTTGATGTCATTTATGGTTCCCGAGGTGTTTCCAA
CACAATTTTTGGGATCA
BM455231 
CTTTTAATAGTTAAAATAGTTAACTATTGGTATGGTAGGAAATGATAAAGTAGACTAGTA
TCTGTATACATTTTCTGCATTTATGACATACCTTTTTCTTCATTTTTTTCAATATTTTAA
TTGAAAAGTTCATCCGAGTTTCATCTAAGTTTTTCAAAGTGATACAAATCTCCAAAAAA
TTTTCCAATATATGTATTGAAAAAATCCAGGTGTAAGTGGCTCTGCGCAGTCCAAACCTG
TGTTGTTCAAGGGTCAACTGTGTATGAATCCAAGCGAAAGCTTTTCTTAACACCTCATAA
GAACTATTTTTTAAAAAACAGGAACTAGCATAGAGTAACCATCACAGGTAAAGTGTAATT
TGTTATCAGCCATCTTTTGCCCATTTCAGTACTGGTAGAAGGCTCAATGGTAAAAATAAA
AACGGACAGTCAGAAGATCTGGAAGTCCTGACCCTGCTTTCACCTGGCATGTGTAATCC
AGTCATGCTCGTATCAGTCTCTGTAGGAGCACTTGAAGGTATTACATAAATGCTATCTAA
CTCTGGGAAACGCCAACATGTGATTGCCTCCAGAGGAATCTTCTTTAAAAAAAAATTCAA
AATGTTATTTCCTTACTAGGATGTCTTTAAAGAATTATAACCCTTACCGTGCCTCCACAT
TAGATAGATCCCTGCCACCAGCACCCATGTGGCCACCAGCAGAGACAGCAGGAGGAGAGG
CAGCCAGCCTCCCGGCTTGCTTTTGTCTGGAAAAAAACAAAGCTTATTCACCTTTGGAAA
AAAATCCACACTTATCTCTTAATTTAAAAACTAAGACTTGGTATACTTTATAGAGGGTTA
TTTATTTTTTATTATTTTTTAGTTTTGAGACAGAGTCTCGCTTTGTTGCCTANGCTGGAG
TGCAGTGGCGCAATCTCGGTTCACTGCAGCCTCCGTTCTCCGGGTTCAAGGCATGCTGG
CTCAGCCTCCTGTATAGCTGGGATTAAAGGCATGTGTTCACGCGGCCCAGCCCCTTTTG
TAAAAGATTTAGATCCCTTTTAAAACCATCAGTCAGGAGGCTCCTTTAAAAAGTCTGGCC
ATCTAATCTTTTTTCCCCCAAAAGGGG
BI492426 
TTTTTTTTTTCTTTTTTCTGAGTAAGAACAGGCTTTATTTGTAAAACCACTCGTGACTC
TTTACAAAGCAGGATACACAGAAGGAAAAAAATACACAGTGCAAAATGGATGTTCTGAG
TGCCACAAGGATCTGCTGAAAAAAGCCAAAGATGTAAGATGGCTGGGTATATATGAGAAT
GAATATTTCACTATATTCTGATTCAATTACCAGTCTCAGTGGCCCAGGATGAGCTTTTGG
TGTGGTCACATGGCCAACATTTGGATAACAAATGAGGAATAATCTCGTGC
BG674622 
AATTTATAGAGTATTGATGTCATTTATGTTTCTGAGGTGTTTCAACACAATTTTGGATCA
GCTGCCTGTTTGCAAAAACATAATATATTTCTGTTAAACAGTTCTTCACCTAACAGCATA
TTGCTCTTATAACTGGTAGAGCTGTTTCAAAGGAAGTTGGTTTCTGGTCCAAGTTTTGAC
CTAAACCATGTCCATCTTCTATTACCAGCACTTACAAGCACTGTGAAAACTGATCATGAC
AAATAAGTAAAATTTGCTACATTAAACATATTGCCTCAGCCATTACTAAGCGTCCACTTG
TAAAGCTGGACACAGTTTTTACTTTATGCTTCATTTTGATTTTTTATCCGTAAGACATAA
ATTAGAAGGCATGAGGTGGCCCTTTAAGGATAATCTGCAAATATACACATTTTAATAGTC
ATCCATCTGAAATCGATCCACATTCCAGAGAAGATTCAGTATTGTGCTGTGTGAAATAAG
CATTCCCAGAAAAAAAACATTTATGCTAATAATACAACATAACCTCTGCATTAAAGAAAA
AGATGCTTTTAGGCCAGGCGCCGTGGCTCACGCCTGTAATCCCTGCACTTTGAGAGGCTG
AGGTGGGTGGATCATGAGGTCAGGAGATCAAGACCATCCTGGCTAACAGGGTGAAACCCC
GTCTCTACTGGGGATATAACAAAGTTAGCTGGGTGTGGTGGTGGGTGCTTGTGGTCCCAG
CTACTCAGGAGGCTGAGGCAGGAGAATGGCGTGAACCCGGAAGGCAGAGGTTGTAGTGAC
GCGAGGTTCACGCCACTGCATTCCAGTCTGGG
BX111256 
CAGGAAGNTAAGAACAGTCCTAAAATCTCTTTGGCTTCTTTGTCCTGATATGCACCGGCA
TTTTCACAGTAGGAACTAGGGTTTCTGTCCAGTTTTTTGGTTCTTTAAGGAATTAATGT
TATTCTGGGTACAACTGCTTACATACATAGCACATATAGATGACATTTTTACAGGCCGTC
TTGTTAGACTGACATACATGGAGGATAGTGCCACCCGCCTCACAAGAACATCAGGTAAGC
TCAGGCACAGAGTGCCCAGGAATCTGTAAGGCTTCGCCCACGCACAAGTCAGGGCTGCCA
GTCACCTGGGTTGTCTTCACTTTATTTGGCTGCGTCTAATGACACCTTCCAACTTTTGAC
 
CCCACCCCTGGACTGTTGTGTAAACATTGTATTTCTCCATCTGTAATGAAAAAGCTAACA
CATCTCTAACTCCAGAGACATTTTCCAGAACATGCTGTTCTCAGGCACTAGTGAGGCGGT
ACCATTATTCCTCATTTGTTATCCAAATGTTGGCCATGTGACCACACCAAAAGCTCATCC
TGGGCCACTGAGACTGGTAATTGAATCAGAATATAGTGAAATATTCATTCTCATATATAC
CCAGCCATCTTACATCTTTGGCTTTTTTCAGCAGATCCTTGTGGCACTCAGAACATCCAT
TTTGCACTGTGTATTTTTTT
BX117618 
AAATTTTTAACTTTTAATAGTTAAAATAGTTAACTATTGGTATGGTAGGAAATGATAAAG
TAGACTAGTATCTGTATACATTTTCTGCATTTATGACATACCTTTTTCTTCATTTTTTTC
AATATTTTAATTGAAAAGTTCATCCGAGTTTCATCTAAGTTTTTTCAAAGTGATACAAAT
CTCCAAAAAAATTTTCCAATATATGTATTGAAAAAATCCAGGTGTAAGTGGCTCTGCGCAG
TCCAAACCTGTGTTGTTCAAGGGTCAACTGTGTATGAATCCAAGCGAAAGCTTTTCTTAA
CACCTCATAAGAACTATTTTTTAAAAAACAGGAACTAGCATAGAGTAACCATCACAGGTA
AAGTGTAATTTGTTATCAGCCATCTTTTGCCCATTTCAGTACTGGTAGAAGGCTCAATGG
TAAAAATAAAAACGGGACAGTCAGAAAAA
AA682806 
TCTGAGTAAGAACAGGCTTTATTGTAAAACCACTCGTGACTCTTTACAAAGCAGGATAC
ACAGAAGGAAAAAAATACACAGTGCAAAATGGATGTTCTGAGTGCCACAAGGATCTGCT
GAAAAAAGCCAAAGATGTAAGATGGCTGGGTATATATGAGAATGAATATTTCACTATATT
CTGATTCAATTACCAGTCTCAGTGGCCCAGGATGAGCTTTTGGTGTGGTCACATGGCCAA
CATTTGGATAACAAATGAGGAATAATGGTACCGCCTCACTAGTGCCTGAGAACAGCATGT
TCTGGAAAATGTCTCTGGAGTTAGAGATGTGTTAGCTTTTTCATTACAGATGGAGAAATA
CAATGTTTACACAACAGTCCAGGGGTGGGGTCAAAG
AI202376 
CTGACTGTCCCGTTTTTATTTTTACCATTGAGCCTTCTACCAGTACTGAAATGGGCAAAA
GATGGCTGATAACAAATTACACTTTACCTGTGATGGTTACTCTATGCTAGTTCCTGTTTT
TTAAAAAATAGTTCTTATGAGGTGTTAAGAAAAGCTTTCGCTTGGATTCATACACAGTTG
ACCCTTGAACAACACAGGTTTGGACTGCGCAGAGCCACCCTCGTGCCGAATT
AI658949 
CTGACTGTCCCGTTTTTATTTTTACCATTGAGCCTTCTACCAGTACTGAAATGGGCAAAA
GATGGCTGATAACAAATTACACTTTACCTGTGATGGTTACTCTATGCTAGTTCCTGTTTT
TTAAAAAATAGTTCTTATGAGGTGTTAAGAAAAGCTTTCGCTTGGATTCATACACAGTTG
ACCCT
BG403405 
GGAAATGATAAAGTAGACTAGTATCTGTATACATTTTCTGCATTTATGACATACCTTTTT
CTTCATTTTTTTCAATATTTAATTGAAAAGTTCATCCGAGTTTCATCTAAGTTTTTTCA
AAGTGATACAAATCTCCAAAAAATTTCCAATATATGTATTGAAAAAATCCAGGTGTAAG
TGGCTCTGCGCAGTCCAAACCTGTGTTGTTCAAGGGTCAACTGTGTATGAATCCAAGCGA
AAGCTTTTCTTAACACCTCATAAGAACTATTTTTTAAAAAACAGGAACTAGCATAGAGTA
ACCATCACAGGTAAAGTGTAATTTGTTATCAGCCATCTTTGCCCATTTCAGTACTGGTAG
AAGGCTCAATGGTAAAAATAAAAACGGGACAGTCAGAAGATCTGGAAGTCCTGACCCTGC
TTTCACCTGGCATGTGTAATCCAGTCATGCTCGTATCAGTCTCTGTAGGAGCACTTGAAG
 
GTATTACATAAATGCTATCTAACTCTGGAAACGCCAACATGTGATTGCCTCCAGAGGAA
TCTTCTTTAAAAAAAAATTCAAAATGTTATTTCCTTACTAGGATGTCTTTAAAGAATTAT
AACCCTTACCGTGCCTCCACATTAGATAGATCCCTGCAACAGACCCATGTGGCACCAGCA
GAGACAGCAGGAGGAGAGGCAGCAGCTCCCGGTTGTTTGTCTGGAAAAACAAAGGTTATC
ACTTTG
BE673417 
CTGACTGTCCCGTTTTTATTTTTACCATTGAGCCTTCTACCAGTACTGAAATGGGCAAAA
GATGGCTGATAACAAATTACACTTTACCTGTGATGGTTACTCTATGCTAGTTCCTGTTTT
TTAAAAAATAGTTCTTATGAGGTGTTAAGAAAAGCTTTCGCTTGGATTCATACACAGTTG
ACCCT
AW021469 
GCACGAGATTATTCCTCATTTGTTATCCAAATGTTGGCCATGTGACCACACCAAAAGCTC
ATCCTGGGCCACTGAGACTGGTAATTGAATCAGAATATAGTGAAATATTCATTCTCATAT
ATACCCAGCCATCTTACATCTTTGGCTTTTTTCAGCAGATCCTTGTGGCACTCAGAACAT
CCATTTTGCACTGTGTATTTTTTTCCCTTCTGTGTATCCTGCTTTGTAAAGAGTCACGAG
TGGTTTTACAAATAAAGCCTGTTCTTACTCAGAAAAAAAAAAAAAAAAAA
CF455736 
NNTTGAACAGGCGTGACGGTCCGGATTCCCGGATGTTGTGCTCTGCCCACAAACAGGCG
TCCCTTTCCCTCTGGATAACAACAAAGCAAGCCGGAGGCTGGCTGCCTCTCCTCCTGC
TGTCTCTGCTGGTGGCCACATGGGTGCTGGTGGCAGGGATCTATCTAATGTGGAGGCACG
AAAGGATCAAGAAGACTTCCTTTTCTACCACCACTACTGCCCCCCATTAAGGTTCTTG
TGGTTTACCCATCTGAAATATGTTTCCATCACACAATTTGTTACTTCACTGAATTTCTTC
AAAACCATTGCAGAAGTGAGGTCATCCTTGAAAAGTGGCAGAAAAAGAAAATAGCAGAGA
TGGGTCCAGTGCAGTGGCTTGCCACTCAAAAGAAGGCAGCAGACAAAGTCGTCTTCCTTC
TTTCCAATGACGTCAACAGTGTGTGCGATGGTACCTGTGGCAAGAGCGAGGGCAGTCCCA
GTGAGAACTCTCAAGACCTCTTCCCCCTTGCCTTTAACCTTTTCTGCAGTGATCTAAGAA
GCCAGATTCATCTGCACAAATACGTGGTGGTCTACTTTAGAGAGATTGATACAAAAGACG
ATTACAATGCTCTCAGTGTCTGCCCCAAGTACCACCTCATGAAGGATGCCACTGCTTTCT
GTGCAGAACTTCTCCATGTCAAGCAGCAGGTGTCAGCAGGAAAAAGATCACAAGCCTGCC
ACGATGGCTGCTGCTCCTTGTAGCCCACCCATGAGAAGCAAGAGACCTTNAAGGCTTCCT
ATCCCACCATTACAG
AW339874 
TTTTTTTTTTTTTCTGAGTAAGAACAGGCTTTATTTGTAAAACCACTCGTGACTCTTTAC
AAAGCAGGATACACAGAAGGAAAAAAATACACAGGGCAAAATGGATGTTCTGAGTGCCA
CAAGGATCTGCTGAAAAAAGCCAAAGATGTAAGATGGCTGGGTATATATGAGAATGAATA
TTTCACTATATTCTGATTCAATTACCAGTCTCAGTGGCCCAGGATGAGCTTTTGGTGTGG
TCACATGGCCAACATTTGGATAACAAATGAGGAATAATGGTACCGCCTCACTAGTGCCTG
AGAACAGCATGTTCTGGAAAATGTCTCTGGAGTTAGAGATGTGTTAGCTTTTTCATTACA
GATGGAGAAATACAATGTTTACACAAC
BG399724 
CATGATGTTCAGTATGATCAGTTAACCTTAACCTCTGAGCATCCTGAAGCAAAATCTAAA
TAATGCAGCTATTACCACTGGTGGTCCAGGCTCTGGTGAAGCCCTCTGAGCCCAGGAGGA
 
AGAGAAAGCATTGTCCAGAGGTAGGAACACAGTCTGGGAGCCCAGAGCTCTGGGAGGAGT
GGGAAAATGCTGCTTCCTGCTGCTTGCTTCTAGGCACCTGCTTCCGCCATCTCACTTACC
ATGGCTAGAGATGGGGGTGAGACTGGGAAGGACAAAAGCAGGAACAGATAAGGGATGG
AAATCAGAAGGGAATATAGAAAGAACTCTGGATGTGGAGAAATGCCGGTACCTGAGCATT
TTGTATCAATGGGAGTACCCTCTGTAACTGCTCAGTAGGTTACAAATGAAGAGTCCACCA
GTATTAGAAACAATTTAAACTTGCCAGTACCAACTGGGATGTGTGCCTTCAATTTGAAAA
TTGTATGTTTTATTTTTTAAATTTGTTAACAGCATTAATTTATAGAGTATTGATGTCATT
TATGTTTCTGAGGTGTTTCAA
BF475787 
TCTGAGTAAGAACAGGCTTTATTTGTAAAACCACTCGTGACTCTTTACAAAGCAGGATAC
ACAGAAGGAAAAAAATACACAGTGCAAAATGGATGTTCTGAGTGCCACAAGGATCTGCT
GAAAAAAGCCAAAGATGTAAGATGGCTGGGTATATATGAGAATGAATATTTCACTATATT
CTGATTCAATTACCAGTCTCAGTGGCCCAGGATGAGCTTTTGGTGTGGTCACATGGCCAA
CATTTGGATAACAAATGAGGAATAATGGTACCGCCTCACTAGTGCCTGAGAAGAGCATGT
TCTGGAAAATGTCTCTGGAGTTAGAGATGTGTTAGCTTTTTCATTACAGATGGAGAAATA
CAATGTTTACACAACAGTCCAGGGGTGGGGTCAAAAGTTGGAAGGTGTCATTAGACGCAG
CCAAATAAAGTGAAGACAACCCAGGTGACTGGCAGCCCTGACTTGTGCGTGGGCGA
BF437145 
CTGACTGTCCCGTTTTTATTTTTACCATTGAGCCTTCTACCAGTACTGAAATGGGCAAAA
GATGGCTGATAACAAATTACACTTTACCTGTGATGGTTACTCTATGCTAGTATCCTGTTT
TTTAAAAAATAGTTCTTATGAGGTGTTAAGAAAAGCTTTCGCTTGGATTCATACACAGTT
GACCCT
H64601 
AGGAAGTTAAGAACAGTCCTAAAATCTCTTTGGCTTCTTTGTCCTGATATGCACCGGCAT
TTTCACAGTAGGAACTAGGGTTTCTGTCCAGTTTTTTGGTTCTTTAAGGAATTAATGTT
ATTCTGGGTACAACTGCTTACATACATAGCACATATAGATGACATTTTTACAGGCCGTCT
TGTTAGACTGACATACATGGAGGATAGTGCCACCCGCCTCACAAGAACATCAGGTAAGCT
CAGGCACAGAGTCCNAGGGNATCTGTAAGGGCTTCGCCCACGCACAAGTCAGGGCTGCCA
GTCACCNGGGTTGTCTTCACTTTATTTGGGCTGCGTCTAATGACACCTTNCCAACTTTTT
GACCCCACCCTGGGGCTTGTTGTGTAAACCATTGTTATTTCTCCCNTCTGTAATGGAAAA
AGGTTAACACNTTTTTAACTTCCGGNGACATTTTTC
AF212365 
gcacgagcga tgtcgctcgt gctgctaagc ctggccgcgc tgtgcaggag cgccgtaccc
cgagagccga ccgttcaatg tggctctgaa actgggccat ctccagagtg gatgctacaa
catgatctaa tccccggaga cttgagggac ctccgagtag aacctgttac aactagtgtt
gcaacagggg actattcaat tttgatgaat gtaagctggg tactccgggc agatgccagc
atccgcttgt tgaaggccac caagatttgt gtgacgggca aaagcaactt ccagtcctac
agctgtgtga ggtgcaatta cacagaggcc ttccagactc agaccagacc ctctggtggt
aaatggacat tttcctacat cggcttccct gtagagctga acacagtcta tttcattggg
gcccataata ttcctaatgc aaatatgaat gaagatggcc cttccatgtc tgtgaatttc
acctcaccag gctgcctaga ccacataatg aaatataaaa aaaagtgtgt caaggccgga
agcctgtggg atccgaacat cactgcttgt aagaagaatg aggagacagt agaagtgaac
ttcacaacca ctcccctggg aaacagatac atggctctta tccaacacag cactatcatc
gggttttctc aggtgtttga gccacaccag aagaaacaaa cgcgagcttc agtggtgatt
ccagtgactg gggatagtga aggtgctacg gtgcagctga ctccatattt tcctacttgt
 
ggcagcgact gcatccgaca taaaggaaca gttgtgctct gcccacaaac aggcgtccct
ttccctctgg ataacaacaa aagcaagccg ggaggctggc tgcctctcct cctgctgtct
ctgctggtgg ccacatgggt gctggtggca gggatctatc taatgtggag gcacgaaagg
atcaagaaga cttccttttc taccaccaca ctactgcccc ccattaaggt tcttgtggtt
tacccatctg aaatatgttt ccatcacaca atttgttact tcactgaatt tcttcaaaac
cattgcagaa gtgaggtcat ccttgaaaag tggcagaaaa agaaaatagc agagatgggt
ccagtgcagt ggcttgccac tcaaaagaag gcagcagaca aagtcgtctt ccttctttcc
aatgacgtca acagtgtgtg cgatggtacc tgtggcaaga gcgagggcag tcccagtgag
aactctcaag actcttcccc ttgcctttaa ccttttctgc agtgatctaa gaagccagat
tcatctgcac aaatacgtgg tggtctactt tagagagatt gatacaaaag acgattacaa
tgctctcagt gtctgcccca agtaccacct catgaaggat gccactgctt tctgtgcaga
acttctccat gtcaagtagc aggtgtcagc aggaaaaaga tcacaagcct gccacgatgg
ctgctgctcc ttgtagccca cccatgagaa gcaagagacc ttaaaggctt cctatcccac
caattacagg gaaaaaacgt gtgatgatcc tgaagcttac tatgcagcct acaaacagcc
ttagtaatta aaacatttta taccaataaa attttcaaat attgctaact aatgtagcat
taactaacga ttggaaacta catttacaac ttcaaagctg ttttatacat agaaatcaat
tacagtttta attgaaaact ataaccattt tgataatgca acaataaagc atcttcagcc
aaaaaaaaaa aaaaaa
AF208110 
cggcgatgtc gctcgtgctg ataagcctgg ccgcgctgtg caggagcgcc gtaccccgag
agccgaccgt tcaatgtggc tctgaaactg ggccatctcc agagtggatg ctacaacatg
atctaatccc cggagacttg agggacctcc gagtagaacc tgttacaact agtgttgcaa
caggggacta ttcaattttg atgaatgtaa gctgggtact ccgggcagat gccagcatcc
gcttgttgaa ggccaccaag atttgtgtga cgggcaaaag caacttccag tcctacagct
gtgtgaggtg caattacaca gaggccttcc agactcagac cagaccctct ggtggtaaat
ggacattttc ctatatcggc ttccctgtag agctgaacac agtctatttc attgggcccc
ataatattcc taatgcaaat atgaatgaag atggcccttc catgtctgtg aatttcacct
caccaggctg cctagaccac ataatgaaat ataaaaaaaa gtgtgtcaag gccggaagcc
tgtgggatcc gaacatcact gcttgtaaga agaatgagga gacagtagaa gtgaacttca
caaccactcc cctgggaaac agatacatgg ctcttatcca acacagcact atcatcgggt
tttctcaggt gtttgagcca caccagaaga aacaaacgcg agcttcagtg gtgattccag
tgactgggga tagtgaaggt gctacggtgc agctgactcc atattttcct acttgtggca
gcgactgcat ccgacataaa ggaacagttg tgctctgccc acaaacaggc gtccctttcc
ctctggataa caacaaaagc aagccgggag gctggctgcc tctcctcctg ctgtctctgc
tggtggccac atgggtgctg gtggcaggga tctatctaat gtggaggcac gaaaggatca
agaagacttc cttttctacc accacactac tgccccccat taaggttctt gtggtttacc
catctgaaat atgtttccat cacacaattt gttacttcac tgaatttctt caaaaccatt
gcagaagtga ggtcatcctt gaaaagtggc agaaaaagaa aatagcagag atgggtccag
tgcagtggct tgccactcaa aagaaggcag cagacaaagt cgtcttcctt ctttccaatg
acgtcaacag tgtgtgcgat ggtacctgtg gcaagagcga gggcagtccc agtgagaact
ctcaagacct cttccccctt gcctttaacc ttttctgcag tgatctaaga agccagattc
atctgcacaa atacgtggtg gtctacttta gagagattga tacaaaagac gattacaatg
ctctcagtgt ctgccccaag taccacttca tgaaggatgc cactgctttc tgtgcagaac
ttctccatgt caagcagcag gtgtcagcag gaaaaagatc acaagcctgc cacgatggct
gctgctcctt gtagcccacc catgagaagc aagagacctt aaaggcttcc tatcccacca
attacaggga aaaaacgtgt gatgatcctg aagcttacta tgcagcctac aaacagcctt
agtaattaaa acattttata ccaataaaat tttcaaatat tactaactaa tgtagcatta
actaacgatt ggaaactaca tttacaactt caaagctgtt ttatacatag aaatcaatta
cagctttaat tgaaaactgt aaccattttg ataatgcaac aataaagcat cttccaaaaa
aaaaaaaaaa aaaaaaaaaa aaaaaaaa
AF208111 
cggcgatgtc gctcgtgctg ataagcctgg ccgcgctgtg caggagcgcc gtaccccgag
agccgaccgt tcaatgtggc tctgaaactg ggccatctcc agagtggatg ctacaacatg
atctaatccc cggagacttg agggacctcc gagtagaacc tgttacaact agtgttgcaa
caggggacta ttcaattttg atgaatgtaa gctgggtact ccgggcagat gccagcatcc
gcttgttgaa ggccaccaag atttgtgtga cgggcaaaag caacttccag tcctacagct
gtgtgaggtg caattacaca gaggccttcc agactcagac cagaccctct ggtggtaaat
ggacattttc ctatatcggc ttccctgtag agctgaacac agtctatttc attggggccc
ataatattcc taatgcaaat atgaatgaag atggcccttc catgtctgtg aatttcacct
caccaggctg cctagaccac ataatgaaat ataaaaaaaa gtgtgtcaag gccggaagcc
tgtgggatcc gaacatcact gcttgtaaga agaatgagga gacagtagaa gtgaacttca
caaccactcc cctgggaaac agatacatgg ctcttatcca acacagcact atcatcgggt
tttctcaggt gtttgagcca caccagaaga aacaaacgcg agcttcagtg gtgattccag
tgactgggga tagtgaaggt gctacggtgc aggtaaagtt cagtgagctg ctctggggag
ggaagggaca tagaagactg ttccatcatt cattgctttt aaggatgagt tctctcttgt
caaatgcact tctgccagca gacaccagtt aagtggcgtt catgggggtt ctttcgctgc
agcctccacc gtgctgaggt caggaggccg acgtggcagt tgtggtccct tttgcttgta
ttaatggctg ctgaccttcc aaagcacttt ttattttcat tttctgtcac agacactcag
ggatagcagt accattttac ttccgcaagc ctttaactgc aagatgaagc tgcaaagggt
ttgaaatggg aaggtttgag ttccaggcag cgtatgaact ctggagaggg gctgccagtc
ctctctgggc cgcagcggac ccagctggaa cacaggaagt tggagcagta ggtgctcctt
cacctctcag tatgtctctt tcaactctag tttttgaagt ggggacacag gaagtccagt
ggggacacag ccactcccca aagaataagg aacttccatg cttcattccc tggcataaaa
agtgntcaaa cacaccagag ggggcaggca ccagccaggg tatgatgggt actacccttt
tctggagaac catagacttc ccttactaca gggacttgca tgtcctaaag cactggctga
aggaagccaa gaggatcact gctgctcctt ttttgtagag gaaatgtttg tgtacgtggt
aagatatgac ctagcccttt taggtaagcg aactggtatg ttagtaacgt gtacaaagtt
taggttcaga ccccgggagt cttgggcatg tgggtctcgg gtcactggtt ttgactttag
ggctttgtta cagatgtgtg accaagggga aaatgtgcat gacaacacta gaggtagggg
cgaagccaga aagaagggaa gttttggctg aagtaggagt cttggtgaga ttttgctgtg
atgcatggtg tgaactttct gagcctcttg tttttcctca gctgactcca tattttccta
cttgtggcag cgactgcatc cgacataaag gaacagttgt gctctgccca caaacaggcg
tccctttccc tctggataac aacaaaagca agccgggagg ctggctgcct ctcctcctgc
tgtctctgct ggtggccaca tgggtgctgg tggcagggat ctatctaatg tggaggcacg
aaaggatcaa gaagacttcc ttttctacca ccacactact gccccccatt aaggttcttg
tggtttaccc atctgaaata tgtttccatc acacaatttg ttacttcact gaatttcttc
aaaaccattg cagaagtgag gtcatccttg aaaagtggca gaaaaagaaa atagcagaga
tgggtccagt gcagtggctt gccactcaaa agaaggcagc agacaaagtc gtcttccttc
tttccaatga cgtcaacagt gtgtgcgatg gtacctgtgg caagagcgag ggcagtccca
gtgagaactc tcaagacctc ttcccccttg cctttaacct tttctgcagt gatctaagaa
gccagattca tctgcacaaa tacgtggtgg tctactttag agagattgat acaaaagacg
attacaatgc tctcagtgtc tgccccaagt accacttcat gaaggatgcc actgctttct
gtgcagaact tctccatgtc aagcagcagg tgtcagcagg aaaaagatca caagcctgcc
acgatggctg ctgctccttg tagcccaccc atgagaagca agagacctta aaggcttcct
atcccaccaa ttacagggaa aaaacgtgtg atgatcctga agcttactat gcagcctaca
aacagcctta gtaattaaaa cattttatac caataaaatt ttcaaatatt actaactaat
gtagcattaa ctaacgattg gaaactacat ttacaacttc aaagctgttt tatacataga
aatcaattac agctttaatt gaaaactgta accattttga taatgcaaca ataaagcatc
ttccaaaaaa aaaaaaaaaa aaaaaaaaaa aaaaaa
AF250309 
atgtcgctcg tgctgctaag cctggccgcg ctgtgcagga gcgccgtacc ccgagagccg
accgttcaat gtggctctga aactgggcca tctccagagt ggatgctaca acatgatcta
atcccgggag acttgaggga cctccgagta gaacctgtta caactagtgt tgcaacaggg
gactattcaa ttttgatgaa tgtaagctgg gtactccggg cagatgccag catccgcttg
ttgaaggcca ccaagatttg tgtgacgggc aaaagcaact tccagtccta cagctgtgtg
aggtgcaatt acacagaggc cttccagact cagaccagac cctctggtgg taaatggaca
ttttcctata tcggcttccc tgtagagctg aacacagtct atttcattgg ggcccataat
attcctaatg caaatatgaa tgaagatggc ccttccatgt ctgtgaattt cacctcacca
ggctgcctag accacataat gaaatataaa aaaaagtgtg tcaaggccgg aagcctgtgg
gatccgaaca tcactgcttg taagaagaat gaggagacag tagaagtgaa cttcacaacc
actcccctgg gaaacagata catggctctt atccaacaca gcactatcat cgggttttct
caggtgtttg agccacacca gaagaaacaa acgcgagctt cagtggtgat tccagtgact
ggggatagtg aaggtgctac ggtgcagctg actccatatt ttcctacttg tggcagcgac
tgcatccgac ataaaggaac agttgtgctc tgcccacaaa caggcgtccc tttccctctg
gataacaaca aaagcaagcc gggaggctgg ctgcctctcc tcctgctgtc tctgctggtg
gccacatggg tgctggtggc agggatctat ctaatgtgga ggcacgaaag gatcaagaag
acttcctttt ctaccaccac actactgccc cccattaagg ttcttgtggt ttacccatct
gaaatatgtt tccatcacac aatttgttac ttcactgaat ttcttcaaaa ccattgcaga
agtgaggtca tccttgaaaa gtggcagaaa aagaaaatag cagagatggg tccagtgcag
tggcttgcca ctcaaaagaa ggcagcagac aaagtcgtct tccttctttc caatgacgtc
aacagtgtgt gcgatggtac ctgtggcaag agcgagggca gtcccagtga gaactctcaa
gacctcttcc cccttgcctt taaccttttc tgcagtgatc taagaagcca gattcatctg
cacaaatacg tggtggtcta ctttagagag attgatacaa aagacgatta caatgctctc
agtgtctgcc ccaagtacca cctcatgaag gatgccactg ctttctgtgc agaacttctc
catgtcaagc agcaggtgtc agcaggaaaa agatcacaag cctgccacga tggctgctgc
tccttgtagc ccacccatga gaagcaagag accttaaagg gttccttttc ccatcattta
caggggaaaa acgtgtgatg atc
AK095091 
catattagag tctacagata tgcctttctt acagcaatcc tgcacccaca taaaagctac
attttcaata caagattaaa aggtattctg caaaatgtgc aaggttttca tgtctgctgg
tgtagctgta gtgatggctt catgaatttt tttctttttt gactatggtc cttacgctgg
attcatttat cttgaaatgg tgaacaatca cagctgcaga ccctcaattt atggtacata
tcaagcaatt tggctttttt tcttgtaatg aaaaaaaaaa gttttttttg cttttttca
tgacactgct tcttgggagc actgccagca ttactagtgg cacttcgtat gggtcctaag
gtgttattga aggtttacga tattgcacta aacacgaaaa ataccagaga accactggag
atacttttta ctgtgatatg taatttactg gagacaggaa ctgctcgttt ggagatggtt
agcatcacag ggtgttttaa gtcgatactt gcaacccttg agctcaccac agtagcaaca
ggaggtggct aggaaattat tcacagcagg acagtacgca ctgcaattaa ttgtatgcag
ttatgattta ataccacatc tttatgctca cgtttctctc aactgtgaat ggtgccatgt
acagttggta tgtgtgtgtt taagttttga taaattttta acttttaata gttaaaatag
ttaactattg gtatggtagg aaatgataaa gtagactagt atctgtatac attttctgca
tttatgacat acctttttct tcattttttt caatatttta attgaaaagt tcatccgagt
ttcatctaag ttttttcaaa gtgatacaaa tctccaaaaa attttccaat atatgtattg
aaaaaatcca ggtgtaagtg gctctgcgca gtccaaacct gtgttgttca agggtcaact
gtgtatgaat ccaagcgaaa gcttttctta acacctcata agaactattt tttaaaaaac
aggaactagc atagagtaac catcacaggt aaagtgtaat ttgttatcag ccatcttttg
cccatttcag tactggtaga aggctcaatg gtaaaaataa aaacgggaca gtcagaagat
ctggaagtcc tgaccctgct ttcacctggc atgtgtaatc cagtcatgct cgtatcagtc
tctgtaggag cacttgaagg tattacataa atgctatcta actctgggaa acgccaacat
gtgattgcct ccagaggaat cttctttaaa aaaaaattca aaatgttatt tccttactag
gatgtcttta aagaattata acccttaccg tgcctccaca ttagatagat ccctgccacc
 
agcacccatg tggccaccag cagagacagc aggaggagag gcagccagcc tcccggcttg
cttttgtctg gaaaaaacaa agcttattca cctttggaaa acaaatccac acttatctct
taatttaaaa actaagactt ggtatacttt atagaggttt atttattttt tattattttt
tagttttgag acagagtctc gctttgttgc ctaggctgga gtgcagtggc gcaatctcgg
ttcactgcag cctccgtctc ccgggttcaa gcaatgctgc ctcagcctcc tgagtagctg
ggattacagg catgtgtcac cgcgcccagc cactttgtag agatttagat ccctttaaaa
ccatcagtca gaagctcttt agatagtctg ccaatcatat ctttttccct agagtgtgca
ggtcttgcat tagattctca aaagggatat gggacccagg aagttaagaa cagtcctaaa
atctctttgg cttctttgtc ctgatatgca ccggcatttt cacagtagga actagggttt
ctgtccagtt tttttggttc tttaaggaat taatgttatt ctgggtacaa ctgcttacat
acatagcaca tatagatgac atttttacag gccgtcttgt tagactgaca tacatggagg
atagtgccac ccgcctcaca agaacatcag gtaagctcag gcacagagtg cccaggaatc
tgtaaggctt cgcccacgca caagtcaggg ctgccagtca cctgggttgt cttcacttta
tttggctgcg tctaatgaca ccttccaact tttgacccca cccctggact gttgtgtaaa
cattgtattt ctccatctgt aatgaaaaag ctaacacatc tctaactcca gagacatttt
ccagaacatg ctgttctcag gcactagtga ggcggtacca ttattcctca tttgttatcc
aaatgttggc catgtgacca caccaaaagc tcatcctggg ccactgagac tagtaattga
atcagaatat agtgaaatat tcattctcat atatacccag ccatcttaca tctttggctt
ttttcagcag atccttgtgg cactcagaac atccattttg cactgtgtat ttttttccct
tctgtgtatc ctgctttgta aagagtcacg agtggtttta caaataaagc ctgttcttac
tcag
BM983744 
TTTTTTTTTTTTTTTTCTGAGTAAGAACAGGCTTTATTTGTAAAACCACTCGTGACTCTT
TACAAAGCAGGATACACAGAAGGAAAAAAATACACAGTGCAAAATGGATGTTCTGAGTG
CCACAAGGATCTGCTGAAAAAAGCCAAAGATGTAAGATGGCTGGGTATATATGAGAATGA
ATATTTCACTATATTCTGATTCAATTACCAGTCTCAGTGGCCCAGGATGAGCTTTTGGTG
TGGTCACATGGCCAACATTTGGATAACAAATGAGGAATAATGGTACCGCCTCACTAGTGC
CTGAGAACAGCATGTTCTGGAAAATGTCTCTGGAGTTAGAGATGTGTTAGCTTTTTCATT
ACAGATGGAGAAATACAATGTTTACACAACAGTCCAGGGGTGGGGTCAAAAGTTGGAAGG
TGTCATTAGACGCAGCCAAATAAAGTGAAGACAACCCAGGTGACTGGCAGCCCTGACTTG
TGCGTGGGCGAAGCCTTACAGATTCCTGGGCACTCTGTGCCTGAGCTTACCTGATGTTCT
TGTGAGGCGGGTGGCACTATCCTCCATGTATGTCAGTCTAACAAGACGGCCTGTAAAAAT
GTCATCTATATGTGCTATGTATGTAAGCAGTTGTACCCAGAATAACATTAATCCTCGTGC
CGAAT
CB305764 
TTTTTTTTTTTTTTTGTTGGGCTGAAGATGCTTTATTATTGCATTATCAAAATGGTTATA
GTTTTCAATTAAAACTGTAATTGATTTCTATGTATAAAACAGCTTTGAAGTTGTAAATGT
AGTTTCCAATCGTTAGTTAATGCTACATTAGTTAGCAATATTTGAAAATTTTATTGGTAT
AAAATGTTTTAATTACTAAGGCTGTTTGTAGGCTGCATAGTAAGCTTCAGGATCATCACA
CGTTTTTTCCCTGTAATTGGTGGGATAGGAAGCCTTTAAGGTCTCTTGCTTCTCATGGGT
GGGCTACAAGGAGCAGCAGCCATCGTGGCAGGCTTGTGATCTTTTTCCTGCTGACACCTG
CTGCTTGACATGGAGAAGTTCTGCACAGAAAGCAGTGGCATCCTTCATGAGGTGGTACTT
GGGGCAGACACTGAGAGCATTGTAATCGTCTTTTGTATCAATCTCTCTAAAGTAGACCAC
CACGTATTTGTGCAGATGAATCTGGCTTCTTAGATCACTGCAGAAAAGGTTAAAGGCAAG
GGGGAAGAGGTCTTGAGAGTTCTCACTGGGACTGCCCTCGCTCTTGCCACAGGTACCATC
GCACACACACTGTTNACGTCATTGGAAAGAAGGAAGACGACTTTGTCTGCTGCCTTCTTTTG
AGTG
BM715988 
TGGTTTTTGTTTTTTTTCATTTTCTGTTGGATTACAGAAAAAGAATGGACCCATTCAG
GTCTCGATTTCCAAAGGTAAAGATGGAAGGCTGGCAGACTGGCTTTGTTACCTGACAT
GCCGTAGGGTGAGCTTAGAGGAAGAAAGAAAACAATTTTATTTGGCCAAAACAGAACAA
ATGCTGAAAAGGAAATCTTGTTTTTTTCCTAAAGCCAAATAGAAATGATTTGGGTATAAT
TTAAGAGTCCTTGTGTTGTACAGATATGGTGACTGATGTAGTTATTAATACTACCAACTT
AGTCATCAAGCCTCAATTTTCCTTTACCTGAAGGATTAAGTGAAAGCTTTTGGAGTTCAT
GATGTTCAGTATGATCAGTTAACCTTAACCTCTGAGCATCCTGAAGCAAAATCTAAATAA
TGCAGCTATTACCACTGGTGGTCCAGGCTCTGGTGAAGCCCTCTGAGCCCAGGAGGAAGA
GAAAGCATTGTCCAGAGGTAGGAACACAGTCTGGAGCCCAGAGCTCTGGAGGAGTGGG
AAAATGCTGCTTCCTGCTGCTTGCTTCTAGGCACCTGCTTCCGCCATCTCACTTACCATG
GCTAGAGATGGGGGTGAGACTGGGGAAGGACACAAGCAGGGAACAGATAAGGATGGAAA
TCAGAAGGGAATATAGAAAGAACTCTGGATGTGGAGACATGCCGGTACCTGAGCATTTTG
TATCAATGGGAGTACCTCT
BM670929 
TTTTTTTTTTTTTTTTTGGCTGAAGATGCTTTATTGTTGCATTATCAAAATGGTTACAG
TTTTCAATTAAAGCTGTAATTGATTTCTATGTATAAAACAGCTTTGAAGTTGTAAATGTA
GTTTCCAATCGTTAGTTAATGCTACATTAGTTAGCAATATTTGAAAATTTTATTGGTATA
AAATGTTTTAATTACTAAGGCTGTTTGTAGGCTGCATAGTAAGCTTCAGGATCATCACAC
GTTTTTTTCCCTGTAATTGGTGGGATAGGAAGCCTTTAAGGTCTCTTGCTTCTCATGGGT
GGGCTACAAGGAGCAGCAGCCATCGTGGCAGGCTTGTGATCTTTTTCCTGCTGACACCTG
CTGCTTGACATGGAGAAGTTCTGCACAGAAAGCAGTGGCATCCTTCATGAGGTGGTACTT
GGGGCAGACACTGAGAGCATTGTAATCGTCTTTTGTATCAATCTCTCTAAAGTAGACCAC
CACGTATTTGTGCAGATGAATCTGGCTTCTTAGATCACTGCAGAAAAGGTTAAAGGCAAG
GGGGAAGAGGTCTTGAGAGTTCTCACTGGGACTTGCCTCGCTCTTGCCACAGGTACCATC
GCACACACTGTTGACGTCATTGGAAAGAAAGAAGACGACTTTGTCTGCTGCCTTCTT
BI792416 
GCTGAAGATGCTTTATTGTTGCATTATCAAAATGGTTACAGTTTTCAATTAAAGCTGTAA
TTGATTTCTATGTATAAAACAGCTTTGAAGTTGTAAATGTAGT
BI715216 
CACGCGTCCGATTTTATACCAATAAAATTTTCAAATATTGCTAACTAATGTAGCATTAAC
TAACGATTGGAAACTACATTTACAACTTCAAAGCTGTTTTATACATAGAAATCAATTACA
GCTTTAATTGAAAACTGTAACCATTTTGATAATGCAACAATAAAGCATCTTCAGCCAAAA
AAAAAAA
N56060 
AGAAAAAGAAAATAGCAGAGATGGGTCCAGTGCAGTGGCTTGCATAAAAAAGAAGGCAGC
AGACAAAGTCGTCTTCCTTCTTTCCAATGACGTCAACAGTGTGTGCGATGGTACCTGTGG
CAAGAGCGAGGGCAGTCCCAGTGAGAACTCTCAAGACCTCTTCCCCCCTTGCCTTTAACC
TTTTCTGCAGTGATCTAAGAAGCCAGATTCATCTGCACAAATACGTGGTGGTCTACTTTA
GAGAGATTGATACAAAAGACGATTACAATGCTCTCAGTGTCTGCCCCAAGTACCACCTCA
TGAAGGATGCCACTGCTTTCTGTGCAGAACTTCTCCATGTCAAGCAGCAGGTTTCAGCAG
G
CB241389 
TTTTTTTTTTTTTTGTTTGGCTGAAGATGCTTTATTGTTGCATTATCAAAATGGTTACAG
TTTTCAATTAAAGCTGTAATTGATTTCTATGTATAAAACAGCTTTGAAGTTGTAAATGTA
GTTTCCAATCGTTAGTTAATGCTACATTAGTTAGCAATATTTGAAAATTTATTGGTATA
AAATGTTTTAATTACTAAGGCTGTTTGTAGGCTGCATAGTAAGCTTCAGGATCATCACAC
GTTTTTTCCCTGTAATTGGTGGGATAGGAAGCCTTTAAGGTCTCTTGCTTCTCATGGGTG
GGCTACAAGGAGCAGCAGCCATCGTGGCAGGCTTGTGATCTTTTTCCTGCTGACACCTGC
TGCTTGACATGGAGAAGTTCTGCACAGAAAGCAGTGGCATCCTTCATGAGGTGGTACTTG
GGGCAGACACTGAGAGCATTGTAATCGTCTTTTGTATCAATCTCTCTAAAGTAGACCACC
ACGTATTTGTGCAGATGAATCTGGCTTCTTAGATCACTGCAGAAAAGGTTAAAGGCAAGG
GGGAAGAGGTCTTGAGAGTTCTCACTGGGACTGCCCTCGCTCTTGCCACAGGTACCATCG
CACACACTGTTGACGTCATTGGAAAGAAGGAAGACGACTTTGTCTGCTGCCTTCTTTTGA
GTGGCAAGCCACTGCACTGGACCCATCTCTGCTATTTTCTTTTTCTNGCACTTTTCAAGG
ATGACTCACTTCTGCAATGGTTTTTGAGAATTCAGTGAAGTACAAATGTGTGATGGAACA
TAT
AV660618 
CGCTCGTGCTGCTAAGCCTGGCCGCGCTGTGCAGGAGCGCCGTACCCCGAGAGCCGACCG
TTCAATGTGGCTCTGAAACTGGGCCATCTCCAGAGTGGATGCTACAACATGATCTAATCC
CCGGAGACTTGAGGGACCTCCGAGTAGAACCTGTTACAACTAGTGTTGCAACAGGGGACT
ATTCAATTTTGATGAATGTAAGCTGGGTACTCCGGGCAGATGCCACACCAGAAGAAACAA
ACGCGAGCTTCAGTGGTGATTCCAGTGACTGGGATAGTGAAGGTGCTACGGTGCAGCTG
ACTCCATATTTTCCTACTTGTGGCAGCGACTGCATCCGACATAAAGGAACAGTTGTGCTC
TGCCCACAAACAGGCGTCCCTTTCCCTCTGGATAACAAC
BX088671 
GCTGAGTGTGATGGTGTAAGCCTGTGGTCCCAGCTACTAGGGAGGCTGAGATGGGATTAC
AGGTGTGAGCCACGGCGCCTGGCCTAAAAGCATCTTTTTCTTTAACGCAGAGGTTATGTT
GTATTATTAGCATAAATGTTTTTTTCTGGGAATGCTTATTTCACACAGCACAATACTGAA
TCTTCTCTGGAATGTGGATCGATTTCAGATGGATGACTATTAAAATGTGTATATTTGCAG
ATTATCCTTAAAGGGCCACCTCATGCCTTCTAATTTATGTCTTACGGATAAAAAATCAAA
ATGAAGCATAAAGTAAAAACTGTGTCCAGCTTTACAAGTGGACGCTTAGTAATGGCTGAG
GCAATATGTTTAATGTAGCAAATTTTACTTATTTGTCATGATCAGTTTTCACAGTGCTTG
TAAGTGCTGGTAATAGAAGATGGACATGGTTTAGGTCAAAACTTGGACCAGAAACCAACT
TCCTTTGAAACAGCTCTACCAGNTATAAGAGCAATATG
CB154426 
CTGTTGACGTCATTGGAAAGAAGGAAGACGACTTTGTCTGCTGCCTTCTTTTGAGTGGCA
AGCCACTGCACTGGACCCATCTCTGCTATTTCTTTTTCTGCCACTTTTCAAGGATGACC
TCACTTCTGCAATGGTTTTGAAGAAATTCAGTGAAGTAACAAATTGTGTGATGGAAACAT
ATTTCAGATGGGTAAACCACAAGAACCTTAATGGGGGCAGTAGTGTGGTGGTAGAAAAG
GAAGTCTTCTTGATCCTTTCTGTGAGAGGAGAAAAGCATTTGTTATCTGTGAACAGCAAA
CAGCAGGCTTTCACTCTGTAAACCATCCCTGACAAATGATCCCTTGCTAGAGAATGTCAG
CTGAGCACCAAGGGCCTTGTTAGTGACAGCAAGGAAAAACATCCTGATGTTCCTTTTGAA
CACATCACCTGAAACACACTGATGCTTAAACCTTAACTTTTTTTTTTTGGAGACACAGT
CTCACTCTGT
CA434589 
TTTTTTTTTTTTTTTTTTTCTGAGTAAGAACAGGCTTTATTTGTAAAACCACTCGTGACTC
TTTACAAAGCAGGATACACAGAAGGAAAAAAATACACAGTGCAAAATGGATGTTCTGAG
TGCCACAAGGATCTGCTGAAAAAAGCCAAAGATGTAAGATGGCTGGGTATATATGAGAAT
GAATATTTCACTATATTCTGATTCAATTACCAGTCTCAGTGGCCCAGGATGAGCTTTTGG
TGTGGTCACATGGCCAACATTTGGATAACAAATGAGGAATAATGGTACCGCCTCACTAGT
GCCTGAGAACAGCATGTTCTGGAAAATGTCTCTGGAGTTAGAGATGTGTTAGCTTTTTCA
TTACAGATGGAGAAATACAATGTTTACACAACAGTCCAGGGGTGGGGTCAAAAGTTGGAA
G
CA412162 
TTTTTTTTTTTTTTTTTTTGGCTGAAGATGCTTTATTGTTGCATTATCAAAATGGTTATAG
TTTTCAATTAAAACTGTAATTGATTTCTATGTATAAAACAGCTTTGAAGTTGTAAATGTA
GTTTCCAATCGTTAGTTAATGCTACATTAGTTAGCAATATTTGAAAATTTATTGGTATA
AAATGTTTTAATTACTAAGGCTGTTTGTAGGCTGCATAGTAAGCTTCAGGATCATCACAC
GTTTTTTCCCTGTAATTGGTGGGATAGGAAGCCTTTAAGGTCTCTTGCTTCTCATGGGTG
GGCTACAAGGAGCAGCAGCCATCGTGGCAGGCTTGTGATCTTTTTCCTGCTGACACCTGC
TGCTTGACATGGAGAAGTTCTGCACAGAAAGCAGTGGCATCCTTCATGAGGTGGTACGTG
GGGCAGACACTGAGAGCATTGTAATCGTCTTTTGTATCAATCTCTCTAAAGTAGACCACC
ACGTATTTGTGCAGATGAATCTGGCTTCTTAGATCACTGCAGAAAAGGTTAAAGGCAAGG
GGGAAGA
CA314073 
TTTTTTTTTTTTTTTTTTTGAAAGGGTCAGGACTTCCAGATCTTCTGACTGTCCCGTTTTT
ATTTTTACCATTGAGCCTTCTACCAGTACTGAAATGGGCAAAAGATGGCTGATAACAAAT
TACACTTTACCTGTGATGGTTACTCTATGCTAGTTCCTGTTTTTTAAAAAATAGTTCTTA
TGAGGTGTTAAGAAAAGCTTTCGCTTGGATTCATACACAGTTGACCCTTGAACAACACAG
GTTTGGACTGCGCAGAGCCACTTACACCTGGATTTTTTCAATACATATATTGGAAAATTT
TTTGGAGATTTGTATCACTTTGAAAAAACTTAGATGAAACTCGGATGAACTTTTCAATTA
AAATATTGAAAAAAATGAAGAAAAAGGTATGTCATAAATGCAGAAAATGTATACAGATAC
TAGTCTACTTTATCATTTCCTACCATACCAATAGTTAACTATTTTAACTATTAAAAGTTA
AAAATTTATCAAAACTTAAACACACACATACCAACTGTACATGGCACCATTCACAGTTGA
GAGAAACGTGAGCATAAAGATGTGGTATTAAATCATAACTGCATACAATTAATTGCAGTG
CGTACTGTCCTGCTGTGAATATTTCCTAGCCCTCGTGCCGAATC
BF921554 
GTGGGTGACCGTGGCTTGCCACTCAAAAGAAGGCAGCAGACAAAGTCGTCTTCCTTCTTT
CCAATGACGTCAACAGTGTGTGCGATGGTACCTGTGGCAAGAGCGAGGGCAGTCCCAGTG
AGAACTCTCAAGACCTCTTCCCCCTTGCCTTTAACCTTTTCTGCAGTGATCTAAGAAGCC
AGATTCATCTGCACAAATACGTGGTGGTCTACTTTAGAGAGATTGATACAAAAGACGATT
ACAATGCTCTCAGTGTCTGCCCCAAGTACCACCTCATGAAGGATGCCACTGCTTTCTGTG
CATAACTTCTCCATGTCAAGCAGCAGGTGTCAGCAGGAAAAAGATCACAAGCCTGCCACG
ATGGCTGCTGCTCCTTGTAGCCCACCCATGAGAAGCAAGAGACCTTAAAGGCTTCCTATC
CCACCAATTACAGGGAAAAAAACGTGTGATGATCCTGAAGCCACGGTCAA
BF920093 
TAGAGGATCCCGGTCGACGGTGGTTCAGTGATCATCACACTTTTTCCCTGTAATAGGTGG
GATAGGAAGCCTTTAAGGTCTCTTGCTTCTCATGGGTGGGCTACAAGGAGCAGCAGCCAT
CGTGGCAGGCTTGTGATCTTTTTCCTGCTGACACCTGCTGCTTGACATGGAGAAGTTATG
CACAGAAAGCAGTGGCATCCTTCATGAGGTGGTACTTGGGGCAGACACTGAGAGCATTGT
 
AATCGTCTTTTGTATCAATCTCTCTAAAGTAGACCACCACGTATTTGTGCAGATGAATCT
GGCTTCTTAGATCACTGCAGAAAAGGTTAAAGGCAAGGGGAAGAGGTCTTGAGAGTTCT
CACTGGGACTGCCCTCGCTCTTGCCACAGGTACCATCGCACACACTGTTGACGTCATTGG
AAAGAAGGAAGACGACTTTGTCTGCTGCCTTCTTTTGAGTGGCAAGCCACGGTCAACCCA
CAAGCCACGGTCAACCCAC
AV685699 
TCTACGTGGTAAGATATGACCTAGCCCTTTTAGGTAAGCGAACTGGTATGTTAGTAACGT
GTACAAAGTTTAGGTTCAGACCCCGGAGTCTTGGGCATGTGGGTCTCGGGTCACTGGTT
TTGACTTTAGGGCTTTGTTACAGATGTGTGACCAAGGGGAAAATGTGCATGACAACACTA
GAGGTAGGGGCGAAGCCAGAAAGAAGGGAAGTTTTGGCTGAAGTAGGAGTCTTGCGACTG
CATCCGACATAAAGGAACAGTTGTGCTCTGCCCACAAACAGGCGTCCCTTTCCCTCTGGA
TAACAACAAAAGCAAGCCGGGAGGCTGGCTGCCTCTCCTCCTGCTGTCTCTGCTGGTGGC
CACATGGGTGCTGGTGGCAGGGATCTATCTAATGTGGAGGCACGAAAGGATCAAGAAGAC
TTCCTTTTCTACCACCACACTACTGCCCCCCATTAAGGTTCTTGTGGTTTACCCATCTGA
AATATGTTTCCATCACACAATTTGTTACTTCACTGAATTTCTTCAAAACCATTGCAGAAG
TGAGGTCATCCTTGAAAGTGGCAGAGTAGCAGAGATGGGTCCAGTGCAGTGGCTTGCCAC
TCGTGCGATGGTCTT
AV650175 
GGCACGAGCACTGGCTGAAGGAAGCCAAGAGGATCACTGCTGCTCCTTTNTTCTAGAGGA
AATGTTTGTCTACGTGGTAAGATATGACCTAGCCCTTTTAGGTAAGCGAACTGGTATGTT
AGTAACGTGTACAAAGTTTAGGTTCAGACCCCGGGAGTCTTGGGCATGTGGGTCTCGGGT
CACTGGTTTTGACTTTAGGGCTNTGTTACAGATGTGTGACCAAGGGGAAAATGTGCATGA
CAACACTAGAGCTGACTCCATATTTTCCTACTTGTGGCAGCGACTGCATCCGACATAAAG
GAACAGTTGTGCTCTGCCCACANACAGGCGTCCCTTTCCCTCTGGATAACAACATAAGCA
AGCCGGGAGGCTGGCTGCCTCTCCTCCTGCTGTCTCTGCTGGTGGCACATGGGTGCTGGT
GGAGGGATCTATCTAATGTGGAGGCACGGATCAAGAAGACTTNCTTNTCTACCACCACAC
TACTGGCCCCAATAAGGGTCTNGTGGNTACCCCATCTGAATATGTTCATACACAATTTGT
ACTCACTGAATTCTCAAAACATTGAGAGTGAGGCATCCTGAAAGTGCGAAAAGANATGCN
AATGGTCAGTGCATGCTGCACTAGCAGCATGGACTT
BX483104 
GATCCCGCGCAGTGGCCCGGCGATGTCGCTCGTGCTGCTAAGCCTGGCCGCGCTGTGCAG
GAGCGCCGTACCCCGAGAGCCGACCGTTCAATGTGGCTCTGAAACTGGGCCATCTCCAGA
GTGGATGCTACAACATGATCTAATCCCCGGAGACTTGAGGACCTCCGAGTAGAACCTGT
TACAACTAGTGTTGCAACAGGGGACTATTCAATTTTGATGAATGTAAGCTGGGTACTCCG
GGCAGATGCCAGCATCCGCTTGTTGAAGGCCACCAAGATTTGTGTGACGGGCAAAAGCAA
CTTCCAGTCCTACAGCTGTGTGAGGTGCAATTACACAGAGGCCTTCCAGACTCAGACCAG
ACCCTCTGGTGGTAAATGGACATTTTCCTACATCGGCTTCCCTGTAGAGCTGAACACAGT
CTATTTCATTGGGGCCCATAATATTCCTAATGCAAATATGAATGAAGATGGCCCTTCCAT
GTCTGTGAATTTCACCTCACCAGGCTGCCTAGACCACATAATGAAATATAAAAAAAAGTG
TGTCAAGGCCGGAAGCCTGTGGGATCCGAACATCACTGCTTGTAAGAAGAATGAGGAGAC
AGTAGAAGTGAACTTCACAACCACTCCCCTGGGAAACAGATACATGGCTCTTATCCAACA
CAGCACTATCATTCGG
CD675121 
GTCTTGCATTAGATTCTCAAAAGGATATGGACCCAGGAAGTTAAGAACAGTCCTAAAA
TCTCTTTGGCTTCTTTGTCCTGATATGCACCGGCATTTTCACAGTAGGAACTAGGGTTTC
 
TGTCCAGTTTTTTTGGTTCTTTAAGGAATTAATGTTATTCTGGGTACAACTGCTTACATA
CATAGCACATATAGATGACATTTTACAGGCCGTCTTGTTAGACTGACATACATGGAGGA
TAGTGCCACCCGCCTCACAAGAACATCAGGTAAGCTCAGGCACAGAGTGCCCAGGAATCT
GTAAGGCTTCGCCCACGCACAAGTCAGGGCTGCCAGTCACCTGGGTTGTCTTCACTTTAT
TTGGCTGCGTCTAATGACACCTTCCAACTTTTGACCCCACCCCTGGACTGTTGTGTAAAC
ATTGTATTTCTCCATCTGTAATGAAAAAGCTAACACATCTCTAACTCCAGAGACATTTTC
CAGAACATGCTGTTCTCAGGCACTAGTGAGGCGGTACCATTATTCCTCATTTGTTATCCA
AATGTTGGCCATGTGACCACACCAAAAGCTCATCCTGGGCCACTGAGACTGGTAATTGAA
TCAGAATATAGTGAAATATTCATTCTCATATATACCCAGCCATCTTACATCTTTGGCTTT
TTTCAGCAGATCCTTGTGGCACTCAGAACATCCATTTTGCACTGTGTATTTTTTTCCCTT
CT
BE081436 
TGTGTAACTCTCAAGACCTCTTCCCCTTGCCTTTAACCTTTTCTGCAGTGATCTAAGAA
GCCAGATTCATCTGCACAAATACGTGGTGGTCTACTTTAGAGAGATTGATACAAAAGACG
ATTACAATGCTCTCAGTGTCTGCCCCAAGTACCACCTCATGGAGGATGCCACTGCTTTCT
GTGCAGAACTTCTCCATGTCAAGTAGCAGGTGTCAGCAGGAAAAAGATCACAAGCCTGCC
ACGATGGCTGCTGCTCCTTGTAGCCCACCCATGAGAAGCAAGAGACCTTAAAGGCTTCCT
ATCCCACCAATTACAGGGAAAAAACGTGTGATGAT
AW970151 
CTGAAATATGTTTCCATCACACAATTTGTTACTTCACTGAATTTCTTCAAAACCATTGCA
GAAGTGAGGTCATCCTTGAAAAGTGGCAGAAAAAGAAAATAGCAGAGATGGGTCCAGTGC
AGTGGCTTGCCACTCAAAAGAAGGCAGCAGACAAAGTCGTCTTCCTTCTTTCCAATGACG
TCAACAGTGTGTGCGATGGTACCTGTGGCAAGAGCGAGGGCAGTCCCAGTGAGAACTCTC
AAGACCTCTTCCCCCTTGCCTTTAACCTTTTCTGCAGTGATCTAAGAAGCCAGATTCATC
TGCACAAATACGTGGTGGTCTACTTTAGAGAGATTGATACAAAAGACGATTACAATGCTC
TCAGTGTCTGCCCCAAGTACCACCTCATGAAGGATGCCACTGCTTTCTGTGCAGAACTTC
TCCATGTCAAGTAGCAGGTGTCAGCAGGAAAAAGATCACAAGCCTGCCACGATGGCTGCT
GCTCCTTGTAGCCCACCCATGAGAAGCAAGAGACCTTAAAGGCTTCCTATCCCACCAATT
ACAGGGAAAAAAACGTGTGATGATCCCTGAAGCTTACTATGCAGCCTACANACAGCCTTA
GTAATAAAACATTTTATCCAATAAAATTTCAAATTTTGCTTAACTATGTGCATAAACTAC
GATTGAAAACTCTTTACACT
AW837146 
CATTGTGGTTGCAGCTGCATAGTAAGCTTCAGGATCATCACACGTTTTTCCCTGTAATT
GGTGGGATAGGAAGCCTTTAAGGTCTCTTGCTTCTCATGGGTGGCTACAAGGAGCAGCA
GCCATCGTGGCAGGCTTGTGATCTTTTTCCTGCTGACACCTGCTGCTTGACATGGAGAAG
TTCTGCACAGAAAGCAGTGGCATCCTTCATGAGGTGGTACTTGGGGCAGACACTGAGAGC
ATTGTAATCGTCTTTTGTATCAATCTCCCTAAAGTAGACCACCACGTATTTGTGCAGATG
AATCTGGCTTCTTAGATCACTGCAGAAAAGGTTAAAGGCAAGGGGGAAGAGGTCTTGAGA
GTTCTCACTGGGACTGCCCTCGCTCTTGCCACAGGTACCATCGCACACACTGTTGACGTC
ATTGGAAAGAAGGAAGACGACTTTGTCTGCTGCCTTCTTTTGAGTGGCAAGCCACTGCAC
TGGACCCATCT
AW368264 
GTGAATAAGCTTTGTTTTTCCAGACAAAAGCAAGCCAGGAGGCTGGCTGCCTCTCCTCC
TGCTGTCTCTGCTGGTGGCCACATGGTTGCTGGTGGCAGGATCTATCTAATGTGGAGGC
ACGGTAAGGGTTATAATTCTTTAAAGTCATCCTAGTAAGGAAATAACATTTGGAATTTTT
 
TTTTAAAGAAGATTCCTCTGGAGGCAATCACCTGTTGGCGTTTCCCAGAGTTAGATAGCA
TTTATGTAATACCTTCAAGTGCTCCTACAGAGACTGATACGAGCATGACTGGATTACACA
TGCCAGGTGAAAGCAGGGCCAGGACTTCCAGATCTTCTGACTGTCCCGTTTTTATTTTTA
CCATTGAGCCTTCTACCAGAACTGAAATGGGCAAAAGATGGCTGATAACAAATTACACTT
TACCTGTGATGGTTACTCTATGCTAGTTCCTGTTTTTAAAAAAATAGTTCTTATGAGGTG
TCAAGAAAAGCTTTCGCTTGGATTCATACACAGTTGACCCTTGAACAACACAG
D25960 
GATCCTGAAGCTTACTATGCAGCCTACAAACAGCCTTAGTAATTAAAACATTTTATACCA
ATAAAATTTTCAAATATTGCTAACTAATGTAGCATTAACTAACGATTGGAAACTACATNN
ACAACTTCAAAGCTGTTTTATACATAGAAATCAATTACAGCTTTAATTGAAAACTATAAC
CATTTTGATAATGCAACANTAAAGCATCTTCAGCCAAA
AV709899 
GCAACTTCCAGTCCTACAGCTGTGTGAGGTGCAATTACACAGAGGCCTTCCAGACTCAGA
CCAGACCCTCTGGTGGTAAATGGACATTTTCCTATATCGGCTTCCCTGTAGAGCTGAACA
CAGTCTATTTCATTGGGGCCCATAATATTCCTAATGCAAATATGAATGAAGATGGCCCTT
CCATGTCTGTGAATTTCACCTCACCAGGCTGCCTAGACCACATAATGAAATATAAAAAAA
AGTGTGTCAAGGCCGGAAGCCTGTGGGATCCGAACATCACTGCTTGTAAGAAGAATGAGG
AGACAGTAGAAGTGAACTTCACAACCACTCCCCTGGGAAACAGATACATGGCTCTTATCC
AACACAGCACTATCATCGGGTTTTCTCAGGTGTTTGAGCCACACCAGAAGAAACAAACGC
GAGCTTCAGTGGTGATTCCAGTGACTGGGGATAGTGAAGGTGCTACGGTGCAGCTGACTC
CATATTTTCCTACTTGTGGCAGCGACTGCATCCGACATAAAGGAACAGTTGTGCTCTGCC
CACAAACAGGCGTNCCTTTTCCTCTGGATAACAACAAAAGCAAGCCGGGAGGCTTGGCTG
CTCTCCTTCTGCTGGCCTTTGCTGTGGCCACATTGGTGCTGGTGGCAGGGATCTATCTAA
TGTGGATGCACGTCTCGTGGTTTACCCATCTGAAATATGTTCN
BX431018 
ATTTTTCCTCTTGTGGCAGCGACTGGCATCCGACATAAAGGAACAGTTGTGCTCTGCCCA
CAAACAGGCGTCCCTTTCCCTCTGGATAACAACAAAAGCAAGCCGGGAGGCTGGCTGCCT
CTCCTCCTGCTGTCTCTGCTGGTGGCCACATGGGTGCTGGTGGCAGGGATCTATCTAATG
TGGAGGCACGAAAGGATCAAGAAGACTTCCTTTTCTACCACCACTACTGCCCCCCATT
AAGGTTCTTGTGGTTTACCCATCTGAAATATGTTTCCATCACACAATTTGTTACTTCACT
GAATTTCTTCAAAACCATTGCAGAAGTGAGGTCATCCTTGAAAAGTGGCAGAAAAAGAAA
ATAGCAGAGATGGGTCCAGTGCAGTGGCTTGCCACTCAAAAGAAGGCAGCAGACAAAGTC
GTCTTCCTTCTTTCCAATGACGTCAACAGTGTGTGCGATGGTACCTGTGGCAAGAGCGAG
GGCAGTCCCAGTGAGAACTCTCAAGACCTCTTCCCCCTTGCCTTTAACCTTTTCTGCAGT
GATCTAAGAAGCCAGATTCATCTGCACAAATACGTGGTGGTCTACTTTAGAGAGATTGAT
ACAAAAGACGATTACAATGCTCTCAGTGTCTGCCCCAAGTACCACCTCATGAAGGATGCC
ACTGCTTTCTGTGCAGAACTTCTCCATGTCAAGCAGCAGGTGTCAGCAGGAAAAAGATCA
CAAGCCTGCCACGATGGCTGCTGCTCCTTGTAGCCCACCCATGAGAAGCAAGAGACCTTA
AGGCTTCTATCCCACCANTACAGGNAAAAACGTGTGATGATCCTGAAGCTTACTATGCAG
CCTACAACAGGCTTAGTATTAAAACATTTATACCCATAAATTTTCAAATTGCT
AL535617 
TAGGTGACACTATAGAACAAGTTTGTACAAAAAAGCAGGCTGGTACCGGTCCGGAATTCC
CGGGATAGTGGMCCGGCGAKGTCGCTCGTGCTGCTAAGCCTGGCCGCGCTGTGCAGGAGC
GCCGTACCCCGAGAGCCGACCGTTCAATGTGGCTCTGAAACTGGGCCATCTCCARAGTGG
ATGSKACAACATGATCTAATCCCGGAGACTTGAGGGACCTCCGAGTAGAACCTGTTACA
 
ACTAGTGTTGCAACAGGGGACTATTCAATTTTGATGAATGTAAGCTGGGTACTCCGGGSA
GATGCCAGCATCCGCTTGTTGAAGGCCACCAAGATTTGTGTGAMGGCAAAAGCAACWTC
CAGTCCTACAGCWGTGTGAGGTAGCAATTACACAGAGAGCACATATCCAGACTCTAGACC
AGACCCTCTGGWGGTAAATGGACATTTTCCTATATCGGCTTCCCTGTAGAGCTGAACACA
GTCTATATTCATTGGGGCCCAWAATAWWCCTAATGCAAATATGAATGAAGATGGCCCTTC
CATGTCTGTGAATTTCACCTCACCAGGCTGCCTAGACCACATAATGAAATAWAAAAAAAA
GTGTGTCAAGGCCGGAAGCCTGTGGATCCGAACATCACTGCTTGTAAGAAGAATGARGA
GACAGTAGAAGTGAACTTCACAACCACTCCCCTGGGAAACAGATAMATKGCTCTTATCCA
ACACARMACTATCATCGGGTTTTCTCAGGTGTTTGAGCCACACCAGAAGAAACAAACGCG
AGCTTCAGTGGTGATTCCAGTGACTGGGGATAGTGAAGGTGCTACGGTGCAGCTGACTCC
ATATTTTCCTACTTGTGGCAGCGWCTGCATCCGACATAAAGGAACAGTTGTGCTCTGCCC
ACAAACAGGCGTCCCTTTYCCTCTGGATAACAACAAAAGCAACYGGGAGSTGGYTGYCT
AL525465 
WAATWAKADDRATANHTGAAAACTATAACCATTTNTGATAATNGNAANAATAAAGCATCT
TCAGCCAAACATCTAGTCTTCCATAGACCATGCATTGCAGTGTACCCAGAWCTGTTTAGC
TAATATTCTATGTTTAATTAATGAATACTAACTCTAAGAACCCCTCACTGATTCACTCAA
TAGCATCTTAAGTGAAAAACCTTCTATTACATGCAAAAAATCATTGTTTTTAAGATAACA
AAAGTAGGGAATAAACAAGCTGAACCCACTTTTACTGGACCAAATGATCTATTATATGTG
TAACCACTTGTATGATTTGGTATTTGCATAAGACCTTCCCTCTACAAACTAGATTCATAT
CTTGATTCTTGTACAGGTGCCTTTTAACATGAACAACAAAATACCCACAAACTTGTCTAC
TTTTGCCTAAAGTTACCTATTAGAGGTCACTGTSAGAGTKCTCAGTTTCTTAGTTACTAT
TTAASTTTTSATGTTCAAAATGAAAATAATTCTKAAGTKGAAAGSGCTCTTGAAGTAACC
TTTTTATAAATGAGTTATTATAATGGTTTACTTAAATAAAAVAGAGGGGKTTTTGCGGTG
GCTCATGCCTCCAATCCCAGCACTTTGGCAAGGCCAAGGCAAAAVGATCGCTCAAGACCA
GGCTACGTCACAAAGCGAGACCTCCATCTCTACAAAAGATTTAAAAAATTAGCTGAGTGT
GATGGTGTGAGCCTGTGGTCCCAGCTACTAGGGAGGCTGAGATGGAGGATCACTTGAGC
CCTGGAGGTCAAGGGTGCAGTAAACGGTGATTGTGCCACTGCACTCCATCCTGGGTGAGA
GCAGACCCTGTCTAAAACAAACAAACGAAAAAACCCCCACAGAATGACAGAACATAAAAG
ATGCACATTTTGTCTTCCAACTTTTTACTCTTCTAAAAGCATCTTTTTTAAATTTTTTAA
ATTTTTTTTTTTTTGAGACAGAGTTTCACTCTGTCACACAGGCTGGAGTGMGTGGCGTGA
CTCGGCTCACTAMAACTCTGCYTCCGGGGTYACSCATCTCCTGCWCAGCTCCTGAGAAGC
KGGAYAMAGGMCCACACAAACCAGTAAYTTTATWTTTTGAAAAAGGGTTYACCTGTASMA
GRAGGCTGAATCCGACMAARTMACCMCCACYYCAAADGAGGAWAAGKGKRSMGGSCBGGC
A
BX453536 
TTATGGGGGCAGTAGTGTGGTGGTAGAAAAGGAAGTCTTCTTGATCCTTTCGTGCCTCC
CATTAGATAGATCCCTGCCACCAGCACCCATGTGGCCACCAGCAGAGACAGCAGGAGGAG
AGGCAGCCAGCCTCCCGGCTTGCTTTTGTTGTTATCCAGAGGGAAAGGACGCCTGTTTG
TGGGCAGAGCACAACTGTTCCTTTATGTCGGATGCAGTCGCTGCCACAAGTAGGAAAATA
TGGAGTCAGCTGCACCGTAGCACCTTCACTATCCCCAGTCACTGGAATCACCACTGAAGC
TCGCGTTTGTTTCTTCTGGTGTGGCTCAAACACCTGAGAAAACCCGATGATAGTGCTGTG
TTGGATAAGAGCCATGTATCTGTTTCCCAGGGGAGTGGTTGTGAAGTTCACTTCTACTGT
CTCCTCATTCTTCTTACAAGCAGTGATGTTCGGATCCCACAGGCTTCCGGCCTTGACACA
CTNTNTTTATATTTCATTATGTGGTCTAGGCAGCCTGGTGAGGTGAAATTCACAGACAT
GGAAGGGCCATCTTCATTCATATTTGCATTAGGAATATTATGGGCCCCAATGAAATAGAC
TGTGTTCAGCTCTACAGGGGAAGCCGATATAGGAAAATGTCCATTTACCACCAGAGGGTC
TGGTCTGAGTCTTGAAGGCCTTTTGTGTTATTGCACCTTACACAGCTGTTAGACTGGGAA
GTTGCTTTTGCCCCGCACACAAATCTTGTGGGCCTTCAACAGCGGATGCTGCCATTTGCC
CCGAAGTCCCCAGCTCAATTCATTAAAAATTGAATAGGCCCCTTGTGGCAACCCTAGTTG
 GTACAGGGTTTTACTTGGGGGCCCCTCTAAGTTTCCCCGGATATAAACAAAGTGTGG
BX453537 
TTATGGGGGCAGTAGTGTGGTGGTAGAAAAGGAAGTCTTCTTGATCCTTTCGTGCCTCC
ACATTAGATAGATCCCTGCCACCAGCACCCATGTGGCCACCAGCAGAGACAGCAGGAGGA
GAGGCAGCCAGCCTCCCGGCTTGCTTTTGTTGTTATCCAGAGGAAAGGACGCCTGTTT
GTGGGCAGAGCACAACTGTTCCTTTATGTCGGATGCAGTCGCTGCCACAAGTAGGAAAAT
ATGGAGTCAGCTGCACCGTAGCACCTTCACTATCCCCAGTCACTGGAATCACCACTGAAG
CTCGCGTTTGTTTCTTCTGGTGTGGCTCAAACACCTGAGAAAACCCGATGATAGTGCTGT
GTTGGATAAGAGCCATGTATCTGTTTCCCAGGGGAGTGGTTGTGAAGTTCACTTCTACTG
TCTCCTCATTCTTCTTACAAGCAGTGATGTTCGGATCCCACAGGCTTCCGGCCTTGACAC
ACTTTTTTTTATATTTCATTATGTGGTCTAGGCAGCCTGGTGAGGTGAAATTCACAGACA
TGGAAGGGCCATCTTCATTCATATTTGCATTAGGAATATTATGGGCCCCAATGAAATAGA
CTGTGTTCAGCTCTACAGGGAAGCCGATATAGGAAAATGTCCATTTACCACCAGAGGGTC
TGGTCTGAGTCTGGAAGGCCTCTGTGTAATTGCACCTCACACAGCTGTAGGACTGGGAGT
TGCTTTTGCCCGTACACAAATCTTGTTGGCCTTCAACAAGCGGATGCTGGCATCTGGCGG
GGGTACCCAGCTTACATTCATCAAAATTGAATAGTCCCCTTGTTGCAACACTAGTTTGTA
AACAGGTTCTACTCCGGGGGTCCCCTCAGTCTCCCGG
AW28945 
CAAATATGAATGAAGATGGCCCTTCCATGTCTGTGAATTTCACCTCACCAGGCTGCCTAG
ACCACATAATGAAATATAAAAAAAAGTGTGTCAAGGCCGGAAGCCTGTGGATCCGAACA
TCACTGCTTGTAAGAAGAATGAGGAGACAGTAGAAGTGAACTTCACAACCACTCCCCTGG
GAAACAGATACATGGCTCTTATCCAACACAGCACTATCATCGGGTTTTCTCAGGTGTTTG
AGCCACACCAGAAGAAACAAACGCGAGCTTCAGTGGTGATTCCAGTGACTGGGGATAGTG
AAGGTGCTACGGTGCAACTGACTCCATATTTTCCTACTTGTGGCAGCGACTGCATCCGAC
ATAAAGGAACAGTTGTGCTCTGCCCACAAACAGGCGTCCCTTTCCCTCTGGATAACAAC
AV728939 
GCAAATATGAATGAAGATGGCCCTTCCATGTCTGTGAATTTCACCTCACCAGGCTGCCTA
GACCACATAATGAAATATAAAAAAAGTGTGTCAAGGCCGGAAGCCTGTGGATCCGAAC
ATCACTGCTTGTAAGAAGAATGAGGAGACAGTAGAAGTGAACTTCACAACCACTCCCCTG
GGAAACAGATACATGGCTCTTATCCAACACAGCACTATCATCGGGTTTTCTCAGGTGTTT
GAGCCACACCAGAAGAAACAAACGCGAGCTTCAGTGGTGATTCCAGTGACTGGGGATAGT
GAAGGTGCTACGGTGCAGCTGACTCCATATTTTCCTACTTGTGGCAGCGACTGCATCCGA
CATAAAGGAACAGTTGTGCTCTGCCCACAAACAGGCGTCCCTTTCCCTCTGGATAACAAC
AV727345

GCAAATATGAATGAAGATGGCCCTTCCATGTCTGTGAATTTCACCTCACCAGGCTGCCTA
GACCACATAATGAAATATAAAAAAAGTGTGTCAAGGCCGGAAGCCTGTGGATCCGAAC
ATCACTGCTTGTAAGAAGAATGAGGAGACAGTAGAAGTGAACTTCACAACCACTCCCCTG
GGAAACAGATACATGGCTCTTATCCAACACAGCACTATCATCGGGTTTTCTCAGGTGTTT
GAGCCACACCAGAAGAAACAAACGCGAGCTTCAGTGGTGATTCCAGTGACTGGGGATAGT
GAAGGTGCTACGGTGCAGCTGACTCCATATTTTCCTACTTGTGGCAGCGACTGCATCCGA
CATAAAGGAACAGTTGTGCTCTGCCCACAAACAGGCGTCCCTTTCCCTCTGGATAACAAC
AAAAGCAAGCCGGGAGGCTGGCTGCCTCTCCTCCTGCTGTCTCTGCTGGTGGCCACATGG
GTGCTGGTGGCAGGGATCTATCTAATGTGGAGGCACGAAAGGATCAAGAAGACTTCCTTT
TTTACCACCACACTACTGTCTCCCATTAAAGATCTTGTGGTTTATCCATCTGAAATATTG
TTCCATTACACATATTGGTACCTAACTGAAATTCTTTAAAACCATTGCAAATTGAGGTCA

CTCTTGAAAGGGCGTG

Sequences identified as those of CHDH
>gi|26011703|gb|CA774243.1|CA774243 in24a07.x1 Human Fetal Pancreas 1B Homo sapiens cDNA clone IMAGE: 3' 
TAAATAACAAACACCACTTTGTTATGAAGACCTTACAAACCTCTTCTTAAGACATTCTTA
CTCTGATCCAGGCAAAAACACTTCAAGGTTTGTAAATGACTCTTTCCTGACATAAATCCT
TTTTTATTAAAATGCAAAATGTTCTTCAGAATAAAACTGTGTAATAATTTTATACTTGGG
AGTGCTCCTTGCACAGAGCTGTCATTTGCCAGTGAGAGCCTCCGACGGGCAGGTACTGT
GCCAGGGCAGCTCTGAAATTATGGATATTCTTATCCTCCTGGTTCCTTCGGTGCCAATGGT
AACCTAATACCAGCCGCAGGGAGCGCCATTTCTCCTAAAGGGCTACACCACTGTCAACAT
TATCCTGGACTCTGTGTCTCTCTCTGTTGGGTCTTGTGGCATCACATCAGGCCAAAATTGC
CAGACCAGGACCCTAAGTGTCTGATAGAGGCGATGATCTTTTCCAAAGTCAGTACTTACA
AACTGGCATTCTTACAGGCTGCACCATTTCCTAGTATGTCTGCTTTAAGCCTGGTTCAACC
TCTCATCGAATATTAAATTTTTCTTTGTA
>gi|23527150|gb|BU679327.1|BU679327 UI-CF-DU1-aau-i-03-0-UI.s1 UI-CF-DU1 Homo sapiens cDNA clone UI-CF-DU1-aau-i-03-0-UI 3' 
TTTTTTTTTTTTTTTCTTACAAAGAAAAATTTAATATTCGATNGAGAGGTTGAACCAGGCT
TAAAGCAGACATACTAGGAAATGGTGCAGCCTGTAAGAATGCCAGTTTGTAAGTACTGA
CTTTGGAAAAGATCATCGCCTCTATCAGACACTTAGGGTCCTGGTCTGGCAATTTTGGCC
TGATGTGATGCCACAAGACCCAACAGAGAGACACAGAGTCCAGGATAATGTTGACAG
TGGTGTAGCCCTTTAGGAGAAATGGCGCTCCCTGCGGCTGGTATTAGGTTACCATTGGCA
CCGAAGGAACCAGGAGGATAAGAATATCCATAATTTCAGAGCTGCCCTGGCACAGTACC
TGCCCCGTCGGAGGCTCTCACTGGCAAATGACAGCTCTGTGCAAGGAGCACTCCCAAGT
ATAAAAATTATTACACAGTTTTATTCTGAAGAACATTTTGCATTTTAATAAAAAGGATT
TATGTCAGGAAAGAGTCATTTACAAACCTTGAAGTGTTTTTGCCTGGATCAGAGTAAGAA
TGTCTTAAGAAGAGGTTTGTAAGGTCTTCATAACANAGTGGTGTTTGTTATTTACAAAAA
AAAAAAAAAAAAAAAATAAAAAAAAAAAAAAAAAACCTCGTGCCGAATTCT
>gi|23274053|gb|BU608029.1|BU608029 UI-CF-FN0-aes-I-02-0-UIs1 UI-CF-FN0 Homo sapiens cDNA clone UI-CF-FN0-aes-1-02-0-UI 3' 
TTTTTTTTTTTTTTTTGTAAATAACAAACACCACTTTGGTTATGAAGACCTTACAAACCTC
TTCTTAAGACATTCTTACTCTGATCCAGGCAAAAACACTTCAAGGTTTGTAAATGACTCTT
 
TCCTGACATAAATCCTTTTTATTAAAATGCAAAATGTTCTTCAGAATAAAACTGTGTAAT
AATTTTATACTTGGGAGTGCTCCTTGCACAGAGCTGTCATTTGCCAGTGAGAGCCTCCG
ACAGGGCAGGTACTGTGCCAGGGCAGCTCTGAAATTATGGATATTCTTATCCTCCTGGTT
CCTTCGGTGCCAATGGTAACCTAATACCAGCCGCAGGGAGCGCCATTTCTCCTAAAGGGC
TACACCACTGTCAACATTATCCTGGACTCTGTGTCTCTCTCTGTTGGGTCTTGTGGCATCA
CATCAGGCCAAAATTGCCAGACCAGGACCCTAAGTGTCTGATAGAGGCGATGATCTTTTC
CAAAGTCAGTACTTACAAACTGGCATTCTTACAGGCTGCACCATTTCCTAGTATGTCTGC
TTTAAGCCTGGTTCAACCTCTCATCGAATATTAAATTTTTCTTTGTAAGAAAAATTTGAAG
TTGTAGAGCATGGTTTTTTGTTTTCCCTTGTCTTAGGAAAGTTTTAAGATGAAATGTTTTT
CC
>gi|11451784|gb|BF439267.1|BF439267 nab62a07.x1 Soares_NSF_F8_9W_OT_PA_P_S1_ Homo sapiens cDNA clone IMAGE:3272340 3' 
TTTTTTTTTTTTTTGGGCCAAAATTTTAATAGTATACAGACAACCTGTTAATTTTTTTTT
TTTTTTTTTGTAAATAACAAACACCACTTTGTTATGAAGACCTTACAAACCTCTTTTTAA
GACATTCTTACTCTGATCCAGGCAAAAACACTTCAAGGTTTGTAAATGACTTTTTCCTGA
CATAAATCCTTTTTTATTAAAATGCAAAATGTTCTTCAGAATAAAACTGTGTAATAATTTT
TATACTTGGGAGTGCTCCTTGCACAGAGCTGTCATTTGCCAGTGAGAGCCTCCGACGGGG
CAGGTACTGTGCCAGGGCAGCTCTGAAATTATGGATATTCTTATCCTCCTGGTTCCTTCGG
TGCCAATGGTAACCTAATACCAGCCGCAGGGAGCGCCATTTCTCCTAAAGGGCTACACC
ACTGTCAACATTATCCTGG
>gi|11448468|gb|BF436153.1|BF436153 nab77h10.x1 Soares_NSF_F8_9W_OT_PA_P_S1 Homo sapiens cDNA clone IMAGE:3273859 3' 
TTTTTTTTGGTCCAAAATTTTAATAGTATACAGACAACCTGTTAATTTTTTTTTTTTTT
TTTTTGTAAATAACAAACACCACTTTGTTATGAAGACCTTACAAACCTCTTCTTAAGACAT
TCTTACTCTGATCCAGGCAAAAACACTTCAAGGTTTGTAAATGACTCTTTCCTGACATAA
ATCCTTTTTATTAAAATGCAAAATGTTCTTCAGAATAAAACTGTGTAATAATTTTTATAC
TTGGGAGTGCTCCTTGCACAGAGCTGTCATTTGCCAGTGAGAGCCTCCGACGGGGCAGGT
ACTGTGCCAGGGCAGCTCTGAAATTATGGATATTCTTATCCTCCTGGTTCCTTCGGTGCCA
ATGGTAACCTAATACCAGCCGCAGGGAGCGCCATTTCTCCTAAAGGGCTACACCACTGTC
AACATTATCCTGGACTC
>gi|10033200|gb|BE672659.1|BE672659 7b71b03.x1 NCI_CGAP_Lu24 Homo sapiens cDNA clone IMAGE:3233645 3' 
TTTGTAAATAACAAACACCACTTTGTTATGAAGACCTTACAAACCTCTTCTTAAGACATT
CTTACTCTGATCCAGGCAAAAACACTTCAAGGTTTGTAAATGACTCTTTCCTGACATAAA
TCCTTTTTATTAAAATGCAAAATGTTCTTCAGAATAAAACTGTGTAATAATTTTTATACT
TGGGAGTGCTCCTTGCACAGAGCTGTCATTTGCCAGTGAGAGCCTCCGACAGGGCAGGT
ACTGTGCCAGGGCAGCTCTGAAATTATGGATATTCTTATCCTCCTGGTTCCTTCGGTGCCA
ATGGTAACCTAATACCAGCCGCAGGGAGCGCCATTTCTCCTAAAGGGCTACACCACTGTC
AACATTATCCTGGACTCTGTGTCTCTCTCTGTTGGGTCTTGTGGCATCACATCAGGCCAAA
ATTGCCAGACCAGGACCCTAAGTGTCTGATAGAGGCGATGATCTTTTCCAAAGTCAGTAC
TTACAAACTGGCATTCTTACAGGCTGCACCATTTCCTAGTATGTCTGCTTTAAGCCTGGTT
CAACC
>gi|6837905|gb|AW341279.1|AW341279 xz97e03.x1 NCI_CGAP_Lu24 Homo sapiens cDNA clone IMAGE:2872156 3' 
TAAATAACTAACACCATTTTGTTATGAAGACCTTACAAACCTCTTCTTAAGACATTCTTAC
TCTGATCCAGGCAAAAACACTTCAAGGTTTGTAAATGACTCTTTCCTGACATAAATCCTT
TTTTTATTAAAATGCAAAATGTTCTTCAGAATAAAACTGTGTAATAATTTTATACTTGGG
AGTGCTCCTTGCACAGAGCTGTCATTTGCCAGTGAGAGCCTCCGACGGGGCAGGTACTGT
GCCAGGGCAGCTCTGAAATTATGGATATTCTTATCCTCCTGGTTCCTTCGGTGCCAATGGT
AACCTAATACCAGCCGCAGGGAGCGCCATTTCTCCTAAAGGGCTACACCACTGTCAACAT
TATCCTGGACTCTGTGTCTCTCTCTGTTGGGTCTTGTGGCATCACATCAGGCCAAAATTGC
CAGACCAGGACCCTAAGTGTCTGATAGAGGCGATGATCTTTTCCAAAGTCAGTACTTACA
AACTGGCATTCTTACAGGCTGCACCATTTCCTAGTATGTCTG
>gi|5543775|gb|AI869807.1|AI869807 wm04b02.x1 NCI_CGAP_Ut4 Homo sapiens cDNA clone IMAGE:2434923 3' 
GTAAATAACAAACACCACTTTGTTATGAAGACCTTACAAACCTCTTCTTAAGACATTCTT
ACTCTGATCCAGGCAAAAACACTTCAAGGTTTGTAAATGACTCTTTCCTGACATAAATCC
TTTTTTATTAAAATGCAAAATGTTCTTCAGAATAAAACTGTGTAATAATTTTTATACTTGG
GAGTGCTCCTTGCACAGAGCTGTCATTTGCCAGTGAGAGCCTCCGACGGGCAGGTACTGT
GCCAGGGCAGCTCTGAAATATGGATATTCTTACCTCCTGGTTCTTTCGGTGCAAATGGTA
ACCTAATACCAGCCGCAGGGAGCGCCATTTCT
>gi|5543768|gb|AI869800.1|AI869800 wm04a03.x1 NCI_CGAP_Ut4 Homo sapiens cDNA clone IMAGE:2434924 3' 
GTAAATAACAAACACCACTTTGTTATGAAGACCTTACAAACCTCTTCTTAAGACATTCTT
ACTCTGATCCAGGCAAAAACACTTCAAGGTTTGTAAATGACTCTTTCCTGACATAAATCC
TTTTTTATTAAAATGCAAAATGTTCTTCAGAATAAAACTGTGTAATAATTTTATACTTGG
GAGTGCTCCTTGCACAGAGCTGTCATTTGCCAGTGAGAGCCTCCGACGGNGCAGGTACTG
TGCCAGGGCAGCTCTGAATTATGGATATTCTTATCCTCCTG
>gi|4610281|gb|AI601252.1|AI601252 ar88c09.x1 Barstead colon HPLRB7 Homo sapiens cDNA clone IMAGE:2152336 3' 
TTTGTAAATAACAAACACCACTTTGTTATGAAGACCTTACAAACCTCTTCTTAAGACATT
CTTACTCTGATCCAGGCAAAAACACTTCAAGGTTTGTAAATGACTCTTTCCTGACATAAA
TCCTTTTTATTAAAATGCAAAATGTTCTTCAGAATAAAACTGTGTAATAATTTTTATACT
TGGGAGTGCTCCTTGCACAGAGCTGTCATTTGCCAGTGAGAGCCTCCGACGGGCAGGT
ACTGTGCCAGGGCAGCTCTGAAATTATGGATATTCTTATCCTCCTGGTTCCTTCGGTGCCA
ATGGTAACCTAATACCAGCCGCAGGGAGCGCCATTTCTCCTAAAGGGCTACACCACTGTC
AACATTATCCTGGACTCTGTGTCTCTCTCTGTTGGGTCTTGTGGCATCACATCAGGCCAAA
ATTGCCAGACCAGGACCCTAAGTGTCTGATAGAGGCGATGATCTTTTCCAAAGTCAGTAC
TTACAAACT
>gi|4330088|gb|AI467998.1|AI467998 tj84e10.x1 Soares_NSF_F8_9W_OT_PA_P_S1 Homo sapiens cDNA clone IMAGE:2148234 3' 
TTTTTTTTTTTTTTTTGGTCCAAAATTTTAATAGTATACAGACAACCTGTTAATTTTTTT
TTTTTTTTTTTTGTAAATAACAAACACCACTTTGTTATGAAGACCTTACAAACCTCTTCTT
AAGACATTCTTACTCTGATCCAGGCAAAAACACTTCAAGGTTTGTAAATGACTCTTTCCT
GACATAAATCCTTTTTATTAAAATGCAAAATGTTCTTCAGAATAAAACTGTGTAATAAT
TTTTATACTTGGGAGTGCTCCTTGCACAGAGCTGTCATTTGCCAGTGAGAGCCTCCGACG
GGGCAGGTACTGTGCCAGGGCAGCTCTGAAATTATGGATATTCTTATCCTCCTGGTTCCT
TCGGTGCCAATGGTAACCTAATACCAGCCGCAGGGAGCGCCATTTCTCCTAAAGGGCTAC
ACCACTGTCAACATTATCC
>gi|4311745|gb|AI459166.1|AI|459166 tj65h07.x1 Soares_NSF_F8_9W_OT_PA_P_S1 Homo sapiens cDNA clone IMAGE:2146429 3' 
TTTTTTTTGGTCCAAAATTTTAATAGTATACAGACAACCTGTTAATTTTTTTTTTTTT
TTTTTGTAAATAACAAACACCACTTTGTTATGAAGACCTTACAAACCTCTTCTTAAGACAT
TCTTACTCTGATCCAGGCAAAAACACTTCAAGGTTTGTAAATGACTCTTTCCTGACATAA
ATCCTTTTTATTAAAATGCAAAATGTTCTTCAGAATAAAACTGTGTAATAATTTTTATAC
 
TTGGGAGTGCTCCTTGCACAGAGCTGTCATTTGCCAGTGAGAGCCTCCGACGGGGCAGGT
ACTGTGCCAGGGCAGCTCTGAAATTATGGATATTCTTATCCTCCTGGTTCCTTCGGTGCCA
ATGGTAACCTAATACCAGCCGCAGGGAGCGCCATT
T
>gi|4222874|gb|AI393327.1|AI393327 tg44a12.x1 Soares_NFL_T_GBC_S1 Homo sapiens cDNA clone IMAGE:2111614 3' 
TTTATATTATTCACCACTTTGTTATGAAGACCTTACAAACCTCTTCTTAAGACATTCTTAC
TCTGATCCAGGCAAAAACACTTCAAGGTTTGTAAATGACTCTTTCCTGACATAAATCCTT
TTTTATTAAAATGCAAAATGTTCTTCAGAATAAAACTGTGTAATAATTTTATACTTGGGA
GTGCTCCTTGCACAGAGCTGTCATTTGCCAGTGAGAGCCTCCGACAGGGCAGGTACTGTG
CCAGGGCAGCTCTGAAATTATGGATATTCTTATCCTCCTGGTTCCTTCGGTGCCAATGGTA
ACCTAATACCAGCCGCAGGGAGCGCCATTTCTCCTAAAGGGCTACACCACTGTCAACATT
ATCCTGGACTCTGTGTCTCTCTCTGTTGGGTCTTGTGGCATCACATCAGGCCAAAATTGCC
AGACCAGGACCCTAAGTGTCTGATAGAGGCGATGATCTTTT
>gi|3933768|gb|AI290994.1|AI290994 qm09e05.x1 NCI_CGAP_Lu5 Homo sapiens cDNA clone IMAGE:18813443' 
TAAATAACAAACACCACTTTGTTATGAAGACCTTACAAACCTCTTCTTAAGACATTCTTA
CTCTGATCCAGGCAAAAACACTTCAAGGTTTGTAAATGACTCTTTCCTGACATAAATCCT
TTTTTATTAAAATGCAAAATGTTCTTCAGAATAAAACTGTGTAATAATTTTATACTTGGG
AGTGCTCCTTGCACAGAGCTGTCATTTGCCAGTGAGAGCCTCCGACGGGGCAGGTACTGT
GCCAGGGCAGCTCTGAAATTATGGATATTCTTATCCTCCTGGTTCCTTCGGTGCCAATGGT
AACCTAATACCAGCCGCAGGGAGCGCCATTTCTCCTAAAGGGCTACACCACTGTCAACAT
TATCCTGGACTCTGTGTCTCTCTCTGTTGGGTCTTGTGGCATCACATCAGGCCAAAATTGC
CAGACCAGGACCCTAAGTGTCTGATAGA
>gi|3844395|gb|AI248998.1|AI248998 qh80f04.x1 Soares_fetal_liver_spleen_1NFLS_S1 Homo sapiens cDNA clone IMAGE:1851007 3' 
TGTAAATAACAAACACCACTTTGTTATGAAGACCTTACAAACCTCTTCTTAAGACATTCT
TACTCTGATCCAGGCAAAAACACTTCAAGGTTTGTAAATGACTCTTTCCTGACATAAATC
CTTTTTTATTAAAATGCAAAATGTTCTTCAGAATAAAACTGTGTAATAATTTTATACTTG
GGAGTGCTCCTTGCACAGAGCTGTCATTTGCCAGTGAGAGCCTCCGACGGGCAGGTACT
GTGCCAGGGCAGCTCTGAAATTATGGATATTCTTATCCTCCTGGTTCCTTCGGTGCCAATG
GTAACCTAATACCAGCCGCAGGGAGCGCCATTTCTCCTAAAGGGCTACACCACTGTCAAC
 
ATTATCCTGGACTCTGTGTCTCTCTCTGTTGGGTCTTGTGGCATCACATCAGGCCAAAATT
GCCAGACCAGGACCCTAAGTGTCTGATAGAGGCGATGATCTTTTCCAAAGTCAGTACTTA
CAAACTGGCATTCTTACAG
>gi|3405022|gb|AI075844.1|AI075844 oz16d08.x1 Soares_fetal_liver_spleen_1NFLS_S1 Homo sapiens cDNA clone IMAGE:1675503 3' 
TTTTTTTTTTGTAAATAACAAACACCACTTTGTTATGAAGACCTTACAAACCTCTTCTTA
AGACATTCTTACTCTGATCCAGGCAAAAACACTTCAAGGTTTGTAAATGACTCTTTCCTG
ACATAAATCCTTTTTATTAAAATGCAAAATGTTCTTCAGAATAAAACTGTGTAATAATTT
TTATACTTGGGAGTGCTCCTTGCACAGAGCTGTCATTTGCCAGTGAGAGCCTCCGACGGG
GCAGGTACTGTGCCAGGGCAGCTCTGAAATTATGGATATTCTTATCCTCCTGGTTCCTTCG
GTGCCAATGGTAACCTAATACCAGCCGCAGGGAGCGCCATTTCTCCTAAAGGGCTACAC
CACTGTCAACATTATCCTGGACTCTGTGTCTCTCTCTGTTGGGTCTTG
>gi|3307963|gb|AI051972.1|AI051972 ow83h10.x1 Soares_fetal_liver_spleen_1NFLS_S1 Homo sapiens cDNA clone IMAGE:1653475 3' 
TTTTTTTTGTAAATAACAAACACCACTTTGTTATGAAGACCTTACAAACCTCTTCTTAAG
ACATTCTTACTCTGATCCAGGCAAAAACACTTCAAGGTTTGTAAATGACTCTTTCCTGAC
ATAAATCCTTTTTATTAAAATGCAAAATGTTCTTCAGAATAAAACTGTGTAATAATTTTT
ATACTTGGGAGTGCTCCTTGCACAGAGCTGTCATTTGCCAGTGAGAGCCTCCGACAGGGC
AGGTACTGTGCCAGGGCAGCTCTGAAATTATGGATATTCTTATCCTCCTGGTTCCTTCGGT
GCCAATGGTAACCTAATACCAGCCGCAGGGAGCGCCATTTCTCCTAAAGGGCTACACCA
CTGTCAACATTATCCTGGACTCTGTGTCTCTCTCTGTTGAGTCTTGTGGCATCACATCAGG
CCAAAATTGCCAGACCAGGACCCTAAGTGTCTGATAGAGGCGATGATCTT
>gi|3254601|gb|AI033648.1|AI033648 ow22e09.x1 Soares_parathyroid_tumor_NbHPA Homo sapiens cDNA clone IMAGE: 1647592 3' 
TGTAAATAACAAACACCACTTGGTTATGAAGACCTTACAAACCTCTTCTTAAGACATTCT
TACTCTGATCCAGGCAAAAACACTTCAAGGTTTGTAAATGACTCTTTCCTGACATAAATC
CTTTTTATTAAAATGCAAAATGTTCTTCAGAATAAAACTGTGTAATAATTTTATACTTG
GGAGTGCTCCTTGCACAGAGCTGTCATTTGCCAGTGAGAGCCTCCGACGGGCAGGTACT
GTGCCAGGGCAGCTCTGAAATTATGGATATTCTTATCCTCCTGGTTCCTTCGGTGCCAATG
GTAACCTAATACCAGCCGCAGGGAGCGCCATTTCTCCTAAAGGGCTACACCACTGTCAAC
ATTATCCTGGACTC
>gi|3229367|gb|AI015031.1|AI015031 ot30f04.s1 Soares_testis_NHT Homo sapiens cDNA clone IMAGE:1618303 3' 
TTTTTCTTACAAAGAAAAATTTAATATTCGATGAGAGGTTGAACCAGGCTTAAAGCAGAC
ATACTAGGAAATGGTGCAGCCTGTAAGAATGCCAGTTTGTAAGTACTGACTTTGGAAAA
GATCATCGCCTCTATCAGACACTTAGGGTCCTGGTCTGGCAATTTTGGCCTGATGTGATG
CCACAAGACCCAACAGAGAGAGACACAGAGTCCAGGATAATGTTGACAGTGGTGTAGCC
CTTTAGGAGAAATGGCGCTCCCTGCGGCTGGTATTAGGTTACCATTGGCACCGAAGAGAC
CAGGAGGATAAGAATATCCATAATTTCAGAGCTGCCCTGGCACAGTACCTGCCCCGTCG
GAGGCTCTCACTGGCAAATGACAGCTCTGTGCAAGGAGCACTCCCAAGTATAAAAATTA
TTACACAGTTTTATTCTG
>gi|3055333|gb|AA915941.1|AA915941 on18d06.s1 NCI_CGAP_Lu5 Homo sapiens cDNA clone IMAGE: 1557035 3' 
TTGTAAATAACAAACACCACTTTGTTATGAAGACCTTACAAACCTCTTCTTAAGACATTC
TTACTCTGATCCAGGCAAAAACACTTCAAGGTTTGTAAATGACTCTTTCCTGACATAAAT
CCTTTTTATTAAAATGCAAAATGTTCTTCAGAATAAAACTGTGTAATAATTTTATACTT
GGGAGTGCTCCTTGCACAGAGCTGTCATTTGCCAGTGAGAGCCTCCGAAGGGGCAGGTA
CTGTGCCAGGGCAGCTCTGAAATTATGGATATTCTTATCCTCCTGGTTCCTTCGGTGCCAA
TGGTAACCTAATACCAGCCGCAGGAGCGCCATTTCTCCTAAAGGGCTACACCACTGTCAA
CATTATCCTGGACTCTGTGTCTCTCTCTGTTGGGTCTTGTGGCATCACATCAGGCCAAAAT
TGCCAGACCAGGACCCTAAGTGTCTGATAGAGGCGATGATCTTTTCCAAAGTCAGTACTT
A
>gi|2953300|gb|AA861160.1|AA861160 ak36b12.s1 Soares_testis_NHT Homo sapiens cDNA clone IMAGE:1408031 3' 
TTTGTAAATAACAAACACCACTTTGTTATGAAGACCTTACAAACCTCTTCTTAAGACATT
CTTACTCTGATCCAGGCAAAAACACTTCAAGGTTTGTAAATGACTCTTTCCTGACATAAA
TCCTTTTTATTAAAATGCAAAATGTTCTTCAGAATAAAACTGTGTAATAATTTTATACT
TGGGAGTGCTCCTTGCACAGAGCTGTCATTTGCCAGTGAGAGCCTCCGAC
>gi|1782040|gb|AA192157.1|AA192157 zq02g05.s1 Stratagene muscle 937209 Homo sapiens cDNA clone IMAGE:628568 3' 
GTAAATAACAAACACCACTTTGTTATGAAGACCTTACAAACCTCTTCTTAAGACATTCTT
ACTCTGATCCAGGCAAAAACACTTCAAGGTTTGTAAATGACTCTTTCCTGACATAAATCC
TTTTTTATTAAAATGCAAAATGTTCTTCAGAATAAAACTGTGTAATAATTTTTATACTTGG
 
GAGTGCTCCTTGCACAGAGCTGTCATTTGCCAGTGAGAGCCTCCGACGGGGCAGGTACTG
TGCCAGGGCAGCTCTGAAATTATGGATATTCTTATCCTCCTGGTTCCTTCGGTGCCAATGG
TAACCTAATACCAGCCGCAGGAGCGCCATTTCTCCTAAAGGGCTACACCACTGTCAACAT
TATCCTGGGACTCTGTGTCTCTCTCTGTTGGGTCTTGTGGCATCACATCAGGCCAAAATTG
GCCAGACCAGGACCCCAAGTGGTCTGATAGAAGGCGATGATCTTTTCCAAAGTCAGTAC
TTACA
>gi|1230953|gb|N73668.1|N73668 yz78h05.s1 Soares_multiple_sclerosis_2NbHMSP Homo sapiens cDNA clone IMAGE:289209 3' 
GGTCCAAAATTTTAATAGTATACAGACAACCTGTTAATTTTTTTTTTTTTTTTTTGTAA
ATAACAAACACCACTTTGTTATGAAGACCTTACAAACCTCTTCTTAAGACATTCTTACTCT
GATCCAGGCAAAAACACTTCAAGGTTTGTAAATCGACTCTTTCCTGACATAAATCCTTTT
TTATTAAAATNGCAAAATTGTTCTTCAGAATAAAACTGTGTAATAATTTTTATACTTGGG
AGTGCTCCTTGCACAGAGCTGTCATTTGCCAGTGAGAGCCTCCGACGGGGCAGGTACTGT
GCCAGGGCAGCTCTGAAATTATGGAAATTCTTATCCCCCTGGTTCCTNCGGTGGCCAATG
GGTAACCTAATACCAGCCCGCGGGAAGCGCCAATTTCNCCCAAAAGGGGGTAAACCACT
GGTNAAACATTA
>gi|2142013|gb|AA437099.1|AA437099 zv53b09.s1 Soares_testis_NHT Homo sapiens cDNA clone IMAGE:757337 3' 
CTTACAAAGAAAAATTTAATATTCGATGAGAGGTTGAACCAGGCTTAAAGCAGACATAC
TAGGAAATGGTGCAGCCTGTAAGAATGCCAGTTTGTAAGTACTGACTTTGGAAAAGATC
ATCGCCTCTATCAGACACTTAGGGTCCTGGTCTGGCAATTTGGCCTGATGTGATGCCAC
AAGACCCAACAGAGAGAGACACAGAGTCCAGGATAATGTTGACAGTGGTGTAGCCCTTT
AGGAGAAATGGCGCTCCCTGCGGCTGGTATTAGGTTACCATTGGCACCGAAGAGACCAG
GAGGATAAGAATATCCATAATTTCAGAGCTGCCCTGGCACAGTACCTGCCCCGTCGGAG
GCTCTCACTGGCAAATGACAGCTCTGTGCAAGGAGCACTCCCAAGTATAAAAATTAT
>gi|3836330|gb|AI240933.1|AI240933 qk01b11.x1 NCI_CGAP_Kid3 Homo sapiens cDNA clone IMAGE: 1867677 3' 
TTTTTTTTTTTTTTTTTTTTGGTCCAAAATTTTAATAGTATACAGACAACCTGTTAATTTT
TTTTTTTTTTTTTTTGGAAATAACAAACACCACTTTGTTATGAAGACCTTACAAACCTCT
TCTTAAGACATTCTTACTCTGATCCAGGCAAAAACACTTCAAGGTTTGGAAATGACTCTT
TCCTGACATAAATCCTTTTTATTAAAATGCAAAAGGTTCTTCAGAATAAAACTGTGTAA
TAATTTTTATACTTGGGAGTGCTCCTTGCACAGAGCTGTCATTTGCCAG
>gi|2669971|gb|AA682690.1|AA682690 zj86f07.s1 Soares_fetal_liver_spleen_1NFLS_S1 Homo sapiens cDNA clone (IMAGE:461797 3' 
AATGCAAAATGTTCTTCAGAATAAAACTGTGTAATAATTTTTATACTTGGGATGTGCTCC
TTGCACAGAGCTGTCATTTGCCAGTGAGAGCCTCGACAGGCAGGTACTGTGCCAGGGCA
GCTCTGAAATTATGGATATTCTTATCCTCCTGGTTCCTTCTGTGCTCAATGGTAACCTAAT
ACCAGCCGCAGGACNCGCCATTTCTCCTAAAGGGCTACACCACTGTCAACATTATC
>gi|1162804|gb|N39597.1|N39597 yy51e04.s1 Soares_multiple_sclerosis_2NbHMSP Homo sapiens cDNA clone IMAGE:277086 3' 
ACAAAGAAAAATTTAATATTCGATGAGAGGTTGAACCAGGCTTAAAGCAGACATACTAG
GAAATGGTGCAGCCTGTAAGAATGCCAGTTTGTAAGTACTGACTTTGGAAAAGATCATC
GCCTCTATCAGACACTTAGGGTCCTGGTCTGGCAATTTTGGCCTGATGTGATGCCACAAG
ACCCAACAGAGAGAGACACAGAGTCCAGGATAATGTTGACAGTGGTGTAGCCCTTTAGG
AGAAATGGCGCTCCCTGCGGCTGGTATTAGGTTACCATTGGCACCGAAGAACCAGGAGG
ATAAGAATATCCATAATTTCAGAGCTTGCCCTGGCACAGTACCTGCCCCGTCGGAGGCTC
TCACTGGGCAAATGGACAGCTCTGTGCAAGGAGCACTCCCAAGTATAANAATTATTACA
CAGTTTTATTCTGAAGAACATTTTGCATTTTAATAAAAANGGA
>gi|3838507|gb|AI243110.1|AI243110 qh26f06.x1 Soares_NFL_T_GBC_S1 Homo sapiens cDNA clone IMAGE:1845827 3'

TTTTTCTTACAAAGAAAAATTTAATATTCGATGAGAGGTTGAACCAGGCTTAAAGCAGAC
ATACTAGGAAATGGTGCAGCCTGTAAGAATGCCAGTTTGTAAGTACTGACTTTGGAAAA
GATCATCGCCTCTATCAGACACTTAGGGTCCTGGTCTGGCAATTTTGGCCTGATGTGATG
CCACAAGACCCAACAGAGAGAGACACAGAGTCCAGGATAATGTTGACAGTGGTGTAGCC
CTTTAGGAGAAATGGCGCTCCCTGCGGCTGGTATTAGGTTACCATTGGCACCGAAGGAAC
CAGGAGGATAAGAATATCCATAATTTCAGAGCTGCCCTGGCACAGTACCTGCCCCGTCG
GAGGCTCTCACTGGCAAATGACAGCTCTGTGCAAGGAGCACTC

Sequences identified as those of QPRT cluster
BC005060 
1 caccagccca gacagctgca agtcaccatg gacgctgaag gcctggcgct gctgctgccg
61 cccgtcaccc tggcagccct ggtggacagc tggctccgag aggactgccc agggctcaac
121 tacgcagcct tggtcagcgg ggcaggcccc tcgcaggcgg cgctgtgggc caaatcccct
181 gggatactgg cagggcagcc tttcttcgat gccatattta cccaactcaa ctgccaagtc
241 tcctggttcc tccccgaggg atcgaagctg gtgccggtgg ccagagtggc cgaggtccgg
301 ggccctgccc actgcctgct gctgggggaa cgggtggccc tcaacacgct ggcccgctgc
361 agtggcattg ccagtgctgc cgccgctgca gtggaggccg ccaggggggc cggctggact
421 gggcacgtgg caggcacgag gaagaccacg ccaggcttcc ggctggtgga gaagtatggg
481 ctcctggtgg gcggggccgc ctcgcaccgc tacgacctgg gagggctggt gatggtgaag
541 gataaccatg tggtggccgc cggtggcgtg gagaaggcgg tgcgggcggc cagacaggcg
601 gctgacttcg ctctgaaggt ggaagtggaa tgcagcagcc tgcaggaggc cgtgcaggca
661 gctgaggctg gtgccgacct tgtcctgctg gacaacttca agccagagga gctgcacccc
721 acggccaccg tgctgaaggc ccagttcccg agtgtggctg tggaagccag tgggggcatc
781 accctggaca acctccccca gttctgcggg ccgcacatag acgtcatctc catggggatg
841 ctgacccagg cggccccagc ccttgatttc tccctcaagc tgtttgccaa agaggtggct
901 ccagtgccca aaatccacta gtcctaaacc ggaagaggat gacaccggcc atgggttaac
961 gtggctcctc aggaccctct gggtcacaca tctttagggt cagtggccaa tggggcacat
1021 ttggcactag cttgagccca actctggctc tgccacctgc tgctcctgtg acctgtcagg
1081 gctgacttca cctctgctca tctcagtttc ctaatctgta aaatgggtct aataaaggat
1141 caaccacatg gggttctgcg gtgataatga gcacatagtg aggggtcagc aaatgtcaga
1201 agttacctgg gacagccggg cacgatggct cacacctgta atcccagcac tttgggaggc
1261 tgaggcggga agatcacttg agttcaggag tttgagacca gcctggccaa catggtgaaa
1321 ccccatctct accaaaaata gaagaattag ctgggtgtgg tggcacgcgc ctgtaatccc
1381 agctacttag gaggctgagg caggagaatc gcttgaaccc aggaagtgga ggttgcagtg
1441 agctgatggt gccactgcac tccagcctgg gtgatagagc gagactctgt ctccaaagaa
1501 gaaacaaaaa aaaaaaaaaa aaaa
BE299670 
1 gtggccacca ctcagctcga tgcaacctac acttcctggg ttcaagcgat tctcctgcct
61 cagcctccta agtagctggg attacaggcg cgtgccacca cacccagcta attcttctat
121 ttttggtaga gatggggttt caccatgttg gccaggctgg tctcaaactc ctgaactcaa
181 gtgatcttcc cgcctcagcc tcccaaagtg ctgggattac aggtgtgagc catcgtgccc
241 ggctgtccca ggtaacttct gacatttgct gacccctcac tatgtgctca ttatcaccgc
301 agaaccccat gtggttgatc ctttattaga cccattttac agattaggaa actgagatga
361 gcagaggtga agtcagccct gacaggtcac aggagcagca ggtggcagag ccagagttgg
421 gctcaagcta gtgccaaatg tgccccattg gccactgacc ctaaaagatg tgtgacccag
481 agggtcctga ggagccacgt taacccatgg ccggtgtcat cctcttccgg tttaggacta
541 gtggattttc gggcactgga gccacctctt tggcaaacta gctttgaggg agaaatcaag
601 ggctggggcc gctgggtcag catccccatg gagatgacgt ctatgtgcgg ccgccgaact
661 gggggaggtt gtccaggttg atgccccact ggcttcccag ccacactcgg gaaccggggc
721 ttccgcacgg tggccgttgg ggtgcagctc ctctggcttg aagttgtcca gcaggccaag
781 gtcggcacgc tcagctgctg cacgctctgc agctgcgtcc cactccactt cgagcaaccg
841 cgctgtttgc cgccaacgct atcaggcacg gggcacctgt atcttacctc ccgcccccgt
901 taggggcggg cccacaggca ttcacgcgac tggtaacccg agccgaccgc cggcaggaga
961 caggcgtgcc acgaggccac cgtcgcagtt tcgtcccgtg cctc
BE273102 
1 atggacgctg aaggcctggc tgctgctgct gcctgccctg tcacccttgc agccctggtg
61 gacagcgtgg ctccgagagg actgcccagt ggctcaacta cgcagccttg gtcagcgggg
121 caggcccctc cgcagtgcgg cgcgttgtgg ggcaagatcc cctgggtgta ctggcagtgg
 
181 cagccgtttc gttcgatgcc gatatttacc cagactcaac ttgccaaggt ctccttggtt
241 ccgtcccctg agggcgatcg aagcttggtt gcctggttgg gccagagttg gcctgagtgt
301 cctgtgtggc cgctgccgca cttgccttgc ttggctgggg ggaacggggt tggccgcgtc
361 aagcactgct tgtgccctgg ctgcatgttg tgcatttgcc catgtgcttg cctggcctgc
421 ttgcatgttg tgagtgcctg cccagtgtgg tgcctgtgct gtgagctggt gcacgtgtgt
481 gcagtggcga ctgagggaat gacccactgc ccgaggggct tcctggcctg tgttggacgc
541 aacgtattgg ggcgtcctgt gtgggcgggg gcgcgcgctc tgcaccggct tacggaccct
601 gggtgagggc tgcgtgcatg cgtgaagggg attaacgcat gttgttttgg cccgcctggt
661 ggcgctgtgt gacagtgcgg tgctggcctg tgccaacatg gcgtgctgac gttcgggtc
BC010033 
1 cagacagctg caagtcacca tggacgctga aggcctggcg ctgctgctgc cgcccgtcac
61 cctggcagcc ctggtggaca gctggctccg agaggactgc ccagggctca actacgcagc
121 cttggtcagc ggggcaggcc cctcgcaggc ggcgctgtgg gccaaatccc ctggggtact
181 ggcagggcag cctttcttcg atgccatatt tacccaactc aactgccaag tctcctggtt
241 cctccccgag ggatcgaagc tggtgccggt ggccagagtg gccgaggtcc ggggccctgc
301 ccactgcctg ctgctggggg aacgggtggc cctcaacacg ctggcccgct gcagtggcat
361 tgccagtgct gccgccgctg cagtggaggc cgccaggggg gccggctgga ctgggcacgt
421 ggcaggcacg aggaagacca cgccaggctt ccggctggtg gagaagtatg ggctcctggt
481 gggcggggcc gcctcgcacc gctacgacct gggagggctg gtgatggtga aggataacca
541 tgtggtggcc gccggtggcg tggagaaggc ggtgcgggcg gccagacagg cggctgactt
601 cgctctgaag gtggaagtgg aatgcagcag cctgcaggag gccgtgcagg cagctgaggc
661 tggtgccgac cttgtcctgc tggacaactt caagccagag gagctgcacc ccacggccac
721 cgtgctgaag gcccagttcc cgagtgtggc tgtggaagcc agtgggggca tcaccctgga
781 caacctcccc cagttctgcg ggccgcacat agacgtcatc tccatgggga tgctgaccca
841 ggcggcccca gcccttgatt tctccctcaa gctgtttgcc aaagaggtgg ctccagtgcc
901 caaaatccac tagtcctaaa ccggaagagg atgacaccgg ccatgggtta acgtggctcc
961 tcaggaccct ctgggtcaca catctttagg gtcagtggcc aatggggcac atttggcact
1021 agcttgagcc caactctggc tctgccacct gctgctcctg tgacctgtca gggctgactt
1081 cacctctgct catctcagtt tcctaatctg taaaatgggt ctaataaagg atcaaccaca
1141 tggggttctg cggtgataat gagcacatag tgaggggtca gcaaatgtca gaagttacct
1201 gggacagccg ggcacgatgg ctcacacctg taatcccagc actttgggag gctgaggcgg
1261 gaagatcact tgagttcagg agtttgagac cagcctggcc aacatggtga aaccccatct
1321 ctaccaaaaa tagaagaatt agctgggtgt ggtggcacgc gcctgtaatc ccagctactt
1381 aggaggctga ggcaggagaa tcgcttgaac ccaggaagtg gaggttgcag tgagctgatg
1441 gtgccactgc actccagcct gggtgataga gcgagactct gtctccaaag aagaaaaaaa
1501 aaaaaaaaaa a
BC018910 
1 gtcctgagca gccaacacac cagcccagac agctgcaagt caccatggac gctgaaggcc
61 tggcgctgct gctgccgccc gtcaccctgg cagccctggt ggacagctgg ctccgagagg
121 actgcccagg gctcaactac gcagccttgg tcagcggggc aggcccctcg caggcggcgc
181 tgtgggccaa atcccctggg gtactggcag ggcagccttt cttcgatgcc atatttaccc
241 aactcaactg ccaagtctcc tggttcctcc ccgagggatc gaagctggtg ccggtggcca
301 gagtggccga ggtccggggc cctgcccact gcctgctgct gggggaacgg gtggccctca
361 acacgctggc ccgctgcagt ggcattgcca gtgctgccgc cgctgcagtg gaggccgcca
421 gggggccggg ctggactggg cacgtggcag gcacgaggaa gaccacgcca ggcttccggc
481 tggtggagaa gtatgggctc ctggtgggcg gggccgcctc gcaccgctac gacctgggag
541 ggctggtgat ggtgaaggat aaccatgtgg tggccgccgg tggcgtggag aaggcggtgc
601 gggcggccag acaggcggct gacttcgctc tgaaggtgga agtggaatgc agcagcctgc
661 aggaggccgt gcaggcagct gaggctggtg ccgaccttgt cctgctggac aacttcaagc
721 cagaggagct gcaccccacg gccaccgtgc tgaaggccca gttcccgagt gtggctgtgg
781 aagccagtgg gggcatcacc ctggacaacc tcccccagtt ctgcgggccg cacatagacg
841 tcatctccat ggggatgctg acccaggcgg ccccagccct tgatttctcc ctcaagctgt
 
901 ttgccaaaga ggtggctcca gtgcccaaaa tccactagtc ctaaaccgga agaggatgac
961 accggccatg ggttaacgtg gctcctcagg accctctggg tcacacatct ttagggtcag
1021 tggccaatgg ggcacatttg gcactagctt gagcccaact ctggctctgc cacctgctgc
1081 tcctgtgacc tgtcagggct gacttcacct ctgctcatct cagtttccta atctgtaaaa
1141 tgggtctaat aaaggatcaa ccacaaaaaa aaaaaaaaaa aaaaaaaaaa aaaaaaaaaa
1201 aaaaaaaaaa aaaaaaaaaa aaaaaaaaaa aaaaaaaaaa
BE902622 
1 agcccagaca gctgcaagtc acatggacgc tgaaggcctg gcgctgctgc tgccgcccgt
61 caccctggca gccctggtgg acagctggct ccgagaggac tgcccagggc tcaactacgc
121 agccttggtc agcggggcag gcccctcgca ggcggcgctg tgggccaaat cccctggggt
181 actggcaggg cagcctttct tcgatgccat atttacccaa ctcaactgcc aagtctcctg
241 gttcctcccc gagggatcga agctggtgcc ggtggccaga gtggccgagg tccgggcccc
301 tgcccactgc ctgctgctgg gggaacgggt ggccctcaac acgctggccg ctgcagtggc
361 attgccagtg ctgccgccgc tgcagtggag gccgcagggg ggccggctgg actgggcacg
421 tggcaggcac gaggaagacc acgccaggct tccggctggt ggagaagtat gggctcctgg
481 tgggcgggcc gctcgcaccg tacgacctgg gagggctggt gatggtgaag gataaccatg
541 tggtggccgc cggtggcgtg gagaaggcgg tgcgggcggc agacaggcgg tgacttcgct
601 ctgaaggtgg aagtggaatg cagcagcctg caggaggccg tgcaggcagt gaggctggtg
661 cgacttgtcc tgctggacaa ttcagccaga ggagctgcac ccaggcacgt tgctgaaggc
721 cagtccgagt gtggtgtgga accaggtggg ggatcacctg gaaacctccc caatctgcgg
N23182 
1 gtggttgatc ctttattaga cccattttac agattaggaa actgagatga gcagaggtga
61 agtcagccct gacaggtcac aggagcagca ggtggcagag cagaggttgg gctcaagcta
121 gtgccaaatg tgccccattg gccactgacc ctaaagatgt gtgacccaga ggncttgagg
181 agccacgtta acccatggcc ggtgtcatcc tcttccggtt taggactagt ggattttggg
241 cactggagcc acctctttgg caaacagctt gaagggagaa atcaagggct ggggccgcct
301 gggtcagcat ccccatggag atgacgtcta tntgcggccc gcagaactgg gggaggttgt
361 ccagggtgat gcccccactg gcttccacag ccacactcgg gaactgggcc ttcagcacgg
421 tggccgtg
N32648 
1 agccaacaca ccannccaga cagctgcaag tcaccatgga cgctgaaggc tggcgctgct
61 gctgccgccc gtcaccctgg cagccctggt ggacagctgg ctccgagaga ctgcccaggg
121 ctcaactacg cagccttggt cagcggggca ggccctcgca ggcggcgctg tgggccaaat
181 cccctggggt actggcaggg cagcctttct tcgatgccat atttacccaa ctcaactgcc
241 aagtctcctg gttcctcccc gagggatcga agctggtgcc ggtgncagag tggcgaaggt
301 ccggggccct gccaactgcc tgctgctggg ggaacggtgg gccctcaaca cgctggcccg
361 ctgcagtggc attncagtgc tgccgccgct g
BE735342 
1 tagtcaggct gggtgttaat cggcaccgag ggcaagtcac catggacgct gaaggctggc
61 gctgctgctg ccgcccgcac cctggcagcc ctggtggaca gctggctccg agaggactgc
121 ccaggctcaa ctacgcagcc ttggtcagcg gggcaggccc ctcgcaggcg gcgctgtggg
181 ccaaatcccc tggggtactg gcagggcagc ctttcttcga tgccatattt acccaactca
241 actgccaagt ctcctggttc ctccccgagg gatcgaagct ggtgccggtg gccagagtgg
301 ccgaggtccg gggccgcctc gcaccgctac gacctgggag ggctggtgat ggtgaaggat
361 aaccatgtgg tggccgccgg tggcgtggag aaggcggtgc gggcggccag acaggcggct
421 gacttcgctc tgaaggtgga agtggaatgc agcagcctgc aggaggccgt gcaggcagct
481 gaggctggtg ccgaccttgt cctgctggac aacttcaagc cagaggagct gcaccccacg
541 gccaccgtgc tgaaggccca gttcccgagt gtggctgtgg aagccagtgg gggcatcacc
 
601 ctggacaact cccccagttc tgcgggccgc acatagacgt catctccatg gggatgctga
661 cccaggcggc ccagcccttg atttctccct caagctgttt tgccacagag gtggctccag
721 tgcccaaaaa tcactagtcc taaaccggaa gaggatgaca ccggcatggg taacgtggct
781 cctcagaccc tctgggtcac acatcttagg gtccaggcca atgggcccat ttggccctac
841 ttgagcccaa tctggtctgc actgctgtct gtgaccgtca gggtagcttc acttctgctc
901 ctctcagttc taacctgtaa cggtcctcta agcgtcaccc caaaaaaaaa aaactcggtc
961 ttttcgtcgg cgaaaggtcg aaggctgtcc gtccgcacct g
BX283118 
1 gacagctgca agtcaccatg gacgctgaag gcctggcgct gctgctgccg cccgtcaccc
61 tggcagccct ggtggacagc tggctccgag aggactgccc agggctcaac tacgcagcct
121 tggtcagcgg ggcaggcccc tcgcaggcgg cgctgtgggc caaatcccct ggggtactgg
181 cagggcagcc tttcttcgat gccatattta cccaactcaa ctgccaagtc tcctggttcc
241 tccccgaggg atcgaagctg gtgccggtgg ccagagtggc cgaggtccgg ggccctgccc
301 actgcctgct gctgggggaa cgggtggccc tcaacacgct ggcccgctgc agtggcantg
361 ccagtgctgc cgccgctgca gtggaggccg ccaggggggc cggctggact gggcacgtgg
421 caggcacgag gaagaccacg ccaggcttcc ggctggtgga gaagtatggg ctcctggtgg
481 gcggggccgc ctcgcaccgc tacgacctgg gaggg
BG769505 
1 ggacgctgaa ggcctggcgc tggtgctgcc gcccgtcacc ctggcagccc tggtggacag
61 ctggctccga gaggactgcc cagggctcaa ctacgcagcc ttggtcagcg gggcaggccc
121 ctcgcaggcg gcgctgtggg ccaaatcccc tggggtactg gcagggcagc ctttcttcga
181 tgccatattt acccaactca actgccaagt ctcctggttc ctccccgagg gatcgaagct
241 ggtgccggtg gccagagtgg ccgaggtccg gggccctgcc cactgcctgc tgctggggga
301 acgggtggcc ctcaacacgc tggcccgctg cagtggcatt gccagtgctg ccgccgctgc
361 agtggaggcc gccagggggg ccggctggac tgggcacgtg gcaggcacga ggaagaccac
421 gccaggcttc cggctggtgg agaagtatgg gctcctggtg ggcggggccg cctcgcaccg
481 ntacgacctg ggagggctgg tgatggtgaa ggataaccat gtggtggccg ccggtggcgt
541 ggagaaggcg gtgcgggcgg cagacaggcg gctgacttcg ctctgaacgg tggcaagtgg
601 actgcagcag cctgcagcga ggccgtgcag gcagctgagg ctggtgccga ccttgtcctg
661 ctggacaact tcaagccaga ggagctgcaa ccccacggcc acgtgctgaa ggccagttcc
721 agagtgtggc tgtggacgcc agtggcggca tcacctggac aacctccccc agttctgcgg
781 gccgcacata gacgtcatct tccatggcga tgctgaccca ggcggcccca gccctggatt
841 tctcctcagc tggttgcaca gaggtgcctc agtgcccaaa tcactcatct aacc
BG750434 
1 ggacagctgc aagtcaccat ggacgctgaa ggcctggcgc tgctgctgcc gcccgtcacc
61 ctggcagccc tggtggacag ctggctccga gaggactgcc cagggctcaa ctacgcagcc
121 ttggtcagcg gggcaggccc ctcgcaggcg gcgctgtggg ccaaatcccc tggggtactg
181 gcagggcagc ctttcttcga tgccatattt acccaactca actgccaagt ctcctggttc
241 ctccccgagg gatcgaagct ggtgccggtg gccagagtgg ccgaggtccg gggccctgcc
301 cactgcctgc tgctggggga acgggtggcc ctcaacacgc tggcccgctg cagtggcatt
361 gccagtgctg ccgccgctgc agtggaggcc gccagggggg ccggctggac tgggcacgtg
421 gcaggcacga ggaagaccac gccaggcttc cggctggtgg agaagtatgg gctcctggtg
481 ggcggggccg cctcgcaccg ctacgacctg ggagggctgg tgatggtgaa cggataacca
541 tgtggtggcc gccggtggcg tggagaaggc ggtgcgggcg gccagaccag gcggctgact
601 tcgctctgaa ggtggaagtg gaatgcagca gcctgcagga ggccgtgcag gcagcttgag
661 gctggtgccg accttgtcct gcttggacaa cttcaaagcc aggaggagct ggacccacgg
721 ccaccgtgct gaaggccagt tcccgagtgt ggctgtggaa gccagtgggg gcatcagcct
781 ggacaagctc cccccagttc ctggagggcc cgcacattaa accgtcatct ccatggggga
841 ttgctgaacc aaggcggacc cagcccttgg atttctcctc aagctgttgc cacaggaggt
901 ggtccagtgg ccaaaatcca ctagtcctaa acgggaagga tgacccgggc catggttacc
961 gggtcctaag acct
BG766440 
1 ggctgcaagt caccatggac gctgaaggcc tggcgctgct gctgccgccc gtcaccctgg
61 cagccctggt ggacagctgg ctccgagagg actgcccagg gctcaactac gcagccttgg
121 tcagcggggc aggcccctcg caggcggcgc tgtgggccaa atcccctggg gtactggcag
181 ggcagccttt cttcgatgcc atatttaccc aactcaactg ccaagtctcc tggttcctcc
241 ccgagggatc gaagctggtg ccggtggcca gagtggccga ggtccggggc cctgcccact
301 gcctgctgct gggggaacgg gtggccctca acacgctggc ccgctgcagt ggcattgcca
361 gtgctgccgc cgctgcagtg gaggccgcca ggggggccgg ctggactggg cacgtggcag
421 gcacgaggaa gaccacgcca ggcttccggc tggtggagaa gtatgggctc ctggtgggcg
481 gggccgcctc gcaccgctac gacctgggag ggctggtgat ggtgaaggat aaccatgtgg
541 tggccgccgg tggcgtggag acaggcggtg cgggcggcca gacaggcggc ttgacttcgc
601 tctgaaggtg gaagcggaat tgcagcagcc tgcaggaggc cgtgcaggca gctgaggctg
661 gtgccgacct tgtcctgctg gacaacttca agccagagga gctgaaccca cggcaccgtg
721 ctgaaagcca gttcccgagt gtggctgtgg aagccagtgg gggcatcacc tggaacacct
781 cccca
BG401877 
1 agccttggtc ctgagcagcc aacacaccag cccagacagc tgcaagtcac catggacgct
61 gaaggcctgg cgctgctgct gccgcccgtc accctggcag ccctggtgga cagctggctc
121 cgagaggact gcccaggctc aactacgcag ccttggtcag cggggcaggc ccctcgcagg
181 cggcgctgtg ggccaaatcc cctggggtac tggcagaggc agcctttctt cgatgccata
241 tttacccaac tcaactgcca agtctcctgg ttcctccccg agggatcgaa gctggtggag
301 aagtatgggc tcctggtggg cggagccgac tcgcaccgct acgaccctga gagggctggt
361 gatggtgaag gataaccatg tggtggccgc cggtggcgtg gagaaggcgg tgcgggcggc
421 cagacaggcg gctgacttcg ctctgaaggt ggaagtggaa tgcagcacgc ctggcaggag
481 ggccgtgcag gcagctgagg ctggtgcgac cttgtcactg ctgagcactt caagccagag
541 aagctgcacc ccagagccac gtgcttgaag ccccagtcca aagtgtggct gtgaaaccag
601 tgcggcttca ccctgtgaca cctcccccag tactgctgcc ggcattagac gtcatctcca
661 tgggatgctg ccaggatgcc agcctgaatc tccctcagct gtgccaaaag tgtccagtcc
721 aatcactatc tacgaaaaga taccggacag gttagggcca agcctggtac acttagacga
781 cccagggcac ttgcatagtg cactaggtga cgagtctgag agagaagacc ggaacataca
841 aggaaagacc agtcagaaat ggagagcagc aagaaaggac ggcccacatg ctaactct
BG337811 
1 gagccaacac accagcccag acagctgcaa gtcaccatgg acgctgaagg cctggcgctg
61 ctgctgccgc ccgtcaccct ggcagccctg gtggacagct ggctccgaga ggactgccca
121 gggctcaact acgcagcctt ggtcagcggg gcaggcccct cgcaggcggc gctgtgggcc
181 aaatcccctg gggtactggc agggcagcct ttcttcgatg ccatatttac ccaactcaac
241 tgccaagtct cctggttcct ccccgaggga tcgaagctgg tgccggtggc cagagtggcc
301 gaggtccggg gccctgccca ctgcctgctg ctgggggaac gggtggccct caacacgctg
361 gcccgctgca gtggcattgc cagtgctgcc gccgctgcag tggaggccgc caggggggcc
421 ggctggactg ggcacgtggc aggcacgagg aagaccacgc caggcttccg gctggtggag
481 aagtatgggc tcctggtggg cggggccgcc tcgcaccgct acgacctggg agggctggtg
541 atggtgaagg ataaccatgt ggtggccgac ggtggcgtgg aagaaggcgg tgcggcggc
601 cagacaggcg gctgacttcg ctctgaaggt ggaagtggaa tgcagcagac tgcaggaggg
661 cgtgcacgca gctgaggctg gctgccgact tgtcctggtg gacaccttca agccagagga
721 gctggacgca aggcaacgtg ctgaaggaca gttccgagtg ttggtgtgga agccacatgg
781 agggcatatc actggaaaaa ctcccccaaa tctgcggaac ggaactaaac gataagcacc
841 aggcggaaat cagaccccag gaaggcccag acccgaaata ctccccaaga ggatggccaa
901 gagggggcac acgcccaaaa cacacaaacc aacgcgaaag aacacggcag gcaaaatggc
961 ccaagacacg ggaccaacct acgcagggcc aaggcaatgg aacatgccaa gagccacgac
1021 ccagcaggac cag
BG338063 
1 gtggacgctg aaggcctggc gctgctgctg ccgcccgtca ccctggcagc cctggtggac
61 agctggctcc gagaggactg cccagggctc aactacgcag ccttggtcag cggggcaggc
121 ccctcgcagg cggcgctgtg ggccaaatcc cctggggtac tggcagggca gcctttcttc
181 gatgccatat ttacccaact caactgccaa gtctcctggt tcctccccga gggatcgaag
241 ctggtgccgg tggccagagt ggccgaggtc cggggccctg cccactgcct gctgctgggg
301 gaacgggtgg ccctcaacac gctggcccgc tgcagtggca ttgccagtgc tgccgccgct
361 gcagtggagg ccgccagggg gccggctgga ctgggcacgt ggcaggcacg aggaagacca
421 cgccaggctt ccggctggtg gagaagtatg ggctcctggt ggggcgggcc gcctcgcacc
481 gctacgacct gggagggctg gtgatggtga aggataacca tgtggtggcc gcggtggcgt
541 gagaagcggt gcgggcggcc agacaggcgg tgacttcgct ctgaaggtgg aagtggaatg
601 cagcagcctg caggaggccg tgcaggcagc tgaggctggt gccgaccttg tcctgctgga
661 caacttcaag ccagaggagc tgcaacccac ggcaccgtgc tgaaggccca gttccgagtg
721 tggtgtggaa cccagtgggg gcatcacccg gaaaaactcc cccag
BG251163 
1 gctgcaagtc accatggacg ctgaaggcct ggcgctgctg ctgccgcccg tcaccctggc
61 agccctggtg gacagctggc tccgagagga ctgcccaggg ctcaactacg cagccttggt
121 cagcggggca ggcccctcgc aggcggcgct gtgggccaaa tcccctgggg tactggcagg
181 gcagcctttc ttcgatgcca tatttaccca actcaactgc caagtctcct ggttcctccc
241 cgagggatcg aagctggtgc cggtggccag agtggccgag gtccggggcc ctgcccactg
301 cctgctgctg ggggaacggg tggccctcaa cacgctggcc cgctgcagtg gcattgccag
361 tgctgccgcc gctgcagtgg aggccgccag gggggccggc tggactgggc acgtggcagg
421 cacgaggaag accacgccag gcttccggct ggtggagaag tatgggctcc tggtgggcgg
481 ggccgcctcg caccgctacg acctgggagg gctggtgatg gtgaaggata accatgtggt
541 ggccgccggt ggcgtggaga aggcggtgcg ggcggcagac aggcggctga cttcgctctg
601 aaggtggaag tggaatgcag cagcctgcag gaggccgtgc aggcagctga ggctggtgcc
661 gacttgctcc tgctggacaa ccttcaagcc agaggagctg cacccacggc acgtgctgaa
721 ggccagttcc cgatgtggct gtggaaccct ggggctcacc tggaaaactc cccatn
BG396079 
1 gcttggtcct gagcagccaa cacaccagcc cagacagctg caagtcacca tggacgctga
61 aggcctggcg ctgctgctgc cgcccgtcac cctggcagcc ctggtggaca gctggctccg
121 agaggactgc ccagggctca actacgcagc cttggtcagc ggggcaggcc cctcgcaggc
181 ggcgctgtgg gccaaatccc ctggggtact ggcagggcag cctttcttcg atgccatatt
241 tacccaactc aactgccaag tctcctggtt cctccccgag ggatcgaagc tggtgccggt
301 ggccagagtg gccgaggtcc ggggccctgc ccactgcctg ctgctggggg aacgggtggc
361 cctcaacacg ctggcccgct gcagtggcat tgccagtgct gccgccgctg cagtggaggc
421 cgccaggggg gccggctgga ctgggcacgt ggcaggcacg aggaagacca cgccaggctt
481 ccggctggtg gagaagtatg ggctcctggt gggcggggcc gctcgcaccg ctacgacctg
541 ggagggctgg tgatggtgaa ggataaccat gtggtggccg ccggtggcgt gtagaaggcg
601 gtgcgggcgg cagacaggcg gtgacttcgc tctgaaggtg gaagtggaat gcagcagctg
661 caggaggccg tgcaaggcag tgaggtggtg ccgacttgtt ctgctggaaa ttcagcaagg
721 agtgacca
BF316915 
1 ggtcaccatg gacgctgaag gcctggcgct gctgctgccg cccgtcaccc tggcagccct
61 ggtggacagc tggctccgag aggactgccc aggcctcaac tacgcagcct tggtcagcgg
121 ggcaggcccc tcgcaggcgg cgctgtgggc caaatcccct ggggtactgg cagggcagcc
181 tttcttcgat gccatattta cccaactcaa ctgccaagtc tcctggttcc tccccgaggg
241 atcgaagctg gtgccggtgg ccagagtggc cgaggtccgg ggccctgccc actgcctgct
301 gctgggggaa cgggtggccc tcaacacgct ggcccgctgc agtggcattg ccagtgctgc
361 cgcgctgcag tggaggccgc cagggggccg gctggactgg gcacgtggca ggcacgagga
421 agaccacgcc aggcttccgg ctggtggaga agtatgggct cctggtgggc ggggcgctcg
481 caccgtacga ctgggagggc tggtgatggt gaaggataac atgtggtggc gcggtggggt
541 ggagaagggt gcgggctggc agaaggcggt gactttcgct ctgaaggtgg aaggtggaat
 
601 gaggagctgt aggaggcgtg caggcagtga ggctggtgca gacttgttct gtggaaattc
661 aagcagagga gtgacccacg gcacgggtga ggcagttccc agtgtggtgg tggagcagtg
721 gggtcacctg aaacctccca ggctgggcga atgagtgttc catgggatcg acagggcag
781 cctgatttcc tcaaggt
BF313098 
1 gcagacagct gcaagtcacc atggacgctg aaggcctggc gctgctgctg ccgcccgtca
61 ccctggcagc cctggtggac agctggctcc gagaggactg cccagggctc aactacgcag
121 ccttggtcag cggggcaggc ccctcgcagg cggcgctgtg ggccaaatcc cctgggtac
181 tggcagggca gcctttcttc gatgccatat ttacccaact caactgccaa gtctcctggt
241 tcctccccga gggatcgaag ctggtgccgg tggccagagt ggccgaggtc cggggccctg
301 cccactgcct gctgctgggg gaacgggtgg ccctcaacac gctggcccgc tgcagtggca
361 ttgccagtgc tgccgccgct gcagtggagg ccgccagggg gccggctgga ctgggcacgt
421 ggcaggcacg aggaagacca cgccaggctt ccggctggtg gagaagtatg gctcctggt
481 gggcggggcc gtctcgcacc gctacgacct gggagggctg gtgatggtga aggataacca
541 tgtggtggcc gccggtggcg tggaaagcag cggtgcgggc ggcaacaggc ggtgacttcg
601 tctgaaggtg gaagtggact gcagcaggcc tgcaggaggc cgtgcacggc agctgagggc
661 tggttgccga ccttgtctgc tgcacacttc aagccaggag gagctgcacc ccacgccacc
721 gtgctgactg cccagtcccg agtgtgggtg tggaacgcag gggggcatca cctggacact
781 cccagttttg cgggccgcat gacgtctctc ctggggtgtt gccagggccc ggctgacttt
841 cctccactgt tgccagtggc ggccaccttc cacggaggga cggcggttgg gcccgcctgc
901 c
BG257831 
1 gcagacaggt tgacctgcaa aaaaagacag gggaggcatg gaaacaaagg acccatcgca
61 aatgttttcc atgctgatct ccaaagtgtc ccacccccag cctggggcct ctgggagcct
121 tggtcctgag cagccaacac accagcccag acagctgcaa gtcaccatgg acgctgaagg
181 cctggcgctg ctgctgccgc ccgtcaccct ggcagccctg gtggacagct ggctccgaga
241 ggactgccca ggctcaacta cgcagccttg gtcagcgggg caggcccctc gcaggcggcg
301 ctgtgggcca aatcccctgg ggtactggca gggcagcctt tcttcgatgc catatttacc
361 caactcaact gccaagtctc ctggttcctc cccgagggat cgaagctggt gccggtggcc
421 agagtgggcc gaggtccggg gccctgccca ctgcctgctg ctgggggaac gggtggacct
481 caacacgctg gccgctgcag tggcattgcc agtgctgccg cgctgcagtg gaggccgcca
541 ggtgggccgg ctggactggc acgtggcagg cacgaggaag acacgccagg ttccggctgg
601 tggagaagta tgggctccct gggtgggcgg ggcagctcga caccggatac gaacctggag
661 gggctggtga tgggtgaagg gatacaccat gtgttggccg cggttgagtg gagaagcggt
721 ggggggaggc caaacaggcg gtgattgtct gaggtggaag tgaatgcaga cactgagagg
781 cagagcagca gtgaaggttg tgagaactgt ctgaggacaa taacacaaag atgaaca
BF312975 
1 ggacagctgc aagtcaccat ggacgctgaa ggcctggcgc tgctgctgcc gcccgtcacc
61 ctggcagccc tggtggacag ctggctccga gaggactgcc cagggctcaa ctacgcagcc
121 ttggtcagcg gggcaggccc ctcgcaggcg gcgctgtggg ccaaatcccc tggggtactg
181 gcagggcagc ctttcttcga tgccatattt acccaactca actgccaagt ctcctggttc
241 ctccccgagg gatcgaagct ggtgccggtg gccagagtgg ccgaggtccg gggccctgcc
301 cactgcctgc tgctggggga acgggtggcc ctcaacacgc tggcccgctg cagtggcatt
361 gccagtgctg ccgccgctgc agtggaggcc gccagggggc cggctggact gggcacgtgg
421 caggcacgag gaagaccacg ccaggcttcc ggctggtgga gaagtatggg ctcctggtgg
481 ggcggggccg cctcgcaccg ctacgacctg ggagggctgg tgatggtgaa ggataccatg
541 tggtggccgc cggtgcgtgg gaggcggtgc gggcggccag acaggcggct gacttcgctc
601 tgaaggtgga gtggaatgca gcagcctgtc aggagggccg tgcaggcagc tgaggctggt
661 gccgaccttt ggtctgctgg taccacttcg agccagagga gctgacccca cgggcaccgt
721 gctgcaggcc ccgttccccg agtgtggctg tggacgcccg tgggggcctc actcttggac
 781 acctccccgg ttctgcgggc gccctagacg tcctctcctg gggtgctgac caggggccc
BG115486 
1 gtcctgagca gccaacacac cagcccagac agctgcaagt caccatggac gctgaaggcc
61 tggcgctgct gctgccgccc gtcaccctgg cagccctggt ggacagctgg ctccgagagg
121 actgcccagg gctcaactac gcagccttgg tcagcggggc aggcccctcg caggcggcgc
181 tgtgggccaa atcccctggg gtactggcag ggcagccttt cttcgatgcc atatttaccc
241 aactcaactg ccaagtctcc tggttcctcc ccgagggatc gaagctggtg ccggtggcca
301 gagtggccga ggtccggggc cctgcccact gcctgctgct gggggaacgg gtggccctca
361 acacgctggc ccgctgcagt ggcattgcca gtgctgccgc cgctgcagtg gaggccgcca
421 ggggggccgg ctggactggg cacgtggcag gcacgaggaa gaccacgcca ggcttccggc
481 tggtggagaa gtatgggctc ctggtgggcg gggccgcctc gcaccgctac gagctgggag
541 ggctggtgat ggtgaaggat aaccatgtgg tggcccgcgg tggcgtggag aaggcggtgc
601 gggccggcag acaggcggtg gacttcgctc tgaaggtgga agtggaatgc aggagcctgc
661 aggaggcgtg aaggcagtcg aggatggtgg cgaccttgtc ctggctggac aaacttcagc
721 cagaggagct ggacaccaca gccaacgggc tgaaggccag gtcccgagag tgcagaggaa
781 ccaaaggggc tacacctggg acactaccca gactgcagac gccaaaaagc gcatccggcg
BG748194 
1 ggcctggcag aggacaggag ggaggcttgg ggagcctggg aagggccggc tgacagcttg
61 atgggccctc cctccctcca cagtcccacc cccagcctgg ggcctctggg agccttggtc
121 ctgagcagcc aacacaccag cccagacagc tgcaagtcac catggacgct gaaggcctgg
181 cgctgctgct gccgcccgtc accctggcag ccctggtgga cagctggctc cgagaggact
241 gcccaggctc aactacgcag ccttggtcag cggggcaggc ccctcgcagg cggcgctgtg
301 ggccaaatcc cctggggtac tggcagggca gcctttcttc gatgccatat ttacccaact
361 caactgccaa gtctcctggt tcctccccga gggatcgaag ctggtgccgg tggccagagt
421 ggccgaggtc cggggccctg cccactgcct gctgctgggg gaacgggtgg ccctcaacac
481 gctggcccgc tgcagtggca ttgccagtgc tgccgccgct gcagtggagg ccgccagggg
541 ggccggctgg actgggcacg tggcaggcac gaggaagacc acgccaggct tccggctggt
601 ggagaagtat gggctcctgg tgggccgggc cgcctcgcac cgctacgacc tgggaggg
BF980859

1 ccccagcctg gggcctctgg gagccttggt cctgagcagg ccaacacacc agcccagaca
61 gctgcaagtc accatggacg ctgaaggcct ggcgctgctg ctgccgcccg tcaccctggc
121 agccctggtg gacagctggc tccgagagga ctgcccaggc tcaactacgc agccttggtc
181 agcggggcag gcccctcgca ggcggcgctg tgggccaaat cccctggggt actggcaggg
241 cagcctttct tcgatgccat atttacccaa ctcaactgcc aagtctcctg gttcctcccc
301 gagggatcga agctggtgcc ggtggccaga gtggccgagg tccgggcccc tgcccactgc
361 ctgctgctgg gggaacgggt ggccctcaac acgctggccc gctgcagtgg cattgccagt
421 gctgccgccg ctgcagtgga ggccgccagg ggggccggct ggactgggca cgtggcaggc
481 acgaggaaga ccacgccagg cttccggctg gtggagaagt atgggctcct ggtgggcggg
541 gccgcctcgc accgctacga cctgggaggg ctggtgatgg tgaaggataa ccatgtggtg
601 gccgccggtg gcgtggagaa agcggtgcgg gcggccagac aggcgggtga cttcgcctct
661 gaatgtggag tggaatgaga gctgcagagg cgtgcagagc tgaggtgggg cacctggtct
721 ggtggacaac tcagcaagga gctggccacg gcacggctga aggccatccg agtgtggcgt
781 gagccggggc gttccgggac ccgcagtcgg gggcaaaagc cccggggggg gccagggccc
841 ccatttcccc caggcgacag ggcgcgcgca ccacgaaaag cgccgcgggg cgccccgcac
901 aagaggcgga aacacacccg cggcgcgaca cgggcgacgc gtctcagctt gtcgcn

Sequences identified as those of HOXB 13 cluster
BF676461 
GGGATTCCCCCGGCTGGGTGGGAGAGCGAGCTGGGTGCCCCCATAGATTCCCCTGCCCG
AACCTCATGAGCCGACCCTCGGCTCCATGGAGCCGGAAATTATGCCACCTTGGATGGAG
CCAAGGATATCGAAGGCTTGTTGGGAGCGGAGGGGGCGGAATCTGGTCGCCCACTCCC
TCTCTGACCAGCCACCCAGCGCGCTACGCTTGATGCCTGTGTCAATATGCCCCCTTGATC
TGCCAGGCTCGGGGAGCGGCCAAAAGCAATGCCCACCCTATGCTCTGGGGGTGCCCAGGG
GACTGTCCCCGGCTCCGTGCCTTATGGTTACTGTGGGCGGGTACATACTCCTGCAGAG
TTGTCCCGGAGCTCGTTGAAACCTTGTGCCGAGGAGAGCCACCCTGGCGGTACCCGGGAA
GACTCCCCAGGGCGGGAAGAGTACCCCAGCGGCCCAATGAGTTGTGCTTCTATCGGGATA
TCCGGGACCTACCAGGCCTATGTGCAGGTACTGGACGTGTCCTGTGCTGCAGACTCTGGG
TGTCCGTGGAGCACCGGACATTGGCTCGCTGTGGCCTGTGGCCGGTACCAGTCTTGGGCT
CTCGGTGTGTGGCTGGACACGCCGGTTGTGTTCGCGGAGACCGCACCCACCAGGTTCCT
TTGGGAGGGCCGCTTTGCAGACTCCGGGGGAGGCCCCTCTGAGGCGGGGCCTTTTCGGGG
GGGCGAAGAAAGCTTTCCGACGCAGGCGCTTGCGGAGCTGGCGGACATCGGGACACTTC
ACCCAGCGAAGCGCGGCTTGGGGCCCCTCTGGGCGCGGTCTCGGTTGACACCGGCGAAGA
GTTTCGGGAGAGGCCCATATCTTCTGGGGAGGGCGTTGCGTCGCCCCCG
BC007092 
ggattccccc ggcctgggtg gggagagcga gctgggtgcc ccctagattc cccgcccccg
cacctcatga gccgaccctc ggctccatgg agcccggcaa ttatgccacc ttggatggag
ccaaggatat cgaaggcttg ctgggagcgg gagggggcgg gaatctggtc gcccactccc
ctctgaccag ccacccagcg gcgcctacgc tgatgcctgc tgtcaactat gcccccttgg
atctgccagg ctcggcggag ccgccaaagc aatgccaccc atgccctggg gtgccccagg
ggacgtcccc agctcccgtg ccttatggtt actttggagg cgggtactac tcctgccgag
tgtcccggag ctcgctgaaa ccctgtgccc aggcagccac cctggccgcg taccccgcgg
agactcccac ggccggggaa gagtacccca gccgccccac tgagtttgcc ttctatccgg
gatatccggg aacctaccag cctatggcca gttacctgga cgtgtctgtg gtgcagactc
tgggtgctcc tggagaaccg cgacatgact ccctgttgcc tgtggacagt taccagtctt
gggctctcgc tggtggctgg aacagccaga tgtgttgcca gggagaacag aacccaccag
gtcccttttg gaaggcagca tttgcagact ccagcgggca gcaccctcct gacgcctgcg
cctttcgtcg cggccgcaag aaacgcattc cgtacagcaa ggggcagttg cgggagctgg
agcgggagta tgcggctaac aagttcatca ccaaggacaa gaggcgcaag atctcggcag
ccaccagcct ctcggagcgc cagattacca tctggtttca gaaccgccgg gtcaaagaga
agaaggttct cgccaaggtg aagaacagcg ctacccctta agagatctcc ttgcctgggt
gggaggagcg aaagtggggg tgtcctgggg agaccaggaa cctgccaagc ccaggctggg
gccaaggact ctgctgagag gcccctagag acaacaccct tcccaggcca ctggctgctg
gactgttcct caggagcggc ctgggtaccc agtatgtgca gggagacgga accccatgtg
acagcccact ccaccagggt tcccaaagaa cctggcccag tcataatcat tcatcctgac
agtggcaata atcacgataa ccagtactag ctgccatgat cgttagcctc atattttcta
tctagagctc tgtagagcac tttagaaacc gctttcatga attgagctaa ttatgaataa
atttggaaaa aaaaaaaaaa aaaaaaaaaa aaaaaa
BM462617 
ATTCCCCCGGCCTGGGTGGGAGAGCGAGCTGGGTGCCCCCTAGATTCCCCGCCCCCGCA
CCTCATGAGCCGACCCTCGGCTCCATGGAGCCCGGCAATTATGCCACCTTGGATGGAGCC
AAGGATATCGAAGGCTTGCTGGGAGCGGAGGGGGCGGAATCTGGTCGCCCACTCCCCT
CTGACCAGCCACCCAGCGGCGCCTACGCTGATGCCTGCTGTCAACTATGCCCCCTTGGAT
CTGCCAGGCTCGGCGGAGCCGCCAAAGCAATGCCACCCATGCCCTGGGGTGCCCCAGGGG
ACGTCCCCAGCTCCCGTGCCTTATGGTTACTTTGGAGGCGGGTACTACTCCTGCCGAGTG
TCCCGGAGCTCGCTGAAACCCTGTGCCCAGGCAGCCACCCTGGCCGCGTACCCCGCGGAG
ACTCCCACGGCCGGGGAAGAGTACCCCAGCCGCCCCACTGAGTTTGCCTTCTATCCGGGA
TATCCGGGAACCTACCAGCCTATGGCCAGTTACCTGGACGTGTCTGTGGTGCAGACTCTG
 
GGTGCTCCTGGAGAACCGCGACATGACTCCCTGTTGCCTGTGGACAGTTACCAGTCCTGG
GCTCTCGCTGGTGGCTGGAACAGCCAGATGTGTTGCCAGGGAGAACAGAACCCACCAGGT
CCCCTTTTGGAAGGCAGCATTTGCAGACTCCAGCGGGCAGCACCCTCCTGACGCCTGCGC
CTTTCGT
BG752489 
GCAGGCGACTTGCGAGCTGGGAGCGATTTAAAACGCTTTGGATTCCCCGGCCTGGGTGG
GGAGAGCGAGCTGGGTGCCCCCTAGATTCCCCGCCCCCGCACCTCATGAGCCGACCCTCG
GCTCCATGGAGCCCGGCAATTATGCCACCTTGGATGGAGCCAAGGATATCGAAGGCTTGC
TGGGAGCGGGAGGGGGGCGGAATCTGGTCGCCCACTCCCCTCTGACCAGCCACCCAGCGG
CGCCTACGCTGATGCCTGCTGTCAACTATCCCCCCTTGGATCTGCCAGGCTCGGCGGAGC
CGCCAAAGCAATGCCACCCATGCCCTGGGGTGCCCCAGGGGACGTCCCCAGCTCCCGTGC
CTTATGGTTACTTTGGAGGCGGGTACTACTCCTGCCGAGTGTCCCGGAGCTCGCTGAAAC
CCTGTGCCCAGGCAGCCACCCTGGCCGCGTACCCCGCGGAGACTCCCACGGCCGGGGAAG
AGTACCCCAGCCGCCCCACTGAGTTTGCCTTCTATCCGGGATATCCGGGAACCTACCAGC
CTATGGCCAGTTACCTGGACGTGTCTGTGGTGCAGACTCTGGGTGCTCCTGGAGAACCGC
GACATGACTCCCTGTTGCCTGTGGACAGTTACCAGTCTTGGGCTCTCGCTGGTGGCTGGA
ACAGCCAGATGTGTTGCCAGGGAGAACAGAAGCCACCAGGTCCCTTTTGGAAGGCAGCAT
CTGCAGACTCCAGCGGGCAGGACCTCCTGACGCCTGCGGCCTTTCGTCGCGAGCGCAAGA
AACGCATTCCGTA
BG778198 
GGATTTAAAACGCTTTGGATTCCCCGGCCTGGGTGGGAGAGCGAGCTGGGTGCCCCCT
AGATTCCCCGCCCCCGCACCTCATGAGCCGACCCTCGGCTCCATGGAGCCCGGCAATTAT
GCCACCTTGGATGGAGCCAAGGATATCGAAGGCTTGCTGGGAGCGGGAGGGGGGCGGAAT
CTGGTCGCCCACTCCCCTCTGACCAGCCACCCAGCGGCGCCTACGCTGATGCCTGCTGTC
AACTATGCCCCCTTGGATCTGCCAGGCTCGGCGGAGCCGCCAAAGCAATGCCACCCATGC
CCTGGGGTGCCCCAGGGACGTCCCCAGCTCCCGTGCCTTATGGTTACTTTGGAGGCGGGT
ACTACTCCTGCCGAGTGTCCCGGAGCTCGCTGAAACCCTGTGCCCAGGCAGCCACCCTGG
CCGCGTACCCCGCGGAGACTCCCACGGCCGGGGAAGAGTACCCCAGCCGCCCCACTGAGT
TTGCCTTCTATCCGGGATATCCGGGAACCTACCAGCCTATGGCCAGTTACCTGGACGTGT
CTGTGGTGCAGACTCTGGGTGCTCCTGGAGAACCGCGACATGACTCCCTGTTGCCTGTGG
ACAGTTACCAGTCTTGGGCTCTCGCTGGTGGCTGGAACAGCCAGATGTGTTGCCAGCGC
AGAACAGAACCCACCAGGTCCCTTTTGGAAGGCAGCATTTGCAGACTCCAGCGGGCAGAA
CCCTCCTGACGCCTGCGCCTTTCGTTCGCGGGCGAAAAA
CB050884 
AAGAAACGCATTCCGTACAGCAAGGGGCAGTTGCGGGAGCTGGAGCGGGAGTATGCGGCT
AACAAGTTCATCACCAAGGACAAGAGGCGCAAGATCTCGGCAGCCACCAGCCTCTCGGAG
CGCCAGATTACCATCTGGTTTCAGAACCGCCGGGTCAAAGAGAAGAAGGTTCTCGCCAAG
GTGAAGAACAGCGCTACCCCTTAAGAGATCTCCTTGCCTGGGTGGGAGGAGCGAAAGTGG
GGGTGTCCTGGGGAGACCAGGAACCTGCCAAGCCCAGGCTGGGGCCAAGGACTCTGCTGA
GAGGCCCCTAGAGACAACACCCTTCCCAGGCCACTGGCTGCTGGACTGTTCCTCAGGAGC
GGCCTGGGTACCCAGTATGTGCAGGGAGACGGAACCCCATGTGACAGCCCACTCCACCAG
GGTTCCCAAAGAACCTGGCCCAGTCATAATCATTCATCCTGACAGTGGCAATAATCACGA
TAACCAGTACTAGCTGCCATGATCGTTAGCCTCATATTTTCTATCTAGAGCTCTGTAGAG
CACTTTAGAAACCGCTTTCATGAATTGAGCTAATTATGAATAAATTTGGAAGGCGAAAAA
AAAAACCTCGTGCC
CB050885 
ATTCGGCACGAGGTTTTTTTTTCGCCTTCCAAATTTATTCATAATTAGCTCAATTCATG
AAAGCGGTTTCTAAAGTGCTCTACAGAGCTCTAGATAGAAAATATGAGGCTAACGATCAT
GGCAGCTAGTACTGGTTATCGTGATTATTGCCACTGTCAGGATGAATGATTATGACTGGG
CCAGGTTCTTTGGGAACCCTGGTGGAGTGGGCTGTCACATGGGGTTCCGTCTCCCTGCAC
ATACTGGGTACCCAGGCCGCTCCTGAGGAACAGTCCAGCAACCAGTGGCCTGGGAAGGGT
GTTGTCTCTAGGGGCCTC
BF965191 
GGGTGGGAGAGCGAGCTGGGTGCCCCCTAGATTCCCCGCCCCCGCACCTCATGAGCCGA
CCCTCGGCTCCATGGAGCCGGCAATTATGCCACCTTGGATGGAGCCAAGGATATCGAAG
GCTTGCTGGGAGCGGAGGGGGGCGGAATCTGGTCGCCCACTCCCCTCTGACCAGCCACC
CAGCGGCGCCTACGCTGATGCCTGCTGTCAACTATGCCCCCTTGGATCTGCCAGGCTCGG
CGGAGCCGCCAAAGCAATGCCACCCATGCCCTGGGGTGCCCCAGGGGACGTCCCCAGCTC
CCGTGCCTTATGGTTACTTTGGAGGCGGGTACTACTCCTGCCGAGTGTCCCGGAGCTCGC
TGAAACCCTGTGCCAGGCAGCCACCCTGGCCGCGTAACCCGACGGAGACTCTCACGTGCG
GGGAAGAGTACCCCTAGCGCCCCACATGAGTTTGCCTTCTATCCGGGATATCCGGACCG
TACCAGCCTATGGCAGTTACCTGGACGTGTCTGTGGTGCCGACTCTGGGTGCTCCTGGAG
AACCGCGGACATGACTCCTTGTTTGCTGTGCGACGCTCACCAGTCTGGGCTCCTCGTCGG
TGGTCGCACTCCCACTTTTTGCCGGGCGACATCCCCCGGGGCCCCTTCCGGAACAGCGAC
CTTGCGAGCCCCCGGGGACACACCCCCGTAAGCGGCCTATCATCGCTGATAAACCTCATC
AGAGGGCACCGAAAGCCGCGACTCTAACCCCCCCACTACGACTCACGACCGCACAGGTAC
TCGAACCGCCCAATATCTGGTTCTAACCCATGGCGCATCTCAGCCGCTAGAGAGCCAACC
AAACGCGCCACGCGCAACCACACTACACCACGGCACCCCTTTCATCTCACTCCCACGCCG
ATCACTCTTCACCCTCCAGAATCATTCCCCTCGCACATCCTACCTATCTCATGCCTCCCA
GTTCACCCCATTCCCTCCCCTAATCTCACCCACACATTCACGCACGTTCTCACTACGCTT
CGCTCCGACCCACATCCTCACCCCCACATTCATACCACTTCACCATCACGACCCCCCCCT
CTCATCGACTCCTGTCTCATTCTCAACCACAGTACTACCAGCTCCAACACACCACTCACC
CCAAGCTATCCATCACCTACACGCTTTCACCCCTCACCGCTCCCAAGTAATTCAGATCAC
TCAAACACAATCTGCTACATACTCATCCCTCCCCCACTCCCAGTACAGTCCAACCACCGA
CCAACTACCTCCGCGCCACCCGCGCCGCCCCACCTCACCGGCCCCAACCGCCCGCACAGG
GCACGCACCCCCCGGCAACCGCGCGATCCGGCCGTACACACTCTTGGGCGGCACGCAGCT
GAGGACATTCCGCGGGAGCGCCCCACCGTGGGCTACGTGGTCGCGACCCGGCGGGGCGC
GTGCGGCGTCGCCCGCCCGCCCGCCGACTGCGACCCAGTCGAG
BU930208 
GGGGCTTTGGATTCCCCCGGCCTGGGTGGGAGAGCGAGCTGGGTGCCCCCTAGATTCCC
CGCCCCCGCACCTCATGAGCCGACCCTCGGCTCCATGGAGCCCGGCAATTATGCCACCTT
GGATGGAGCCAAGGATATCGAAGGCTTGCTGGGAGCGGAGGGGGCGGAATCTGGTCGC
CCACTCCCCTCTGACCAGCCACCCAGCGGCGCCTACGCTGATGCCTGCTGTCAACTATGC
CCCCTTGGATCTGCCAGGCTCGGCGGAGCCGCCAAAGCAATGCCACCCATGCCCTGGGGT
GCCCCAGGGGACGTCCCCAGCTCCCGTGCCTTATGGTTACTTTGGAGGCGGGTACTACTC
CTGCCGAGTGTCCCGGAGCTCGCTGAAACCCTGTGCCCAGGCAGCCACCCTGGCCGCGTA
CCCCGCGGAGACTCCCACGGCCGGGGAAGAGTACCCCAGCCGCCCCACTGAGTTTGCCTT
CTATCCGGGATATCCGGGAACCTACCAGCCTATGGCCAGTTACCTGGACGTGTCTGTGGT
GCAGACTCTGGGTGCTCCTGNAGAACCGCGACATGACTCCCTGTTGCCTGTGGACAGTTA
CCAGTCTTGGGCTCTCGCTGGTGGCCTGGAACAGCCCAGATGTGTTTGCCCAGGGNAGAA
CACGAACCCCACCCGGTTCCCCCTTTTGGAAAGGGCAGCCATTTTGGCCAGCCTTCCAA
GCGGGGCCAACCACCCCCTCCCCTGGACAGGCCCTGGT
AA807966 
GCGGCCGCAAGAAACGCATTCCGTACAGCAAGGGGCAGTTGCGGACTGGAGCGGGAGTA
TGCGGCTAACAAGTTCATCACCAAGGACAAGAGGCGCAAGATCTCGGCAGCCACCAGCCT
CTCGGAGCGCCAGATTACCATCTGGTTTCAGAACCGCCGGGTCAAAGAGAAGAAGGTTCT
CGCCAAGGTGAAGAACAGCGCTACCCCTTAAGAGATCTCCTTGCCTGGGTGGAGGAGCG
AAAGTGGGGGTGTCCTGGGGAGACCAGGAACCTGCCAAGCCCAGGCTGGGGCCAAGGACT
CTGCTGAGAGGCCCCTAGAGACAACACCCTTCCCAGGCCACTGGCTGCTGGACTGTTCCT
CAGGAGCGGCCTGGGTACCCAGTATGTGCAGGGAGACGGAACCCCATGTGACAGCCCATT
CCACCAGGGTTCCCAAAGAACCTGGCCCAGTCATAATCATTCATCCTGACAGTGGC
AI884491 
AGCGGCCGCAAGAAACGCATTCCGTACAGCAAGGGGCAGTTGCGGAGCTGGAGCGGGAG
TATGCGGCTAACAAGTTCATCACCAAGGACAAGAGGCGCAAGATCTCGGCAGCCACCAGC
CTCTCGGAGCGCCAGATTACCATCTGGTTTCAGAACCGCCGGGTCAAAGAGAAGAAGGTT
CTCGCCAAGGTGAAGAACAGCGCTACCCCTTAAGAGATCTCCTTGCCTGGGTGGGAGGAG
CGAAAGTGGGGGTGTCCTGGGGAGACCAGGAACCTGCCAAGCCCAGGCTGGGGCCAAGGA
CTCTGCTGAGAGGCCCCTAGAGACAACACCCTTCCCAGGCCACTGGCTGCTGGACTGTTC
CTCAGGAGCGGCCTGGGTACCCAGTATGTGCAGGGAGACGGAACCCCATGTGACAGCCCA
CTCCACCAGGGTTCCCAAAGAACCTGGCCCAGTCATAATCATTCATCCTGACAGTGGCAA
TAATCACGATAACCAGTACTAGCTGCCATGATCGTTAGCCTCATATTTTCTATCTAGAGC
TCTGTAGAGCAC
AA652388 
GCGGCCGCAAGAAACGCATTCCGTACAGCAAGGGGCAGTTGCGGACTGGAGCGTGAGTA
TGCGGCTAACAAGTTCATCACCAAGGACAAGAGGCGCAAGATCTCGGCAGCCACCAGCCT
CTCGGAGCGCCAGATTACCATCTGGTTTCAGAACCGCCGGGTCAAAGAGAAGAAGGTTCT
CGCCAAGGTGAAGAACAGCGCTACCCCTTAAGAGATCTCCTTGCCTGGGTGGGAGGAGCG
AAAGTGGGGGTGTCCTGGGGAGACCAGGAACCTGCCAAGCCCAGGCTGGGGCCAAGGACT
CTGCTGAGAGGCCCCTAGAGACAACACCCTTCCCAGGCCACTGGCTGCTGGACTGTTCCT
CAGGAGCGGCCTGGGTACCCAGTATGTGCAGGGAGACGGAACCCCATGTGACAGCCCACT
CCACCAGGGTTCCCAAAGAACCTGGCC
BF446158 
TTTTTTTTTTTTTTTTTTTTCGCCTTCCAAATTTATTCATAATTAGCTCAATTCATGAAAG
CGGTTTCTAAAGTGCTCTACAAAGCTCTAAATAAAAAATATGAGGCTAACGATCATGGCA
GCTAGTACTGGTTATCGGGATTATTGCCACTGTCAGGATGAATGATTATGACTGGGCCAG
GTTCTTTGGGAACCCTGGTGGAGTGGGCTGTCACATGGGGTTCCGTCTCCCTGCACATAC
TGGGTACCCAGGCCGTTCCTGAGGAACAGTCCACCACCCAGTGGCCTGGAAGGGTGTTG
TCTCTAGGGGCCTCTCAACAAAGTCCTTGGCCCCAGCCTGGGCTTGGCAGGTTCCTGGTC
TCCCCAGGACACCCCCCACTTTCGCTCCTCCCACCCAGGCAAGGAGATCTCTTAAGGGG
AA657924 
GACGCNAGGTATGCGGCTAACAAGTTCATCACCAAGGACAAGAGGCGCAAGATCTCGGCA
GCCACCAGCCTCTCGGAGCGCCAGATTACCATCTGGTTTCAGAACCGCCGGGTCAAAGAG
AAGAAGGTTCTCGCCAAGGTGAAGAACAGCGCTACCCCTTAAGAGATCTCCTTGCCTGGG
TGGGAGGAGCGAAAGTGGGGGTGTCCTGGGGAGACCAGGAACCTGCCAAGCCCAGGCTGG
GGCCAAGGACTCTGCTGAGAGGCCCCTAGAGACAACACCCTTCCCAGGCCACTGGCTGCT
GGACTGTTCCTCAGGAGCGGCCTGGGTACCCATGTATGTGCAGGGAGACGGAACCCCATG
 TGACAGCCCACTCCACCAGNGTTCCTAAAGAACCCTGGCCAGTCA
AA644637 
GCAGGCGACTTGCGAGCTGGGAGCGGTTTAAAACGCTTTGGATTCCCCGGCCTGGGTGG
GGAGAGCGAGCTGGGTGCCCCCTAGATTCCCCGCCCCCGCACCTCATGAGCCGACCCTCG
GTCCATGGACACGGCAATTATGCCACCTTGGATGGAGCCAAGGATATCGAAGGCTTGCTG
GGAGCGGGAGGGGGGCGGAATCTGGTCGCCCACTCCCCTCTGACCAGCCACCCAGCGGCG
CCTACGCTGATGCCTGCTGTCAACTATGCCCCCTTGGATCTGCCAGGCTCGGCGGACTCT
NAAAGCATATGCCACCCNATGCCCTGGGGTGCCCCAGGGGAACGTCCCCAGCTCCCGTGC
CTTATGGTT
BF222357 
GCGGCCGCAAGAAACGCATTCCGTACAGCAAGGGGCAGTTGCGGAGCTGGAGCGGGAGT
ATGCGGCTAACAAGTTCATCACCAAGGACAAGAGGCGCAAGATCTCGGCAGCCACCAGCC
TCTCGGAGCGCCAGATTACCATCTGGTTTCAGAACCGCCGGGTCAAAGAGAAGAAGGTTC
TCGCCAAGGTGAAGAACAGCGCTACCCCTTAAGAGATCTCCTTGCCTGGGTGGAGGAGC
GAAAGTGGGGGTGTCCTGGGAGACCAGGAACCTGCCAAGCCCAGGCTGGGGCCAAGGAC
TCTGCTGAGAGGCCCCTAGAGACAACACCCTTCCCAGGCCACTGGCTGCTGGACTGTTCC
TCAGGAGCGGCCTG
AA527613 
GTCGACGAACAGCGCTACCCCTTAAGAGATCTCCTTGCCTGGTGGAGGAGCGAAAGTG
GGGGTGTCCTGGGGAGACCGGAACTGCCAAGCCCAGGCTGGGCAAGGACTCTGCTGAG
AGGCCCCTAGAGACAACACCCTTCCCAGGCCACTGCTGCTGGACTGTTCCTCAGGAGCGG
CCTGGGTACCCAGTATGTGCAGGGAGACGGAACCCCATGTGACAGCCCACTCCACCAGGG
TTCCCAAAGAACCTGGCCCAGTCATAATCATTCATCCTGACAGTGGCAATAATCACGATA
ACCAGTACTCAGCTGCCATGATCGTTAGCCTCATATT
AA533227 
GCGTCGACCCCTTGAAGAGATCTCCTTGCCTGGGTGGAGGAGCGAAAGTGGGGGTGTCC
TGGGGAGACCAGGAACCTGCCAAGCCCAGGCTGGGGCCAAGGACTCTGCTGAGGCCCC
TAGAGACAACACCCTTCCCAGGCCACTGGCTGCTGGACTGTTCCTCAGGAGCGGCCTGGG
TACCCAGTATGTGCAGGGAGACGGAACCCCATGTGACAGCCCACTCCACCAGGGTTCCCA
AAGAACCTGGCCCAGTCATAATCATTCATCCTGACAGTGGCAATAATCACGATAACCAGT
ACTAGCTGCCATGATCGTTAGCCTCATATTTTCTATCTAGAGCTCTGTAGAGCACTTGTA
GAAACCGCTTTCATGAATTGAGCTAATTATGAATAGATTTGGAAGGGGAAAAAGTGGAA
AAAGTTTTGCCCAAAGTGGGTCGTTTACGTCG
AA456069 
CTCCCTGGCAACACATCTGGCTGTTCCAGCACCAGCGAGACCCAAGACTGGTAACTGTCC
ACAGGCAACAGGGAGTCATGTCGCGGTTCTCCAGGAGCACCCAGAGTCTGCACCACAGAC
ACGTCCAGGTAACTGGCCATAGCTGAGTAGGTTCCCGGATATCCCGGATAGAAGGCAAAC
TCAGTGGGGCGGCTGGGGTACTCTTCCCCGGCCGTGGAGAGTCTCCGCGGGGTACGGCCC
AGGGTGGCTGCCTGGGCATCAGGGTTTCAGCGAGCTCCGGGACACTCGGCAGGAGTAGTA
CCCGCCTCCAAAGTAACCATAAGGCACGGGAGCTGGGGACGTCCCTGGGGCACCCCAG
AA455572 
TTTAAAACGCTTTGGATTCCCCGGCCTGGGTGGGAGAGCGAGCTGGGTGCCCCCTAGA
TTCCCCGCCCCCGCACCTCATGAGCCGACCCTCGGTCCATGGAGCCGGCGAATTATGCCA
CCTTGGATGGAGCCAAGGATATCGAAGGCTTGCTGGAGCGGAGGGGGCGGAATCTGG
TCGCCCACTCCCCTCTGACCAGCCACCCAGCGGCGCTACGTGATGCCTGCTGTCAACTAT
GCCCTTGGATCTGCCAGCTCGCGGAGCCAAAGCAATGCCACCCATGCCCTGGGGTGCCCC
AGGTGACGTCCCCAGCTCCCGTGCCTTATGGTTACTTTGGAGGCGGGTACTACTCCTGCC
GAGTGTCCCGGAGCTCGCTGAAACCCTGTGCCCAGGCAGCCACCCTGGCCGCGTACCCCG
CGATGACTCCCACGGCCGGGGAAGAGTACCCCAGCCGCCCCACTGAGTTTGCCT
BX117624 
CAGGCGACTTGCGAGTCTGGGAGCGATTTAAAACGCTTTGGATTCCCCGGCCTGGGTGG
GGAGAGCGAGCTGGGTGCCCCCTAGATTCCCCGCCCCCGCACCTCATGAGCCGACCCTCG
GCTCCATGGAGCCCGGCAATTATGCCACCTTGGATGGAGCCAAGGATATCGAAGGCTTGC
TGGGAGCGGGAGGGGGGCGGAATCTGGTCGCCCACTCCCCTCTGACCAGCCACCCAGCGG
CGCCTACGCTGATGCCTGCTGTCAACTATGCCCCCTTGGATCTGCCAGGCTCGGCGGAGC
CGCCAAAGCAATGCCACCCATGCCCTGGGGTGCCCCAGGGGACGTCCCCAGCTCCCGTGC
CTTATGGTTACTTTGGAGGCGGGTACTACTCCTGCCGAGTGTCCCGGAGCTCGCTGAAAC
CCTGTGCCCAGGCAGCCACCCTGGCCGCGTACCCCGCGGAGACTCCCACGGCCGGGGAAG
AGTACCCCAGCCGCCCCACTGAGTTTGCCTTCTATCCGGGATATCCGGGAACCTACCAGC
CTATGGCCAGTTACCTTGGACGTGTCTGTGGTGCAGACTCTGGGTGCTCCTGGAGAACCG
CGACATGACTCCCTGNTGCCTGTGGACAGTTACCAGTCTTGGGCTCTCGCTGGTGGCTGG
AACAGCCAGATGTGTTGNCAGGGAGAACAGAACCCACCAGGTCCCTTTTGGAAGGCAGAT
TTGCAGACTNCAGCGGGCA
BQ673782 
AGGCAGCCACCCTGGCCGCGTACCCCGCGGAGACTCCCACGGCCGGGAAGAGTACCCCA
GCCGCCCCACTGAGTTTGCCTTCTATCCGGATATCCGGAACCTACCAGCCTATGGCCA
GTTACCTGGACGTGTCTGTGGTGCAGACTCTGGGTGCTCCTGGAGAACCGCGACATGACT
CCCTGTTGCCTGTGGACAGTTACCAGTCTTGGGCTCTCGCTGGTGGCTGGAACAGCCAGA
TGTGTTGCCAGGGAGAACAGAACCCACCAGGTCCCTTTTGGAAGGCAGCATTTGCAGACT
CCAGCGGGCAGCACCCTCCTGACGCCTGCGCCTTTCGTCGCGGCCGCAAGAAACGCATTC
CGTACAGCAAGGGGCAGTTGCGGGAGCTGGAGCGGGAGTATGCGGCTAACAAGTTCATCA
CCAAGGACAAGAGGCGCAAGATCTCGGCAGCCACCAGCCTCTCGGAGCGCCAGATTACCA
TCTGGTTTCAGAACCGCCGGGTCAAAGAGAAGAAGGTTCTCGCCAAGGTGAAGAACAGCG
CTACCCCTTAAGAGATCTCCTTGCCTGGGTGGAGGAGCGAAAGTGGGGGTGTCCTGGGG
AGACCAGGAACCTGCCAAGCCCCAGGCTGGGGCCAAGGACTCTGCTGAGAGGCCCCTAGA
GACAACACCCTTCCCAGGCCACTGGCTGCTGGACTGTTCCTCAGGAGCGGCCTGAGTACC
CCGTATGTGCAGGGGAGACGGAACCCCCTGTGACCAGCCCCCCTCCACCCGTGGTCTCCC
AGATAACCTGGCCCCCACTCATAAATCATTTCTTCCCGGCCGGGGGCCAATCATTCCCC
GAACTACCCCGGTACCTTATACAATTAGATTGGACATGAATCCTCTCGGGGGCATTCCCT
ATGGCGCTGAGGCCCCTCACACCT
AI814453 
GGGTGCTGTCCTCTGGAGTCTGCAAATGCTGCCTTCCAAAAGGACCTGGTGGGTTCTGT
TCTCCCTGGCAACACATCTGGCTGTTCCAGCCACCAGCGAGAGCCCAAGACTGGTAACTG
TCCACAGGCAACAGGGAGTCATGTCGCGGTTCTCCAGGAGCACCCAGAGTCTGCACCACA
GACACGTCCAGGTAACTGGCCATAGGCTGGTAGGTTCCCGGATATCCCGGATAGAAGGCA
AACTCAATGGGGCGGCTGGGGTACTCTTCCCCGGCCGTGGGAGTCTCCGCGGGGTACGCG
GCCAGGGTGGCTGCCTGGGCACAGGGTTTCAGCGAGCTCCGGGACACTCGGCAGGAGTAG
TACCCGCCTCCAAAGTAACCATAAGGCACGGAGCTGGGGACGTCCCCTGGGGCACCCCA
 
NGGCATGGGTGGCATTGCTTTGGCGGCTCCGCCGAGCCTGGCAGATCCAAGGGGGCATAG
TTGACAGCAGGCATCAGCGTAGGCGCCGCTGGGTGGCTGGTCAAAAGGAGTGGCGACCA
NATTCCGCCCCCCTCCCGCTTCCCAG
AI417272 
GGGTGCTGCCCGCTGGAGTCTGCAAATGCTGCCTTCCAAAAGGGACCTGGTGGGTTCTGT
TCTCCCTGGCAACACATCTGGCTGTTCCAGCCACCAGCGAGAGCCCAGGACTGGTAACTG
TCCACAGGCAACAGGGAGTCATGTCGCGGTTCTCCAGGAGCACCCAGAGTCTGCACCACA
GACACGTCCAGGTAACTGGCCATAGGCTGGTAGGTTCCCGGATATCCCGGATAGAAGGCA
AACTCAGTGGGGCGGCTGGGGTACTCTTCCCCGCCGTGGGAGTCTCCGCGGGGTACGCGG
CCAGGGTGGCTGCCTGGGCACAGGGTTTCAGCGAGCTCCGGGACACTCGGCAGGAGTAGT
ACCCGCCTCCAAAGTAACCATAAGGCACGGGAGCTGGGGACGTCCCCTGGGGCACCCCAG
GGCATGGGTGGCATTGCTTTGGCGGCTCCGCCGAGCCTGGCAGATCCAAGGNGGCATAGT
TGACAGCAGGCATCAGCGTANGCGCCGCTGGGTGGCTGTCAAGAGG
AA535663 
TCGACGTTACCTGGACGTGTCTGTGGTGCAGACTCTGGGTGCTCCTGGAGAACCGCGACA
TGACTCCCTGTTGCCTGTGGACAGTTACCAGTCTTGGGCTCTCGCTGGTGGCTGGAACAG
CAGATGTGTTGCCAGGGAGAACAGAACCCACCAGGTCCCTTTTGGAAGGCAGCATTTGCA
GACTCCAGCGGGCAGCACCCTCCTGACGCCTGCGCCTTTCGTCGCGGCCGCAAGAAACGC
ATTCCGTACAGCAAGGGGCAGTTGCGGACTGGAGCGGGAGTATGCGGCTAACAAGTTCA
TCACCAAGGACAAGAGGCGCAAGATCTCGGCAGCCACCAGCCTCTCGGAGCGCCAGATTA
CCATCTGGTTTCAGAACCGCCGGGTCAAAGAGAAGAAGGTTCTCGCCAAGGTGAAGAACA
GCGCTACCCCTTAAGAGATCTCCTTGCCTGGGTGGGAGGAGCGAAAGTGTG
AI400493 
GTCAGGAGGGTGCTGCCCGCTGGAGTCTGCAAATGCTGCCTTCCAAAAGGACCTGGTGG
GTTCTGTTCTCCCTGGCAACACATCTGGCTGTTCCAGCCACCAGCGAGAGCCCAGGACTG
GTAACTGTCCACAGGCAACAGGGAGTCATGTCGCGGTTCTCCAGGAGCACCCAGAGTCTG
CACCACAGACACGTCCAGGTAACTGGCCATAGGCTGGTAGGTTCCCGGATATCCCGGATA
GAAGGCAAACTCAGTGGGGCGGCTGGGGTACTCTTCCCCGGCCGTGGAGTCTCCGCGGG
GTACGCGGCCAGGGTGGCTGCCTGGGCACAGGGTTTCAGCGAGCTCCGGGACACTCGGCA
TGAGTAGACCCGCCTTCCAAGTAACCATAAGGCACGGGAGCTGGTAACGTCCCCTGGGGC
ACCCCANGGCCATGGGTGCATTGCTTTGGCGGCTCCGCCGAGCCCTGCAGATCCAAGGTG
GGCATATTGACAGCAGGCATTCACGTATGCGCCCCCTGGGTGGCTGTCATATTGGGGATT
GCGAC
AW779219 
GCAGGCGTCAGGAGGGTGCTGCCCGCTGGAGTCTGCAAATGCTGCCTTCCAAAAGGACC
TGGTGGGTTCTGTTCTCCCTGGCAACACATCTGGCTGTTCCAGCCACCAGCGAGAGCCCA
AGACTGGTAACTGTCCACAGGCAACAGGGAGTCATGTCGCGGTTCTCCAGGAGCACCCAG
AGTCTGCACCACAGACACGTCCAGGTAACTGGCCATAGGCTGGTAGGTTCCCGGATATCC
CGGATAGAAGGCAAACTCAGTGGGGCGACTGGGGTACTCTTCCCGGCCGTGGGGAGTCTC
CGCGGGGTACGCGGCCAGGGGTGGCTGCCTGGGCACCAGGGGTTTCAGCGAGCTCCGGGA
CACTCNGCAGGAAANTAGTACCCGCCTCCCAAAGTAACCATAAGCACCGGACTGNGGGNN
GGACGTCCCCTGGGGCAC
AA594847 
GCGACCGGACGAAAGGAGGCGTCAGGAGGGTGCTGCCCGCTGGAGTCTGCAAATGCTGCC
TTCCAAAAGGACCTGGTGGGTTCTGTTCTCCCTGGCAACACATCTGGCTGTTCCAGCAC
CAGCGAGACCCAAGACTGGTAACTGTCCACAGGCAACAGGGAGTCATGTCGCGGTTCTCC
AGGAGCACCCAGAGTCTGCACCACAGACACGTCCAGGTAACTGGCCATAGCTAGGTAGGT
TCCCGGATATCCCGGATAGAAGGCAAACTCAGTGGGGCGACTGGGGTACTCTTCCCCGGC
CGTGGGAGTCTCCGCGGGGTACGCCCATGGGTGGCTGCCTGGGCACAGGGTTTCAGCGAG
CTCCGGGACA
AI150430 
GCAGGCGTCAGGAGGGTGCTGCCCGCTGGAGTCTGCAAATGCTGCCTTCCAAAAGGGACC
TGGTGGGTTCTGTTCTCCCTGGCAACACATCTGGCTGTTCCAGCCACCAGCGAGAGCCCA
AGACTGGTAACTGTCCACAGGCAACAGGGAGTCATGTCGCGGTTCTCCAGGAGCACCCAG
AGTCTGCACCACAGACACGTCCAGGTAACTGGCCATAGGCTGGTAGGTTCCCGGATATCC
CGGATAGAAGGCAAACTCAGTGGGGCGACTGGGGTACTCTTCCCCGGCCGTGGGAGTCTC
CGCGGGGTACGCGGCCAGGGTGGCTGCCTGGGCACAGGGTTTCAGCGAGCTCCGGGACAC
TCGGCAGGAGTAGTACCCGCCTCCAAAGTAACCATAAGGCACGGAGCTGGATGCGTCCC
CTAGGGCACCCCATGGCATGGGTGGCATTGCTTTGGCGGCTCCGCCGAGCCTGGCAGATC
CAAGGAGGCACTGTT
AA494387 
GGGTGCTGCCCGCTGGAGTCTGCAAATGCTGCCTTCCAAAGGACCTGGTGGGTTCTGT
TCTCCCTGGCAACACATCTGGCTGTTCCAGCCACCAGCGAGACCCAAGACTGGTAACTGT
CCACAGGCAACAGGGAGTCATGTCGCGGTTCTCCAGGAGCACCCAGAGTCTGCACCACAG
ACACGTCCAGGTAACTGGCCATAGGCTGGTAGGTTCCCGGATATCCCGGATAGAAGGCAA
ACTCAGTGGGGCGGCTGGGGTACTCTTCCCCGGCCGTGGGAGTCTCCGCGGGGTACGCGT
CCAGGGTGGCTGCCTGGGCACAGGGTTTCAGCGAGCTCCGGGACACTCGGCAGGAGTAGT
ACCCGCCTCCAAAGTAACCATAAGGCACGGGAGCTGGGGACGTCCCTG
AA662643 
GGGTGCTGCCCGCTGGAGTCTGCAAATGCTGCCTTCCAAAAGGACCTGGTGGGTTCTGT
TCTCCCTGGCAACACATCTGGCTGTTCCAGCCACCAGCGAGACCCAAGACTGGTAACTGT
CCACAGGCAACAGGGAGTCATGTCGCGGTTCTCCAGGAGCACCCAGAGTCTGCACCACAG
ACACGTCCAGGTAACTGGCCATAGGTGGTAGGTTCCCGGATATCCCGGATAGAAGGCAAA
CTCAGTGGGGCGGCTGGGGTACTCTTCCCCGGCCGTGGGAGTCTCCGCGGGGTACGCGGC
CAGGGTGGCTGCCTGGGCACAGGGTTTCAGCGAGCTCCGGGACA
AI935940 
GGGTGCTGCCCGCTGGAGTCTGCAAATGCTGCCTTCCAAAGGACCTGGTGGGTTCTGT
TCTCCCTGGCAACACATCTGGCTGTTCCTGCCACCAGCGAGAGCCCAAGACTGGTAACTG
TCCACAGGCAACAGGGAGTCATGTCGCGGTTCTCCAGGAGCACCCAGAGTCTGCACCACA
GACACGTCCAGGTAACTGGCCATAGGCTGGTAGGTTCCCGGATATCCCGGATAGAAGGCA
AACTCAGTGGGGCGGCTGGGGTACTCTTCCCCGGCCGTGGGAGTCTCCGCGGGGTACGCG
GCCAGGGTGGCTGCCTGGGCACAGGGTTTCAGCG
AA532530 
GGGTGCTGCCCGCTGGAGTCTGCAAATGCTGCCTTCCAAAAGGACCTGGTGGGTTCTGT
TCTCCCTGGCAACACATCTGGCTGTTCCAGCCACCAGCGAGACCCAAGACTGGTAACTGT
 
CCACAGGCAACAGGGAGTCATGTCGCGGTTCTCCAGGAGCACCCAGAGTCTGCACCACAG
ACACGTCCAGGTAACTGGCCATAGGTNGGTAGGTTCCCGGATATCCCGGATAGAAGGCAA
ACTCAGTGGGGCGGCTGGGGTACTCTTCCCCGGCCGTGGAGTCTCCG
AA857572 
CTCCCTGGCAACACATCTGGCTGTTCCAGCACCAGCGAGAGCCAAGACTGGTAACTGTCC
ACAGGCAACAGGGAGTCATGTCGCGGTTCTCCAGGAGCACCCAGAGTCTGCACCACAGAC
ACGTCCAGGTAACTGGCCATAGGTCGGTAGGTTCCCGGATATCCCGGATAGAAGGCAAAC
TCAGTGGGGCGACTGGGGTACTCTTCCCCGGCCGTGGAGTCTCCGCGGGGTACGGCNAC
AGGGTGGCTGCCTGGGCACAGGGTTTCAGCGAGCTCCGGGACACTCGGCAGGAGTAGTAN
CCGCCTCAAAGTAACCATAANGCACGGGAGCTGGGGACGTCCC
AI261980 
ACGAAAGGCGCAGGCGTCAGGAGGGTGCTGCCCGCTGGAGTCTGCAAATGCTGCCTTCCA
AAAGGGACCTGGTGGGTTCTGTTCTCCCTGGCAACACATCTGGCTGTTCCAGCCACCAGC
GAGAGCCCAAGACTGGTAACTGTCCACAGGCAACAGGGAGTCATGTCGCGGTTCTCCAGG
AGCACCCAGAGTCTGCACCACAGACACGTCCAGGTAACTGGCCATAGGCTGGTAGGTTCC
CGGATATCCCGGATAGAAGGCAAACTCAGTGGGGCGACTGGGGTACTCTTCCCCGGCCCG
GGGAGTCTCCGCGGGGTACGCGGCCAGGGTGGCTGCCTGGGCACAGGGTTTCAGCGAGCT
CCGGGACACTCGGCGGAGNTAGTACCCGCCTCCAAAGTAACCATAAGGCACGGGAGCTGG
GGAACCGTCCCCTGGGGCACC
BE888751.1 
GAGCGAGCTGGGTGCCCCCTAGATTCCCCGCCCCCGCACCTCATGAGCCGACCCTCGGCT
CCATGGAGCCCGGCAATTATGCCACCTTGGATGGAGCCAAGGATATCGAAGGCTTGCTGG
GAGCGGGAGGGGGGCGGAATCTGGTCGCCCACTCCCCTCTGACCAGCCACCCAGCGGCGC
CTACGCTGATGCCTGCTGTCAACTATGCCCCCTTGGATCTGCCAGGCTCGGCGGAGCCGC
CAAAGCAATGCCACCCATGCCCTGGGGTGCCCCAGGGACGTCCCCAGCTCCCGTGCCTTA
TGGTTACTTTGGAGGCGGGTACTACTCCTGCCGAGTGTCCCGGAGCTCGCTGAAACCCTG
TGCCCAGGCAGCCACCCTGGCCGCGTACCCCGCGGAGACTCCCACGGCCGGGGAAGAGTA
CCCCAGCCGCCCCACTGAGTTTGCCTTCTATCCGGGATATCCGGGAACCTACCAGCCTAT
GGCCAGTTACCTGGACGTGTCTGTGGTGCAGACTCTGGGTGCTCCTGGAGAACCGCGACA
TGACTCCCTGTTGCCTGTGGACAGTTACCAGTCTTGGGCTCTCGCTGGTGGCTGGAACAG
CCAGATGTGTTGCCAGGGAGAACAGAACCCACCAGGTCCCTTTTTGGAAGGCAGCATTTG
CAGACTCCAGCGGCAGGACCTCCTGAACGCCTGCGCCTTTCGTCGCGGCGTCTAAAGTAA
TCCTCGAGG
AI378797 
GCGGCCGCGGCCCACCACCAACTGCTCGCCACCGACCCCACTACTCGCCACCGACCCGCT
GCTCGGAGCTTCGGTTCTGCGGGTTGTCCAGACTTCAGGCCTGTGCGCTCAATCGTGGAG
AATGCGCCGGCAGGCCCCCCACCCCCAGCCTAAGGTGCAGGAAGGACCAGCACGAACCCG
CTGGCTTTGCTGCGCGGCCAGGAGATGAGTCCCACCGGGCACTGAGCCCAGGTACAGGAC
ATCAGAGAATGAACACAGAGGCAGAGGCCCTCATGTCCCTCTCAGAGTCCCGGCTCTGCA
NAGAGCCCGTCTGTCTCCAGCTTCCAGAATTCCGCACTGTGAATCTGTCTACGTGGACTG
GGAAAACAGGGTTGGCACCACTCTGCCACTCCGTTTGTGCCTGGGAAGGGCTAAGTATGC
AAGGCTACAAACATCTACTTCACTGGGATCCCAAATGCTCAACAAACCATGACCTGCTNT
GGTCAGAACCACCAGAAATATT
AA234220 
GCAGGCGACTTGCGAGCTGGGAGCACTTTAAAACGCTTTGGATTCCCCGGCCTGGGTGG
GGAGAGCGAGCTGGGTGCCCCCTAGATTCCCCGCCCCCGCACCTCATGAGCCGACCCTCG
GCTCCATGGAGCCTGGCATATTATGCCACCTTGGTATGGAGCCAAGGATATCGAAGGCTT
GCTGGGAGCGGAGGGGGCGGAATCTGGTCGCCCACTCCCCTCTGACCAGCCACCCAGC
GGCGCCTACGCTGATGCCTGCTGTCAACTATGCCCCCTTGGA
AA236353 
GCCCGCTGGAGTCTGCAAATGCTGCCTTCCAAAAGGACCTGGTGGGTTCTGTTCTCCCT
GGCAACACATCTGGCTGTTCCAGCCACCAGCGAGACGCCAAGACTGGTAACTGTCCACAG
GCAACAGGGAGTCATGTCGCGGTTCTCCAGGAGCACCCAGAGTCTGCACCACAGACACGT
CCAGGTAACTGGCCATAGGTNGGTAGGTTCCCGGATATCCCGGATAGAAGGCAAACTCAG
TGGGGCGGCTGGGGTACTCTTCCCCGGCCGTGGAGTCTCCGCGGGGTACGCGCACAGGG
TGGCTGCCTGGGCACAGGGTTTCAGCGAGCTCCGGGACACTCGGCAGGAGTAGTACCCGC
CTCCAAAGTAACCATAAGGCA
AA588193 
AACTGCTCGCCACCGACCCCACTACTCGCCACCGACCCGCTGCTCGGAGCTTCGGTTCTG
CGGGTTGTCCAGACTTCAGGCCTGTGCGCTCAATCGTGGAGAATGCGCCGGCAGCCCCCA
CCCCCAGCCTAAGGTGCAGGAAGGACCAGCACGAACCCGCTGGCTTTGCTGCGCGGCCAG
GAGATGAGTCCCACCGGGCACTGAGCCCAGGTACAGGACATCAGAGAATGAACACAGAGG
CAGAGGCCCTCATGTCCCTCTCAGAGTCCCGGCTCTGCAAAGAGCCCGTCTGTCTCCAGC
TTCCAGAATTCCGCACTGTGAATCTGTCTACGTGGACTGGGAAAACAGGGTTGGCACCAC
TCTGCCACTCCGTTTGTGCCTGGGAAGGGCTAAGTATGCAAGGCT
AI821103 
GATCCCTTTGCAGGGAAGCTTTCTCTCAGACCCCCTTCCATTACACCTCTCACCCTGGTA
ACAGCAGGAAGACTGAGGAGAGGGGAACGGCAGATTCGTTGTGTGGCTGTGATGTCCGT
TTAGCATTTTTCTCAGCTGACAGCTGGGTAGGTGGACAATTGTAGAGGCTGTCTCTTCCT
CCCTCCTTGTCCACCCCATAGGGTGTACCCACTGGTCTTGGAAGCACCCATCCTTAATAC
GATGATTTTTCTGTCGTGTGAAAATGAAGCCAGCAGGCTGCCCCTAGTCAGTCCTTCCTT
CCAGAGAAAAAGAGATTTGAGAAAGTGA
AI821851 
TTTTTTTTTTTTTTTTTTTCTTTTTCACTTTCTCAAATCTCTTTTTCTCTGGAAGGAAG
GACTGACTAGGGGCAGCCTGCTGGCTTCATTTCACACGACAAAAAATCATCGTATTAA
GGATGGGTGCTTCCAAAACCAGTGGGTACACCCTATGGGGGGACAAGGAGGAGGAAGA
GACAGCCTCTACAATTGTCCACCTACCCAGCTGTCAGCTGAGAAAAATGCTAAACGGACA
TCACAGCCACACAACGAATCTGCCCGTTCCCCTCTCCTCAGTCTTCCTGCTGTTACCAGG
GTGAGAGGTGTAATGGAAGG
AA635855 
TTTTTTTTTTTTTTTTTTTTTCTTTTTCACTTTCCCAAATCTCTTTTTCTCTGGAAGGAAG
GACTGACTAGGGGCAGCCTGCTGGCTTCATTTCACACGACAGAAAAATCATCGTATTAA
GGATGGGTGCTTCCAAGACCAGTGGGTACACCCTATGGGGTGGACACAGGAGGAGGAAG
AGACAGCCTCTACAATTGTCCACCTACCCAGCTGTCAGCTGAGAAAAATGCTAAACGGAC
ATCACAGCCACACAACGAATCTGCCCGTTCCCCTCTCCTCAGTCTTCCTGCTGTTACCAG
 GGTGAGAGGTGTAATGGAAGG
AI420753 
GCGGCCGCGGCCCACCACCAACTGCTCGCCACCGACCCCACTACTCGCCACCGACCCGCT
GCTCGGAGCTTCGGTTCTGCGGGTTGTCCAGACTTCAGGCCTGTGCGCTCAATCTTGGAG
AATGCGCCGGCAGGCCCCCCACCCCCAGCCTAAGGTGCAGGAAGGACCAGCACGAACCCG
CTGGCTTTGCTGCGCGGCCAGGAGATGAGTCCCACCGGGCACTGAGCCCAGGTACAGGAC
ATCAGAGAATGAACACAGAGGCAGAGGCCCTCATGTCCCTCTCAGAGTCCCGGCTCTGCA
AAGAGCCCGTCTGTCTCCAGCTTCCAGAATTCCGCACTGTGAATCTGTCTACGT
BG180547 
CACGCGTCGATCCCAGTGAAGTAGATGTTTGTAGCCTTGCATACTTAGTCCTTCCCAGGC
ACAAACGGAGTGGCAGAGTGGTGCCAACCCTGTTTTCCCAGTCCACGTAGACAGATTCAC
AGTGCGGAATTCTGGAAGCTGGAGACAGACGGCTCTTTGCAGAGCCGGACTCTGAGAG
GGACATGAGGGCCTCTGCCTCTGTGTTCATTCTCTGATGTCCTGTACCTGGGCTCAGTGC
CCGGTGGGACTCATCTCCTGGCCGCGCAGCAAAGCCAGCGGGTTCGTGCTGGTCCTTCCT
GCACCTTAGGCTGGGGGTGGGGGGCCTGCCGGCGCATTCTCCACGATTGAGCGCACAGGC
CTGAAGTCTGGACAACCCGCAGAACCGAAGCTCCGAGCAGCGGGTCGGTGGCGAGTAGTG
GGGTCGGTGGCGAGCAGTTGGTGGTGGG
AA468306 
TCGACCTCGCCAAGGTGAAGAACAACGCTACCCCTTAAGAGATCTCCTTGCCTGGGTGGG
AGGAGCGAAAGTGGGGGTGTCCTGGGGAGACCAGGAACCTGCCAAGCCCAGGCTGGGGCC
AAGGACTCTGCTGAGAGGCCCCTAGAGACAACACCCTTCCCAGGCCACTGGCTGCTGGAC
TGTTCCTCAGGAGCGGCCTGGGTACCCAGTATGTGCAGGGAGA
AA468232 
TTTTTTACTGGTTATCGTGGTTATTGCCACTGTCAGGATGAATGATTATGACTGGGCCAG
GTTCTTTGGGAACCCTGGTGGAGTGGGCTGTCACATGGGGTTCCGTCTCCCTGCACATAC
TGGGTACCCAGGCCGCTCCTGAGGAACAGTCCAGCAG
CB050115 
GGCCCACCACCAACTGCTCGCCACCGACCCACTACTCGCCACCGACCCGCTGCTCGGAG
CTTCGGTTCTGCGGGTTGTCCAGACTTCAGGCCTGTGCGCTCAATCGTGGAGAATGCGCC
GGCAGGCCCCCCACCCCCAGCCTAAGGTGCAGGAAGGACCAGCACGAACCCGCTGGCTTT
GCTGCGCGGCCAGGAGATGAGTCCCACCGGGCACTGAGCCCAGGTACAGGACATCAGAGA
ATGAACACAGAGGCAGAGGCCCTCATGTCCCTCTCAGAGTCCCGGCTCTGCAAAGAGCCC
GTCTGTCTCCAGCTTCCAGAATTCCGCACTGTGAACCTCGTGCC
CB050116 
GGCACGAGGTTCACAGTGCGGAATTCTGGAAGCTGGAGACAGACGGGCTCTTTGCAGAGC
CGGGACTCTGAGAGGACATGAGGCCTCTGCCTCTGTGTTCATTCTCTGATGTCCTGTA
CCTGGGCTCAGTGCCCGGTGGACTCATCTCCTGGCCGCGCAGCAAAGCCAGCGGGTTCG
TGCTGGTCCTTCCTGCACCTTAGGCTGGGGGTGGGGGCCTGCCGGCGCATTCTCCACGA
TTGAGCGCACAGGCCTGAAGTCTGGACAACCCGCAGAACCGAAGCTCCGAGCAGCGGGTC
GGTGGCGAGTAGTGGGGTCGGTGGCGAGCAGTTGGTGGTGGGCC
AA661819 
GCTGCTCGGAGCTTCGGTTCTGCGGGTTGTCCAGACTTCAGGCCTGTGCGCTCAATCGTG
GAGAATGCGCCGGCAGCCCCCACCCCCAGCCTAAGGTGCAGGAAGGACCAGCACGAACCC
GCTGGCTTTGCTGCGCGGCCAGGAGATGAGTCCCACCGGCACTGAGCCAGGTACAGGACA
TCAGAGAATGAACACAGAGGCAGAGGCCTCATGTCCCTCTCAGAGTCCCGGCTCTGCAAA
GAGCCGTACTGTCTCCAGCTTCCAGAATTCCGCACTGTGAATCTGTCTACGTGGACTGGG
AAAAC
CF146837 
CACGAGGATTTTCTATCTAGAGCTCTGTAGAGCACTTTAGAAACCGCTTTCATGAATTGA
GCTAATTATGAATAAATTTGGAAGGCGATCCCTTTGCAGGGAAGCTTTCTCTCAGACCCC
CTTCCATTACACCTCTCACCCTGGTAACAGCAGGAAGACTGAGGAGAGGGGAACGGGCAG
ATTCGTTGTGTGGCTGTGATGTCCGTTTAGCATTTTTCTCAGCTGACAGCTGGGTAGGTG
GACAATTGTAGAGGCTGTCTCTTCCTCCCTCCTTGTCCACCCCATAGGGTGTACCCACTG
GTCTTGGAAACACCCATCCTTAATACGATGATTTTTCTGTCGTGTGAAAATGAAGCCAGC
AGGCTGCCCCTAGTCAGTCCTTCCTTCCAGAGAAAAAGAGATTTGAGAAAGTGCCTGGGT
AATTCACCATTAATTTCCTCCCCCAAACTCTCTGAGTCTTCCCTTAATATTTCTGGTGGT
TCTGACCAAAGCAGGTCATGGTTTGTTGAGCATTTGGATCCCAGTGAAGTAGATGTTTG
TAGCCTTGCATACTTAGCCCTTCCCAGGCACAAACGGAGTGGCAGAGTGGTGCCAACCCT
GTTTTCCCAGTCCACGTAGACAGATTCACAGTGCGGAATTCTGGAAGCTGGAGACAGACG
GGCTCTTTGCAGAGCCGGGACTCTGAG
CF146763 
CACGAGGATTTTCTATNCTAGAGCTCTGGTAGAGCACTTTANAAACCGCTTTCATGAATT
GAGCTAATTATGAATAAATTTGGAAGGCGATCCCTTTGCAGGAAGCTTTCTCTCAGACC
CCCTTCCATTACACCTCTCACCCTGGTAACAGCAGGAAGACTGAGGAGAGGGGAACGGGC
AGATTCGTTGTGTGGCTGTGATGTCCGTTTAGCATTTTTCTCAGCTGACAGCTGGGTAGG
TGGACAATTGTAGAGGCTGTCTCTTCCTCCCTCCTTGTCCACCCCATAGGGTGTACCCAC
TGGTCTTGGAAACACCCATCCTTAATACGATGATTTTTCTGTCGTGTGAAAATGAAGCCA
GCAGGCTGCCCCTAGTCAGTCCTTCCTTCCAGAGAAAAAGAGATTGAGAAAGTGCCTGGG
TAATTCACCATTAATTTCCTCCCCCAAACTCTCTGAGTCTTCCCTTAATATTTCTGGTGG
TTCTGACCAAAGCAGGTCATGGTTTGTTGAGCATTTGGGATCCCAGTGAAGTAGATGTTT
GTAGCCTTGCATACTTAGCCCTTCCCAGGCACAAACGGAGTGGCAGAGTGGTGCCAACCC
TGTTTTCCCAGTCCACGTAGACAGATTCACAGTGCGGAATTCTGGAAGCTGGAGACAGAC
GGGCTCTTTGCAGAGCCGGGACTCTGA
CF144902 
CACGAGGGAAGCCAGCAGGCTGCCCCTAGTCAGTCCTTCCTTCCAGAGAAAAAGAGATTT
GAGAAAGTGCCTGGGTAATTCACCATTAATTTCCTCCCCCAAACTCTCTGAGTCTTCCCT
TAATATTTCTGGTGGTTCTGACCAAAGCAGGTCATGGTTTGTTGAGCATTTGGGATCCCA
GTGAAGTAGATGTTTGTAGCCTTGCATACTTAGCCCTTCCCAGGCACAAACGGAGTGGCA
GAGTGGTGCCAACCCTGTTTTCCCAGTCCACGTAGACAGATTCACAGTGCGGAATTCTGG
AAGCTGGAGACAGACGGGCTCTTTGCAGAGCCGGACTCTGAGAGGACATGAGGGCCTC
TGCCTCTGTGTTCATTCTCTGATGTCCTGTACCTGGGCTCAGTGCCCGGTGGACTCATC
TCCTGGGCGCGCAGCAAAGCCAGCGGGTTCGTGCTGGTCCTTCCTGCACCTTA
CF141511.1 
CACGAGGCCTGGTAACAGCAGGAAGACTGAGGAGAGGGGAACGGGCAGATTCGTTGTGTG
GCTGTGATGTCCGTTTAGCATTTTTCTCAGCTGACAGCTGGGTAGGTGGACAATTGTAGA
GGCTGTCTCTTCCTCCCTCCTTGTCCACCCCATAGGGTGTACCCACTGGTCTTGGAAACA
CCCATCCTTAATACGATGATTTTTCTGTCGTGTGAAAATGAAGCCAGCAGGCTGCCCCTA
GTCAGTCCTTCCTTCCAGAGAAAAAGAGATTTGAGAAAGTGCCTGGGTAATTCACCATTA
ATTTCCTCCCCCAAACTCTCTGAGTCTTCCCTTAATATTTCTGGTGGTTCTGACCAAAGC
AGGTCATGGTTTGTTGAGCATTTGGGATCCCAGTGAAGTAGATGTTTGTAGCCTTGCATA
CTTAGCCCTTCCCAGGCACAAACGGAGTGGCAGAGTGGTGCCAACCCTGTTTTCCCAGTC
CACGTAGACAGATTCACAGTGCGGAATTCTGGAA
CF139563.1 
CACGAGGTCTTCCCTTAATATTTCTGGTGGTTCTGACCAAAGCAGGTCATGGTTTGTTGA
GCATTTGGGATCCCAGTGAAGTAGATGTTTGTAGCCTTGCATACTTAGCCCTTCCCAGGC
ACAAACGGAGTGGCAGAGTGGTGCCAACCCTGTTTTCCCAGTCCACGTAGACAGATTCAC
AGTGCGGAATTCTGGAAGCTGGAGACAGACGGGCTCTTTGCAGAGCCGGGACTCTGAGAG
GGACATGAGGGCCTCTGCCTCTGTGTTCATTCTCTGATGTCCTGTACCTGGGCTCAGTGC
CCGGTGGGACTCATCTCCTGGCCGCGCAGCAAAGCCAGCGGGTTCGTGCTGGTCCTTCCT
GCACCTTAGGCTGGGGGTGGGGGGCCTGCCGGCGCATTCTCCACGATTGAGCGCACAGGC
CTGAAGTCTGGACAACCCGCAGAACCGAAGCTCCGAGCAGCGGGTCGGTGGCGAGTA
CF139372 
CACGAGGATTTCTGGTGGTTCTGACCAAAGCAGGTCATGGTTTGTTGAGCATTTGGGATC
CCAGTGAAGTAGATGTTTGTAGCCTTGCATACTTAGCCCTTCCCAGGCACAAACGGAGTG
GCAGAGTGGTGCCAACCCTGTTTTCCCAGTCCACGTAGACAGATTCACAGTGCGGAATTC
TGGAAGCTGGAGACAGACGGGCTCTTTGCAGAGCCGGGACTCTGAGAGGGACATGAGGGC
CTCTGCCTCTGTGTTCATTCTCTGATGTCCTGTACCTGGGCTCAGTGCCCGGTGGGACTC
ATCTCCTGGCCGCGCAGCAAAGCCAGCGGGTTCGTGCTGGTCCTTCCTGCACCTT
CF139319 
CACGAGGAAGGCGATCCCTTTGCAGGGAAGCTTTCTCTCAGACCCCCTTCCATTACACCT
CTCACCCTGGTAACAGCAGGAAGACTGAGGAGAGGGAACGGCAGATTCGTTGTGTGGC
TGTGATGTCCGTTTAGCATTTTTCTCAGCTGACAGCTGGGTAGGTGGACAATTGTAGAGG
CTGTCTCTTCCTCCCTCCTTGTCCACCCCATAGGGTGTACCCACTGGTCTTGGAAACACC
CATCCTTAATACGATGATTTTTCTGTCGTGTGAAAATGAAGCCAGCAGGCTGCCCCTAGT
CAGTCCTTCCTTCCAGAGAAAAAGAGATTTGAGAAAGTGCCTGGGTAATTCACCATTAAT
TTCCTCCCCCAAACTCTCTGAGTCTTCCCTTAATATTTCTGGTGGTTCTGACCAAAGCAG
GTCATGGTTTGTTGAGCATTTGGGATCCCAGTGAAGTAGATGTTTGTAGCCTTGCATACT
TAGCCCTTCC
CF139275 
CACGAGGTGGATTCCCCCGGCCTGGGTGGGAGAGCGAGCTGGGTGCCCCCTAGATTCCC
CGCCCCCGCACCTCATGAGCCGACCCTCGGCTCCATGGAGCCCGGCAATTATGCCACCTT
GGATGGAGCCAAGGATATCGAAGGCTTGCTGGGAGCGGGAGGGGGCGGAATCTGGTCGC
CCACTCCCCTCTGAGCAGCCACCCAGCGGCGCCTACGCTGATGCCTGCTGTCAACTATGC
CCCCTTGGATCTGCCAGGCTCGGCGGAGCCGCCAAAGCAATGCCACCCATGCCCTGGGGT
GCCCCAGGGGACGTCCCCAGCTCCCGTGCCTTATGGTTACTTTGGAGGCGGGTACTACTC
CTGCCGAGTGTCGCGGAGCTCGCTGAAACCCTGTGCCCAGGCA
CF122893 
CACGAGGATTTTCTATCTAGAGCTCTGTAGAGCACTTTAGAAACCGCTTTCATGAATTGA
GCTAATTATGAATAAATTTGGAAGGCGATCCCTTTGCAGGAAGCTTTCTCTCAGACCCC
CTTCCATTACACCTCTCACCCTGGTAACAGCAGGAAGACTGAGGAGAGGGGAACGGGCAG
ATTCGTTGTGTGGCTGTGATGTCCGTTTAGCATTTTTCTCAGCTGACAGCTGGGTAGGTG
GACAATTGTAGAGGCTGTCTCTTCCTCCCTCCTTGTCCACCCCATAGGGTGTACCCACTG
GTCTTGGAAACACCCATCCTTAATACGATGATTTTTCTGTCGTGTGAAAATGAAGCCAGC
AGGCTGCCCCTAGTCAGTCCTTCCTTCCAGAGAAAAAGAGATTTGAGAAAGTGCCTGGGT
AATTCACCATTAATTTCCTCCCCCAAACTCTCTGAGTCTTCCCTTAATATTTCTGGTGGT
TCTGACCAAAGCAGGTCATGGTTTGTTGAGCATTTGGGATCCCAGTGAAGTANATGTTTG
TAGCCTTGCATACTTAGCCCTT
AI972423 
CATTTTCACACGACTGTAAAATCATCGTATTAAGGATGGGTGCTTCCAAGACCAGTGGGT
ACACCCTATGGGGTGGACAAGGAGGGAGGAAGAGACAGCCTCTACAATTGTCCACCTACC
CAGCTGTCAGCTGAGAAAAATGCTAAACGGACATCACAGCCACACAACGAATCTGCCCGT
TCCCCTCTCCTCAGTCTTCCTGCTGTTACCAGGGTGAGAGGTGTAATGGAAGGGGGTCTG
AGAGAAAGCTTCCCTGCAAAGGGATCGCCTTCCAAATTTATTCATAATTAGCTCAATTCA
TGAAAGCGGTTTCTAAAGTGCTCTACAGAGCTCTAGATAGAAAATATGAGGCTAACGATC
ATGGCAGCTAGTACTGGTTATCGTGATTATTGCCACTGTCAGGATGAATGATTATGACTG
GGCCAGGTTCTTTGGGAACCCTGGTGGAGTGGGCTGTCACATG
AI918975 
TGCAGCTAGTACTGGTTATCGTGATTATTGCCACTGTCAGGATGAATGATTATGACTGGG
CCAGGTTCTTTGGGAACCCTGGTGGAGTGGCTGTCACATGGGGTTCCGTCTCCCTGCAC
ATACTGGGTACCCAGGCCGCTCCTGAGGAACAGTCCAGCACAGGGTTTCAGCGAGCTCCG
GGACACTCGGCCTCGTGC
AI826991 
TTTTTTTTTTTTTTTTTTTTTCTTTTTCACTTTCTCAAATCTCTTTTTCTCTGGAAGGAAG
GACTGACTAGGGGCAGCCTGCTGGCTTCATTTTCACACCACAAAAAATCATCGTATTAA
GGATGGGTGCTTCCAAAACCAGTGGGTACACCCTATGGGGTGGACAAGGAGGGAGGAAAA
AACAGCCTCTACAATTGTCCACCTACCCAGCTGTCAGCTGAAAAAAATGCTAAACGGACA
TCACAGCCACACAACGAATCTGCCCGTTCCCCTCTCCTCAGTCTTCCTGCTGTTACCAGG
GTGAAAGGTGTAATGGAAGG
AI686312 
ACCGACCCCACTACTTGCCACCGACCCGCTGCTCGGAGCTTCGGTTCTGCGGGTTGTCCA
GACTTCAGGCCTGTGCGCTCAATCGTGGAGAATGCGCCGGCAGGCCCCCACCCCCAGCC
TAAGGTGCAGGAAGGACCAGCACGAACCCGCTGGCTTTGCTGCGCGGCCAGGAGATGAGT
CCCACCGGGCACTGAGCCCAGGTACAGGACATCAGAGAATGAACACAGAGGCAGAGGCCC
TCATGTCCCTCTCAGAGTCCCGGCTCTGCAAAGAGCCCGTCTGTCTCCAGCTTCCAGAAT
TCCGCACTGTGAATCTGTCTACGTGGACTGGGAAAACAGGGTTGGCACCACTCTGCCACT
CCGTTTGTGCCTGGAAGGGCTAAGTATGCAAGGCTACAAACATCTACTTCACTGGGATC
C
AI655923 
TTTTTTTTTTTTTTTCCCTGCAAAGGATCGCCTTCCAAATTTATTCATAATTAGCTCAA
TTCATGAAAGCGGTTTCTAAAGTGCTCTACAGAGCTCTAGATAGAAAATATGAGGCTAAC
GATCATGGCAGCTAGTACTGGTTATCGTGATTATTGCCACTGTCAGGATGAATGATTATG
ACTGGGCCAGGTTCTTTGGGAACCCTGGTGGAGTGGGCTGTCACATGGGGTTCCGTCTCC
CTGCACATACTGGGTACCCAGGCCGCTCCTGA
CF146922 
CACGAGGCGACTTGCGAGCTGGGAGCGATTTAAAACGCTTTGGATTCCCCGGCCTGGGTG
GGAGAGCGAGCTGGGTGCCCCCTAGATTCCCCGCCCCCGCACCTCATGAGCCGACCCTC
GGCTCCATGGAGCCCGGCAATTATGCCACCTTGGATGGAGCCAAGGATATCGAAGGCTTG
CTGGGAGCGGGAGGGGGGCGGAATCTGGTCGCCCACTCCCCTCTGACCAGCCACCCAGCG
GCGCCTACGCTGATGCCTGCTGTCAACTATGCCCCCTTGGATCTGCCAGGCTCGGCGGAG
CCGCCAAAGCAATGCCACCCATGCCCTGGGGTGCCCCAGGGACGTCCCCAGCTCCCGTG
CCTTATGGTTACTTTGGAGGCGGGTACTACTCCTGCCGAGTGTCCCGGAGCTCGCTGAAA
CCCTGTGCCCAGGCAGCCACCCTGGCCGCGTACCCCGCGGAGACTCCCACGGCCGGGGAA
GAGTACCCCAGCCGCCCCACTGAGTTTGCCTTCTATCCGGGATATCCGGGAACCTACCAG
CCTATGGCCAGTTACCTGGACGTGTCTGTGGTGCAGACTCTGGGTGCTCCTGGAGAACGC
GACATGACTCCCTGTTGCCTGTGGACAGTTACCAGTCTTGGGCTCTCGCTGGTGGCTGGA
ACAGCCAGATGTGTTGCCA
BF476369 
GCGGCCGCGGCCCACCACCAACTGCTCGCCATTCGACCCACTACTCGCCACCGACCCGC
TGCTCGGAGCTTCGGTTCTGCGGGTTGTCCAGACTTCAGGCCTGTGCGCTCAATCGTGGA
GAATGCGCCGGCAGGCCCCCCACCCCCAGCCTAAGGTGCAGGAAGGACCAGCACGAACCC
GCTGGCTTTGCTGCGCGGCCAGGAGATGAGTCCCACCGGGCACTGAGCCCAGGTACAGGA
CATCAGAGAATGAACACAGAGGCAGAGGCCCTCATGTCCCTCTCAGAGTCCCGGCTCTGC
AAAGAGCCCGTCTGTCTCCAGCTTCCAGAATTCCGCACTGTGAATCTGTCTACGTGGACT
GGGAAAACAGGGTTGGCACCACTCTGCCACTCC
BF057410 
GCGGCCGCGGCCCACCACCAACTGCTCGCCACCGACCCACTACTCGCCACCGACCCGCT
GCTCGGAGCTTCGGTTCTGCGGGTTGTCCAGACTTCAGGCCTGTGCGCTCAATCGTGGAG
AATGCGCCGGCAGGCCCCCCACCCCCAGCCTAAGGTGCAGGAAGGACCAGCACGAACCCG
CTGGCTTTGCTGCGCGGCCAGGAGATGAGTCCCACCGGGCACTGAGCCCAGGTACAGGAC
ATCAGAGAATGAACACAGAGGCAGAGGCCCTCATGTCCCTCTCAGAGTCCCGGCTCTGCA
AAGAGCCCGTCTGTCTCCAGCTTCCAGAATTCCGCACTGTGAATCTGTCTACGTGGACTG
GGAAAACAGGGTTGGCACCACTCTGCCACTCCGTTTGTGCCTGGGAAGGGCTAAGTATGC
AAGGCTACAAACATCTACTTCACTGGGATCCCAAATGCTCAACAAACCATGACCTGCTNT
GGTCAGAACCACCAGAAATATTAA
BE645544 
GCGGCCGCGGCCCACCACCAACTGCTCGCCACCGACCCCACTACTCGCCACCGACCCGCT
GCTCGGAGCTTCGGTTCTGCGGGTTGTCCAGACTTCAGGCCTGTGCGCTCAATCGTGGAG
AATGCGCCGGCAGGCCCCCCACCCCCAGCCTAAGGTGCAGGAAGGACCAGCACGAACCCG
CTGGCTTTGCTGCGCGGCCAGGAGATGAGTCCCACCGGGCACTGAGCCCAGGTACAGGAC
ATCAGAGAATGAACACAGAGGCAGAGGCCCTCATGTCCCTCTCAGAGTCCCGGCTCTGCA
AAGAGCCCGTCTGTCTCCAGCTTCCAGAATTCCGCACTGTGAATCTGTCTACGTGGACTG
GGAAAACAGGGTTGGCACCACTCTGCCACTCCGTTTGTGCCTGGGAAGGGCTAAGTATGC
AAGGCTACAAACATCTACTTCACTGGGATCC
BE645408 
TCCTCCCTCTAAGAAAGGCGCAAGCGTCAAGAGGGTGCTGCCCGCTGGTTTCTGCAAATG
CTGCCTTCCAAAAAGGACCTGGTGGGTTCTGTTCTCCCTGGCAACACATCTGGCTGTTCC
AGCCACCAGCGAGAGCCCAAGACTGGTAACTGTCCACAGGCAACAGGGAGTCATGTCGCG
GTTCTCCAGGAGCACCCAGAGTCTGCACCACAGACACGT
BE388501 
TTAATACGATGATTTTTCTGTCGTGTGAAAATGAAGCCAGCAGGCTGCCCCTAGTCAGTC
CTTCCTTCCAGAGAAAAAGAGATTTGAGAAAGTGCCTGGGTAATTCACCATTAATTTCCT
CCCCCAAACTCTCTGAGTCTTCCCTTAATATTTCTGGTGGTTCTGACCAAAGCAGGTCAT
GGTTTGTTGAGCATTTGGGATCCCAGTGAAGTAGATGTTTGTAGCCTTGCATACTTAGCC
CTTCCCAGGCACAAACGGAGTGGCAGAGTGGTGCCAACCCTGTTTTCCCAGTCCACGTAG
ACAGATTCACAGTGCGGAATTCTGGAAGCTGGAGACAGACGGGCTCTTTGCAGAGCCGGG
ACTCTGAGAGGGACATGAGGGCCTCTGCCTCTGTGTTCATTCTCTGATGTCCTGTACCTG
GGCTCAGTGCCCGGTGGGACTCATCTCCTGGCCGCGCAGCAAAGCCAGCGGGTTCGTGCT
GGTCCTTCCTGCACCTTAGGCTGGGGGTGGGGGGCCTGCCGGCGCATTCTCCACGATTGA
GCGCACAGGCCTGAAGTCTGGACAACCCGCAGAACCGAAGCTCCGAGCAGCGGGTCGGTG
GCGAGTAGTGGGGGTCGGTGGCGAACAAGTGGTGGTGGGCCGGGGCCGCATAACTCGAGG
ACTTTCCTCCCGGAGCAGTCCCTAAAAACCCGGGGGCGC
CF147366 
GACGAGGACAATTGTAGAGGCTGTCTCTTCCTCCCTCCTTGTCACCCCATAGGGTGTACC
ACTGGTCTTGGAAGCACCCATCCTTAATACGATGATTTTTCTGTCGTGTGAAAATGAAGC
CAGCAGGCTGCCCCTAGTCAGTCCTTCCTTCCAGAGAAAAAGAGATTTGAGAAAGTGCCT
GGGTAATTCACCATTAATTTCCTCCCCCAAACTCTCTGAGTCTTCCCTTAATATTTCTGG
TGGTTCTGACCAAAGCAGGTCATGGTTTGTTGAGCATTTGGGATCCCAGTGAAGTAGATG
TTTGTAGCCTTGCATACTTAGCCCTTCCCAGGCACAAACGGAGTGGCAGAGTGGTGCCAA
CCCTGTTTTCCCAGTCCACGTAGACAGATTCACAGTGCGGAATTCTGGAAGCTGGAGACA
GACGGGCTCTTTGCAGAGCCGGGACTCTGAGAGGACATGAGGCCTCTGCCTCTGTGTT
CATTCTCTGATGTCCTGTACCTGGGCTCAGTGCCCGGTGGACTCATCTCCTGGCCGCGC
AGCAAAGCCAGCGGGTTCGTGCTGGTCCTTCCTGC
CF147143 
CACGAGGCGACTTGCGAGCTGGGAGCGATTTAAAACGCTTTGGATTCCCCCGGCCTGGGT
GGGGAGAGCGAGCTGGGTGCCCCCTAGATTCCCCGCCCCCGCACCTCATGAGCCGACCCT
CGGCTCCATGGAGCCCGGCAATTATGCCACCTTGGATGGAGCCAAGGATATCGAAGGCTT
GCTGGGAGCGGGAGGGGGGCGGAATCTGGTCGCCCACTCCCCTCTGACCAGCCACCCAGC
GGCGCCTACGCTGATGCCTGCTGTCAACTATGCCCCCTTGGATCTGCCAGGCTCGGCGGA
GCCGCCAAAGCAATGCCACCCATGCCCTGGGGTGCCCCAGGGGACGTCCCCAGCTCCCGT
GCCTTATGGTTACTTTGGAGGCGGGTACTACTCCTGCCGAGTGTCCCGGAGCTCGCTGAA
ACCCTGTGCCCAGGCAGCCACCCTGGCCGCGTACCCCGCGGAGACTCCCACGGCCGGGGA
AGAGTACCCAGCCGCCCCACTGAGTTTGCCTTCTATCCGGGATATCCGGAACCTACCAG
CCTATGGCCAGTTACCTGGACGTGTCTGTGGTGCAGACTCTGGGTGCTCCTGGAGAACGC
GACATGACTCCCTGTTGCCTGTGGACAGTTACCAATCTTGGGCTCTCGCTGGTGGCTGGA
ACAGCCAGATGTGTTGCCAGGGAG
BT007410 
atggagcccg gcaattatgc caccttggat ggagccaagg atatcgaagg cttgctggga
gcgggagggg ggcggaatct ggtcgcccac tcccctctga ccagccaccc agcggcgcct
acgctgatgc ctgctgtcaa ctatgccccc ttggatctgc caggctcggc ggagccgcca
aagcaatgcc acccatgccc tggggtgccc caggggacgt ccccagctcc cgtgccttat
ggttactttg gaggcgggta ctactcctgc cgagtgtccc ggagctcgct gaaaccctgt
gcccaggcag ccaccctggc cgcgtacccc gcggagactc ccacggccgg ggaagagtac
cccagccgcc ccactgagtt tgccttctat ccgggatatc cgggaaccta ccagcctatg
gccagttacc tggacgtgtc tgtggtgcag actctgggtg ctcctggaga accgcgacat
gactccctgt tgcctgtgga cagttaccag tcttgggctc tcgctggtgg ctggaacagc
cagatgtgtt gccagggaga acagaaccca ccaggtccct tttggaaggc agcatttgca
gactccagcg ggcagcaccc tcctgacgcc tgcgcctttc gtcgcggccg caagaaacgc
attccgtaca gcaaggggca gttgcgggag ctggagcggg agtatgcggc taacaagttc
atcaccaagg acaagaggcg caagatctcg gcagccacca gcctctcgga gcgccagatt
accatctggt ttcagaaccg ccgggtcaaa gagaagaagg ttctcgccaa ggtgaagaac
agcgctaccc cttag
BC007092 
ggattccccc ggcctgggtg gggagagcga gctgggtgcc ccctagattc cccgccccg
cacctcatga gccgaccctc ggctccatgg agcccggcaa ttatgccacc ttggatggag
ccaaggatat cgaaggcttg ctgggagcgg gaggggggcg gaatctggtc gcccactccc
ctctgaccag ccacccagcg gcgcctacgc tgatgcctgc tgtcaactat gcccccttgg
atctgccagg ctcggcggag ccgccaaagc aatgccaccc atgccctggg gtgccccagg
ggacgtcccc agctcccgtg ccttatggtt actttggagg cgggtactac tcctgccgag
tgtcccggag ctcgctgaaa ccctgtgccc aggcagccac cctggccgcg taccccgcgg
agactcccac ggccggggaa gagtacccca gccgccccac tgagtttgcc ttctatccgg
gatatccggg aacctaccag cctatggcca gttacctgga cgtgtctgtg gtgcagactc
tgggtgctcc tggagaaccg cgacatgact ccctgttgcc tgtggacagt taccagtctt
gggctctcgc tggtggctgg aacagccaga tgtgttgcca gggagaacag aacccaccag
gtcccttttg gaaggcagca tttgcagact ccagcgggca gcaccctcct gacgcctgcg
cctttcgtcg cggccgcaag aaacgcattc cgtacagcaa ggggcagttg cgggagctgg
agcgggagta tgcggctaac aagttcatca ccaaggacaa gaggcgcaag atctcggcag
ccaccagcct ctcggagcgc cagattacca tctggtttca gaaccgccgg gtcaaagaga
agaaggttct cgccaaggtg aagaacagcg ctacccctta agagatctcc ttgcctgggt
gggaggagcg aaagtggggg tgtcctgggg agaccaggaa cctgccaagc ccaggctggg
gccaaggact ctgctgagag gcccctagag acaacaccct tcccaggcca ctggctgctg
gactgttcct caggagcggc ctgggtaccc agtatgtgca gggagacgga accccatgtg
acagcccact ccaccagggt tcccaaagaa cctggcccag tcataatcat tcatcctgac
agtggcaata atcacgataa ccagtactag ctgccatgat cgttagcctc atattttcta
tctagagctc tgtagagcac tttagaaacc gctttcatga attgagctaa ttatgaataa
atttggaaaa aaaaaaaaaa aaaaaaaaaa aaaaaa
U57052 
cgggtgcccc ctagattccc cgcccccgca cctcatgagc cgaccctcgg ctccatggag
cccggcaatt atgccacctt ggatggagcc aaggatatcg aaggcttgct gggagcggga
ggggggcgga atctggtcgc ccactcccct ctgaccagcc acccagcggc gcctacgctg
atgcctgctg tcaactatgc ccccttggat ctgccaggct cggcggagcc gccaaagcaa
tgccacccat gccctggggt gccccagggg acgtccccag ctcccgtgcc ttatggttac
tttggaggcg ggtactactc ctgccgagtg tcccggagct cgctgaaacc ctgtgcccag
gcagccaccc tggccgcgta ccccgcggag actcccacgg ccggggaaga gtaccccagc
cgccccactg agtttgcctt ctatccggga tatccgggaa cctaccacgc tatggccagt
tacctggacg tgtctgtggt gcagactctg ggtgctcctg gagaaccgcg acatgactcc
 
ctgttgcctg tggacagtta ccagtcttgg gctctcgctg gtggctggaa cagccagatg
tgttgccagg gagaacagaa cccaccaggt cccttttgga aggcagcatt tgcagactcc
agcgggcagc accctcctga cgcctccgcc tttcgtcgcg gccgcaagaa acgcattccg
tacagcaagg ggcagttgcg ggagctggag cgggagtatg cggctaacaa gttcatcacc
aaggacaaga ggcgcaagat ctcggcagcc accagcctct cggagcgcca gattaccatc
tggtttcaga accgccgggt caaagagaag aaggttctcg ccaaggtgaa gaacagcgct
accccttaag agatctcctt gcctgggtgg gaggagcgaa agtgggggtg tcctggggag
accaggaacc tgccaagccc aggctggggc caaggactct gctgagaggc ccctagagac
aacacc
U81599 
tcctaatacg actcactata gggctcgagc ggccgcccgg gcaggtcgaa tgcaggcgac
ttgcgagctg ggagcgattt aaaacgcttt ggattccccc ggcctgggtg gggagagcga
gctgggtgcc ccctagattc cccgcccccg cacctcatga gccgaccctc ggctccatgg
agcccggcaa ttatgccacc ttggatggag ccaaggatat cgaaggcttg ctgggagcgg
gaggggggcg gaatctggtc gcccactccc ctctgaccag ccacccagcg gcgcctacgc
tgatgcctgc tgtcaactat gcccccttgg atctgccagg ctcggcggag ccgccaaagc
aatgccaccc atgccctggg gtgccccagg ggacgtcccc agctcccgtg ccttatggtt
actttggagg cgggtactac tcctgccgag tgtcccggag ctcgctgaaa ccctgtgccc
aggcagccac cctggccgcg taccccgcgg agactcccac ggccggggaa gagtacccca
gtcgccccac tgagtttgcc ttctatccgg gatatccggg aacctaccac gctatggcca
gttacctgga cgtgtctgtg gtgcagactc tgggtgctcc tggagaaccg cgacatgact
ccctgttgcc tgtggacagt taccagtctt gggctctcgc tggtggctgg aacagccaga
tgtgttgcca gggagaacag aacccaccag gtcccttttg gaaggcagca tttgcagact
ccagcgggca gcaccctcct gacgcctgcg cctttcgtcg cggccgcaag aaacgcattc
cgtacagcaa ggggcagttg cgggagctgg agcgggagta tgcggctaac aagttcatca
ccaaggacaa gaggcgcaag atctcggcag ccaccagcct ctcggagcgc cagattacca
tctggtttca gaaccgccgg gtcaaagaga agaaggttct cgccaaggtg aagaacagcg
ctacccctta agagatctcc ttgcctgggt gggaggagcg aaagtggggg tgtcctgggg
agaccagaaa cctgccaagc ccaggctggg gccaaggact ctgctgagag gcccctagag
acaacaccct tcccaggcca ctggctgctg gactgttcct caggagcggc ctgggtaccc
agtatgtgca gggagacgga accccatgtg acaggcccac tccaccaggg ttcccaaaga
acctggccca gtcataatca ttcatcctca cagtggcaat aatcacgata accagt
CB120119 
ATTTTTCTGTCGTGTGAAAATGAAGCCAGCAGGCTGCCCCTAGTCAGTCCTTCCTTCCAG
AGAAAAAGAGATTTGAGAAAGTGCCTGGGTAATTCACCATTAATTTCCTCCCCCAAACTC
TCTGAGTCTTCCCTTAATATTTCTGGTGGTTCTGACCAAAGCAGGTCATGGTTTGTTGAG
CATTTGGGATCCCAGTGAAGTAGATGTTTGTAGCCTTGCATACTTAGCCCTTCCCAGGCA
CAAACGGAGTGGCAGAGTGGTGCCAACCCTGTTTTCCCAGTCCACGTAGACAGATTCACA
GTGCGGAATTCTGGAAGCTGGAGACAGACGGGCTCTTTGCAGAGCCGGGACTCTGAGAGG
GACATGAGGGCCTCTGCCTCTGTGTTCATTCTCTGATGTCCTGTACCTGGGCTCAGTGCC
CGGTGGGACTCATCTCCTGGCTGCGCAGCAAAGCCAGCGGGTTCGTGCTGGTCCTTCCTG
CACCTTAGGCTGGGGGTGGGGGGCCT
CB125764 
ATTTTTCTGTCGTGTGAAAATGAAGCCAGCAGGCTGCCCCTAGTCAGTCCTTCCTTCCAG
AGAAAAAGAGATTTGAGAAAGTGCCTGGGTAATTCACCATTAATTTCCTCCCCCAAACTC
TCTGAGTCTTCCCTTAATATTTCTGGTGGTTCTGACCAAAGCAGGTCATGGTTTGTTGAG
CATTTGGGATCCCAGTGAAGTAGATGTTTGTAGCCTTGCATACTTAGCCCTTCCCAGGCA
 
CAAACGGAGTGGCAGAGTGGTGCCAACCCTGTTTTCCCAGTCCACGTAGACAGATTCACA
GTGCGGAATTCTGGAAGCTGGAGACAGACGGCTCTTTGCAGAGCCGGACTCTGAGAGG
GACATGAGGGCCTCTGCCTCTGTGTTCATTCTCTGATGTCCTGTACCTGGCTCAGTGCC
CGGTGGGACTCATCTCCTGGCTGCGCAGCAAAGCCAGCGGGTTCGTGCTGGTCCTTCCTG
CACCTTAGGCTGGGGGTGGGGGGGCCTGCCGGCGCATTCTCCACGATTGAGCGCACAGG
CCTGAAGTCTGGACAACCCGCAGAACCGAAGCTCCGAGCAGCGGGTCGGTGGCGAGT
AU098628 
ATTTAAAACGCTTTGGATTCTTTCGTCCTGCGTGGGGAGAGCGAGCTGGGTGCCCCCTAG
ATTCCCCGCCCCCGCACCTCATGAGCCGACCCTCGGCTCCATGGAGCCCGGCACTTATGC
CACCTTGGATGGAGCCAAGGATATCGAAGGCTTGCTGGAGCGGGAGGGGGCGGAATCT
GGTCGCCCACTCCCCTCTGACCAGCCACCCAGCGGCGCCTACGCTGATGCCTGCTGTCAA
TTATGCCCCCTTGCATCTGCCAGGCTCGGCGGAGCCGCCAAAGCAATGCCACCCATGCCC
CB126130 
ATTTTTCTGTCGTGTGAAAATGAAGCCAGCAGGCTGCCCCTAGTCAGTCCTTCCTTCCAG
AGAAAAAGAGATTTGAGAAAGTGCCTGGGTAATTCACCATTAATTTCCTCCCCCAAACTC
TCTGAGTCTTCCCTTAATATTTCTGGTGGTTCTGACCAAAGCAGGTCATGGTTTGTTGAG
CATTTGGGATCCCAGTGAAGTAGATGTTTGTAGCCTTGCATACTTAGCCCTTCCCAGGCA
CAAACGGAGTGGCAGAGTGGTGCCAACCCTGTTTTCCCAGTCCACGTAGACAGATTCACA
GTGCGGAATTCTGGAAGCTGGAGACAGACGGGCTCTTTGCAGAGCCGGGACTCTGAGAGG
GACATGAGGGCCTCTGCCTCTGTGTTCATTCTCTGATGTCCTGTACCTGGGCTCAGTGCC
CGGTGGGACTCATCTCCTGGCTGCGCAGCAAAGCCAGCGGGTTCGTGCTGGTCCTTCCTG
CACCTTAGGCTGGGGGTGGGGGGCCTGC
BI023924 
AGGCCGCACCCAGTCTTAAGGTGCAGTGAAGGACAGCACGAACCCGCTGTGCTTTGCTGC
GCGGCAGGAGATGAGTCCCACCGGGCACTGAGCCCAGGTACAGGACATCAGAGAATGAAC
ACAGAGGCAGAGGCCCTCATGTCCCTCTCAGAGTCCCGGCTCTGCAAAGAGCCCGTCTGT
CTCCAGCTTCCAGAATTCCGCACTGTGAATCTGTCTACGTGGACTGNGAAAACAGGGTTG
GCACCACTCTGCCACTCCGTTTGTGCCTNGGGGCGGCAGAGGG
BM767063.1 
AAAAACGCTTTGGATTCCCCCGGCCTGGGTGGGAGAGCGAGCTGGGTGCCCCCTAGATT
CCCCGCCCCCGCACCTCATGAGCCGACCCTCGGCTCCATGGAGCCCGGCAATTATGCCAC
CTTGGATGGAGCCAAGGATATCGAAGGCTTGCTGGGAGCGGGAGGGGGGCGGAATCTGGT
CGCCCACTCCCCTCTGACCAGCCACCCAGCGGCGCCTACGCTGATGCCTGCTGTCAACTA
TGCCCCCTTGGATCTGCCAGGCTCGGCGGAGCCGCCAAAGCAATGCCACCCATGCCCTGG
GGTGCCCCAGGGGACGTCCCCAGCTCCCGTGCCTTATGGTTACTTTGGAGGCGGGTACTA
CTCCTGCCGAGTGTCCCGGAGCTCGCTGAAACCCTGTGCCCAGGCAGCCACCCTGGCCGC
GTACCCCGCGGAGACTCCCACGGCCGGGGAAGAGTACCCCAGCCGCCCCACTGAGTTTGC
CTTCTATCCGGGATATCCGGGAACCTACCAGCCTATGGCCAGTTACCTGGACGTGTCTGT
GGTGCAGACTCTGGGTGCTCCTGGAGAACCGCGACATGACTCCCTGTTGCCTGTGGACAG
TTACCAGTCTTGGGCTCTCGCTGGTGGCTGGAACAGCCAGATGTGTTGCCA
BM794275 GCAGACTCTGGGTGCTCCTGGAGAACCGCGACGTGACTCCCTGTTGCCTGTGGACAGTTA 
CCACTCTTGGGCTCTCGCTGGTGGCTGGAACAGCCAGATGTGTTGCCAGGGAGAACAGAA
CCCACCAGGTCCCTTTTGGAAGGCAGCATTTGCAGACTCCAGCGGCAGCACCCTCCTGA
CGCCTGCGCCTTTCGTCGCGGCCGCAAGAAACGCATTCCGTACAGCAAGGGGCAGTTGCG
GGAGCTGGAGCGGGAGTATGCGGCTAACAAGTTCATCACCAAGGACAAGAGGCGCAAGAT
CTCGGCAGCCACCAGCCTCTCGGAGCGCCAGATTACCATCTGGTTTCAGAACCGCCGGGT
CAAAGAGAAGAAGGTTCTCGCCAAGGTGAAGAACAGCGCTACCCCTTAAGAGATCTCCTT
GCCTGGGTGGAGGATCTAAAGTGGGGGTGTCCTGGGGAGACCAGGAACCTGCCAAGCCC
AGGCTGGGGCCAAGGACT
BQ363211 
ACGCTGCACTGCGTTTCAAAGAGAAGAAGGTTCTCGCCAAGGTGAAGAACAGCGCTACCC
CTTAAGAGATCTCCTTGCTTGGGTGGAGGAGCGAAAGTGGGGTGTCCTGGGAGACCA
GGAACCTGCCATCACCAGGCTGGGCCCAAGGACTCTGCTGAGAGGCCCCTAGAGACAACA
CCCTTCCCAGGCCATTGCTTGCTGGACTGTGCCTCAGGAGCGGCCTGGGTACC
BM932052 
GAGTTTTCCAATTTCCAAAGAAAAATTTAGGTTTCCTGCAGCCGTGACATATGTGTGTGC
ACTGGATGGGTTAATGTGTGTGTGTGTGTGTATGCGCATGTATTGGAGTGGGGGCA
GAAACGTGTTTCCAGAATTTGCCTGTAGAATCTAAAAGAGTGGCCAAGAGTCTGGAAATG
CATGAAGACTGGACGTATGTGATGGTGGGCAAAGGCCTGACTGTGTGTGGTGTGTGGGTA
TGTTTGCAGATTCGCGGTGTGAGAGCAGTGATGGGTGAGGGTGGCCTTCAGGAGCCAAG
GCTGATCGGTGGTGAGAGAACAAGCCGGAAGCCAGGGTGCTGTCCTGGTATGCTTTGGAG
GAACAGGATTGCACGTGCGCCTGTAGGGTGACCTGTGTGCACCTGTGAGATGACTTAGCT
TGGGGCTTGCAAGGCCTGGGTCTGCATGGGTGGGTATCTGACCATGCCTTTTCCTCCCTC
CCTTTCACGCCGCGCAGACTCCAGCGGCAGCACCCTCCTGACGCCTGCGCCTTTCGTC
AA357646.1 
CCGGCCTGGGTGGGAGAGCGAGCTGGGTGCCCCTAGATTCCCCGCCCCGCACCTCAT
GAGCCGACCCTCGGCTCCATGGAGCCGGCAATTATGCCACCTTGGATGGAGCCAAGGAT
ATCGAAGGCTTGCTGGGAGCGGGAGGGGGCGGAATCTGGTCGCCCACTCCCCTCTGACC
AW609525 
ACCGCGGGTCAAATTTATTCATAATTAGCTCAATCATGAAAGCGGTTCTAAAGTGCTCTA
CAGAGCTCTAGATAGAAAATATGAGGCTAACGATCATGGCAGCTAGTACTGGTTATCGTG
ATTATGGCCACTGTCAGGATGAATGATAATGACTGGGCCAGGTCCTTTGGAAACCCTGGT
GGAGTGGGCTGTCACATGGGGTCCCGTCTCCCTGCACATACTGGGTACCCAGGCCGCTCC
TGAGGAACAGTCCAGCAGCCAGTGGCCTGGGAAGGGTGTGGTCTCTAGGGGCCTCTCAGC
AGAGTCCTTGGCCCCAGCCTGGGCTTGGCAGGTCCCTGGTCTCCCCAGGACACCCCCACT
TTCGCTCCTCCCACCCAGGCAAGGAGATCTCTTAAGGGGTAGCGCTGTTCTTCACCTTGG
CGAGAACCTTCTTCTCTTTGAACCGGCGGTGCGGCGTGGGTACCGAGC
CB126919 
ATTTTTCTGTCGTGTGAAAATGAAGCCAGCAGGCTGCCCCTAGTCAGTCCTTCCTTCCAG
AGAAAAAGAGATTTGAGAAAGTGCCTGGGTAATTCACCATTAATTTCCTCCCCCAAACTC
TCTGAGTCTTCCCTTAATATTTCTGGTGGTTCTGACCAAAGCAAGTCATGGTTTGTTGAG
CATTTGGGATCCCAGTGAAGTAGATGTTTGTAGCCTTGCATACTTAGCCCTTCCCAGGCA
 
CAAACGGAGTGGCAGAGTGGTGCCAACCCTGTTTTCCCAGTCCACGTAGACAGATTCACA
GTGCGGAATTCTGGAAGCTGGAGACAGACGGGCTCTTTGCAGAGCCGGACTCTGAGAGG
GACATGAAGGCCTCTGCCTCTGTGTTCATTCTCTGATGTCCTGTACCTGGGCTCAGTGCC
CGGTGGGACTCATCTCCTGGCTGCGCAGCAAAGCCAGCGGGTTCGTGCTGGT
AW609336 
CCAACGAGAAGAAGGTTCTCGCAAGGTGAAGAACAGCGCTACCCCTTAAGAGATCTCCTT
GCGTGGGTGGAGGAGCGAAAGTGGGGTGTCCTGGGAGACCAGGAACCTGCCAGCCCA
GGCTGAGGCCAAGGACTCTGCTGAGAGGCCCCTAGAGACAACACCCTTCCCAGGCCACTG
GATGCTGAACTGTCCCTCAGGAGCGGCCTGGGTACCCAGTATGTGCAGGGAGACGGAACC
CCATGTGACAGCCCACTCCACCAGGGTTCCCAAAGAACCTGGCCCCAGTCATAATCATTC
ATCCTGACAGTGGCAATAATCACGATAACCAGTACTAGCTGCCATGATCGTAAGCCTCAT
ATTTGCTATCTAGAGCTCTGTAGAGCACTTTAGAAACCGCTTTCATGAATTGAGCTAATT
ATGACTCAATTTGAACCGGCGTCCGGCGTG
AW609244 
ACGCGCACCGCGGTCAAGAGAAGAAGGTTCTCGCAAGGTGAAGAACAGCGCTACCCCTTA
AGAGATCTCCTTGCGTGGGTGGAGGAGCGAAAGTGGGGGTGTCCTGGGAGACCAGGAA
CCTGCCAAGCCCAGGCTGTGGCCAAGGACTCTGCTGAGAGGCCCCTATGAGACAACACCC
TTCCCAGGCCACTGGCTGCTGGGACTGTTCCTCAGGAGCGGCCTGGGTACCCGAGTAATG
TGCAGGGGAGACGGAACCCCATGTGACAGCCCACTCCACCAGGGTTCCCAAAAGAACCCT
GGCCCAGTCATAATCATTCATCCTGACAGTGGCAATAATCACGATAACCAGTACTAGCTG
CCATGATCGTAAGCCTCATATTTGCTATCTAGAGCTCTGTAGAGCCCTTTAGAAACCGCT
TTCATGAATGGAGCTAAATTATGAATACATTTGAACCGGCGATCCGACGTGA
BF855145 
CTAGAGGATCCCGGAAGCAACTGCAACAGGTTCCCAAAGAACCGGGCCAGTCATAATCAT
TCATCCTGACAGGGCAATAATCACGATAACCAGTACTAGCTGCCATGATCGTTAGCCTCA
TATTTTCTATCTAGAGCTCTGTAGAGCACTTTAGAAACCGCTTTCATGAATGGAGCTAAT
TATGAATAAATTTGGAAGGCGATCCCTTGGCAGGGAAGCTTTCTCTCAGACCCCCTTCCA
TTACACCTCTCACCCTGGTAACAGCAGGAAGACTGAGGAGAGGGGAACGGCAGATTCGT
GGTGTTGCAGTGTGCTTCCG
AU126914 
GAGCGAATGCAGGCGACTTGCGAGCTGGGAGCGATTTAAAACGCTTTGGATTCCCCCGGC
CTGGGTGGGAGAGCGAGCTGGGTGCCCCCTAGATTCCCCGCCCCCGCACCTCATGAGCC
GACCCTCGGCTCCATGGAGCCCGGCAATTATGCCACCTTGGATGGAGCCAAGGATATCGA
AGACTTGCTGGGAGCGGGAGGGGGCGGAATCTGGTCGCCCACTCCCCTCTGACCAGCCA
CCCAGCGGCGCCTACGCTGATGCCTGCTGTCAACTATGCCCCCTTGGATCTGCCAGGCTC
GGCGGAGCCGCCAAAGCAATGCCACCCATGCCCTGGGGTGCCCCAGGGGACGTCCCCAGC
TCCCGTGCCTTATGGTTACTTTGGAGGCGGGTNCTACTCCTGCCGAGTGTCCCGGAGCTC
GCTGAAACCCTGTGCCCANNCANCCACCCTGGCCGCGTN
CB126449 
CTCTGCCTCTGTGTTCATTCTCTGATGTCCTGTACCTGTGCTCAGTGCCCGGTGGGACTC
ATCTCCTGGCTGCGCAGCAAAGCCAGCGGGTTCGTGCTGGTCCTTCCTGCACCTTCGGCT
GGGGGTGGGGGCCTGCCGGCGCATTCTCCACGATT
AW582404 
ACGCTGCACCGCCGGTCCAAGAGAAGAAGGTTCTCGCCAAGGTGAAGAACAGCGCTACCC
CTTTAAGAGATCTCCTTGCTGGGGTGGAGGAGCGAAAGTGGGGGTGTCTGGGAGACCA
GGAACCTGCCAGCCCCAGGCTGGGCCCAAGGACTCTGCTGAGAGGCCCCTAGAGACAACA
CCCTTCCCAGGCCACTGTCTGCTGGACTGTTCCTCAGGAGCGGCCTGGGTACNCAGTATG
TGCAGGGAGACGGAACCCCATGTGACAGCCCACTCCACCAGGGTTCCCAAAGAACCTGGC
CCAGTCATAATCATTCATCCTGACAGTGGCAATAATCACGATAACCAGTACTAGCTGCCA
TGATCGTTAGCCTCATATTTTCTATCTAGAGCTCTGTAGAGCACTTTAGAAACCGCTTTC
ATGAATTGAGCTACTTATGAATCACTTTGAACCGGCGGTGCGGCGTG
BX641644

GGGGGAGAGCGAGCTGGGTGCCCCCTAGATTCCCCGCCCCCGCACCTCATGAGCCGACCC
TCGGCTCCATGGAGCCCGGCAATTATGCCACCTTGGATGGAGCCAAGGATATCGAAGGCT
TGCTGGGAGCGGGAGGGGGGCGGAATCTGGTCGCCCACTCCCCTCTGACCAGCCACCCAG
CGGCGCCTACGCTGACGCCTGCTGTCAACTATGCCCCCTTGGATCTGCCAGGCTCGGCGG
AGCCGCCAAAGCAATGCCACCCATGCCCTGGGGTGCCCCAGGGGACGTCCCCAGCTCCCG
TGCCTTATGGTTACTTTGGAGGCGGGTACTACTCCTGCCGAGTGTCCCGGAGCTCGCTGA
AACCCTGTGCCCAGGCAGCCACCCTGGCCGCGTACCCCGCGGAGACTCCCACGGCCGGGG
AAGAGTACCCCAGCCGCCCCACTGAGTTTGCCTTCTATCCGGGATATCCGGGAACCTACC
AGCCTATGGCCAGTTACCTGGACGTGTCTGTGGTGCAGACTCTGGGTGCTCCTGGAGAAC
CGCGACATGACTCCCTGTTGCCTGTGGACAGTTACCAGTCTTGGGCTCTCGCTNGTGGCT
GGAACAGCCAGATGTGTTGCCAGGGAGAACAGAACCCACCAGGTCCCTTTTGGAAGGCAG
CATTTG

Sequences from Table 4 not disclosed above
AW006861 (IMAGE Clone ID: :2497262) 
GCTGAGTTCTGAAGCTTCTGAGTTCTGCAGCCTCACCTCTGAGAAAACCTCTTTTCCACC
AATACCATGAAGCTCTGCGTGACTGTCCTGTCTCTCCTCATGCTAGTAGCTGCCTTCTGC
TCTCTAGCGCTCTCAGCACCAATGGGCTCAGACCCTCCCACCGCCTGCTGCTTTTCTTAC
ACCGCGAGGAAGCTTCCTCGCAACTTTGTGGTAGATTACTATGAGACCAGCAGCCTCTGC
TCCCAGCCAGCTGTGGTATTCCAAACCAAAAGAAGCAAGCAAGTCTGTGCTGATCCCAGT
GAATCCTGGGTCCAGGAGTACGTGTATGACCTGGAACTGAACTGAGCTGCTCAGAGACAG
GAAGTCTTCAGGGAAGGTCACCTGAGCCCGGATGCTTCTCCATGAGACACATCTCCTCCA
TACTCAGGACTCCTCTCCGCAGTTCCTGTCCCTTCTCTTAATTTAATCTTTTTTATGTGC
CGTGTTATTGTATTAGGTGTCATTTCCATTATTTATATTAGTTTAGCCAAAGGATAAGTG
TCCCCTATGGGGATGGTCCACTGTCACTGTTTCTCTGCTGTTGCAAATACATGGATAACA
CATTTGATTCTGTGTGTTTTCATAATAAAACTTTAAAATAAAATGCAAAAAAAAAAAAAA
AAAA
X59770 
GCCACGTGCTGCTGGGTCTCAGTCCTCCACTTCCCGTGTCCTCTGGAAGTTGTCAGGAGC
AATGTTGCGCTTGTACGTGTTGGTAATGGGAGTTTCTGCCTTCACCCTTCAGCCTGCGGC
ACACACAGGGGCTGCCAGAAGCTGCCGGTTTCGTGGAGGCATTACAAGCGGGAGTTCAG
GCTGGAAGGGGAGCCTGTAGCCCTGAGGTGCCCCCAGGTGCCCTACTGGTTGTGGCCTC
TGTCAGCCCCCGCATCAACCTGACATGGCATAAAAATGACTCTGCTAGGACGGTCCCAGG
 
AGAAGAAGAGACACGGATGTGGGCCCAGGACGGTGCTCTGTGGCTTCTGCCAGCCTTGCA
GGAGGACTCTGGCACCTACGTCTGCACTACTAGAAATGCTTCTTACTGTGACAAAATGTC
CATTGAGCTCAGAGTTTTTGAGAATACAGATGCTTTCCTGCCGTTCATCTCATACCCGCA
AATTTTAACCTTGTCAACCTCTGGGGTATTAGTATGCCCTGACCTGAGTGAATTCACCCG
TGACAAAACTGACGTGAAGATTCAATGGTACAAGGATTCTCTTCTTTTGGATAAAGACAA
TGAGAAATTTCTAAGTGTGAGGGGGACCACTCACTTACTCGTACACGATGTGGCCCTGGA
AGATGCTGGCTATTACCGCTGTGTCCTGACATTTGCCCATGAAGGCCAGCAATACAACAT
CACTAGGAGTATTGAGCTACGCATCAAGAAAAAAAAAGAAGAGACCATTCCTGTGATCAT
TTCCCCCCTCAAGACCATATCAGCTTCTCTGGGGTCAAGACTGACAATCCCGTGTAAGGT
GTTTCTGGGAACCGGCACACCCTTAACCACCATGCTGTGGTGGACGGCCAATGACACCCA
CATAGAGAGCGCCTACCCGGGAGGCCGCGTGACCGAGGGGCCACGCCAGGAATATTCAGA
AAATAATGAGAACTACATTGAAGTGCCATTGATTTTTGATCCTGTCACAAGAGAGGATTT
GCACATGGATTTTAAATGTGTTGTCCATAATACCCTGAGTTTTCAGACACTACGCACCAC
AGTCAAGGAAGCCTCCTCCACGTTCTCCTGGGGCATTGTGCTGGCCCCACTTTCACTGGC
CTTCTTGGTTTTGGGGGGAATATGGATGCACAGACGGTGCAAACACAGAACTGGAAAAGC
AGATGGTCTGACTGTGCTATGGCCTCATCATCAAGACTTTCAATCCTATCCCAAGTGAAA
TAAATGGAATGAAATAATTCAAACACAAAAAAAAAAAAAAAAAAAA
AB000520 
GGATCCAAGCTATTGTCCTGCCCATGGCTTCCCATCTCAGGACGCTCTCTGGCCGCTATC
ATCCCAGCAGTGGAGTTCAGCCCACTACTCTGAACCAGCCGCAGGTGGCTGCTATGGGAC
TGAAGCCATGAATGGTGCCGGCCCTGGCCCCGCCGCAGCCGCCCCGGTCCCAGTCCCGGT
CCCGGTCCCGGACTGGCGGCAGTTCTGCGAGCTGCATGCGCAGGCGGCCGCCGTGGACTT
TGCGCACAAGTTCTGCCGTTTCCTGCGGGACAACCCAGCTTACGACACGCCCGACGCCGG
CGCCTCCTTCTCCCGCCACTTCGCCGCCAACTTCCTGGACGTCTTCGGCGAGGAGGTGCG
CCGCGTGCTGGTGGCTGGGCCGACGACTCGGGGCGCGGCCGTGAGCGCAGAGGCCATGGA
GCCGGAGCTCGCGGACACCTCTGCACTCAAGGCGGCGTCCTACGGCCACTCGCGGAGCTC
GGAGGACGTGTCCACGCACGCGGCCACCAAGGCCCGCGTTCGCAAGGGCTTCTCGCTGCG
CAACATGAGCCTGTGCGTGGTGGACGGCGTGCGCGACATGTGGCACCGGCGCGCCTCGCC
CGAGCCCGACGCGGCAGCTGCCCCGCGCACCGCCGAGCCCCGCGACAAGTGGACGCGGCG
CCTGAGGCTGTCGCGGACGCTGGCTGCCAAGGTGGAGCTGGTGGACATTCAACGCGAGGG
GGCGCTGCGCTTCATGGTGGCCGACGACGCGGCCGCGGGCTCCGGGGGCTCGGCTCAGTG
GCAGAAGTGCCGCCTGCTCCTGCGCAGGGCTGTGGCCGAGGAACGCTTCCGCCTGGAGTT
CTTCGTGCCGCCCAAAGCCTCCAGGCCCAAGGTCAGCATCCCACTGTCAGCCATCATTGA
GGTCCGCACCACCATGCCCCTGGAAATGCCAGAGAAGGATAACACATTCGTCCTCAAGGT
AGAGAATGGAGCCGAATACATCTTGGAGACCATCGACTCTCTGCAGAAGCACTCGTGGGT
AGCTGACATCCAGGGCTGCGTGGACCCCGGTGACAGTGAGGAAGACACCGAGCTCTCCTG
TACCCGAGGAGGCTGTCTGGCCAGCCGCGTGGCCTCCTGCAGCTGTGAGCTCCTGACTGA
TGCAGTCGACCTGCCCCGCCCCCCAGAGACGACAGCCGTGGGTGCAGTGGTGACAGCCCC
CCACAGCCGAGGTCGAGATGCCGTCAGAGAATCCCTGATCCACGTCCCGCTAGAGACCTT
TCTGCAGACCCTGGAATCCCCGGGCGGCAGCGGCAGTGACAGCAATAACACAGGGGAACA
GGGTGCAGAGACGGATCCCGAGGCTGAACCCGAGCTGGAGCTATCCGACTACCCATGGTT
CCACGGGACACTGTCCCGGGTCAAGGCTGCTCAACTGGTTCTGGCAGGGGGCCCCCGGAA
CCACGGCCTCTTCGTGATCCGCCAAAGTGAGACTCGGCCTGGGGAGTACGTGCTGACCTT
CAACTTCCAGGGCAAGGCCAAGCACCTGCGCCTGTCCCTGAACGGCCACGGCCAGTGTCA
CGTACAGCATCTGTGGTTCCAGTCTGTGCTTGACATGCTCCGCCACTTCCACACACACCC
CATCCCACTGGAGTCAGGGGGCTCGGCCGACATCACCCTTCGCAGCTATGTGCGGGCCCA
GGACCCCCCACCAGAGCCGGGCCCACGCCCCCTGCCGCGCCCGCGTCCCCGGCCTGCTG
GAGCGACTCGCCCGGCCAGCACTACTTCTCCAGCCTCGCCGCGGCCGCCTGCCCGCCTGC
CTCGCCCTCCGACGCCGCCGGCGCCTCCTCGTCTTCCGCCTCGTCGTCCTCTGCCGCGTC
GGGGCCCGCCCCCCGCGCCCCGTCGAGGGCCAGCTCAGCGCGCGGAGCCGCAGCAACAG
CGCCGAGCGCCTGCTGGAGGCCGTGGCCGCCACCGCCGCCGAGGAGCCCCCGGAGGCCGC
 
GCCCGGCCGCGCGCGCCGTGGAGAACCAGTACTCCTTCTACTAGCCCGCGGCGCCGCC
CGGGTGGGACACGCCAAGCTCTTCAGTGAAGACACGATGTTATTAAAAGCCTGTTTTAGG
GACTGCAAAA
AI820604 (IMAGE Clone Id: 1605108 
GATTCCAGCACGGGCTTCGCAGACTGCAGGACACAGAGGCACGCGTGCACATCATGTCTT
CTAAGGAATTTGAACACTGTTGAGAAGACTGTGTACAAGAGAGATGTGCCATGTCAGCCT
TGCAAGGGACAGCGTGAAAACTACCCATCTCCGGTCACCAAGTTGCAGGAGGCCAGGAGC
CAGGAGGGGAAACCGCTCAGTTTGCAAAACGTCGCTTCCACAAGCCTGATGGCTGAAACT
GCTCACTGTACCCTGAAACCAGCTTTACCTACAGCTTCTGAGATAAACTGCTGCAACTCT
GGGACCCACGATGCCTATCACAGTGGCTCATCAATGGAACCTGCCGGCTCCCAACCCTTC
CTAGGGCCCATGAACTCTCTGAAAAGAGGAACAGAAATATTTCTCCTTTTTGTAAAATCT
TTAACCTTCCCTTTGTTCTTCATGTACACGCTGAACTGCAATTCTTCTTCCCAAATAAAA
CATTAAATTTAAAAAA
AI087057 (IMAGE Clone ID:1671188) 
GGCCCCGGAGGGAGAGTAACCCGGCCCATCCATCCGTCGCCCGGTTCTTGGGAACTACT
TTCAGGGGCTTCTTGCCGTCCCCTCATCAGCTCTGTGCGAACCCTCTGTCGGCAGCCATT
GAGGAGACCCTGCCCCCTGGACCCTGACCACATATAGATTGAGGCCGAGGAGTGGCTGCC
CTGTCCCTTTTATGACAGCCCGCAGAAGCCCCGGGGTGAGGCATGGAGGAGGCAGGCGAC
AGCTGACAGGGACCCTGTTGGCCTCCAGCATGTCCAGCCAGCCGGGCAGGATTTCTCTGC
TTCTGGCTGGCAGCCAGGAACTGAGTATGACAATGTTGTACTAAAGAAAGGCCCAAAGTG
ACAGAGGCAGCAGAGGGATGGTCCACCGCCCCTTGGCTTCTGCTGGTGACTCCTCCTGGC
CACTGCATCAGAAGAACCTCCTCTGCCCCTTCTGGAGCCCGAGGCCTGGCCTGTCTTCGT
TGGGGCTGATAAATTGCCTCTCCCAGGGCCTGCTGGGTGAGTCACCATCCCAAAGCAGGA
AGGGTGCCCTGGAGAGAACCACCCTCCTCCTACTCTTTTTCCACTTCCTCCTCTTTCTTT
CCCCAGCTGAGGAGGAACCTGGGGCATTTAGGGCAGAGGACAAAAGGATGTCAGCAATTG
CTTGGGCTGCTTGGCTATGCAAGCCTCCTGCCTGCTGATGGCCACTTCAGGGACAGCCTG
GGCCCAGGCACCCAGGGGGATGGCGGCAGCTTCCTGCACCTTTCAGATTTCTTGGTGGCA
TTAAAGCATTTTCAGAACAAAAAAAAAAAAAAAAAAAAAAAAA
AJ272267 
GGCGGGCCTGGACGGCCGCGTGCTGTACTGGCCACGCGGCCGCGTCTGGGTGGCTCCTC
ATCCCTCAATGCCATGGTCTACGTCCGTGGGCACGCCGAGGACTACGAGCGCTGGCAGCG
CCAGGGCGCCCGCGGCTGGGACTACGCGCACTGCCTGCCCTACTTCCGCAAGGCGCAGGG
CCACGAGCTGGGCGCCAGCCGGTACCGGGGCGCCGATGGCCCGCTGCGGGTGTCCCGGGG
CAAGACCAACCACCCGCTGCACTGCGCATTCCTGGAGGCCACGCAGCAGGCCGGCTACCC
GCTCACCGAGGACATGAATGGCTTCCAGCAGGAGGGCTTCGGCTGGATGGACATGACCAT
CCATGAAGGCAAACGGTGGAGCGCGGCCTGTGCCTACCTGCACCCAGCACTGAGCCGCAC
CAACCTCAAGGCCGAGGCCGAGACGCTTGTGAGCAGGGTGCTATTTGAGGGCACCCGTGC
AGTGGGCGTGGAGTATGTTAAGAATGGCCAGAGCCACAGGGCTTATGCCAGCAAGGAGGT
GATTCTGAGTGGAGGTGCCATCAACTCTCCACAGCTGCTCATGCTCTCTGGCATCGGGAA
TGCTGATGACCTCAAGAAACTGGGCATCCCTGTGGTGTGCCACCTACCTGGGGTTGGCCA
GAACCTGCAAGACCACCTGGAGATCTACATTCAGCAGGCATGCACCCGCCCTATCACCCT
CCATTCAGCACAGAAGCCCCTGCGGAAGGTCTGCATTGGTCTGGAGTGGCTCTGGAAATT
CACAGGGGAGGGAGCCACTGCCCATCTGGAAACAGGTGGGTTCATCCGCAGCCAGCCTGG
GGTCCCCCACCCGGACATCCAGTTCCATTTCCTGCCATCCCAAGTGATTGACCACGGGCG
GGTCCCCACCCAGCAGGAGGCTTACCAGGTACATGTGGGGCCCATGCGGGCACGAGTGT
GGGCTGGCTCAAACTGAGAAGTGCCAATCCCCAAGACCACCCTGTGATCCAGCCCAACTA
CTTGTCAACAGAAACTGATATTGAGGATTTCCGTCTGTGTGTGAAGCTCACCAGAGAAAT
 
TTTTGCACAGGAAGCCCTGGCTCCGTTCCGAGGGAAAGAGCTCCAGCCAGGAAGCCACAT
TCAGTCAGATAAAGAGATAGATGCCTTTGTGCGGCAAAAGCCGACAGCGCCTACCACCC
CTCGTGCACCTGTAAGATGGGCCAGCCCTCCGATCCCACTGCCGTGGTGGATCCGCAGAC
AAGGGTCCTCGGGGTGGAAAACCTCAGGGTCGTCGATGCCTCCATCATGCCTAGCATGGT
CAGCGGCAACCTGAACGCCCCCACAATCATGATCGCAGAGAAGGCAGCTGACATTATCAA
GGGGCAGCCTGCACTCTGGGACAAAGATGTCCCTGTCTACAAGCCCAGGACGCTGGCCAC
CCAGCGCTAAGACAGTTGCTGCTGGAGGATGACCAGGGAAGCCCCCTGATAAGCCAAGAG
GGCCAGCACAGCCCTTGCTCCCAGGCTCCTGCCTGAAACTATCTAGCACACTAGGACCCA
GGTGGTACCCTACTCAGTGGCTGAGAATTGGATAAAGTCTTKGGGAAATGAGACAAGTAC
TGGGCAGTGAATCCAGCTCCTTTTCCCCAGCCTTTCCCTGTGGCCATTTGGGAAGGCC
AGCATTYCAGCCTGAGATGTTCCTCCCTGCCTCCTGGGGGGGCARAAGGGVTAGGWTGGT
TAACTCCTGCCGCATCCTTCCCTGCCTCCTGGAGGGACAGAAGGGGAGGATGGTTAACTC
CTGCCGCATCCTTTTTCTTGTGTTCACGTGGCATTCTCTAACCCAGGGCAGTGGTTCCTT
CCCAGGCCATGCACAGAGGCTGGGTGCCTGCCAGACCCACGGAGGGTTCGCGAAGGAAGG
GGCATCCTCCTTCTTGAGCTGCAAGCTTTAGCTGAGGCAGTAAGTCACACAGTAGTTAGT
TCAGCCTGGGCTGGCACATAAGTCCCCAGTGTCCCTGTTGAGAGGGGAAAGTTGCCTGCT
GGTTGAAAAACTGGCTTTTCCTTTCTCGCTGCCTAATTTCACTCTCAGAGTGAGGCAGGT
AACTGGGGCTCCACTGGGTCACTCTGAGAGGGTTGTGGCTCTGGTTCTTATTAAACCAGG
GCCAGGTGCAGGGCTCACACCTGTAATCCCAGCACTTTGGGAAGGTCACTTGAGCTCAGG
AGTTCAAGACCAGCCTGGGCAACATAGTGAGACCTTGTCTCTGGAAAACAATTAGCTGGG
CATGGTGGTACACACCTGTAGTCCCAGCTACTTGGGAGGCTGAGGCGGGAGGATGGCTTT
AGCCCAGGAGGTTGAGGCTCCTGTGAACCCTGATGGCACCACTGCACTCCAGCCTGGGTG
ACAGGGTGAGACCCTGTCTCAAAAAAAAA
N30081 (IMAGE Clone ID: 258695) 
CCGCCGTTGNCAAAGGGCCCAGAATATGGGCCATGGACNATCTCCATGCCTGGGGAAATT
CCCTCGGGTCTTTTGGNTAACCNCCTTATAGAAAGGTAATGNCATGGAGTCTCTACAGGG
NGCACAAGGTGGACTAATTGATACGAAGAGCCCTGTAAATATGTGGGCAGCGGCAGATTT
TGACCATTTGGACCGAACTGTATTTGACACAGCGCAATATCTGGAACTGGTTGGTCAAAA
ACCTGCTTGTCTTGTTAAATTTCCTCTGTCCAAGGACATGGAATCTCTCTCTAATTTTAC
TTCAAATTTCCCTTTCCTTCATTTCTCTAAAAACGTTAAATAAGAAAGAAGATTGTAAAG
CCAGCATTTGAAGCCTAAGTATTGAAAGTCTTTGACAATTTCTGAAATCAGACTTGACAT
CTTTCCCCCGCCTTGCAAATTTCTTGAAGAAATAAGAAGCTACATGTAAGCATCATCATG
TTTATTAAATTACAATGAGAACTCTCACTCAATCTTGACCAGAGCAGACTCTTAACTTGG
AAGCAGAGTCCCTCTAAAGGTAACTCTTGTGGTCACTCAATATTGTATTGGCATTTGCAT
ATTAAATAGACATTTCAGTAGCATTT
AI700363 (IMAGE Clone ID: 2327403) 
TGGCCCGCGGTCGCGGTGGATCCTAGCCCTGTCTCCTCTCCTGGGAAGGAGTGAGGGTG
GGACGTGACTTAGACACCTACAAATCTATTTACCAAAGAGGAGCCCGGGACTGAGGAAA
AGGCCAAAGAGTGTGAGTGCATGCGGACTGGGGGTTCAGGGGAAGAGGACGAGGAGGAGG
AAGATGAGGTCGATTTCCTGATTTAAAAAATCGTCCAAGCCCCGTGGTCCAGCTTAAGGT
CCTCGGTTACATGCGCCGCTCAGAGCAGGTCACTTTCTGCCTTCCACGTCCTCCTTCAAG
GAAGCCCCATGTGGGTAGCTTTCAATATCGCAGGTTCTTACTCCTCTGCCTCTATAAGCT
CAAACCCACCAACGATCGGGCAAGTAAACCCCCTCCCTCGCCGACTTCGGAACTGGCGAG
AGTTCAGCGCAGATGGGCCTGTGGGGAGGGGGCAAGATAGATGAGGGGGAGCGGCATGGT
GCGGGGTGACCCCTTGGAGAGAGGAAAAAGGCCACAAGAGGGGCTGCCACCGCCACTAAC
GGAGATGGCCCTGGTAGAGACCTTTGGGGGTCTGGAACCTCTGGACTCCCCATGCTCTAA
CTCCCACACTCTGCTATCAGAAACTTAAACTTGAGGATTTTCTCTGTTTTTCACTCGCAA
TAAATTCAGAGCAAACAAAAAAAAAAAAAAAA
AL117406 
CAATAGGCCGGCTTTTGAACTGCTTCGCAGGGGACTTGGAACAGCTGGACCAGCTCTTGC
CCATCTTTTCAGAGCAGTTCCTGGTCCTGTCCTTAATGGTGATCGCCGTCCTGTTGATTG
TCAGTGTGCTGTCTCCATATATCCTGTTAATGGAGCCATAATCATGGTTATTTGCTTCA
TTTATTATATGATGTTCAAGAAGGCCATCGGTGTGTTCAAGAGACTGGAGAACTATAGCC
GGTCTCCTTTATTCTCCCACATCCTCAATTCTCTGCAAGGCCTGAGCTCCATCCATGTCT
ATGGAAAAACTGAAGACTTCATCAGCCAGTTTAAGAGGCTGACTGATGCGCAGAATAACT
ACCTGCTGTTGTTTCTATCTTCCACACGATGGATGGCATTGAGGCTGGAGATCATGACCA
ACCTTGTGACCTTGGCTGTTGCCCTGTTCGTGGCTTTTGGCATTTCCTCCACCCCCTACT
CCTTTAAAGTCATGGCTGTCAACATCGTGCTGCAGCTGGCGTCCAGCTTCCAGGCCACTG
CCCGGATTGGCTTGGAGACAGAGGCACAGTTCACGGCTGTAGAGAGGATACTGCAGTACA
TGAAGATGTGTGTCTCGGAAGCTCCTTTACACATGGAAGGCACAAGTTGTCCCCAGGGGT
GGCCACAGCATGGGGAAATCATATTTCAGGATTATCACATGAAATACAGAGACAACACAC
CCACCGTGCTTCACGGCATCAACCTGACCATCCGCGGCCACGAAGTGGTGGGCATCGTGG
GAAGGACGGGCTCTGTAGGTTTTTACTGAGCACCTACTATGTGCCTGGAACCGAAAGGG
AAGTCCTCCTTGGGCATGGCTCTCTTCCGCCTGGTGGAGCCCATGGCAGGCCGGATTCTC
ATTGACGGCGTGGACATTTGCAGCATCGGCCTGGAGGACTTGCGGTCCAAGCTCTCAGTG
ATCCCTCAAGATCCAGTGCTGCTCTCAGGAACCATCAGATTCAACCTAGATCCCTTTGAC
CGTCACACTGACCAGCAGATCTGGGATGCCTTGGAGAGGACATTCCTGACCAAGGCCATC
TCAAAGTTCCCCAAAAAGCTGCATACAGATGTGGTGGAAAACGGTGGAAACTTCTCTGTG
GGGGAGAGGCAGCTGCTCTGCATTGCCAGGGCTGTGCTTCGCAACTCCAAGATCATCCTT
ATCGATGAAGCCACAGCCTCCATTGACATGGAGACAGACACCCTGATCCAGCGCACAATC
CGTGAAGCCTTCCAGGGCTGCACCGTGCTCGTCATTGCCCACCGTGTCACCACTGTGCTG
AACTGTGACCACATCCTGGTTATGGGCAATGGAAGGTGGTAGAATTTGATCGGCCGGAG
GTACTGCGGAAGAAGCCTGGGTCATTGTTCGCAGCCCTCATGGCCACAGCCACTTCTTCA
CTGAGATAAGGAGATGTGGAGACTTCATGGAGGCTGGCAGCTGAGCTCAGAGGTTCACAC
AGGTGCAGCTTCGAGGCCCACAGTCTGCGACCTTCTTGTTTGGAGATGAGAACTTCTCCT
GGAAGCAGGGGTAAATGTAGGGGGGGTGGGATTGCTGGATGGAAACCCTGGAATAGGCT
ACTTGATGGCTCTCAAGACCTTAGAACCCCAGAACCATCTAAGACATGGGATTCAGTGAT
CATGTGGTTCTCCTTTTAACTTACATGCTGAATAATTTTATAATAAGGTAAAAGCTTATA
GTTTTCTGATCTGTGTTAGAAGTGTTGCAAATGCTGTACTGACTTTGTAAAATATAAAAC
TAAGGAAAACTCAAAAAAAAAAAA
M92432 
CCCACAGGGGACCGGCCCTGTGACCCCTCACCGGGGCCGTGGCCCGAGCCCGGACTT
CCCTAAGCCGGCAATGACCGCCTGCGCCCGCCGAGCGGTGGCTTCCGGACCCGGGCT
CTGCGGTCCCGCGTGGTGGGCTCCGTCCCTGCCCCGCCTCCCCCGGCCCTGCCCCGGCT
CCCGCTCCTGCTGCTCCTGCTTCTGCTGCAGCCCCCCGCCCTCTCCGCCGTGTTCACGGT
GGGGGTCCTGGGCCCCTGGGCTTGCGACCCCATCTTCTCTCGGGCTCGCCCGGACCTGGC
CGCCCGCCTGGCCGCCGCCCGCCTGAACCGCGACCCCGGCCTGGCAGGCGGTCCCCGCTT
CGAGGTAGCGCTGCTGCCCGAGCCTTGCCGGACGCCGGGCTCGCTGGGGGCCGTGTCCTC
CGCGCTGGCCCGCGTGTCGGGCCTCGTGGGTCCGGTGAACCCTGCGGCCTGCCGGCCAGC
CGAGCTGCTCGCCGAAGAAGCCGGGATCGCGCTGGTGCCCTGGGGCTGCCCCTGGACGCA
GGCGGAGGGCACCACGGCCCCTGCCGTGACCCCCGCCGCGGATGCCCTCTACGCCCTGCT
TCGCGCATTCGGCTGGGCGCGCGTGGCCCTGGTCACCGCCCCCCAGGACCTGTGGGTGGA
GGCGGGACGCTCACTGTCCACGGCACTCAGGGCCCGGGGGCTGCCTGTCGCCTCCGTGAC
TTCCATGGAGCCCTTGGACCTGTCTGGAGCCCGGGAGGCCCTGAGGAAGGTTCGGGACGG
GCCCAGGGTCACAGCAGTGATCATGGTGATGCACTCGGTGCTGCTGGGTGGCGAGGAGCA
GCGCTACCTCCTGGAGGCCGCAGAGGAGCTGGGCCTGACCGATGGCTCCCTGGTCTTCCT
GCCCTTCGACACGATCCACTACGCCTTGTCCCCAGGCCCGGAGGCCTTGGCCGCACTCGC
CAACAGCTCCCAGCTTCGCAGGGCCCACGATGCCGTGCTCACCCTCACGCGCCACTGTCC
 
CTCTGAAGGCAGCGTGCTGGACAGCCTGCGCAGGGCTCAAGAGCGCCGCGAGCTGCCCTC
TGACCTCAATCTGCAGCAGGTCTCCCCACTCTTTGGCACCATCTATGACGGTCTTCTT
GCTGGCAAGGGGCGTGGCAGAAGCGCGGGCTGCCGCAGGTGGCAGATGGGTGTCCGGAGC
AGCTGTGGCCCGCCACATCCGGGATGCGCAGGTCCCTGGCTTCTGCGGGACCTAGGAGG
AGACGAGGAGCCCCCATTCGTGCTGCTAGACACGGACGCGGCGGGAGACCGGCTTTTTGC
CACATACATGCTGGATCCTGCCCGGGGCTCCTTCCTCTCCGCCGGTACCCGGATGCACTT
CCCGCGTGGGGATCAGCACCCGGACCTGACCCCTCGTGCTGGTTCGATCCAAACAACAT
CTGCGGTGGAGGACTGGAGCCGGGCCTCGTCTTTCTTGGCTTCCTCCTGGTGGTTGGGAT
GGGGCTGGCTGGGGCCTTCCTGGCCCATTATGTGAGGCACCGGCTACTTCACATGCAAAT
GGTCTCCGGCCCCAACAAGATCATCCTGACCGTGGACGACATCACCTTTCTCCACCCACA
TGGGGGCACCTCTCGAAAGGTGGCCCAGGGGAGTCGATCAAGTCTGGGTGCCCGCAGCAT
GTCAGACATTCGCAGCGGCCCCAGCCAACACTTGGACAGCCCCAACATTGGTGTCTATGA
GGGAGACAGGGTTTGGCTGAAGAAATTCCCAGGGGATCAGCACATAGCTATCCGCCCAGC
AACCAAGACGGCCTTCTCCAAGCTCCAGGAGCTCCGGCATGAGAACGTGGCCCTCTACCT
GGGGCTTTTCCTGGCTCGGGGAGCAGAAGGCCCTGCGGCCCTCTGGGAGGGCAACCTGGC
TGTGGTCTCAGAGCACTGCACGCGGGGCTCTCTTCAGGACCTCCTCGCTCAGAGAGAAAT
AAAGCTGGACTGGATGTTCAAGTCCTCCCTCCTGCTGGACCTTATCAAGGGAATAAGGTA
TCTGCACCATCGAGGCGTGGCTCATGGGCGGCTGAAGTCACGGAACTGCATAGTGGATGG
CAGATTCGTACTCAAGATCACTGACCACGGCCACGGAGACTGCTGGAAGCACAGAAGGT
GCTACCGGAGCCTCCCAGAGCGGAGGACCAGCTGTGGACAGCCCCGGAGCTGCTTAGGGA
CCCAGCCCTGGAGCGCCGGGGAACGCTGGCCGGCGACGTCTTTAGCTTGGCCATCATCAT
GCAAGAAGTAGTGTGCCGCAGTGCCCCTTATGCCATGCTGGAGCTCACTCCCGAGGAAGT
GGTGCAGAGGGTGCGGAGCCCCCCTCCACTGTGTCGGCCCTTGGTGTCCATGGACCAGGC
ACCTGTCGAGTGTATCCTCCTGATGAAGCAGTGCTGGGCAGAGCAGCCGGAACTTCGGCC
CTCCATGGACCACACCTTCGACCTGTTCAAGAACATCAACAAGGGCCGGAAGACGAACAT
CATTGACTCGATGCTTCGGATGCTGGAGCAGTACTCTAGTAACCTGGAGGATCTGATCCG
GGAGCGCACGGAGGAGCTGGAGCTGGAAAAGCAGAAGACAGACCGGCTGCTTACACAGAT
GCTGCCTCCGTCTGTGGCTGAGGCCTTGAAGACGGGGACACCAGTGGAGCCCGAGTACTT
TGAGCAAGTGACACTGTACTTTAGTGACATTGTGGGCTTCACCACCATCTCTGCCATGAG
TGAGCCCATTGAGGTTGTGGACCTGCTCAACGATCTCTACACACTCTTTGATGCCATCAT
TGGTTCCCACGATGTCTACAAGGTGGAGACAATAGGGGACGCCTATATGGTGGCCTCGGG
GCTGCCCCAGCGGAATGGGCAGCGACACGCGGCAGAGATCGCCAACATGTCACTGGACAT
CCTCAGTGCCGTGGGCACTTTCCGCATGCGCCATATGCCTGAGGTTCCCGTGCGCATCCG
CATAGGCCTGCACTCGGGTCCATGCGTGGCAGGCGTGGTGGGCCTCACCATGCCGCGGTA
CTGCCTGTTTGGGGACACGGTCAACACCGCCTCGCGCATGGAGTCCACCGGGCTGCCTTA
CCGCATCCACGTGAACTTGAGCACTGTGGGGATTCTCCGTGCTCTGGACTCGGGCTACCA
GGTGGAGCTGCGAGGCCGCACGGAGCTGAAGGGCAAGGGCGCCGAGGACACTTTCTGGCT
AGTGGGCAGACGCGGCTTCAACAAGCCCATCCCCAAACCGCCTGACCTGCAACCGGGGTC
CAGCAACCACGGCATCAGCCTGCAGGAGATCCCACCCGAGCGGCGACGGAAGCTGGAGAA
GGCGCGGCCGGGCCAGTTCTCTTGAGAAGTGAGGCCCGGCCCCGGACAGGGTCTGGCCC
TGCTCCCTGTCCCATCTGCAGTGGACCCCAGGCACCCCCCTTTGAGGAGGTGGGGTGAAC
TGCTCCTTGGCAGGGATTTGTGACACTGCATTGCTGGGCTGTGTTCCTCGGGCTCTTCTG
GACCTTGCACCGTGGATACCAGGCCATGTGCCATGGTATTTGGGTCCTGGAGGGTGGGT
GAAATAAAGGCATACTGTCTT
AL050227 
CTTTCACAGAAAGAAAGTAACAGGCATAATTCCTGTTGATGAGGCTGGATTGTTTTTAA
GAGGAGAGATAATAACTTCATATTTTAAAGTGCCAGTAGCCTAATATGTGAAACAGATC
AGAATCTGTTGTGTAGTAAGTCTGCTTTGTTGAAGAATTTATTATGGAGTAAAGATAAG
AAGGAAAGAGATCACCATCAGAAACAAGTCAGCCTTTTCATGCTTTTTTGAGCATTTTTG
GAGATGATTCCACTTCTCAAGTTATTATCATTTGTGCATCTCTTCAATGCTATTGTTAAA
TGCTTTAGAATTAGAATATTTTGATCCTTTAATTAAAGTAAGCCAAACGTCTAGGCAAAA
 
ACAGCCAATCATTAAACTTTAATAGTAATTCAAATATAGATTTCTCATACAGTTTTCCAT
GTCTGTAGAAATCAAAGTTGTAATGTTAAGCAGAGGAAATGCGTGTGATTTACTAATAC
ACTTCAACGTTCTACTTTTGAAAGGATACTCATGTGGGTGGGCAGAGAACATAGAAAAA
GATATGATGGAAAACCTGTCCATTTTCTACCTGTTAACCTTCATCATTTTGTGCAGGCCC
TGGAAGCAAAGAGAGGAAGGGACCGACTGCATTTATCTTTGAACACTTGAGCATCAGTAG
TACTACTGAGTGGCCAGGGGTCTTGTCTGTCAAAGCAAATGATAAGTTCACTCAGGCCAT
TATTGACTGCTGAACTCTCTTCCTTCCCAACTCTTCCTTGAAAGAGAAAAAAATACTTTG
CCTTCTTGCTCTCCTTATCAAATGTTTTTGTACAAATAGTGTAAGCCTGTTTAAGCAAAC
CAATTAAAATAGGCACTGATTATTTTGATCTGTTTGTAACAAATGAATGTAAGTACTATT
TACATGGTGTGCCTAGGAGGAGCTGAAATCATTGGCACTTTAATCCATATTGTAAAGATC
AGTATCAAAAGCATAGTGTTCTTCACCTCTCCTCCTCAGCATCCATCTCTATATACTTGA
TTAAATGGAAAAGTCTCTTTTATCACCTCTATGTAAAGTTTTATGGGTAGTTATCGTCAG
TGTATTTAAATATATCTTCTAGTATGTTTTAAAGGCTGGTCTTCAATACTGTGGAGACAA
AAAATAAAAGAGCGTATGAAAAGTACGTTAGACTTTTGCTGGCATTCAAGTCATGGCTAG
TCTGTGTATTTAATAAATGTGTGTTATTTATGTCGTGTTTGTCAATGGAAAATAAAGTTG
AATATTCTGAAAAAAAAAAAAAAA
AW613732 (IMAGE Clone ID: 2953502) 
CCTANAAGTNCCATTTTGGCAAGGATAAACTCCCATGACAANCTCCCANTACTGCATGTG
AATGAATAAGAAACAAGAANTGACCACACCAAAGCCTCCCTGGCTGGTGTTACANGGGAT
CAGGTCCACAGTGGTGCAGATTCAACCACCACCCAGGGAGTGCTTGCAGACTCTGCATAG
ATGTTGCTGCATGCGTCCCATGTGCCTGTCAGAATGGCAGTGTTTAATTCTCTTGAAAGA
AAGTTATTTGCTCACTATCCCCAGCCTCAAGGAGCCAAGGAAGAGTCATTCACATGGAAG
GTCCGGGACTGGTCAGCCACTCTGACTTTTCTACCACATTAAATTCTCCATTACATCTCA
CTATTGGTAATGGCTTAAGTGTAAAGAGCCATGATGTGTATATTAAGCTATGTGCCACAT
ATTTATTTTTAGACTCTCCACAGCATTCATGTCAATATGGGATTAATGCCTAAACTTTGT
AAATATTGTACAGTTTGTAAATCAATGAATAAAGGTTTTGAGTGTAAAAAAAAAAAAAAA
AAAAAAA
BC007783 (IMAGE Clone ID: 4308472) 
GGCACGAGGGCAAAGAGTAGTCAGTCCCTTCTTGGCTCTGCTGACACTCGAGCCCACATT
CCATCACCTGCTCCCAATCATGCAGGTCTCCACTGCTGCCCTTGCCGTCCTCCTCTGCAC
CATGGCTCTCTGCAACCAGGTCCTCTCTGCACCACTTGCTGCTGACACGCCGACCGCCTG
CTGCTTCAGCTACACCTCCCGGCAGATTCCACAGAATTTCATAGCTGACTACTTTGAGAC
GAGCAGCCAGTGCTCCAAGCCCAGTGTCATCTTCCTAACCAAGAGAGGCCGGCAGGTCTG
TGCTGACCCCAGTGAGGAGTGGGTCCAGAAATACGTCAGTGACCTGGAGCCGAGTGCCTG
AGGGGTCCAGAAGCTTCGAGGCCCAGCGACCTCAGTGGGCCCAGTGGGGAGGAGCAGGAG
CCTGAGCCTTGGGAACATGCGTGTGACCTCCACAGCTACCTCTTCTATGGACTGGTTATT
GCCAAACAGCCACACTGTGGGACTCTTCTTAACTTAAATTTTAATTTATTTATACTATTT
AGTTTTTATAATTTATTTTTGATTTCACAGTGTGTTTGTGATTGTTTGCTCTGAGAGTTC
CCCCTGTCCCCTCCACCTTCCCTCACAGTGTGTCTGGTGACAACCGAGTGGCTGTCATCG
GCCTGTGTAGGCAGTCATGGCACCAAAGCCACCAGACTGACAAATGTGTATCAGATGCTT
TTGTTCAGGGCTGTGATCGGCCTGGGGAAATAATAAAGATGTTCTTTTAAACGGTAAAAA
AAAA
X81896 
AGAAAACTATTTCTAAATATTAACACTGAAAATGTTTTGTTAGCTTTTCCTTCTTTCTC
TCCAGAAGAAACATGGATAGATGATAGCTGTTTCATTGTTTGTTTTTGTCAAGCATATTC
ACTTTCCTCCTTGTCCTCTGATTCTGAGCAAAGGGCCTCAGACTCTGAACTTCCCTCAAG
TGCCGTTGTTATGTGAACTCTTCCATTCAGATTCCAGAGAGGTTCTCATGCTCCCCCCCC
 
CTCCTTATTTGTAGCAATCGTAGCAACTAATTCCACTAAGTACAAGGGAGTTTTTACAC
TCCTCCATTTTATAGCATCTGCATTTTTTTTTTTGTTAGGTACATGTATACACCTGCC
TGAGTATAAATACTCTCTCTACCTAATAATAACATCAACCAACATCTTTTCCAAATTAGG
GCCACAGAACAGCAACATTTGTCTGACAGTAGTATAAAGAATAATGATAGCTCTATCCTT
AAGAAGTATTTCCTTTCCTTTTTATATAGTCCCGTTAGGGTTTAAAACCATATTGATCAA
CTAGAAAGAAAAATATGAAAAGAGAAAAATATTTTAATTTAAAAATTGTAATACATTGAT
TTATAAAATGCCTTCTCTGATACTTTTGAAACAGATGTGAAAAACAGAAAAAGAAAAAAT
TGTCTGAAATGTTTATTTTGCAAAACAGTGCAATAGAATCTAGTTATGCCTTCATCACTG
TTGACAGTAAATACTGACAGCCCCTTGCAGTGTGTTAGTTTTAGATCACTCTGTTTTAGT
TGAGAGAAATGTTTTATATCATGGTTTTTATATGAATACAAATTATTTCTCAAAGATTTA
TAGCACACACTATTCTCAGGAATTCTGTATTACATGAATGCTGCTTATATATTTTCATAT
TCTAACTTGTCTTTTCAAGCAAATAACTAATATATATGTGCATGCAGTCTGCCTTGACAA
GTTGTTCCAAGCTGAAGAGCTTTCACTGTACAATGTGTGGAAAATCACCATAGATCATGG
CTGAAATAGTTTGTAATTGTCTGAGTCTGTGCACGTACTTTTAGATAAAATGCTGCTGAG
TGACTGCATGATGAGATACAACTTCTGAATGCTGCACATTCTTCCAAAATGATCCTTAGC
ACAATCTATTGTATGATGGAATGAATAGAAAACTTTTTCACTCAATAAATTATTATTTGA
TATGGTAAAAAAAAAA
BC004960 (IMAGE Clone ID: 3632495) 
CCCAAGGTTGTTATATCTTCATGTCCTCATTTCTTAGGAGGTACCTTCAGAACCAATAG
TGACCCCTAACTTCTCTGGTGGTCGGTTCCATGAAAGGCAAAGGAGTGTGAGAGAGGAGT
GGATGGTCAACCTCCCACTGCCATGGTAACATGGGTGCTGGCTGATGGGAGCAGAAAATA
ATTTAGTGAAAGTCTGTGGGGGCAGTCACAAGATGTCTGAGAAAACTGGCGAGCCAGCTG
CTGAAAACAGGGACAAGGAAGCCTCCGTGGCTGGAGCCCAAATCACACTGCAGACCCAGA
CACCGTGACCACCACCATGGACTCCAGAGAGAGCAGCTTATAGTACTCAATCAGCTGCCA
CTACCACCATCCAGAACACCAGATGTTGTAGCCATGGCTGCAGCAGGAATGGATGTCCCA
CTGTCCCTGCTCCTCGGTGTGACTTGCTCCCAAGTTCAGGGCAGGTCCATCTGATTGGCT
GAGTCTGGAATGTCTGCCTGTGCCTCAGCTGTGAGGGAGGCAGGGAAAGTAAGCCTTTTC
AGCTTCTGTCGTGGGAGGTGGGCTCTGCCTCCTACCAAGAATCAAAGGGTGGAGGATCTT
CAAACACAGGAAAAGAACCCGGATCCTGGCACCCCCAAATTTTCAGAGTCCATTTCAGAG
CATAAGAAATTGAGGGTCCAAGATCATTCATGTAAGAAGTTTAGAGGGGAAGAAAAGAA
TGATAAACGAAAAGAACAGCAATAGTAAAGGATCTTTTCTTTGTTTCAGTAAGATGAAGA
GGCCTGAGCAGTTTCGTGGAGGGGAAGAAACAGGAAAACCTCTTCAAAAGACAAAAAGCT
GGCACTGCATTCTCTCTCTGTAGCAGGACAGAACTGTCTAAAGACAAGACCCCTTTGGCC
AAAATAAAGGAACCTGAAACATTAAAAAAAAAAAAAAAAAAAAAAAAAAAAAAAAAAAAA
AAAAAAAAAAAAAAAAAAAAAAAAAACCTCGGG
AK027250

AAATTTAATTAATTATAAACTCAGTCTCTTGGTTGCACCAGCCACATTTCAGATGCTCAA
TAGCCACATGTGGCTAGTGGGTACCATATTGGACAGGGCAGCTATAGAATATTTCCATCA
TTGCAGAAAGTTCTATTGGATAGTACCATAATCTTTTTATAGTAACTTGGAAATACTATT
TGATATTAGATGTTAGACCACAAAAAGAAGAAAAATGTTAGGACTATTTCAGATATAAAA
AGGAACTGAATTGTGACATAATTAGCATCTTACATTCCATACAGTTGAATACCTTATGCT
GTGACAACCATAGTTAATCATTTCAGTGCTGTTCAACATACATACCTATCAGCAGTGTGT
TTAGACCAGGGGTCTGCAAACTTTCTGTGAATGGACAAAGAGTAAATACTTTAGTAAATG
TCTTAGGCTTTGTGGCCTACATGATCTTTGTTGCAAGTACTCAACTCTGCCATTATAGAG
TTAAAGCAGCCATACACAATATATAAACAAAATGGGCATAGTTGTATTTCAGTAAAACTT
TATTTACAAAGACAGGCGGTAGGCCAGATTTGGCTTGCATGCTGTAGAGCTGTGGTCTAA
ATTTTATTCATAGACTTTCTTTGCAAATACAGTGTGAGTATTGTTCCATTTACAGTATTA
TTATTTTTTAGATACCTGGTTTTTAGATTCTTGCCTGGTAACTTTTTACTGAAAATACAA
GAATTTCGTACTGCATTTGCATCTCCGAGATTAGGGAGCACCTGTCAGGATATGTTGTTC

TATCAGGGTTACTTCTGTTGACTACCTCTTAGATTTTGATACAGTTATATTGTTGAGTTT
CATTTTCATATATTCTTGTAGTGTCTGCTTGCCTGTGACTTCTGGTAAAATAAAATAAGC
CTTTGAAAATATTTAGCATGGTATTTAACATTTCTAAATATTATGGCATTTTGACATA
TTTTAGTCAGCGAAGACATCTGCCCCTTTGGTGTTTCTACTTGCTTATGATTGAGATTTT
ACAAGCCCTTCAAACTCCGTTTTAAAGGAATTTATTGTAAAACATTAACTTTAATAAATT
AGTGTTTTCACAGATCAGATCATTATACTTGGAACTTCTAAATCATGCAATTTCTGAATA
AGGACATAAGGCTAGATTCATTTTTCTTAATAGAGAAAAAGGAAATTTCTGATTTATCAC
TTTTCTAGTTGATAAGTAGGATTCAAAACGTTTGATATGTAAGTATTTATATAAGACTAA
TGTAATTTAAAGTTCTGTATTATTGTGATTAATCATACAGAAATTCAGGAACTGATCAGA
AGTGAGATTCTTTTCCACATCTGGTTAATGTAGTGAGTTGACACCCTGTGGGTGGTAAAG
CATTATAAACATTTCATCTTGAACCATGATTTATACACATCTGTGTTATAAGGAGGCTT
GAGTACATATACCAATGAAGAGATATTCAGCATTTGTCTATTTGATAAGGAATTAAATGT
CCTAGTGATTATAAAGTAAAACCACAGACCAATTTGCAAATGATCTTCAATGTTAAGCAC
TTGCTCTAAGATTAAAATTCCTTTTCTTTTTAAGGTTAAGGGTGTGTACGTATGGCAGTG
ATGTCTATGTTGAGATTAACTTATGTATTGAGGAAAATTTGAAGTTTATTTTTTCGATGA
ATAAGGCTGTCAAATGATTTAGTATAGATTAATGACATCTTTTTAGAAATATTAAAGTG
AGTATTCCTCATTATGTCATCATTTCTGATAATTAGAGTGCTAATTTGAATGTTAGATAA
TGTTTCCACATCTATACCTATTTCTTTCTAGGGCACTTCTGACCCTGGGGCTTGGGGATG
GCCTTTAGGCCACAGTAGTGTCTGTGTTAAGTTCACTAAATGTGTATTTAATGAGAAACA
TTCCTATGTAAAAATGTGTGTATGTGAACGTATGCATACATTTTTATTGTGCACCTGTAC
ATTGTGAAGAAGTAGTTTGGAAATTTGTAAAGCACAAACCATAAAAGAGTGTGGAGTTAT
TAAATGATGTAGCACAAATGTAATGTTTAGCTTATAAAAGGTCCTTTCTATTTTCTATGG
CAAAGACTTTGACACTTGAAAAATAAAACCAATATTTGATTTATTTTTGTAAGTATTTAG
GATATTATTTTAAATAAATGATTGTCCATTATCAATAAAAAAAAAAAAAAAAA

Sequences from Table 5 not disclosed above
NM_014298 
GTCCTGAGCAGCCAACACACCAGCCCAGACAGCTGCAAGTCACCATGGACGCTGAAGGCC
TGGCGCTGCTGCTGCCGCCCGTCACCCTGGCAGCCCTGGTGGACAGCTGGCTCCGAGAGG
ACTGCCCAGGGCTCAACTACGCAGCCTTGGTCAGCGGGGCAGGCCCCTCGCAGGCGGCGC
TGTGGGCCAAATCCCCTGGGGTACTGGCAGGGCAGCCTTTCTTCGATGCCATATTTACCC
AACTCAACTGCCAAGTCTCCTGGTTCCTCCCCGAGGGATCGAAGCTGGTGCCGGTGGCCA
GAGTGGCCGAGGTCCGGGGCCCTGCCCACTGCCTGCTGCTGGGGGAACGGGTGGCCCTCA
ACACGCTGGCCCGCTGCAGTGGCATTGCCAGTGCTGCCGCCGCTGCAGTGGAGGCCGCCA
GGGGGGCCGGCTGGACTGGGCACGTGGCAGGCACGAGGAAGACCACGCCAGGCTTCCGGC
TGGTGGAGAAGTATGGGCTCCTGGTGGCGGGGCCGCCTCGCACCGCTACGACCTGGGAG
GGCTGGTGATGTTGAAGGATAACCATGTGGTGCCCCCCGGTGGCGTGGAGAAGGCGGTGC
GGGCGGCCAGACAGGCGGCTGACTTCGCTCTGAAGGTGGAAGTGGAATGCAGCAGCCTGC
AGGAGGTCGTCCAGGCAGCTGAGGCTGGCGCCGACCTTGTCCTGCTGGACAACTTCAAGC
CAGAGGAGCTGCACCCCACGGCCACCGCGCTGAAGGCCCAGTTCCCGAGTGTGGCTGTGG
AAGCCAGTGGGGGCATCACCCTGGACAACCTCCCCCAGTTCTGCGGGCCGCACATAGACG
TCATCTCCATGGGGATGCTGACCCAGGCGGTCCCAGCCCTTGATTTCTCCCTCAAGCTGT
TTGCCAAAGAGGTGGCTCCAGTGCCCAAAATCCACTAGTCCTAAACCGGAAGAGGATGAC
ACCGGCCATGGGTTAACGTGGCTCCTCAGGACCCTCTGGGTCACACATCTTTAGGTCAG
TGAACAATGGGGCACATTTGGCACTAGCTTGAGCCCAACTCTGGCTCTGCCACCTGCTGC
TCCTGTGACCTGTCAGGGCTGACTTCACCTCTGCTCATCTCAGTTTCCTAATCTGTAAAA
TGGGTCTAATAAAGGATCAACCAAAAAAAAAAAAAAAAAA
AF033199 
CGGGGCATGCTGCTTCCCTTCACCTTCCACCATGATTGTAAGTTTCCTGAGGCCTCCCCA
GGTGTGCTTCTGTACAGCCTGTGGAATGTTACCAAAGACGTTGGAAGAGGTGGCTATGGG
ACATCACCTGGGAGAAGTGGAAGCAAATGGACACTGTTCAGAAGTCCATATACAGAAACA
TACTTGGAAAAATATAGAAACCTGGTTTTGCTAGATGGGAAGCTTGCAGCTGGGGCCAAG
ACATCAAGAGTAGAGCAGCAGGACATTTCAAAAGAAGATTAACTCAAAGATTAGAGATGG
AAGAACTTGCAAAGAGAAAGTCTGTACCGGAAGAAATCTGGAAATCTAGAGGCCAGTTTA
AGAATCAGCAGCTAAACAAGGAGAATAATCTAGGGCAAGAGATAGCTACCTGCACAAAAA
TTCCTACCAGAAAAAGAGACATAGAATCTAATGAATTTGTGAAAAATTTTACTGTAAGAT
CAATACTTGTTGCAGAACAGATAGATCCTATGGAAGAGAATTGTCATAAATATGGTACAT
GTTGAAAGATGCTCAAACAAAACTCAGATTTAATTATACAAAGAAAGTATGATGGAAAAA
AAAAAACCTTGTAAATATAGTGAATGTGGGAGAACCTTCAGAGGCCACATCACTCTTGTT
CAGCATCAAATAACTCATTGTGGAGAGAGACCCTGTAAATGTACTGAGTGTAGAAAGGGA
TTTAATCAGAGTTCCCACTTAAGAAATAATCAGAGAAAAACTCTTTCAGGAGAAAAGCCC
TACAAATGCAGTGAGTGTGGGAAGGCCTTCAGTTATTGCTTAGTTCTTAATCAACACCAG
AGAATTCACAGTGGAGAGAAACCTTATGAGGGTACTGAATGTGGCAAGACATTCATTCAG
TCGTACATACCTTACTCAGCATCAAAGAATTCACACACTGGTGAGAAGCCCTATACATGT
CTTGAATGTGGAAGGCTTTTTAGTCAGAACACACATCTTACTCTACATCAGAGAATCCAT
ACTGGAGAGAAACCTTATGAATGCAATGAATGTGGTAGGTCCTTTAGTCAGACTGCACAT
CTTACTCAACATCAAAGAATGTATACAGGAGAAAAACTCTATGAATGTAATGAATGTGAG
AAAGCCTTCCATGATCACTCAGCTCTTATTCAACATCATATTGTCCATACTGCAGAGAAA
CCCTATGATATCATGACTGGGAAAACTTTCAGTTACTGTTCAGACCTCATTCAACATCAG
AGAATGCACACTGGAGAGAAACCATACAAATGCAATGAATGTGGAATGCCTTTAGTGAT
TGTTCATCCCTTATTCAGCATCAAAGAACTCACACTGGAGAAGAGCCTTATGAATGTAAG
CAATGTGGAAAAGCCTTTAGCAGAAGCACATACCTTACTCAACATCAGAGAAGTCACGCA
GGAGAGAAACAGTATAAATGCAATGAATGTGAGAAAACTTTCAGCCTGAGTTCATTCCTT
ACACAGCATATGAGGGTTCAGACTGGAGAAAAACCCTACAAATATAATGAATATGGAAAA
GCTTTTAGTGACTGCTCAGGACATTTTCAGAGAACTCACACTGGAGAGAAGCCCTGTGAA
TGTAATGACTGTGGAAACCTTTCAGTTTCTGTTCAGCCCTAATTCAACATAAGAGAATT
CATACCAGAAAGAAGCCCTGACTGTACCTTCATACCAGTAAATGCACTGACTGTGGAAAA
GCCTTCAGTGATTGGTTAGCACTTGTTCAACATCAGATAACTCAACACTGGAGAAAAACC
GTATAAATGTACTGAATGTGGAAAAGCCTTCAGTTGGAGTACAGACCTCAAAAATCACCA
GAAAACTCATACTAGTGAAAAATCCTATAAATGTAATGAATGTAGAAAGGCCTTTAGTTA
CTGCTCTGGTCTTATTCAATGTCAGGTCATTCATACTATAGAAAAACCTTATGAATACGG
TAAATGTGGCAAAGCCTTTAGGCAGAGGACAGACCTTAAAAAACATCAGAAAATGCATAC
CGAAGAGAAACCCTATGAATGTAATGAATGTGGAAAGCCTTTAGCCAGAGCACATATCT
TACAAAACACCAAAAAATTCATAGTGAAGAGAAATCAAATATACATACTGAGTGTGGGGA
AACCATTAGACAAAACTCTTCTTTTACAACAATAAAACCTCACACTGGAGAGTTCTCTG
AATGCCTTAAGAATTTGGTTAATATGGAGACCCTTCCCAGGGAAACAGAAGGAGGATCGT
GAAAACCGTTGACTACTTGAATGATCACATGGTTTAGTGGAGAGAGCATGATTCTGGGTT
TTAAAAGTCATGGATCTCAATCTCAGCTCCTATTACTAACTAGATCTTTTACTTTGGGGT
AAGTCACTTCATATCTTTAGGCCTTAATTTCCTCATCTGAAAACTGGAAGGCCTGACTTG
ACTTGTTGAGCTTAAGATCCTCAATTATTATATTTACTAGGAATTCAAGTTTCTATAGAT
GTGGTTCAGAATTGTGACTTATTTATTGTACATCAGGTGTGATTCACAAGTGAGCTTGTA
GTAGTTATTAAGGAGTCAATAAAGATATGATATAAAAAAAAAAAAAAAAAA
AI688494 (IMAGE Clone ID: 2330499) 
CATTTCATCTTCATTGGATAGTGTTACATAGTAATATATTTATGTTTTCTTTTAATCATT
TCATAACTTGGAAAATACTAACATAGTCAAAACTCTAGGGTAGGTGATACATGAGTTTCT
GTAGTAATCTGGTTGGAGACATGTTGTAATTCTGTATATATATGTACATTTATCCCATGC
ATGTTATGCCTAAACTAAGACGGATACCCCTGAATTAAGAGGTGCTGTTATACATTGACC
AGGCTTAAGAATATCTCTTTAAAGTGTGTCGACATTTAATTGACCTTTGGAAGTTCATTC
TGTTAATCATACTCAAAGTGCTAAAGCTATGGTTGACTGCTCTGGTGTTTTTATATTCAT
 
TCGTGCTTTAGCATATAAATTCTTCAGCATAATTGCTACTTATTTAGCAAGAGTTTCCTT
TATTTGAAAATGTGAGTTGTGCTTGTATTTTTGTGTCTTTCTTTCTTTCTTTCTTTTTTT
AAACTTTGCTTCAGGCTGGGTAGTGGTAGAGGTTTGAATTAAAATGTTTTCCTGTCAGTA
AAAAAAAAAAA
AL157459 
GAGCGAGCCCAGCAGCTTGCCCTTGACAGGTGGGGGCTGGCTGGGGCCTTAATGTGAAAA
GACAGTGGCAGGCAGCTGGAGTAGAGCGAGCCCAGCAGCCCTAAAGGCTGCCTTCATGG
CCATCTAGCCCCAGTTCAGGGCAGCATCCATAGCCCACAAGCCAGCGTGGGTGGGGCGGG
GGTGGTCCCACAGCTGGGTTCCACCTGAAGAGCCTCCGTGCCTCGGAGCAGGAGAGGCAG
GCTATGGCTGTCACCCTCCCTCCTGCCTGTGTCCCAGTGAGAACTGACCTGAGTCCCCTT
CCAAACCCAGACCCACCTCCTGCCCCAGGCCCACTGAAGCATGTTCCATTTCTAAAAAGC
CCAGAGTTCAGTGTGTCCCAAGGAAAACCCAAAGTGGAGGTGCTCAGGTCCAGGGGAGTC
CAGTGGGCAGGACCCTTGGCAGGCAAGCCCCTCCCTTCACTCCCAGGACCTACCTTCTGC
TAGTAAAGGACTGGCTTCATTCTAATTATGGCCCACAGACTGCCCCGGAGACCTGGAGGA
CAGCAGTGCTGGCACTTGGGTGTCCATGGGCCCGTCTGCCGGCTCTGCCTGTGCTGCAAG
TGTTGGCCGTGGGTCCAGCCAACAACTCCCTACGTCCTGTGTGGGGCCCTGCCCAAGTGG
ATGAGGCATTCCTTGAGGAGTATCATTTTCCCTGACAATCCCCATCACCTTTAGGGGTTC
CCTGCTTGGCTCCTTTCCAGCTGAAAAACTAGACCTGTGCCATTGGGGAAGCTGGACAAA
GTCTAGGGGGCCCGCCTGGTAGAGGGTCCCGGGAAGCTGGATCTGTCAGCCTCGGCCCTG
AGGCCCCTGTTAACTCAAGACTGTGAGCTGCCTCTAGGTGGTCACGTCTGGGAGCTAGCT
TGTATGGCTTCTGACCAGTATCAGGATTTCTGTTCTGAGAGCAGCGTGGGCAGCAAGGCA
GGGCAGCCCAGAGGTGGCAGCGGCAGGCAATCTGGTCACTAGGTCTTTGTGATGCCAAAA
ATAAAAGAGGGTGGGGTGGGTGCTTTCTGTTCCTCTGATTGGATGGAGTCCGCCAGCAGG
CATGGGGCTACATTCCAGTGCCTGACTATAGGGAGGCACTCCTGATTCCATGGAGCAGCC
CGGACTTTGAGAATGGGCTCTGGTTTGCGGGGGCAGGCGTACCAGACTGCAAGACCCCC
CAGTACCTCACCGTGCCAAATAGGAAGAGGTGGCCTTGGTGTAGCCAAATGGATCTTTTT
AACAGTGTGCCTTTGGGAGGGACCCATGTCCATGGCTTCGTTGAGGGCCATCCATATGC
CAGCTGGGGGCCAGCCCACAGTGGCCATATTGGCTGCAGCAGGAATGGTGCCCACCTCGG
CGAATTGAAGGGCTAAGAGTCCCAGATAGCTAGGCCAGAGCTGGAAGCAGACAGTAAGGG
GAAGAGCTGCTCCCACAGGAGAGGGAGAGATTCCAGCTCACTGCGCAGCCTGGGAGGAGG
CGTGGATCCTGGCACGCTGAGCCTCAGGCACCAGCCTCCCTGTGCTCGACAGCAAAGTCT
TGACTCCTTCCTGCTGAGCACTGTGCTACCTTCACTGCTCCAAAGCCAGACTAACAGCTC
TCCAAGCCCTTGGGGTGACTCGGCTTCCAGGAGCTGTTGGAGAAATGAGGATGTCTGTCC
CTGTCTGCCTGGGCAGGCCAGATTCCTCCCCAGCAGCCGGGTCTCTCCAGACCCTGATTC
GGTGCCTTTCTGTTTACCAGCTACTTCAATCCCAAAGTTTGAATCTGCAGATACCTTACT
CCCAGCCACTTTGCCTTCTTACTGTGTTGTGTGTTTTTCCTGGTGCTTCAAGAGCGTGTG
CAGGGCAAGTGCCGTCACTGGGAACTGCACCAGATGCTCAGACTTGGTTGTCTTATGTTT
ACCAATAAATAAAAGTAGACTTTTTCTATTTTTATTTGCTGCTATTTGTGTGTGTGTTTG
TGTTTGTGTAGCTAGGTATCTGGCACTTCTGACGATGCATTGTTGCTTTTTTCCCGAAGG
TCCCGCAGGAACTGTGGCAATGGTGTGTGTGTGAAATGGTGTGTTAACCGCGTTTTGTTT
GCTCCTGTATTGAATAGGAAGCAGTGGCCAGTCTGTCTTCCTTAGAGATGTTAGCATATT
TTTATATGTATATATTTTGTACCAAAAAAGAGTGTTCCTTGTTTTGGTTACACTCGAAAT
TCTGACCTAGCTGGAGAGGGCTCTGGCCGAGAGCTTTCACTAAGGGGAGACTTCAGGGG
AGGATCAAGCTTTGAACCAAAGCCAATCACTGGCTTGATTTGTGTTTTTTAATTAAAAAA
AAAATCATTCATGTATGCCACTTCTAAAAAAAAAAAAAAAAAAAAAAAA
BC002480 (IMAGE Clone ID: 3350037)

GGCACGAGGCTGAGACCGGTGCGCCGCGCTAGTGGCCGCTCTTCCGCGGCTAGCGGG
CGGTGGGGGCGCCAGCAGCGCGGAAGGCGGGCACGCGGGCCATGGCTCCCTGGCGGAGG
CCGAGCACTCGGCGCTGAACCCGCTGCGCGCGGTGTGGCTCACGCTGACCGCCGCCTTCC

TGCTGACCCTACTGCTGCAGCTCCTGCCGCCCGGCCTGCTCCCGGCTGCGCGATCTTCC
AGGACCTGATCCGCTATGGAAAACCAAGTGTGGGAGCCGTCGCGCCCCGCCGCCTGCC
GAGCCTTTGATGTCCCCAAGAGATATTTTTCCCACTTTATATCATCTCAGTGCTGTGGA
ATGGCTTCCTGCTTTGGTGCCTTACTCAATCTCTGTTCCTGGAGCACCTTTTCCAAGCT
GGCTTCATGGTTTGCTCAGAATTCTCGGGGCGGCACAGTTCCAGGGAGGGGAGCTGGCAC
TGTCTGCATTCTTAGTGCTAGTATTTCTGTGGCTGCACAGCTTACGAAGACTCTTCGAGT
GCCTCTACGTCAGTGTCTTCTCCAATGTCATGATTCACGTCGTGCAGTACTGTTTTGGAC
TTGTCTATTATGTCCTTGTTGGCCTAACTGTGCTGAGCCAAGTGCCAATGGATGGCAGGA
ATGCCTACATAACAGGGAAAAATCTATTGATGCAAGCACGGTGGTTCCATATTCTTGGGA
TGATGATGTTCATCTGGTCATCTGCCCATCAGTATAAGTGCCATGTTATTCTCGGCAATC
TCAGGAAAAATAAAGCAGGAGTGGTCATTCACTGTAACCACAGGATCCCATTTGGAGACT
GGTTTGAATATGTTTCTTCCCCTAACTACTTAGCAGAGCTGATGATCTACGTTTCCATGG
CCGTCACCTTTGGGTTCCACAACTTAACTTGGTGGCTAGTGGTGACAAATGTCTTCTTTA
ATCAGGCCCTGTCTGCCTTTCTCAGCCACCAATTCTACAAAAGCAAATTTGTCTCTTACC
CGAAGCATAGGAAAGCTTTCCTACCATTTTTGTTTTAAGTTAACCTCAGTCATGAAGAAT
GCAAACCAGGTGATGGTTTCAATGCCTAAGGACAGTGAAGTCTGGAGCCCAAAGTACAGT
TTCAGCAAAGCTGTTTGAAACTCTCCATTCCATTTCTATACCCCACAAGTTTTCACTGAA
TGAGCATGGCAGTGCCACTCAAGAAAATGAATCTCCAAAGTATCTTCAAAGAATAAATAC
TAATGGCAGAAAAAAAAAAAAAAAAAAAAAAAAAAAAAAAAAAAA

## Claims

1. A method to determine the risk of cancer recurrence in a subject afflicted with ER+ breast cancer if treated with tamoxifen, said method comprising assaying a sample of ER+ breast cancer cells from said subject for the expression of human HOXB13 and IL17BR mRNAs to determine a ratio of HOXB13 and IL17BR expression levels, wherein said ratio is indicative of lack of cancer recurrence in said subject if treated with tamoxifen.

2. The method of claim 1, wherein said ratio is indicative of the lack of recurrence of cancer via metastasis or survival outcome.

3. The method according to any one of claims 1 and 2, wherein said sample of breast cancer cells is obtained by a minimally invasive technique or selected from core biopsy, excisional biopsy, a ductal lavage sample, a fine needle aspiration sample, or cells microdissected from said sample.

4. The method of any one of claims 1 to 3, wherein said assaying for expression comprises detection of nucleic acids prepared by mRNA amplification from said sample of breast cancer cells or detection of nucleic acids from said sample of breast cancer cells by quantitative PCR.

5. The method of any one of claims 1 to 3, wherein said assaying is by hybridization to a polynucleotide comprising sequences of at least 15 nucleotides from the 3' untranslated region, the coding region, or the 5' untranslated region, of human HOXB13 and IL17BR genes.

6. The method of any one of claims 1 to 3, wherein said assaying for expression comprises assaying for inactivation or methylation of the HOXB13 gene.

7. The method of claim 4, wherein said assaying is for the expression levels of HoxB 13 and IL17BR and comprises real-time PCR and determining a ratio of HoxB 13 and IL17BR RNA expression levels expressed as a ΔCt of the Ct values for HoxB 13 and IL17BR RNA expression levels.

## Patentansprüche

1. Verfahren zur Bestimmung des Risikos für das Wiederauftreten von Krebs bei einem Subjekt, das an ER+-Brustkrebs leidet, wenn es mit Tamoxifen behandelt wird, wobei das Verfahren Analysieren einer Probe von ER+-Brustkrebszellen von dem Subjekt auf die Expression von humanen HOXB13- und IL17BR-mRNAs, um das Verhältnis der HOXB13- und IL17BR-Expressionslevel zu bestimmen, umfasst, wobei das Verhältnis für die Abwesenheit des Wiederauftretens von Krebs bei dem Subjekt, wenn es mit Tamoxifen behandelt wird, indikativ ist.

2. Verfahren gemäß Anspruch 1, wobei das Verhältnis für die Abwesenheit des Wiederauftretens von Krebs via Metastasenbildung oder Überlebensfähigkeit indikativ ist.

3. Verfahren gemäß einem der Ansprüche 1 und 2, wobei die Probe von Brustkrebszellen durch eine minimal invasive Technik erhalten wird oder ausgewählt ist aus einer Kernbiopsie, Exzisionsbiopsie, einer Duktus-Lavage-Probe, einer Probe aus einer Aspiration mit feiner Nadel oder mikrodissezierten Zellen aus der Probe.

4. Verfahren gemäß einem der Ansprüche 1 bis 3, wobei das Analysieren auf Expression eine Detektion von Nukleinsäuren, hergestellt durch mRNA-Amplifikation aus der Probe von Brustkrebszellen, oder eine Detektion von Nukleinsäuren aus der Probe von Brustkrebszellen durch quantitative PCR umfasst.

5. Verfahren gemäß einem der Ansprüche 1 bis 3, wobei das Analysieren durch Hybridisierung an ein Polynukleotid, umfassend Sequenzen von wenigstens 15 Nukleotiden aus der 3'-untranslatierten Region, der codierenden Region oder der 5'-untranslatierten Region von humanen HOXB13- und IL17BR-Genen, erfolgt.

6. Verfahren gemäß einem der Ansprüche 1 bis 3, wobei das Analysieren auf Expression Analysieren auf Inaktivierung oder Methylierung des HOXB13-Gens umfasst.

7. Verfahren gemäß Anspruch 4, wobei das Analysieren auf die Expressionslevel von HOXB13 und IL17BR erfolgt und eine Echtzeit-PCR und Bestimmung des Verhältnisses von HOXB13- und IL17BR-RNA-Expressionsleveln, ausgedrückt als ΔCt der Ct-Werte für HoxB13- und IL17BR-RNA-Expressionslevel, umfasst.

## Revendications

1. Procédé permettant de déterminer le risque de récidive de cancer chez un sujet atteint d'un cancer du sein ER+ en cas de traitement par tamoxifène, lequel procédé comporte le fait de tester, dans un échantillon de cellules de cancer du sein ER+ issues dudit sujet, l'expression des ARNm HOXB13 et IL17BR humains, afin de déterminer un rapport des niveaux d'expression de HOXB13 et IL17BR, lequel rapport est une indication de l'absence de récidive du cancer chez ledit sujet en cas de traitement par tamoxifène.

2. Procédé conforme à la revendication 1, dans lequel ledit rapport est une indication de l'absence de récidive du cancer par métastases ou de l'issue de survie.

3. Procédé conforme à l'une des revendications 1 et 2, pour lequel ledit échantillon de cellules de cancer du sein a été obtenu au moyen d'une technique invasive à un degré minime, ou est choisi parmi une biopsie au trocart, une excision-biopsie, un échantillon obtenu par lavage des canaux galactophores, un échantillon obtenu par aspiration à l'aide d'une aiguille fine, ou des cellules obtenues par micro-dissection d'un tel échantillon.

4. Procédé conforme à l'une des revendications 1 à 3, dans lequel ledit test d'expression comporte la détection des acides nucléiques préparés par amplification d'ARNm à partir dudit échantillon de cellules de cancer du sein, ou la détection des acides nucléiques dans ledit échantillon de cellules de cancer du sein par PCR quantitative.

5. Procédé conforme à l'une des revendications 1 à 3, dans lequel ledit test est réalisé par hybridation à un polynucléotide comprenant des séquences d'au moins 15 nucléotides provenant de la région 3' non traduite, de la région codante, ou de la région 5' non traduite, des gènes HOXB 13 et IL 17BR humains.

6. Procédé conforme à l'une des revendications 1 à 3, dans lequel ledit test d'expression comporte un test d'inactivation ou de méthylation du gène HOXB13.

7. Procédé conforme à la revendication 4, dans lequel ledit test vise à déterminer les niveaux d'expression d'HOXB13 et d'IL17BR et comporte une opération de PCR en temps réel et le fait de déterminer un rapport des niveaux d'expression des ARN HOXB13 et IL17BR, qui est exprimé en termes de différence ΔCt des valeurs de Ct observées pour les niveaux d'expression des ARN HOXB13 et IL17BR.
